# EUROPEAN PATENT APPLICATION

(11) **EP 4 170 047 A1**
(43) Date of publication of application: **26.04.2023**
(21) Application number: 21204456.4
(22) Date of filing: 25.10.2021
(51) Int. Cl.: C12Q 1/6869, C12Q 1/6888

(54) **DNA BASED IDENTIFICATION OF SEAFOOD SPECIES IN SAMPLES**

(71) Applicant: FFoQSI GmbH, 3430 Tulln (AT); Österreichische Agentur für Gesundheit und Ernährungssicherheit GmbH, 1220 Wien (AT); LVA GmbH, 3400 Klosterneuburg (AT)
(72) Inventor: GENSE, Kristina, 1210 Vienna (AT); PETERSEIL, Verena, 1220 Vienna (AT); LICINA, Alma, 1180 Vienna (AT)
(74) Representative: Loidl, Manuela Bettina

(57) **Abstract**

A method for identifying seafood species in a sample, comprising the steps of a) isolating DNA from the sample, b) amplifying fragments of the isolated DNA with one or more primer sets, selected from the group of ps 1 for identifying seafood species of the family of Crustacean, ps 2 for identifying seafood species of the family of Cephalopods, ps 3 for identifying seafood species of the family of Gastropoda, ps 4 for identifying seafood species of the family of Veneridae, ps 5 for identifying seafood species of the family of Ostreidae, ps 6 for identifying seafood species of the family of Pectinidae, and ps 7 for identifying seafood species of the family of Mytilidae, c) sequencing the amplified DNA fragments of step b), and d) identifying the seafood species by comparison of the sequences obtained by steps a) to c) with reference sequences of seafood species. Further provided is a primer library and a kit.

## Description

### FIELD OF THE INVENTION

The present invention relates to the technical field of DNA barcoding, particularly to a method for the identification of seafood species in samples comprising the steps of isolating DNA from a sample, amplifying fragments of the isolated DNA using specific primers, sequencing of the amplified DNA fragments, and identifying the seafood species through sequence comparison with reference sequences. Further provided herein is a primer library and a kit.

### BACKGROUND OF THE INVENTION

Food adulteration is a worldwide problem in various food products, e.g., in farm animal, wild animal, seafood, and also plant products. The term of food adulteration is not uniformly defined but in general, it describes misdeclaration of food intending to gain an economic benefit without limits (Robson, K. et al, 2021). Seafood has a high risk of fraud and seafood products are often mislabelled. According to Pardo, M. et al. (2016), up to 27 % of the seafood is mislabelled worldwide. Food adulteration includes, but is not limited to, replacement (a (valuable) ingredient is replaced by one of a lower value), relabelled or incorrectly labelled food. Incorrect labelling can result when different local names are used for the same species, when the same name is used for different species, or due to translation errors.

However, correct labelling of seafood products is important for traceability issues, protection of endangered species, mitigation of illegal fishing, and for individual reasons of end consumers (Rodriguez, E.M. and Ortea, I., 2017).

Correct declaration of seafood is regulated in the European Union. Thereby, international and national regulations exist to ensure legal trade in seafood and seafood products. The EU directive 1379/2013 regulates market organization of fishery and aquaculture products, including correct declaration of seafood. To comply with legal regulations, labels must include both the local trade name in the official language(s) and the correct scientific Latin name (Regulation (EU) No 1379/2013; Regulation (EU) No 1169/2011).

Regardless of clear and strict requirements for species declaration, incorrect labelling of e.g., bivalve products, has repeatedly been detected in Europe (Näumann, G. et al., 2012; Fernandes, T. et al., 2020). In German and Swiss studies, more than half of the products declared to contain "Jakobsmuschel" (or "Jacobsmuschel") were labelled incorrectly. Although the German name "Jakobsmuschel" (or "Jacobsmuschel") may only be used for scallop species of the genus *Pecten,* species of other genera (particularly *Placopecten* and *Mizuhopecten*) were identified in these products (Näumann, G. et al., 2012; Stephan, R. et al., 2014).

Compliance with regulations is especially important since seafood is gaining importance in human nutrition. In 2019, 107.6 billion US $ were made with the marketing of seafood (crustaceans and molluscs), compared to 8.1 billion US $ 30 years ago. In 2019, 1.03 million tons of mussels, scallops, and oysters were caught in nature and more than 10 million tons were cultivated in aquaculture, earning a profit of millions of US dollars. Worldwide, 6.1 million tonnes of crustaceans were caught and 10.5 million tonnes were cultivated in 2019. In the same year, 6.4 million tonnes of molluscs were caught and 17.6 million tonnes were cultivated.

Crustaceans and molluscs are divided into numerous genera comprising a high number of species with a worldwide distribution. A class of molluscs are for example bivalves, wherein *Mytilidae* (mussels), *Pectinidae* (scallops), and *Ostreidae* (oysters) are the most important bivalve species for human consumption. Each of these bivalve species is divided into several genera comprising a high number of species which makes correct identification of seafood species difficult using known methods.

In the case of seafood, especially for bivalves, morphological characteristics such as shell, colour and size may allow correct species classification. However, after shell removal or mechanical processing, classification by morphology may be hampered or even be impossible (Espiñeira, M. et al., 2009; Fernández, A. et al., 2000).

Recently, matrix-assisted laser desorption ionization time of flight mass spectrometry (MALDI-TOF MS) has been shown to be suitable for accurate species identification of scallops (Stephan, R. et al., 2014). However, MALDI-TOF MS instruments are expensive and do not allow high throughput analysis. Therefore, this methodology is less applicable for routine analyses and for the fast identification of several seafood species.

DNA metabarcoding methods have been recently developed for the identification of mammalian and poultry species in food (Dobrovolny S. et al., 2019).

However, methods which provide comprehensive information on the plurality of seafood species which are present in a food sample are still missing. Thus, there is an urgent need in the field for improved means of identifying several different seafood species in complex and processed foodstuffs suitable for food authentication in routine analysis.

### SUMMARY OF THE INVENTION

It is the objective of the present invention to provide improved means and methods for the identification of several different seafood species in food samples.

The objective is solved by the subject matter of the present invention.

The present invention provides a method which is highly suitable for the identification of seafood species of different origin and processing degree in complex food samples. The inventors of the present invention surprisingly discovered a library of primers which can be incorporated in a fast and reliable metabarcoding method for the identification of a plurality of seafood species in food samples.

According to the invention, there is provided a method for identifying seafood species in a sample, comprising the steps of
a) isolating DNA from the sample,
b) amplifying fragments of said DNA with one or more primer sets (ps) selected from the group of
   i. ps 1 for identifying seafood species of the family of Crustacean comprising one or more primer pairs of one forward primer selected from any one of SEQ ID NOs: 1 and 2, and the reverse primer SEQ ID NO: 15 and/or the reverse complement sequences of the primer sequences;
   ii. ps 2 for identifying seafood species of the family of Cephalopods comprising one or more primer pairs of one forward primer selected from any one of SEQ ID NOs: 3 to 5, and the reverse primer SEQ ID NO: 16 and/or reverse complement sequences of the primer sequences;
   iii. ps 3 for identifying seafood species of the family of Gastropoda comprising one or more primer pairs of the forward primer SEQ ID NO: 6, and one reverse primer selected from any one of SEQ ID NOs: 17 to 18 and/or reverse complement sequences of the primer sequences;
   iv. ps 4 for identifying seafood species of the family of Veneridae comprising one or more primer pairs of one forward primer selected from any one of SEQ ID NOs: 7 to 11, and one reverse primer selected from any one of SEQ ID NOs: 19 to 21 and/or reverse complement sequences of the primer sequences;
   v. ps 5 for identifying seafood species of the family of Ostreidae comprising the forward primer SEQ ID NO: 12 and the reverse primer SEQ ID NO: 22 and/or reverse complement sequences of the primer sequences;
   vi. ps 6 for identifying seafood species of the family of Pectinidae comprising the forward primer SEQ ID NO: 13 and the reverse primer SEQ ID NO: 23 and/or reverse complement sequences of the primer sequences; and
   vii. ps 7 for identifying seafood species of the family of Mytilidae comprising one or more primer pairs of the forward primer SEQ ID NO: 14, and one reverse primer selected from any one of SEQ ID NOs: 24 to 25 and/or reverse complement sequences of the primer sequences,
c) sequencing the amplified DNA fragments of step b), and
d) identifying the seafood species by comparison of the sequences obtained by steps a) to c) with reference sequences of seafood species.

Specifically, amplifying of fragments of DNA in step b) is performed by a polymerase chain reaction (PCR), preferably by a PCR comprising 25-30 cycles, more preferably by 25 cycles, and preferably at an annealing temperature of 60-65 °C, more preferably at an annealing temperature of 62°C.

Specifically, amplifying fragments of the DNA in step b) is performed with at least 1, 2, 3, 4, 5, 6, or 7 primer sets.

Specifically, the amplified DNA fragments of step b) are 16S rDNA fragments, preferably the amplified DNA fragments comprise 120bp-220bp of the 16S rDNA.

Specifically, the reference sequences of seafood species comprise the DNA sequence of the 16S rDNA of said seafood species.

More specifically, the reference sequences are SEQ ID NOs: 28 to 1153.

Specifically, the identified species of the family of Crustacean are selected from the following Group 1: *Varuna litterata, Hemisquilla ensigera, Gonodactylus smithii, Pullosquilla thomassini, Chorisquilla trigibbosa, Telmessus acutidens, Lithodes aequispinus, Panulirus echinatus, Jasus paulensis, Jasus caveorum, Parastacus pilimanus, Parastacus brasiliensis, Parastacus defossus, Parastacus nicoleti, Gonodactylus graphurus, Jasus lalandii, Lopholithodes mandtii, Lithodes spp., Lithodes maja, Jasus edwardsii, Panulirus regius, Panulirus pascuensis, Panulirus laevicauda, Panulirus gracilis, Panulirus guttatus, Panulirus femoristriga, Chionoecetes* spp., *Paralomis granulosa, Panulirus* spp., *Scyllarus arctus, Palinurus elephas, Episesarma mederi, Austropotamobius torrentium, Cycloachelous granulatus, Eriocheir recta, Cervimunida johni, Achelous floridanus, Portunus sayi, Portunus anceps,, Palinurus mauritanicus, Palinurus charlestoni, Pseudosquilla ciliata, Pleuroncodes monodon, Portunus ventralis, Achelous spinicarpus, Callinectes toxotes, Callinectes danae, Callinectes ornatus, Callinectes marginatus, Callinectes affinis, Callinectes rathbunae, Callinectes bocourti, Callinectes similis, Callinectes bellicosus, Callinectes arcuatus, Metanephrops armatus, Metanephrops mozambicus, Metanephrops japonicus, Metanephrops spp., Metanephrops binghami, Parastacus pugnax, Paranephrops zealandicus, Callinectes exasperatus, Palinurus* spp., *Sagmariasus verreauxi, Metanephrops rubellus, Metanephrops challengeri, Metanephrops neptunus, Metanephrops australiensis, Metanephrops arafurensis, Metanephrops boschmai, Metanephrops formosanus, Metanephrops sinensis, Lithodes ferox, Oratosquillina interrupta, Odontodactylus japonicus, Miyakella nepa, Erugosquilla woodmasoni, Clorida decorata, Dictyosquilla foveolata, Anchisquilla fasciata, Scyllarides herklotsii, Astacus astacus, Portunus hastatus, Achelous ordwayi, Carcinus maenas, Portunus inaequalis, Astacoides madagascariensis, Erimacrus isenbecki, Hemisquilla australiensis, Austrosquilla tsangi, Fallosquilla fallax, Echinosquilla guerinii, Coronis scolopendra, Chorisquilla tweediei, Chorisquilla hystrix, Chorisquilla excavate, Busquilla plantei, Alima pacifica, Alima orientalis, Alachosquilla vicina, Gonodactylellus espinosus, Gonodactylellus affinis, Kempella mikado, Hemisquilla californiensis, Haptosquilla trispinosa, Haptosquilla glyptocercus, Gonodactylus platysoma, Gonodactylaceus falcatus, Gonodactylus childi, Gonodactylellus annularis, Odontodactylus scyllarus, Odontodactylus latirostris, Odontodactylus havanensis, Odontodactylus cultrifer, Neogonodactylus oerstedii, Neogonodactylus bredini, Neogonodactylus bahiahondensis, Lysiosquillina sulcata, Squilla rugosa, Raoulserenea* spp., *Raoulserenea oxyrhyncha, Pseudosquillopsis marmorata, Raoulserenea komaii, Protosquilla folini, Ibacus alticrenatus, Scyllarides nodifer, Scyllarides haanii, Scyllarides brasiliensis, Taku spinosocarinatus, Jasus frontalis, Procambarus paeninsulanus, Puerulus sewelli, Panulirus polyphagus, Panulirus longipes., Panulirus interruptus, Panulirus marginatus, Ibacus peronii, Ibacus chacei, Faxonella clypeata, Fallicambarus kountzeae, Arenaeus mexicanus, Cambarus tartarus, Chionoecetes tanneri, Thenus unimaculatus, Thenus indicus, Haptosquilla hamifera, Lithodes turritus, Bouchardina robisoni, Troglocambarus maclanei, Hobbseus yalobushensis, Hobbseus prominens, Charybdis* spp., *Hobbseus petilus, Faxonella creaseri, Thranita danae., Monomia petrea, Neogonodactylus wennerae, Xiphonectes pseudohastatoides, Gonodactylellus viridis, Gonodactylaceus ternatensis, Belosquilla laevis, Procambarus okaloosae, Procambarus morrisi, Procambarus milleri, Procambarus mancus, Procambarus lunzi, Hobbseus cristatus, Procambarus acutissimus, Faxonius pagei, Manningia pilaensis, Pontastacus leptodactylus, Procambarus zonangulus, Procambarus youngi, Procambarus seminolae, Procambarus pycnogonopodus, Procambarus orcinus, Procambarus pallidus, Alima maxima, Scyllarides deceptor, Monomia argentata, Xiphonectes pulchricristatus, Paralithodes platypus, Lopholithodes foraminatus, Faughnia formosae, Faughnia profunda, Bathysquilla crassispinosa, Eriocheir sinensis, Harpiosquilla harpax, Callinectes sapidus, Squilla mantis, Portunus trituberculatus, Panulirus japonicus, Cancer pagurus, Chionoecetes japonicus, Scylla tranquebarica, Scylla serrata, Eriocheir japonica, Eriocheir hepuensis, Cherax destructor, Squilla empusa, Lysiosquillina maculata, Gonodactylus chiragra, Panulirus homarus, Homarus americanus, Panulirus ornatus, Oratosquilla oratoria, Panulirus stimpsoni, Charybdis japonica, Scylla paramamosain, Scylla olivacea, Cherax quadricarinatus, Cherax cainii, Paralithodes brevipes, Paralithodes camtschaticus, Scyllarides latus, Procambarus clarkii, Procambarus fallax, Homarus gammarus, Thenus orientalis, Lithodes nintokuae, Cherax cairnsensis, Cherax dispar, Cherax quinquecarinatus, Cherax robustus, Cherax monticola, Cherax glaber, Cherax holthuisi, Astacopsis gouldi, Portunus pelagicus, Paranephrops planifrons, Nephrops norvegicus, Ibacus ciliatus, Charybdis feriata, Metanephrops sibogae, Panulirus cygnus, Metanephrops thomsoni, Faxonius limosus, Squilloides leptosquilla, Cherax bicarinatus, Austropotamobius pallipes, Cherax tenuimanus, Cherax boesemani, Charybdis (Charybdis) natator, Procambarus acutus, Pacifastacus leniusculus, Munida gregaria, Panulirus versicolor, Faxonius rusticus, Portunus sanguinolentus, Procambarus alleni, Metacarcinus magister, Puerulus angulatus, Lupocycloporus gracilimanus, Monomia gladiator, Varuna yui, Panulirus argus, Munida isos, Scyllarides squammosus, Cambaroides similis, Charybdis bimaculata, Cambarus robustus, Thalamita sima, Thranita crenata, Orconectes luteus, Orconectes punctimanus, Orconectes sanbornii, Cherax* spp., *Cherax crassimanus, Cherax preissii, Munida spinosa, Munida asprosoma, Munida leagora, Munida alonsoi, Munida taenia, Munida gordoae, Munida zebra, Munida distiza, Munida psamathe, Munida thoe, Munida guttata, Munida stia, Munida ommata, Munida roshanei, Munida compressa, Munida clinata, Munida chydaea, Munida compacta, Munida eclepsis, Munida tyche, Munida philippinensis, Munida armilla, Munida mesembria, Munida spilota, Munida benguela, Munida endeavourae, Munida agave, Munida idyia, Munida militaris, Munida flinti, Munida congesta, Munida rubridigitalis, Munida iris, Munida microphthalma, Munida rufiantennulata, Munida pusilla, Munida remota, Munida leptosyne, Munida rosula, Munida munin, Munida valida, Munida proto, Enriquea leviantennata, Munida multilineata, Munida pagesi, Munida stomifera, Munida quadrispina, Munida tiresias, Munida psylla, Munida heteracantha, Paralomis formosa, Paralomis spinosissima, Paralomis birsteini, Paralomis hirtella, Scyllarus subarctus, Scyllarus pygmaeus, Scyllarus chacei, Scyllarus caparti, Scyllarus americanus, Episesarma palawanense, Episesarma singaporense, Austropotamobius fulcisianus orientalis, Achelous tumidulus, Achelous asper, Achelous sebae, Portunus acuminatus, Achelous tuberculatus, Achelous iridescens, Portunus xantusii, Achelous depressifrons, Achelous rufiremus, Achelous gibbesii, Portunus minimus, Achelous stanfordi, Achelous brevimanus, Portunus affinis, Achelous angustus, Achelous binoculus, Oratosquillina inornata, Oratosquillina asiatica, Oratosquillina anomala, Oratosquillina perpensa, Erugosquilla graham, Busquilla quadraticauda, Kempella stridulans, Gonodactylaceus graphurus, Gonodactylaceus randalli, Carcinus aestuarii, Menippe rumphii, Menippe nodifrons, Menippe* spp., *Procambarus liberorum, Procambarus toltecae, Procambarus curdi, Procambarus digueti, Procambarus nigrocinctus, Procambarus versutus, Procambarus gibbus, Cambarus pecki, Procambarus geminus, Charybdis acuta, Creaserinus fodiens, Fallicambarus jeanae, Creaserinus gordoni, Creaserinus caesius, Fallicambarus dissitus, Creaserinus danielae, Fallicambarus oryktes, Fallicambarus byersi, Creaserinus burrisi, Creaserinus gilpini, Fallicambarus harpi, Fallicambarus macneesei, Fallicambarus petilicarpus, Fallicambarus wallsi, Fallicambarus strawni, Fallicambarus devastator, Fallicambarus houstonensis, Fallicambarus hortoni, Arenaeus cribrarius, Cambarus* spp., *Cambarus deweesae, Cambarus striatus, Cambarus graysoni, Cambarus monongalensis, Cambarus pyronotus, Cambarus maculatus, Cambarus latimanus, Cambarus strigosus, Cambarus parrishi, Cambarus bouchardi, Cambarus fasciatus, Cambarus harti, Cambarus nerterius, Cambarus setosus, Cambarus batchi, Cambarus halli,, Cambarus unestami, Cambarus reburrus, Cambarus gentry, Cambarus hubbsi, Cambarus friaufi, Cambarus obeyensis, Cambarus cracens, Cambarus asperimanus, Cambarus hobbsorum, Cambarus williami, Cambarus howardi, Cambarus obstipus, Cambarus girardianus, Cambarus cryptodytes, Cambarus sciotensis, Cambarus georgiae, Cambarus pristinus, Cambarus aculabrum, Cambarus englishi, Cambarus brachydactylus, Cambarus cumberlandensis, Cambarus dubius, Cambarus reflexus, Cambarus scotti, Cambarus longirostris, Cambarus hubrichti, Monomia lucida, Faughnia serenei, Harpiosquilla melanoura, Harpiosquilla annandalei, Cherax cuspidatus, Cherax paniaicus, Cherax lorentzi, Cherax albertisii, Cherax rotundus, Cherax leckii, Cherax murido, Cherax wasselli, Cherax parvus, Cherax pallidus, Cherax cartalacoolah, Cherax rhynchotus, Cherax pulcher, Cherax peknyi, Cherax setosus, Cherax misolicus, Cherax warsamsonicus, Cherax snowden, Cherax boschmai, Cherax nucifraga, Cherax barrette, Oratosquilla fabricii, Astacopsis tricornis, Thalamita admete, Faxonius virilis, Thranita prymna, Astacopsis franklinii, Cambaroides schrenckii, Orconectes australis, Thalamita chaptalii, Zygita longifrons, Thalamita picta, Thalamita seurati, Thranita pelsarti, Orconectes barri, Faxonius ronaldi, Faxonius neglectus, Orconectes compressus, Orconectes forceps, Orconectes pellucidus, Neoeriocheir leptognathus, Penaeus kerathurus, Penaeus marginatus, Penaeus longistylus, Penaeus plebejus, Metapenaeopsis liui, Metapenaeopsis lamellata, Metapenaeopsis acclivis, Metapenaeopsis commensalis, Atypopenaeus stenodactylus, Aristeus antillensis, Solenocera vioscai, Penaeus chinensis, Penaeus spp., Metapenaeopsis barbata, Penaeus esculentus, Heteropenaeus longimanus, Atypopenaeus dearmatus, Funchalia taaningi, Xiphopenaeus kroyeri, Trachypenaeopsis mobilispinis, Rimapenaeus similis, Parapenaeus politus, Solenocera membranacea, Alcockpenaeopsis hungerfordii, Batepenaeopsis tenella, Pandalus platyceros, Metapenaeus moyebi, Metapenaeus joyneri, Pandalus montagui, Penaeus brasiliensis, Aristeus antennatus, Heterocarpus laevigatus, Heterocarpus lepidus, Funchalia villosa, Hemipenaeus carpenter, Mesopenaeus tropicalis, Pelagopenaeus balboae, Penaeus hathor, Metapenaeopsis provocatoria, Aristeus virilis, Aristeus alcock, Penaeus aztecus, Heterocarpus abulbus, Penaeus setiferus, Cerataspis monstrosus, Pleoticus robustus, Aristaeopsis edwardsiana, Solenocera necopina,Parapenaeus cayrei, Parapenaeus fissurus, Parapenaeus investigatoris, Parapenaeus fissuroides, Parapenaeus americanus, Heterocarpus ensifer, Kishinouyepenaeopsis cornuta, Parapenaeus perezfarfantae, Parapenaeus murrayi, Parapenaeus longipes, Parapenaeus spp., Heterocarpus chani, Heterocarpus sibogae, Heterocarpus dorsalis, Metapenaeopsis andamanensis, Metapenaeopsis coniger, Macrobrachium idella, Trachysalambria brevisuturae, Trachysalambria aspera, Trachysalambria albicoma, Euphausia superba, Solenocera hextii, Hymenopenaeus equalis, Rimapenaeus constrictus, Crangon crangon, Trachypenaeus anchoralis, Megokris spp., Trachysalambria longipes, Trachysalambria starobogatovi, Trachysalambria nansei, Trachysalambria malaiana, Trachysalambria spp., Trachysalambria parvispina, Crangon uritai, Pandalus borealis, Metapenaeus monoceros, Pandalus nipponensis, Hadropenaeus lucasii, Ganjampenaeopsis uncta, Solenocera annectens, Solenocera melantho, Parapenaeopsis stylifera, Penaeus japonicus, Penaeus brevirostris, Penaeus notialis, Penaeus duorarum, Penaeus schmitti, Artemesia longinaris, Penaeus subtilis, Penaeus stylirostris, Penaeus vannamei, Macrobrachium rosenbergii, Penaeus monodon, Pandalus hypsinotus, Heterocarpus* spp., *Pandalus jordani, Macrobrachium bullatum, Penaeus merguiensis, Metapenaeus ensis, Acetes chinensis, Macrobrachium nipponense, Penaeus californiensis, Macrobrachium lanchesteri, Pleoticus muelleri, Metapenaeus affinis, Hymenopenaeus neptunus, Penaeus indicus, Aristaeomorpha foliacea, Solenocera* spp., *Mierspenaeopsis hardwickii, Penaeus latisulcatus, Penaeus semisulcatus, Penaeus isabelae, Sicyonia lancifer, Metapenaeopsis dalei, Metapenaeopsis gerardoi, Parapenaeus longirostris, Pandalus eous, Pandalus miyakei, Pandalus japonicas, Pandalus glabrus, Pandalus teraoi, Pandalus ivanovi, Pandalus coccinatus, Pandalus formosanus, Pandalus chani, Pandalus* spp., *Pandalus longirostris, Pandalus latirostris, Metapenaeus* spp., *Palaemon* spp., *Palaemon serratus, Macrobrachium nipponense, Palaemon capensis, Palaemon sinensis ,Palaemon annandalei, Palaemon gravieri, Palaemon serenus, Palaemon carinicauda, Palaemon pugio, Palaemon pandaliformis, Palaemon elegans, Palaemon longirostris, Palaemon peringueyi, Palaemon debilis, Palaemon carteri, Palaemon ritteri, Palaemon orientis, Macrobrachium gracilirostre, Palaemon vulgaris, Palaemon serrifer, Palaemon varians, Palaemon macrodactylus, Palaemon tonkinensis, Palaemon xiphias, Palaemon ivonicus, Palaemon pacificus, Palaemon atrinubes, Palaemon intermedius, Palaemon concinnus, Palaemon yuna, Palaemon antennarius, Palaemon dolospinus, Palaemon gracilis, Palaemon mundusnovus, Palaemon suttkusi, Palaemon zariquieyi, Macrobrachium australiense, Palaemon semmelinkii, Palaemon litoreus, Palaemon septemtrionalis, Palaemon guangdongensis, Palaemon hancocki, Palaemon vietnamicus, Palaemon texanus, Palaemon ortmanni, Palaemon turcorum, Palaemon kadiakensis, Macrobrachium asperulum, Macrobrachium australe, Macrobrachium olfersii, Macrobrachium jelskii, Macrobrachium villosimanus, Macrobrachium equidens, Macrobrachium potiuna, Macrobrachium malcolmsonii, Macrobrachium superbum, Macrobrachium striatum, Macrobrachium latidactylus, Macrobrachium hancocki, Macrobrachium acanthurus, Macrobrachium inflatum, Macrobrachium crenulatum, Macrobrachium carcinus, Macrobrachium americanum, Macrobrachium latimanus, Macrobrachium mammillodactylus, Macrobrachium faustinum, Macrobrachium heterochirus, Macrobrachium scabriculum, Macrobrachium digueti, Macrobrachium tenellum, Macrobrachium idae, Macrobrachium formosense, Macrobrachium dienbienphuense, Macrobrachium placidulum, Macrobrachium sintangense, Macrobrachium niphanae, Macrobrachium totonacum, Macrobrachium tuxtlaense, Macrobrachium vicconi, Macrobrachium villalobosi, Macrobrachium amazonicum, Macrobrachium canarae, Macrobrachium tratense, Macrobrachium forcipatum, Macrobrachium hirsutimanus, Macrobrachium borellii, Macrobrachium brasiliense, Macrobrachium aemulum, Macrobrachium handschini, Macrobrachium horstii, Macrobrachium ferreirai, Macrobrachium lanatum, Macrobrachium novaehollandiae, Macrobrachium tolmerum, Macrobrachium iheringi, Macrobrachium saigonense, Macrobrachium nattereri, Macrobrachium aracamuni, Macrobrachium inpa, Macrobrachium depressimanum, Macrobrachium surinamicum, Macrobrachium denticulatum, Macrobrachium pilimanus, Macrobrachium ohione, Macrobrachium hainanense, Macrobrachium lepidactyloides, Macrobrachium jaroense, Macrobrachium esculentum, Macrobrachium maculatum, Macrobrachium edentatum, Macrobrachium grandimanus, Macrobrachium malayanum, Macrobrachium meridionale, Macrobrachium neglectum, Macrobrachium platycheles, Macrobrachium naso, Macrobrachium placidum, Macrobrachium yui, Macrobrachium shokitai, Macrobrachium sundaicum, Macrobrachium rude, Macrobrachium lamarrei, Macrobrachium sankolli, Macrobrachium gangeticum, Trachysalambria palaestinensis, Euphausia pacifica, Euphausia lucens, Euphausia vallentini, Euphausia triacantha, Euphausia longirostris, Euphausia similis, Euphausia recurve, Euphausia krohni, Euphausia frigida, Euphausia gibboides, Euphausia eximia, Euphausia americana, Euphausia tenera, Euphausia pseudogibba, Euphausia hemigibba, Euphausia brevis, Hymenopenaeus debilis* and *Nematopalaemon tenuipes* (Group 1).

Specifically, the identified species of the family of Cephalopods are selected from the following Group 2: *Loligo forbesii, Nototodarus sloanii, Sepia* spp., *Sepia lorigera, Sepia pardex, Rossia pacifica, Berryteuthis magister, Eledone massyae, Sepia robsoni, Loligo reynaudii, Doryteuthis (Amerigo) pealeii, Doryteuthis (Amerigo) gahi, Sepiola rondeletii,* , *Adinaefiola ligulata, Sepia smithi, Sepia elliptica, Eledone palari, Eledone moschata, Rossia palpebrosa, Gonatus madokai, Gonatus kamtschaticus, Eledone cirrhosa, Sepia elegans, Rossia bipillata, Sepiola atlantica, Lolliguncula (Lolliguncula) panamensis, Octopus maya, Illex illecebrosus, Nototodarus gouldi, Gonatopsis octopedatus, Illex coindetii, Berryteuthis anonychus, Gonatus fabricii, Lusepiola birostrata, Octopus tetricus, Uroteuthis (Photololigo) sibogae, Doryteuthis (Doryteuthis) pleii, Doryteuthis sanpaulensis, Doryteuthis (Amerigo) surinamensis, Octopus hubbsorum, Macrotritopus defilippi, Octopus insularis, Loliolus (Nipponololigo) sumatrensis, Sepia recurvirostra, Sepia madokai, Sepia kobiensis, Amphioctopus aegina, Sepia officinalis, Sepioteuthis lessoniana, Todarodes pacificus, Octopus vulgaris, Heterololigo bleekeri, Octopus sinensis, Octopus americanus, Narrowteuthis nesisi, Ommastrephes bartramii, Sepiella japonica, Uroteuthis (Photololigo) edulis, Doryteuthis (Amerigo) opalescens, Architeuthis dux, Dosidicus gigas, Sepia esculenta, Amphioctopus fangsiao, Loligo vulgaris, Sepiola* spp., *Octopus mimus, Octopus* spp., *Octopus bimaculoides, Uroteuthis (Photololigo) chinensis, Uroteuthis (Photololigo) duvaucelii, Illex argentinus, Sepia aculeata, Sepiella inermis, Sepia lycidas, Sepia latimanus, Sepia apama, Sepia pharaonis, Loliolus (Nipponololigo) beka, Alloteuthis subulata, Nototodarus hawaiiensis, Sepia orbignyana, Sepia papuensis, Rossia macrosoma, Lolliguncula (Lolliguncula) brevis, Lolliguncula (Loliolopsis) diomedeae, Afrololigo mercatoris, Octopus bimaculatus, Octopus cyanea, Callistoctopus ornatus, Enteroctopus megalocyathus,, Sasakiopus salebrosus, Octopus berrima, Amphioctopus marginatus, Octopus maorum, Octopus fitchi, Amphioctopus neglectus, Loliolus (Nipponololigo) uyii, Loliolus (Nipponololigo) japonica, Bathyteuthis abyssicola, Semirossia patagonica, Cistopus taiwanicus, Sthenoteuthis oualaniensis, Watasenia scintillans, Gonatopsis okutanii, Uroteuthis (Aestuariolus) noctiluca, Sepioteuthis australis, Sepioteuthis sepioidea, Amphioctopus kagoshimensis, Amphioctopus membranaceus, Amphioctopus exannulatus, Amphioctopus rex* and *Sepia peterseni* (Group 2).

Specifically, the identified species of the family of Gastropoda are selected from the following Group 3: *Helix pomatia, Achatina fulica, Helix aspersa, Helix aspersa maxima, Helix thessalica, Helix lucorum, Helix nicaeensis, Achatina reticulata, Helix aperta, Helix albescens, Tyrrhenaria ceratina, Helix vladika, Helix* spp., *Pleurodonte discolor, Pleurodonte lychnuchus, Erctella mazzullii, Erctella cephalaeditana, Pleurodonte formosa, Helix christophi, Helix nordmanni, Pleurodonte nucleola, Pleurodonte parilis, Gonostomopsis auridens, Caracolus caracollus, Lacteoluna selenina, Cernuella cisalpine, Cochlicella acuta, Disculella maderensis, Dialeuca nemoraloides, Monadenia fidelis, Cepaea nemoralis, Sphincterochila candidissima, Microphysula ingersolli, Helicodonta obvoluta* and *Cernuella virgata* (Group 3).

Specifically, the identified species of the family of Veneridae are selected from the following Group 4: *Tridacna mbalavuana, Siliqua alta, Megangulus zyonoensis, Megangulus venulosus, Donax faba, Donax cuneatus, Donax kiusiuensis, Mactra quadrangularis, Ensis ensis, Chamelea gallina, Spisula subtruncata, Polititapes rhomboides, Callista chione, Venerupis corrugata, Polititapes aureus, Venus crebrisulca, Mercenaria campechiensis, Antigona lamellaris, Ameghinomya antiqua, Ameghinomya* spp., *Callista erycina, Venerupis aspera, Paphia philippiana, Venus casina, Ensis* spp., *Mactra stultorum, Ensis macha, Siliqua minima, Ensis leei, Polititapes durus, Cerastoderma glaucum, Tridacna* spp., *Donax longissimus, Solen vaginoides, Venus verrucosa, Ezocallista brevisiphonata,, Procardium indicum, Cardium maxicostatum, Cardium costatum, Acanthocardia paucicostata, Acanthocardia echinata, Acanthocardia aculeata, Solen* spp., *Ruditapes philippinarum, Corculum cardissa, Spisula solida, Scrobicularia plana, Mactra spp., Chamelea striatula, Ensis siliqua, Serripes groenlandicus, Tridacna elongatissima, Tridacna rosewateri, Meretrix lamarckii, Meretrix lusoria, Paphia euglypta, Meretrix* spp., *Acanthocardia tuberculata, Tridacna maxima, Lutraria rhynchaena, Meretrix lyrata, Arctica islandica, Solen strictus, Paratapes undulatus, Paratapes textilis, Paphia amabilis, Solen grandis, Lutraria maxima, Donax vittatus, Donax variegatus, Donax trunculus, Donax semistriatus, Ruditapes decussatus, Cerastoderma edule, Tridacna squamosa, Mactra chinensis* and *Mercenaria mercenaria* (Group 4).

Specifically, the identified species of the family of Ostreidae are selected from the following Group 5: *Magallana bilineata, Magallana gigas, Crassostrea virginica, Magallana* spp., *Magallana angulata, Magallana sikamea, Magallana ariakensis, Ostrea denselamellosa, Magallana nippona, Ostrea edulis, Crassostrea* spp., *Crassostrea tulipa, Ostrea angasi, Magallana belcheri, Crassostrea rhizophorae,, Talonostrea talonata, Crassostrea corteziensis, Ostrea* spp., *Ostrea chilensis, Ostrea algoensis, Ostrea megodon, Saccostrea cuccullata, Saccostrea palmula, Saccostrea malabonensis, Saccostrea scyphophilla, Saccostrea kegaki,* and *Saccostrea* spp. (Group 5).

Specifically, the identified species of the family of Pectinidae are selected from the following Group 6: *Euvola* spp., *Mimachlamys crassicostata, Gloripallium pallium, Flexopecten glaber,, Aequipecten opercularis, Nodipecten nodosus, Scaeochlamys livida, Pecten* spp., *Talochlamys multistriata, Patinopecten caurinus, Chlamys behringiana, Placopecten septemradiatus, Pecten maximus, Zygochlamys delicatula, Chlamys hastata, Ylistrum japonicum, Talochlamys gemmulata, Zygochlamys patagonica, Argopecten purpuratus, Argopecten irradians, Azumapecten farreri, Mizuhopecten yessoensi, Placopecten magellanicus, Chlamys islandica, Argopecten ventricosus, Mimachlamys varia, Amusium pleuronectes, Mimachlamys sanguinea, Talochlamys dichroa, Mimachlamys gloriosa, Mimachlamys cloacata, Mimachlamys asperrima, Annachlamys striatula, Decatopecten radula, Bractechlamys vexillum, Aequipecten glyptus, Scaeochlamys lemniscata, Chlamys rubida, Karnekampia sulcata, Crassadoma gigantea,* and *Ylistrum balloti* (Group 6).

Specifically, the identified species of the family of Mytilidae are selected from the following Group 7: *Mytilus* spp., *Perna perna, Mytilus unguiculatus, Perna viridis, Mytilus californianus, Mytilus trossulus, Mytilus galloprovincialis, Mytilus edulis and Perna canaliculus* (Group 7).

Further provided herein is a kit for identifying seafood species in a sample, comprising one or more primer sets selected from the group of
i. ps 1 for identifying seafood species of the family of Crustacean comprising one or more primer pairs of one forward primer selected from any one of SEQ ID NOs: 1 and 2, and the reverse primer SEQ ID NO: 15 and/or the reverse complement sequences of the primer sequences;
ii. ps 2 for identifying seafood species of the family of Cephalopods comprising one or more primer pairs of one forward primer selected from any one of SEQ ID NOs: 3 to 5, and the reverse primer SEQ ID NO: 16 and/or reverse complement sequences of the primer sequences;
iii. ps 3 for identifying seafood species of the family of Gastropoda comprising one or more primer pairs of the forward primer SEQ ID NO: 6, and one reverse primer selected from any one of SEQ ID NOs: 17 to 18 and/or reverse complement sequences of the primer sequences;
iv. ps 4 for identifying seafood species of the family of Veneridae comprising one or more primer pairs of one forward primer selected from any one of SEQ ID NOs: 7 to 11, and one reverse primer selected from any one of SEQ ID NOs: 19 to 21 and/or reverse complement sequences of the primer sequences;
v. ps 5 for identifying seafood species of the family of Ostreidae comprising the forward primer SEQ ID NO: 12 and the reverse primer SEQ ID NO: 22 and/or reverse complement sequences of the primer sequences;
vi. ps 6 for identifying seafood species of the family of Pectinidae comprising the forward primer SEQ ID NO: 13 and the reverse primer SEQ ID NO: 23 and/or reverse complement sequences of the primer sequences; and
vii. ps 7 for identifying seafood species of the family of Mytilidae comprising one or more primer pairs of the forward primer SEQ ID NO: 14, and one reverse primer selected from any one of SEQ ID NOs: 24 to 25 and/or reverse complement sequences of the primer sequences,
optionally further comprising PCR components, buffers, reagents and/or an instruction manual.

Further provided herein is a library of primer sequences comprising any one of SEQ ID NOs: 1 to 25.

### DETAILED DESCRIPTION

Unless indicated or defined otherwise, all terms used herein have their usual meaning in the art, which will be clear to the skilled person. Reference is for example made to the standard handbooks, such as Sambrook et al, "Molecular Cloning: A Laboratory Manual" (4th Ed.), Vols. 1 -3, Cold Spring Harbor Laboratory Press (2012); Krebs et al., "Lewin's Genes XI", Jones & Bartlett Learning, (2017); Berg et al, "Stryer Biochemie" Springer Verlag, 2018; and Murphy & Weaver, "Janeway's Immunobiology" (9th Ed., or more recent editions), Taylor & Francis Inc, 2017.

The subject matter of the claims specifically refers to artificial products or methods employing or producing such artificial products, which may be variants of native (wild-type) products. Though there can be a certain degree of sequence identity to the native structure, it is well understood that the materials, methods and uses of the invention, e.g., specifically referring to isolated nucleic acid sequences, amino acid sequences, expression constructs, transformed host cells and modified proteins and enzymes, are "man-made" or synthetic, and are therefore not considered as a result of "laws of nature".

The terms "comprise", "contain", "have" and "include" as used herein can be used synonymously and shall be understood as an open definition, allowing further members or parts or elements. "Consisting" is considered as a closest definition without further elements of the consisting definition feature. Thus "comprising" is broader and contains the "consisting" definition.

The term "about" as used herein refers to the same value or a value differing by +/-5 % of the given value.

As used herein and in the claims, the singular form, for example "a", "an" and "the" includes the plural, unless the context clearly dictates otherwise.

The method of the present invention relates to the field of DNA barcoding. Compared with existing morphological identification methods, the method of the present invention is not affected by the experience of inspectors or the morphological changes after processing, which greatly improves the feasibility of sample detection. Compared with the existing DNA barcoding techniques, the present invention solves the problem of false negative results in highly processed foods caused by the difficulty in amplifying DNA of bivalve organisms by the existing DNA barcodes. Therefore, the DNA barcode and its application method established by the present invention are important supplements to the existing DNA barcoding techniques, and can be used to simultaneously identify a plurality of seafood species of the family of Crustacean, Cephalopods, Gastropoda, Veneridae, Ostreidae, Pectinidae, and Mytilidae even if present in very low amounts.

The DNA barcoding identification technique is mainly used for species identification by using relatively short DNA fragments in the organism with sufficient variation that can represent and map this species by means of PCR amplification, sequencing and alignment, etc. DNA barcoding aims at detecting a broad range of species by using universal primer systems. DNA metabarcoding allows the identification of multiple species in food samples in one and the same sequencing run.

DNA barcodes commonly contain conserved regions at both ends, serving as binding sites for universal primers, and a variable part in between the primer binding sites, for differentiation between the species of interest. Due to its high copy number and robustness, mitochondrial DNA (mtDNA) is preferred over genomic DNA. The mtDNA regions most commonly used for species identification are cytochrome c oxidase subunit I (COI), cytochrome b (cyt b), and 16S ribosomal DNA (16S rDNA).

According to the invention, seafood species are identified based on a barcoding method. The term "seafood species" refers to species of the following families: Crustacean, Cephalopods, Gastropoda, Veneridae, Ostreidae, Pectinidae, and Mytilidae. In biological classification, the taxonomic rank is the relative level of a group of organisms in a taxonomic hierarchy. As used herein, the following taxonomic ranks are referred to as according to their relative level of a group of organisms in descending taxonomic hierarchy: family, genus, species. As an example, the species *Pecten jacobaeus* and *Placopecten magellanicus* belong to the genus Pecten and the genus Pecten belongs to the family of Pectinidae.

According to a specific embodiment, the seafood species identified according to the invention are the species described herein selected from the group of families of Crustacean, Cephalopods, Gastropoda (snails), Veneridae (venus clams), Ostreidae (oysters), Pectinidae (scallops), and Mytilidae (mussels).

The term "sample" refers to samples which may be taken from different seafood, foodstuff, different origin of the foodstuff and various processing degrees of the food. For example, the sample is taken from sauces, soups, seafood mix, chips, pastes, seafood in cans e.g. mussels in various sauces, raw or frozen seafood, or raw or frozen seafood ingredients.

According to one embodiment of the invention, DNA is isolated from a sample. DNA can be isolated from a sample e.g., by using special kits for DNA isolation/extraction or by using the CTAB (ionic detergent cetyltrimethylammonium bromide)-method. The method for isolating DNA according to the invention enables the isolation of DNA from different samples, specifically from food samples.

According to one embodiment of the invention, the amplification of DNA fragments of isolated DNA is performed by PCR (polymerase chain reaction). Variations of PCR may be used, e.g. real time PCR with intercalating dyes.

In another embodiment, in order to improve the PCR amplification according to the invention, concentrations of compounds commonly used in PCR technology may be adapted. For example, the concentration of magnesium chloride, commonly used in PCR amplification assays, may be increased or decreased depending on the specific setup.

In yet another embodiment, PCR methods rely commonly on thermal cycling, wherein the DNA is replicated by repeated cycles of heating and cooling permitting different temperature-dependent reactions. In general, one PCR-cycle comprises the steps of denaturation, annealing, and extension. Accordingly, the PCR method according to the invention comprises 25-30 cycles for the amplification of DNA fragments. Specifically, the PCR method according to the invention comprises 25, 26, 27, 28, 29, or 30 cycles.

In yet another specific embodiment, the PCR method according to the invention comprises an annealing temperature of 60-65 °C, specifically 60, 61, 62, 63, 64, or 65 °C. More specifically, the annealing temperature is 62 °C.

According to one embodiment, in the PCR assay of the invention, DNA fragments of different seafood species are amplified in one single PCR assay due to the usage of multiple primer pairs. Thereby, a primer pair is consisting of one forward and one reverse primer. Multiplex PCR is enabled since multiple primer pairs are used within a single PCR mixture to produce amplicons of different DNA sequences. Thereby, several different DNA sequences can be amplified simultaneously. By amplifying multiple different DNA fragments at once, additional information is gained from a single test-run.

According to one embodiment of the invention, fragments of the 16S rDNA of the mtDNA region are amplified from isolated DNA samples. Thus, the mtDNA region used for seafood species identification is the 16S rDNA.

In a specific embodiment, the 16S rDNA fragments comprise 130bp to 220 bp. The length of 16S rDNA fragments vary among the seafood species to be identified. The 16S rDNA fragments comprise 190bp to 220bp, 150bp to 160bp, or 130bp to 150 bp. Specifically, for species of the family of Crustacean, the 16S rDNA fragments comprise 198-220bp. Specifically, for species of the family of Cephalopods, the 16S rDNA fragments comprise 194-220bp. Specifically, for species of the family of Gastropoda, the 16S rDNA fragments comprise 154-157bp. Specifically, for species of the family of Veneridae, the 16S rDNA fragments comprise 128-149bp. Specifically, for species of the families of Ostreidae, Pectinidae, and Mytilidae, the 16S rDNA fragments comprise 133-148bp.

According to the invention, a primer pair consisting of one forward primer and one reverse primer binds to a conserved region at both ends of the 16S rDNA fragment which is targeted to be amplified by PCR. In order to identify the seafood species according to the invention, a PCR product has to be obtained for the species to be identified. Due to high sequence variability between closely related seafood species of certain families, more than one primer pair is needed to obtain a PCR product for all the species of the family.

Specifically, the term "primer set" as used herein refers to the set of all the primer pairs which are needed for the identification of seafood species of a specific family, wherein the families are the following: Crustacean, Cephalopods, Gastropoda, Veneridae, Ostreidae, Pectinidae, and Mytilidae. As an example, three primer sets are needed for the identification of seafood species belonging the three families, Pectinidae, Ostreidae, and Mytilidae and each primer set is specific for one of the families.

More specifically, depending on the specific family, the primer set comprises one primer pair consisting of one forward primer and one reverse primer or comprises more than one primer pair. In the case that the primer set comprises more than one primer pair, each of the primer sequences of the primer set can be used to form multiple primer pairs. For example, if one primer set comprises two forward primer sequences and one reverse primer sequence, two primer pairs are formed. These two primer pairs are formed by selecting one forward primer from the two forward primer sequences and combining the forward primer sequence with the reverse primer sequence.

According to one embodiment of the invention, the primers sequences of the primer sets specific for the identification of seafood species of the families Crustacean, Cephalopods, Gastropoda, Veneridae, Ostreidae, Pectinidae, and Mytilidae are given in Table 1. The forward primer SEQ ID NOs: 1 to 14 and reverse primer SEQ ID NOs: 15 to 25 are given in 5'-3' direction. The reverse complement of the primer sequences can also be used in the method provided herein.

Specifically, primer set (ps) 1 is used for the identification of species of the family of Crustacean. In ps 1, one or more, specifically one or two primer pairs are formed by selecting a forward primer from forward primer sequences SEQ ID NOs: 1 and 2 and combining the selected forward primer with the reverse primer given in reverse primer sequence SEQ ID NO: 15. The primer set for the identification of Crustacean comprises SEQ ID NOs: 1, 2, and 15.

Specifically, ps 2 is used for the identification of species of the family of Cephalopods. In ps 2, one or more, specifically one, two, or three primer pairs are formed by selecting a forward primer from SEQ ID NOs: 3 to 5 and combining the selected forward primer with the reverse primer given in reverse primer sequence SEQ ID NO: 16. The primer set for the identification of Cephalopods comprises SEQ ID NOs: 3, 4, 5, and 16.

Specifically, ps 3 is used for the identification of species of the family of Gastropoda. In ps 3, one or more, specifically one or two primer pairs are formed by forward primer from SEQ ID NO: 6 and combining the forward primer with one reverse primer selected from reverse primer sequences SEQ ID NOs: 17 to 18. The primer set for the identification of Gastropoda comprises SEQ ID NOs: 6, 17, and 18.

Specifically, ps 4 is used for the identification of species of the family of Veneridae. In ps 4, one or more, specifically one, two, three, four, or five primer pairs are formed by selecting a forward primer from SEQ ID NOs: 7 to 11 and combining the selected forward primer with one reverse primer selected from reverse primer sequences SEQ ID NOs: 19 to 21. The primer set for the identification of Veneridae comprises SEQ ID NOs: 7, 8, 9, 10, 11, 19, 20, and 21.

Specifically, ps 5 is used for the identification of species of the family of Ostreidae. In ps 5, one primer pair is formed by the forward primer SEQ ID NO: 12 and reverse primer sequence SEQ ID NO: 22. The primer set for the identification of Ostreidae comprises SEQ ID NOs: 12 and 22.

Specifically, ps 6 is used for the identification of species of the family of Pectinidae. In ps 6, one primer pair is formed by the forward primer SEQ ID NO: 13 and reverse primer sequence SEQ ID NO: 23. The primer set for the identification of Pectinidae comprises SEQ ID NOs: 13 and 23.

Specifically, ps 7 is used for the identification of species of the family of Mytilidae. In ps 7, one or more, specifically one or two primer pairs are formed by the forward primer SEQ ID NO: 14 and combining the forward primer with one reverse primer sequence selected from reverse primer sequences SEQ ID NOs: 24 to 25. The primer set for the identification of Mytilidae comprises SEQ ID NOs: 14, 24, and 25.

Specifically, the amplification of DNA fragments according to the invention is performed with at least 1, 2, 3, 4, 5, 6, or 7 primer sets.

In one embodiment, the concentrations of the primers may be adapted to the specific combination of forward and reverse primers in a primer set for a single species. For example, if the PCR amplification of Mytilidae is performed with two primer pairs, namely primer pair 1 being SEQ ID NO: 14 and SEQ ID NO: 24, and primer pair 2 being SEQ ID NO: 14 and SEQ ID NO: 25, then the concentration in the PCR assay of SEQ ID NO: 14 may be twice as high as the concentration of each SEQ ID NO: 24 and SEQ ID NO: 25.

According to the invention, the amplified sequences are sequenced in order to determine the nucleotide sequence of the amplified 16S rDNA fragment. Specifically, Sanger sequencing or next generation sequencing (NGS) is applied as sequencing technology.

According to the invention, the sequenced DNA fragments are identified based on a comparison with reference sequences of the respective species. Specifically, the variable part in between the primer binding sites is used for differentiation between the species of interest. Methods for sequence comparison are commonly known in the art. Specifically, BLASTn provided by NCBI (National Center for Biotechnology Information) can be used for sequence comparison of a sequenced DNA fragment with a reference sequence database in order to identify the seafood species in the sample. More specifically, the sequences DNA fragments obtained by the method of the invention are compared to reference sequences comprising the 16S rDNA sequence of the targeted seafood species.

According to a specific embodiment, the reference sequences of the invention are SEQ ID NOs: 28 to 1153. More specifically, the sequenced DNA fragments are identified based on a comparison with a database or a library comprising reference sequences of the species identified according to the invention. In this case, the library of reference sequences comprises SEQ ID NOs: 28 to 1153.

According to one embodiment of the invention, a library of primer sequences is provided herein comprising SEQ ID NOs: 1 to 25 and/or the reverse complement sequences thereof. The sequences of said primer sequences (SEQ ID NOs: 1 to 25) are given in the Table 1. The primer library of the invention may comprise all primers listed in Table 1. Alternatively, the primer library comprises primers of primer set 1, 2, 3, 4, 5, 6, and/or 7.

As used herein, the term "Fwd" refers to a forward and the term "Rev" refers to a reverse primer. Alternatively, also the term "For" may be used as abbreviation for a forward primer.

The nomenclature of the nucleotide sequences of the invention follows the general guidelines of the IUPAC nucleotide code. Specifically, nucleotide code A refers to adenine, C refers to cytosine, G refers to guanine, T refers to thymine, and W refers to A or T.

According to a specific embodiment, if a primer sequence comprises a "W" at a specific position in the nucleotide sequence, said primer is used as a mixture of two sequences, wherein one primer has an adenine at the specific position and the other primer has thymine at the specific position.

**Table 1**

| Species | Type | Name | Sequence 5' - 3' | SEQ ID NO |
|---|---|---|---|---|
| Crustacean | Fwd | For_Ga1 | 5' GGGGGACGATAAGACCCTATAAA 3' | 1 |
| Crustacean | Fwd | For_Ga4 | 5' AATGGGAAGACAAGACCCTATAAA 3' | 2 |
| Crustacean | Rev | Rev_Kr45 | 5' ATTACGCTGTTATCCCTAAAGTAACTT 3' | 15 |
| Cephalopods | Fwd | For_Ti1+2 | 5' GGGACGAGAAGACCCTAWTGA 3' | 3 |
| Cephalopods | Fwd | For_Ti1+2_A | 5' GGGACGAGAAGACCCTAATGA 3' | 4 |
| Cephalopods | Fwd | For_Ti1+2_T | 5' GGGACGAGAAGACCCTATTGA 3' | 5 |
| Cephalopods | Rev | Rev_Ti5 | 5'ATTACGCTGTTATCCCTATGGTAACT 3' | 16 |
| Gastropoda | Fwd | For_Sc6 | 5' TGACTGTGCAAAGGTAGCATAAT 3' | 6 |
| Gastropoda | Rev | Rev_Sc7 | 5' AAGTTTCTAGGGTCTTCTCGTCT 3' | 17 |
| Gastropoda | Rev | Rev_Sc8 | 5' AAACTCTAAGGGTCTTCTCGTCT 3' | 18 |
| Veneridae | Fwd | For_Mu1 | 5' TTGGCCTTTAATTGGGGTCC 3' | 7 |
| Veneridae | Fwd | For_Mu2 | 5' GGTAGCGCGATAATTTGTCTCTTAA 3' | 8 |
| Veneridae | Fwd | For_Mu4 | 5' CTTAATTGGAGAAGGGTATGAATGG 3' | 9 |
| Veneridae | Fwd | For_Mu7 | 5' GTTTAACGGCCGCAGTTGTC 3' | 10 |
| Veneridae | Fwd | For_Mu15 | 5' TACCGCAGGGATAACAGCG 3' | 11 |
| Veneridae | Rev | Rev_Mu8 | 5' GCTCGACAGGGTCTTCTCGTCT 3' | 19 |
| Veneridae | Rev | Rev_Mu13 | 5' CAAAAATTCAGGTTTTTTCACTTG 3' | 20 |
| Veneridae | Rev | Rev_Mu40 | 5' TCATGTAAGAAATTAAAAAACGAACAG 3' | 21 |
| Ostreidae | Fwd | For_Mu25 | 5' GGTAGCGAAATTCCTTGCCTT 3' | 12 |
| Ostreidae | Rev | Rev_Mu26 | 5' AAAGTTGCACGGGGTCTT 3' | 22 |
| Pectinidae | Fwd | For_Mu21 | 5' TGCTAAGGTAGCTAAATTATGGCC 3' | 13 |
| Pectinidae | Rev | Rev_Mu23 | 5' CTTCACGGGGTCTTCTCGTC 3' | 23 |
| Mytilidae | Fwd | For_Mu27 | 5' CCTTTTGCATAAGGGTTTTTCAAG 3' | 14 |
| Mytilidae | Rev | Rev_Mu29 | 5' CGAATAGTATCTAGCCGCCATTC 3' | 24 |
| Mytilidae | Rev | Rev_Mu30 | 5' GCAAATAGCATATCACTTTCACCTC 3' | 25 |

According to one embodiment of the invention, a kit for identifying seafood species in a sample is provided, wherein the kit comprises the primer library of the invention. Specifically, the kit comprises primer sequences of one or more of the primer sets selected from primer set 1, primer set 2, primer set 3, primer set 4, primer set 5, primer set 6, and primer set 7. More specifically, the kit comprises at least 1, 2, 3, 4, 5, 6, or 7 of the primer sets according to the invention. The kit may also comprise the reverse complement sequences of the primer sequences.

Said kit may further comprise PCR components, buffers, reagents and/or an instruction manual. Specifically, the kit may comprise as PCR components a polymerase, a master mix, and/or magnesium chloride.

### EXAMPLES

The examples described herein are illustrative of the present invention and are not intended to be limitations thereon. Many modifications and variations may be made to the techniques described and illustrated herein without departing from scope of the invention. Accordingly, it should be understood that the examples are illustrative only and are not limiting upon the scope of the invention.

### Example 1 ― Pectinidae, Ostreidae, and Mytilidae

In Example 1, the identification of seafood species of the family of Pectinidae, Ostreidae, and Mytilidae (together known as "bivalves") in raw and processed food products is described. The method was developed on the Illumina MiSeq^{®} and iSeq^{®} platforms.

### Materials and Methods

### Sample collection and storage

86 commercial food products were collected from regional supermarkets, fish markets, and delicacy shops (Table 2). Table 2 shows the declaration, origin and processing condition of the 86 commercial food products. Samples were either fresh, deep-frozen, or in processed condition. Each sample was given a specific ID number, with the letter "O" referring to oysters, "S" to scallops, "M" to mussels, and "Mi" to mixed-species seafood. Samples were stored at -20°C until DNA extraction.

**Table 2**

| **Sample ID** | **Declaration on the product** | | ***** | | |
|---|---|---|---|---|---|
| | **Scientific/Latin name** | **Product description** | **Product origin** | **Purchase oriqin** | **Treatment** |
| O1 | *Crassostrea gigas* | Giant Oyster | France | delicacy shops | raw |
| O2 | *Ostrea edulis* | Oyster | Denmark | fish market | raw |
| O3 | *Crassostrea gigas* | Oyster/Gillardeau | Denmark | fish market | raw |
| O4 | not declared | Oyster | Pacific * | delicacy shops | raw |
| O5 | *Crassostrea gigas* | Oyster in sunflower oil | Korea | delicacy shops | processed |
| O6 | *Crassostrea gigas* | Oyster in sunflower oil | Korea | regional supermarket | processed |
| O7 | *Crassostrea gigas* | Oyster in water | Korea | delicacy shops | processed |
| O8 | not declared | Oyster sauce | Thailand | regional supermarket | processed |
| O9 | not declared | Oyster sauce | Thailand | delicacy shops | processed |
| O10 | not declared | Oyster sauce | China | delicacy shops | processed |
| M11 | *Mytilus edulis* | Mussel | Spain | delicacy shops | processed |
| M12 | *Mytilus galloprovincialis* | Blue Mussel | Spain | regional supermarket | frozen |
| M13 | *Perna canaliculus* | New Zealand green-lipped mussel | New Zealand | delicacy shops | frozen |
| M14 | *Mytilus spp.* | Blue Mussel | not declared | not declared | frozen |
| M15 | *Mytilus edulis* | Blue Mussel | North Atlantic* | delicacy shops | raw |
| M16 | *Mytilus edulis* | Bouchot mussel | France | fish market | raw |
| M17 | not declared | Grilled blue mussel | Italy | delicacy shops | processed |
| M18 | *Mytilus chilensis* | Blue Mussel | Spain | delicacy shops | frozen |
| M19 | not declared | Blue Mussel | not declared | fish market | raw |
| M20 | *Mytilus spp.* | Blue Mussel | not declared | fish market | raw |
| M21 | *Mytilus edulis* | Mussel | Denmark | delicacy shops | processed |
| M22 | *Mytilus galloprovincialis* | Blue Mussel | Italy | delicacy shops | processed |
| M23 | not declared | Mussel with sherry vinegar | Spain | delicacy shops | processed |
| M24 | *Mytilus chilensis* | Blue Mussel in tomato sauce | Denmark | regional supermarket | processed |
| M25 | not declared | Mussel in marinade sauce | Spain | regional supermarket | processed |
| M26 | not declared | Grilled blue mussel | Italy | regional supermarket | processed |
| M27 | *Mytilus chilensis* | Mussel in marinade | Spain | regional supermarket | processed |
| M28 | not declared | Dry cat food with green lipped mussel | Germany | delicacy shops | processed |
| M29 | *Mytilus galloprovincialis* | Blue mussel in tomato sauce | Spain | regional supermarket | processed |
| M30 | *Mytilus galloprovincialis* | Blue mussel a la mariniere | Spain | regional supermarket | processed |
| M31 | not declared | Blue mussel in organic marinade | Germany | delicacy shops | processed |
| M32 | not declared | Marinated blue mussels | Spain | delicacy shops | processed |
| M33 | *Mytilus chilensis* | Mussel in Escabeche | Denmark | delicacy shops | processed |
| M34 | *Mytilus chilensis* | Mussel | Denmark | delicacy shops | processed |
| M35 | *Mytilus chilensis* | Mussel in tomato sauce | Denmark | delicacy shops | processed |
| M36 | *Mytilus galloprovincialis* | Blue mussel marinated | Germany | delicacy shops | processed |
| M37 | *Mytilus edulis* | Mussel in honey mustard sauce | Denmark | delicacy shops | processed |
| M38 | not declared | Blue mussel in marinade | Spain | delicacy shops | processed |
| M39 | *Mytilus chilensis* | Blue mussel | Germany | not declared | processed |
| M40 | *Mytilus edulis* | Blue mussel | Netherlands | not declared | raw |
| S41 | *Placopecten magellanicus* | Deep-sea scallop | France | regional supermarket | cooled |
| S42 | *Mizuhopecten yessoensis* | Yesso scallop | North Atlantic | fish market | cooled |
| S43 | *Pecten maximus* | Great scallop | Pacific * | delicacy shops | cooled |
| S44 | *Pecten spp.* | Great scallop | not declared | not declared | frozen |
| S45 | *Placopecten magellanicus* | Deep-sea scallop | not declared | not declared | frozen |
| S46 | *Pecten jacobaeus* | Great scallop | Croatia | delicacy shops | frozen |
| S47 | *Zygochlamys patagonica* | Scallop "á la Bretonne" | France | delicacy shops | processed |
| S48 | *Patinopecten yessoensis* | Great scallop/ Yesso scallop | Pacific * | fish market | cooled |
| S49 | *Placopecten magellanicus* | Great scallop | Pacific * | delicacy shops | cooled |
| S50 | *Argopecten purpuratus* | Pacific scallop | Peru | delicacy shops | frozen |
| S51 | not declared | Great scallop | not declared | fish market | cooled |
| S52 | *Patinopecten yessoensis* | Great scallop | Pacific * | delicacy shops | cooled |
| S53 | *Pecten sp.* | Great scallop | not declared | fish market | cooled |
| S54 | *Placopecten magellanicus* | Great scallop | Pacific * | delicacy shops | cooled |
| S55 | *Aequipecten opercularis* | Scallop in sauce | Spain | delicacy shops | processed |
| S56 | not declared | Great scallop | not declared | restaurant | processed |
| S57 | *Placopecten magellanicus* | Great scallop | Austria | fish market | cooled |
| S58 | not declared | Rillettes de Saint-Jacques | France | delicacy shops | processed |
| S59 | not declared | Small scallop in galician sauce | Spain | delicacy shops | processed |
| S60 | not declared | Deep-sea scallop | Germany | not declared | frozen |
| S61 | *Patinopecten yessoensis* | Great scallop | Pacific * | delicacy shops | cooled |
| Mi62 | *Mytilus chilensis* | Seafood mix | France | regional supermarket | frozen |
| Mi63 | not declared | Sauce with seafood | Italy | regional supermarket | processed |
| Mi64 | *Mytilus chilensis, Mytilus edulis* | Seafood mix | Germany | delicacy shops | processed |
| Mi65 | not declared | Bouillabaise Marsille | Germany | delicacy shops | processed |
| Mi66 | *Mytilus chilensis* | Seafood mix | France | regional supermarket | processed |
| Mi67 | *Mytilus spp.* | Seafood mix | Chile | regional supermarket | processed |
| Mi68 | *Mytilus galloprovincialis* | Sea fruit salad in sunflower oil | Italy | regional supermarket | processed |
| Mi69 | *Mytilus chilensis* | Seafood mix | France | delicacy shops | processed |
| Mi70 | not declared | Sea fruit salad fantasy | Italy | regional supermarket | processed |
| Mi71 | not declared | Seafood mix | Italy | regional supermarket | processed |
| Mi72 | *Mytilus chilensis* | Seafood mix | Croatia | delicacy shops | processed |
| Mi73 | not declared | Seafood mix | not declared | not declared | unknown |
| Mi74 | not declared | Seafood mix | not declared | not declared | unknown |
| Mi75 | not declared | Pizza Frutti di mare | Austria | restaurant | processed |
| Mi76 | not declared | Paella | Germany | delicacy shops | processed |
| Mi77 | *Mytilus edulis, Mytilus chilensis* | Paella | Germany | regional supermarket | processed |
| Mi78 | *Mytilus chilensis* | Seafood all'Olio | France | regional supermarket | processed |
| Mi79 | *Mytilus chilensis* | Seafood mix | Spain | delicacy shops | processed |
| Mi80 | *Mytilus chilensis* | Seafood mix | Austria | delicacy shops | processed |
| Mi81 | not declared | Sea fruit salad | Italy | delicacy shops | processed |
| Mi82 | *Zygochlamys patagonica, Chlamys opercularis* | Scallop terrine | France | delicacy shops | processed |
| Mi83 | not declared | Terrine of salmon and great scallop | Austria | delicacy shops | processed |
| Mi84 | *Mytilus chilensis* | Seafood mix | Germany | delicacy shops | processed |
| Mi85 | not declared | Instant noodle seafood, mild | Korea | delicacy shops | processed |
| Mi86 | not declared | Instant noodle seafood, spicy | Korea | delicacy shops | processed |

| | | | | | |
|---|---|---|---|---|---|
| * In case the country of production was unknown, the fishing region was specified | | | | | |

Eleven out of the 86 samples ("reference samples"), comprising three mussel, six scallop, and two oyster species, were used for method development. Table 3 shows the bivalve species used for development of the DNA metabarcoding method. Identity of bivalve species in these reference samples (samples M12, M13 and M27 for mussels; samples S42, S46, S47, S49, S50, and S55 for scallops; samples O2 and O3 for oysters; Table 2) was verified by subjecting DNA extracts to Sanger sequencing (Microsynth, Balgach, Switzerland) and matching the sequences against the public databases provided by the National Center for Biotechnology Information (NCBI).

**Table 3**

| ***Scientific name*** | **Commercial name (German)** | **Commercial name (English)** |
|---|---|---|
| **Mytilidae** | **Miesmuscheln** | **mussels** |
| *Mytilus edulis* | Gemeine Miesmuschel | Blue mussel |
| *Mytilus galloprovincialis* | Mittelmeer-Miesmuschel | Mediterranean mussel |
| *Perna canaliculus* | Neuseeland-Miesmuschel | New Zealand green-lipped mussel |
| **Pectinidae** | **Kammmuscheln** | **scallops** |
| *Placopecten magellanicus* | Atlantischer Tiefseescallop | Atlantic deep-sea scallop |
| *Mizuhopecten yessoensis* | Japanische Kammmuschel | Yesso scallop |
| *Pecten jacobaeus* | Jakobsmuschel | Great scallop |
| *Zygochlamys patagonica* | Patagonische Kammmuschel | Patagonian scallop |
| *Argopecten purpuratus* | Purpur-Kammmuschel | Purple scallop |
| *Aequipecten opercularis* | Kleine Pilgermuschel | Queen scallop |
| **Ostreidae** | **Austern** | **oysters** |
| *Magallana gigas* | Pazifische Felsenauster | Pacific oyster |
| *Ostrea edulis* | Europäische Auster | European flat oyster |

### DNA extraction and quantification

Raw material was cut into smaller pieces or homogenized. To 2.0 gram of each sample, 10 mL of a hexadecyltrimethylammonium bromide (CTAB) buffer was added. After addition of 80 µL proteinase K, the mixture was incubated on an lntelli-Mixer^{™} RM2 (LTF Labortechnik) overnight at 50°C.

For DNA isolation, a commercial kit (Maxwell^{®} 16 FFS Nucleic Acid Extraction System Custom-Kit, Promega, Madison, USA) was used according to the manufacturer's instruction. DNA concentration was determined fluorometrically (Qubit^{®} 2.0 fluorometer, Thermo Fisher Scientific, Oregon, USA). For higher concentrations, the Qubit^{®} dsDNA broad range assay kit (2 to 1000 ng) and for lower concentrations, the Qubit^{®} dsDNA high sensitivity assay kit (0.2 to 100 ng) was used. DNA purity was assessed from the ratio of the absorbance at 260 nm and 280 nm (QIAxpert spectrophotometer, software version 2.2.0.21, Qiagen, Hilden, Germany). DNA extracts were stored at -20°C until further use.

### DNA extract mixtures

Ternary DNA extract mixtures were prepared by mixing DNA extracts (DNA concentration 5 ng/mL) from *Pecten* spp., *Magallana gigas* and *Mytilus galloprovincialis,* representing the three bivalve species Pectinidae, Ostreidae, and Mytilidae, respectively. Individual DNA extracts were mixed in a ratio of 98.0:1.5:0.5 (v/v/v).

In addition, DNA extract mixtures consisting of DNA from species belonging to one bivalve species were prepared. In these mixtures, DNA from one species was present as the main component, DNA from the other species as minor components (1.0% each). Since only two oyster species were available, the DNA extract mixture representing the bivalve species Ostreidae contained the closely related scallop (*Placopecten magellanicus)* as major component (98.0%) and DNA from the two oyster species as minor components (1.0% each).

In addition to mixtures consisting of DNA from bivalve species only, a DNA extract mixture containing another mollusc species was prepared. DNA extract from a squid species *(Sepiella inermis)* was chosen as the main component (97.0%) and DNA from the bivalve species *Placopecten magellanicus, Ostrea edulis* and *Perna canaliculus* were present as minor components (1.0% each).

### Reference sequences

A 150 bp fragment of the mitochondrial 16S rDNA gene was used as DNA barcode. Reference sequences for commonly consumed bivalve species and some exotic seafood species, that are permitted for consumption in Austria ("Codex Alimentarius Austriacus" chapter B35, (see Bundesministerium für Arbeit, Soziales, Gesundheit und Konsumentenschutz, Codexkapitel / B 35 / Fische, Krebse, Weichtiere und daraus hergestellte Erzeugnisse: BMGF-75210/0026-II/B/13/2017, 2007), were downloaded from the NCBI databases by using CLC Genomics Workbench 10.1.1 (Qiagen). If available, complete reference sequences from the RefSeq database were preferentially downloaded due to their reliability. In case complete reference sequences were not available, all DNA sequences of the mitochondrial 16S rDNA available for one and the same species, submitted by individual scientists, were aligned and checked for similarity and unidentified nucleotides. Subsequently, the DNA sequence with the highest quality (e.g. without unknown nucleotides, full-length of the DNA barcode) was chosen as a reference sequence.

The reference sequences according to the invention are given in Table 4. This list of sequences comprises all reference sequences used for the identification of all seafood species of the invention and thus, does not only comprise the sequences for example 1 but for the seafood species identified according to the invention.

**Table 4**

| Access ion No. | Species | Sequence (nucleotide) | SEQ ID NO |
|---|---|---|---|
| NC_03 0633 | Mytilus chilensis | CCTTTTGCATAAGGGTTTTTCAAGATCAATTTAAGAATTTAATTTTC CCGAATAAAAGAGAGTTATTTTTTGGAGTTAGGAAATCGTGGTAGA GATTTGATTAAACTAAAGAATGGCGGCTAGATACTATTCG | 28 |
| NC_02 6288 | Perna perna | CCTTTTGCATAAGGGTTTTTCAAGAAAATTTACTTATGTATTTTCCC GAAAAGGAAGAAGCTATCGTTGTGAGTGTGAATACCGTGGTAAAG TTATGACCAACTATACGATAAAAGCTAGATACTAATTGCCT | 29 |
| NC_02 4733 | Mytilus unguicula tus | CCTTTTGCATAAGGGTTTTTCAAGATAAATTTAAGCATTTAAATTTC CCGAATAAAAGAGAGTTATTCTTTTGAGTTAAAAAATCGTGGTAGA GATTAATATAAACTAAAGAATGGCGGCTAGATACTATTCG | 30 |
| NC_01 8362 | Perna viridis | CCTTTTGTATAAGGGTTTTTTTAGAAAATGAGTGTATAACTGTTTCC CGAAAAAAGATAAGTTACTTGAATTTAATTTATTTTTCGTGGTAAAG AAAAATAAAAAAATCAAGTAAAAGTGATATGCTATTTG | 31 |
| NC_01 5993 | Mytilus california nus | CCTTTTGCATAAGGGTTTTTCAAGATAAATTTAAATATTTAAATTTC CCGAATAAAAAAGAGTTATTCCTTTGAGTTAAAAAATCGTGGTAGA GATTTATATAAACTAAGGAATAGCGGCTAGATACTATTCG | 32 |
| NC_00 7687 | Mytilus trossulus | CCTTTTGCATAAGGGTTTTTCAAGACAAGTTTAAGAATTTAATTTTC CCGAATAAAAGAGAGTTATTTTTTTGAGTTAAGAAATCGTGGCAGA GATTTAATTAAACTAAAGAATAGCGGCTAGATACTATTCG | 33 |
| NC_00 6886 | Mytilus galloprovi ncialis | CCTTTTGCATAAGGGTTTTTCAAGATAAATTTAAGAATTTAATTTTC CCGAATAAAAGAGAGTTATTTTTTTGAGTTAGGAAATCGTGGTAGA GATTTGATTAAACTAAAGAATGGCGGCTAGATACTATTCG | 34 |
| NC_00 6161 | Mytilus edulis | CCTTTTGCATAAGGGTTTTTCAAGATCAATTTAAGAATTTAATTTTC CCGAATAAAAGAGAGTTATTTTTTTGAGTTAGGAAATCGTGGTAGA GATTTGATTAAACTAAAGAATGGCGGCTAGATACTATTCG | 35 |
| NC_05 4242 | Perna canaliculu s | CCTTTTGTATAAGGGTTTTTCAAGAAACAAAACGTATGTTTGTATC CCGAAAAAAATAAAAGTTACTTTTTTTCTTATATTTAACGTGGCAAA GTTAAATTATAAAAAGAGGTGAAAGTGATATGCTATTTGC | 36 |
| KP100 301 | Mytilus platensis | CCTTTTGCATAAGGGTTTTTCAAGATCAATTTAAGAATTTAATTTTC CCGAATAAAAGAGAGTTATTTTTTGGAGTTAGGAAATCGTGGTAGA GATTTGATTAAACTAAAGAATGGCGGCTAGATACTATTCG | 37 |
| NC_01 3997 | Magallan a bilineata | GGTAGCGAAATTCCTTGCCTTTTGATTGTAGGCCTGCATGAATGG TTTAACGAGGGTTTAGCTGTCTCTAAATTTTTAATTGAAATTGTATT GAAGGTGAAGATACCTTCATTTAAAAGTTAGACAAAAAGACCCCGT GCAACTTT | 38 |
| NC_00 1276 | Magallan a gigas | GGTAGCGAAATTCCTTGCCTTTTGATTGTGGGCCTGCATGAATGG TTTAACGAGGGTTTGACTGTCTCTAAATTTTTTATTGAAATTGTACT GAAGGTGAAGATACCTTCATTTAAAAGTTAGACAAAAAGACCCCGT GCAACTTT | 39 |
| NC_00 7175 | Crassostr ea virginica | GGTAGCGAAATTCCTTGCCTTTTGATTGTGGGCCTGCATGAATGG TTTGACGAGGGCTTTGCTGTCTCTTGATTTTTTATTGAAATTGTAGT GTAGGTGAAAATACCTTCATAAGAAAGTTAGACAAGAAGACCCCG TGCAACTTT | 40 |
| NC_01 1518 | Magallan a hongkong ensis | GGTAGCGAAATTCCTTGCCTTTTGATTGTAGGCCTGCATGAATGG TTTAACGAGGGTTTAACTGTCTCTAAATTTTTTATTGAAATTGTACT GAAGGTGAAGATACCTTCATTTAAAAGTTAGACAAAAAGACCCCGT GCAACTTT | 41 |
| NC_01 2648 | Magallan a angulata | GGTAGCGAAATTCCTTGCCTTTTGATTGTGGGCCTGCATGAATGG TTTAACGAGGGTTTAACTGTCTCTAAATTTTTTATTGAAATTGTACT GAAGGTGAAGATACCTTCATTTAAAAGTTAGACAAAAAGACCCCGT GCAACTTT | 42 |
| NC_01 2649 | Magallan a sikamea | GGTAGCGAAATTCCTTGCCTTTTGATTGTAGGCCTGCATGAATGG TTTAACGAGGGTTTAACTGTCTCTAAATTTTTTATTGAAATTGTACT GAAGGTGAAGATACCTTCATTAAAAAGTTAGACAAAAAGACCCCG TGCAACTTT | 43 |
| NC_01 2650 | Magallan a ariakensi s | GGTAGCGAAATTCCTTGCCTTTTGATTGTAGGCCTGCATGAATGG TTTAACGAGGGTTTAACTGTCTCTTGATTTAAAATTGAAATTGTACT GAAGGTGAAGATACCTTCATTAAAAAGTCAGACAAAAAGACCCCG TGCAACTTT | 44 |
| NC_01 5231 | Ostrea dense lam ellosa | GGTAGCGAAATTCCTTGCCTTTTAATTGTAGGCCTGCATGAATGG CTTAACGAGGGCTTAACTGTCTCTGGTTTACAGGGTCTAAATTGG ATTAAAGGTGAAGATACCTTTATATAAAAGTCAGACAAGAAGACCC CGTGCAACTTTT | 45 |
| NC_01 5248 | Magallan a nippona | GGTAGCGAAATTCCTTGCCTTTTGATTGTGGGCCTGCATGAATGG TTTAACGAGGGTTTGACTGTCTCTAAGTTTTTTATTGAAATTGTATT GAAGGTGAAGATACCTTCATTTAAAAGTTAGACAAAAAGACCCCGT GCAACTTT | 46 |
| NC_01 6180 | Ostrea edulis | GGTAGCGAAATTCCTTGCCTTTTAATTGTAGGCCTGCATGAATGGT TTGACGAGGGCTTAACTGTCTCTAGTTTGTAAGTCTAAATTGGATT AAAGGTGAAGATACCTTTATAAAAAAGTCAGACAAGAAGACCCCG TGCAACTTT | 47 |
| NC_02 2688 | Ostrea lurida | GGTAGCGAAATTCCTTGCCTTTTAATTGTAGGCCTGCATGAATGG CTTGACGAGGGTTTAACTGTCTCTGGTTTGTAAGGTCTAAATTGGA TTAAAGGTGAAGATACCTTTATATAAAAGTCAGACAAGAAGACCCC GTGCAACTTT | 48 |
| NC_02 7653 | Crassostr ea tulipa | GGTAGCGAAATTCCTTGCCTTTTAATTGTAGGCCAGCATGAATGG TTTGACGAGGGCCTCACTGTCTCTTAGTTCTATGTTGAAATTGTAG TGTAGGTGAAGATACCTTCATAAAAAAGTAAGACAAAAAGACCCC GTGCAACTTT | 49 |
| AF052 063 | Ostrea angasi | GGTAGCGAAATTCCTTGCCTTTTAATTGTAGGCCTGCATGAATGGT TTGACGAGGGCTTAACTGTCTCTAGTTTGTAAGTCTAAATTGGACT AAAGGTGAAGATACCTTTATAAAAAAGTCAGACAAGAAGACCCCG TGCAACTTT | 50 |
| NC_03 7851 | Magallan a belcheri | GGTAGCGAAATTCCTTGCCTTTTGATTGTAGGCCTGCATGAATGG TTTAACGAGGGTTTAACTGTCTCTAAATTTTTAGTTGAAATTGTATT GAAGGTGAAGATACCTTCATTAAGAAGTTAGACAAAAAGACCCCG TGCAACTTT | 51 |
| FJ717 607 | Crassostr ea rhizophor ae | GGTAGCGAAATTCCTTGCCTTTTGATTGTGGGCCAGCATGAATGG TTTGACGAGGGCTTTGCTGTCTCTCGATTTTTTATTGAAATTGTAG TGTAGGTGAAAATACCTTCATAAGAAAGTTAGACAAGAAGACCCC GTGCAACTTT | 52 |
| HQ711 627 | Crassostr ea brasiliana | GGTAGCGAAATTCCTTGCCTTTTAATTGTAGGCCAGCATGAATGG TTTGACGAGGGCCTCACTGTCTCTTAGTTCTATGTTGAAATTGTAG TGTAGGTGAAGATACCTTCATAAAAAAGTAAGACAAAAAGACCCC GTGCAACTTT | 53 |
| KX364 275 | Talonostr ea talonata | GGTAGCGAAATTCCTTGCCTTTTAATTGTAGGCCTGCATGAATGGT TTAACGAGGGTTAAGCTGTCTCTAAGTTTATAAGTTGAAATTGTAT TAAAGGTGAAGATACCTTTATTTTGAAGTTAGACAAAAAGACCCCG TGCAACTTT | 54 |
| KT317 088 | Crassostr ea cortezien sis | GGTAGCGAAATTCCTTGCCTTTTAATTGTAGGCCAGCATGAATGG TTTGACGAGGGCCTCACTGTCTCTCAGCTCTATATTGAAATTGCAG TGGAGGTGAAGATACCTCCATAAGAAAGTAAGACAAGAAGACCCC GTGCAACTTT | 55 |
| AY510 450 | Magallan a rivularis | GGTAGCGAAATTCCTTGCCTTTTGATTGTAGGCCTGCATGAATGG TTTAACGAGGGTTTAACTGTCTCTAAATTTTTTATTGAAATTGTACT GAAGGTGAAGATACCTTCATTTAAAAGTTAGACAAAAAGACCCCGT GCAACTTT | 56 |
| KT317 130 | Ostrea angelica | GGTAGCGAAATTCCTTGCCTTTTAATTGTAGGCCTGCATGAATGG CTTGACGAGGGTTTAACTGTCTCTGGTTTGTAAGGTCTAAATTGGA TTAAAGGTGAAGATACCTTTATATAAAAGTCAGACAAGAAGACCCC GTGCAACTTT | 57 |
| AF052 075 | Ostrea permollis | GGTAGCGAAATTCCTTGCCTTTTAATTGTAGGCCTGCATGAATGG CTTGACGAGGGTTTAACTGTCTCTGGTTTGTAAAGTCTAAATTGGA TTAAAGGTGAAGATACCTTTATATAAAAGTCAGACAAGAAGACCCC GTGCAACTTT | 58 |
| JF808 186 | Ostrea chilensis | GGTAGCGAAATTCCTTGCCTTTTAATTGTAGGCCTGCATGAATGG CTTGACGAGGGTTTAACTGTCTCTGGTTTATAAAGTCTAAATTGGA TTAAAGGTGAAGATACCTTTATACAAAAGTCAGACAAGAAGACCCC GTGCAACTTT | 59 |
| AF052 062 | Ostrea algoensis | GGTAGCGAAATTCCTTGCCTTTTAATTGTAGGCCTGCATGAATGG CTTGACGAGGGCTTAACTGTCTCTAGTTTTAAAGTCTAAATTGGAT TAAAGGTGAAGATACCTTTATAAAAAAGTCAGACAAGAAGACCCC GTGCAACTTT | 60 |
| AF052 073 | Ostrea puelchan a | GGTAGCGAAATTCCTTGCCTTTTAATTGTAGGCCTGCATGAATGG CTTGACGAGGGTTTAACTGTCTCTGGTTTGTAAAGTCTAAATTGGA TTAAAGGTGAAGATACCTTTATATAAAAGTCAGACAAGAAGACCCC GTGCAACTTT | 61 |
| KX364 274 | Ostrea megodon | GGTAGCGAAATTCCTTGCCTTTTAATTGTAGGCCTGCATGAATGG CTTGACGAGGGTTTAACTGTCTCTGGTTTGTAAGGTCTAAATTGGA TTAAAGGTGAAGATACCTTTATATGAAAGTCAGACAAGAAGACCCC GTGCAACTTT | 62 |
| NC_02 7724 | Saccostre a cuccullata | GGTAGCGAAATTCCTTGCCTTTTAATTGTAGGCCTGCATGAATGG ATTGACGAGGGTCAAGCTGTCTCTTGCCTTGAATAACGAAATTGG ACTAAAGGTGAAGATACCTTTATAGAAAAGTTAGACAAGAAGACCC CGTGCAGCTTT | 63 |
| KT317 278 | Saccostre a palmula | GGTAGCGAAATTCCTTGCCTTTTAATTGTAGGCCTGCATGAATGG ATTGACGAGGGTCAAACTGTCTCTTGCTTTGTATAGCGAAATTGGA CTAAAGGTGAAGATACCTTTATATAAAAGTTAGACAAGAAGACCCC GTGCAGCTTT | 64 |
| KX961 677 | Saccostre a malabone nsis | GGTAGCGAAATTCCTTGCCTTTTAATTGTAGGCCTGCATGAATGG ATTGACGAGGGTCAAGCTGTCTCTTGCCTTGATGACGAAATTGGA CTAAAGGTGAAGATACCTTTATAGAAAAGTTAGACAAGAAGACCC CGTGCAGCTTT | 65 |
| NC_01 3998 | Saccostre a scyphoph illa | GGTAGCGAAATTCCTTGCCTTTTAATTGTAGGCCTGCATGAATGG ATTGACGAGGGCCATTCTGTCTCTTGCCTTATGAAGTGAAATTGG ATTAAAGGTGAAGATACCTTTATTAAAAAGTTAGACAAGAAGACCC CGTGCAGCTTT | 66 |
| NC_03 6483 | Saccostre a glomerata | GGTAGCGAAATTCCTTGCCTTTTAATTGTAGGCCTGCATGAATGG ATTGACGAGGGTCAAACTGTCTCTTGCTTTGTATGACGAAATTGGA CTGAAGGTGAAGATACCTTCATGTGAAAGTTAGACAAGAAGACCC CGTGCAGCTTT | 67 |
| NC_03 0533 | Saccostre a kegaki | GGTAGCGAAATTCCTTGCCTTTTAATTGTAGGCCTGCATGAATGG ATTGACGAGGGTCAAACTGTCTCTTGCTTTGTATGTCGAAATTGGA CTGAAGGTGAAGATACCTTCATATAAAAGTTAGACAAGAAGACCC CGTGCAGCTTT | 68 |
| NC_03 6478 | Saccostre a echinata | GGTAGCGAAATTCCTTGCCTTTTAATTGTAGGCCTGCATGAATGG ATTGACGAGGGTCAAACTGTCTCTTGCTTTGTATGACGAAATTGGA CTGAAGGTGAAGATACCTTCATGTGAAAGTTAGACAAGAAGACCC CGTGCAGCTTT | 69 |
| NC_03 6479 | Saccostre a mytiloides | GGTAGCGAAATTCCTTGCCTTTTAATTGTAGGCCTGCATGAATGG ATTGACGAGGGTCAAACTGTCTCTTGCTTTGTATGACGAAATTGGA CTGAAGGTGAAGATACCTTCATGTGAAAGTTAGACAAGAAGACCC CGTGCAGCTTT | 70 |
| AJ972 431 | Euvola vogdesi | TGCTAAGGTAGCTAAATTATGGCCTATTAATTGTAGGTCCTGTGAA TGGTTTGACGAGTTTCCAACTGTCTCTAGTTTTTTTTGGTGAACTT GAATTGGATGTGCAAATGCTTCCATGGGTAAGAAAGACGAGAAGA CCCCGTGAAG | 71 |
| NC_01 1608 | Mimachla mys crassicost ata | TGCTAAGGTAGCTAAATTATGGCCCATTAATTGTGGGTCTTGCGAA CGGCTTGACGAGTTCCCTACTGTCTCAAAGTTGTTTTGGTGAACTT GAATTGGATGTGTAAATGCTTCCATGAGTGAGAAAGACGAGAAGA CCCCGTGAAG | 72 |
| EU379 464 | Gloripalliu m pallium | TGCTAAGGTAGCTAAATTACGGCCTATTAATTGTAGGTCCTGTGAA TGGTTTGACGAGCCTCCAACTGTCTCTAGTTTATTTTGGTGAACTT GAATTGGATGTGCAAATGCTTCCATAGGTAAGAAAGACGAGAAGA CCCCGTGAAG | 73 |
| AJ243 574 | Flexopect en glaber | TGCTAAGGTAGCTAAGTTATGGTCTTTTAATTTTAGGCCCTGTGAA AGGATTGACGAGCTTGAGTCTGTCTCTATCTAAATTTTATGAAATT GAATTTTAAGTGCAAATGCTTAAGTGGACATGAAAGACGAGAAGA CCCCGTGAAG | 74 |
| AJ245 394 | Pecten jacobaeu s | TGCTAAGGTAGCTAAATTATGGCCTATTAATTGTAGGTCCTGTGAA TGGTTTGACGAGTTTCCAACTGTCTCTAGTTTTTTTGGTGAACTT GAATTGGATGTGCAAATGCTTCCATGGGTAAGAAAGACGAGAAGA CCCCGTGAAG | 75 |
| AY650 055 | Pecten novaezel andiae | TGCTAAGGTAGCTAAATTATGGCCTATTAATTGTAGGTCCTGTGAA TGGTTTGACGAGTTTCCAACTGTCTCTAGTTTTTTTTGGTGAACTT GAATTGGATGTGCAAATGCTTCCATGGGTAAGAAAGACGAGAAGA CCCCGTGAAG | 76 |
| EU379 473 | Euvola raveneli | TGCTAAGGTAGCTAAATTATGGCCTATTAATTGTAGGTCCTGTGAA TGGTTTGACGAGTTTCCAACTGTCTCTAGTTTTTTTTGGTGAACTT GAATTGGATGTGCAAATGCTTCCATGGGTAAGAAAGACGAGAAGA CCCCGTGAAG | 77 |
| AM494 408 | Aequipect en operculari s | TGCTAAGGTAGCTAAATTATGGCCCTCTAATTGGGGGCCCTGTGA ATGGATTGACGAGCCTATAACTGTCTCTAGCTGAATTATATGAAAT TGAATTTTAAGTGCAAATGCTTAAATATAAGTGATAGACGAGAAGA CCCCGTGAAG | 78 |
| HM630 517 | Euvola perula | TGCTAAGGTAGCTAAATTATGGCCTATTAATTGTAGGTCCTGTGAA TGGTTTGACGAGTTTCCAACTGTCTCTAGTTTTTTTTGGTGAACTT GAATTGGATGTGCAAATGCTTCCATGGGTAAGAAAGACGAGAAGA CCCCGTGAAG | 79 |
| GQ342 275 | Nodipect en nodosus | TGCTAAGGTAGCTAAATTATGGCCTATTAATTGTAGGTCCTGTGAA TGGTTTGACGAGTCTTCAACTGTCTCTAGTTTTTTTGATGAACTTG AATTGGATGTGCAAATGCTTCCATGGGTAAGAAAGACGAGAAGAC CCCGTGAAG | 80 |
| GQ166 559 | Scaeochl amys livida | TGCTAAGGTAGCTAAATTATGGCCCATTAATTGTGGGTCCTGTGAA TGGTTTGACGAGTCCTCGACTGTCTCAAGGTTGTTTTGGTGAACTT GAATTGAATGTGCAAATGCTTTCATGGGTAAGAAAGACGAGAAGA CCCCGTGAAG | 81 |
| FN667 667 | Pecten keppelian us | TGCTAAGGTAGCTAAATTATGGCCTATTAATTGTAGGTCCTGTGAA TGGTTTGACGAGTTTCCAACTGTCTCTAGTTTTTTTTGGTGAACTT GAATTGGATGTGCAAATGCTTCCATGGGTAAGAAAGACGAGAAGA CCCCGTGAAG | 82 |
| FN667 665 | Talochla mys multistriat a | TGCTAAGGTAGCTAAATTATGGCCTATTAATTGTAGGTCCTGTGAA TGGTTTGACGAGTCCTCTACTGTCTCAAGGTTGTTTTGGTGAACTT GAATGGAACGTGCAAATGCTTTCATGAGAGAGAAAGACGAGAAGA CCCCGTGAAG | 83 |
| FJ263 642 | Patinopec ten caurinus | TGCTAAGGTAGCTAAATTATGGCCCATTAATTGTGGGTCCTGTGAA TGGTTTGACGAGTCCTCTACTGTCTCAAGGTTGTTTTGGTGAACTT GAATTGAACGTGCAAATGCTTTCATGGGAAAGAAAGACGAGAAGA CCCCGTGAAG | 84 |
| FJ263 641 | Chlamys behringia na | TGCTAAGGTAGCTAAATTATGGCCCATTAATTGTGGGTCCTGTGAA TGGTTTGACGAGTCCTCGACTGTCTCAAGGTTGTTTTGGTGAACTT GAATTGAATGTGCAAATGCTTTCATGAGAAAGAAAGACGAGAAGA CCCCGTGAAG | 85 |
| EU379 475 | Placopect en septemra diatus | TGCTAAGGTAGCTAAATTATGGTCTATTAATTGTAGGTCTTGTGAA TGGTTTGACGAGTCTTCGACTGTCTCTAGGTTGTTTTGGTGAACTT GAATTGGATGTGTAAATGCTTCCGTGGGTGAGAAAGACGAGAAGA CCCCGTGAAG | 86 |
| KP900 975 | Pecten maximus | TGCTAAGGTAGCTAAATTATGGCCTATTAATTGTAGGCCCTGTGAA TGGTTTGACGAGTTTCCAACTGTCTCTAGTTTTTTTTGGTGAACTT GAATTGGATGTGCAAATGCTTCCATGGGTAAGAAAGACGAGAAGA CCCCGTGAAG | 87 |
| KP900 974 | Pecten albicans | TGCTAAGGTAGCTAAATTATGGCCTATTAATTGTAGGTCCTGTGAA TGGTTTGACGAGTTTCCAACTGTCTCTAGTTTTTTTTGGTGAACTT GAATTGGATGTGCAAATGCTTCCATGGGTAAGAAAGACGAGAAGA CCCCGTGAAG | 88 |
| KP300 542 | Zygochla mys delicatula | TGCTAAGGTAGCTAAATTATGGCCCATTAATTGTGGGTCCTGTGAA TGGTTTGACGAGTCCTCGACTGTCTCGAGATTACTTTGGTGAACTT GAATTAAACGTGCAAATGCTTTTATGGGTAAGAAAGACGAGAAGA CCCCGTGAAG | 89 |
| KF982 789 | Chlamys hastata | TGCTAAGGTAGCTAAATTATGGCCCATTAATTGTGGGTCCTGTGAA TGGTTTGACGAGTCCTCGACTGTCTCAAGGTTGTTTTGGTGAACTT GAATTGAACGTGCAAATGCTTTCATGAGAAAGAAAGACGAGAAGA CCCCGTGAAG | 90 |
| KF982 785 | Ylistrum japonicu m | TGCTAAGGTAGCTAAATTATGGCCTATTAATTGTAGGTCCTGTGAA TGGTTTGACGAGTCTTTAACTGTCTCTAGTTTGTTTTGGTGAAATT GAATTGGATGTGCAAATGCTTCCGTAGGTAAGAAAGACGAGAAGA CCCCGTGAAG | 91 |
| JF339 109 | Pecten fumatus | TGCTAAGGTAGCTAAATTATGGCCTATTAATTGTAGGTCCTGTGAA TGGTTTGACGAGTTTCCAACTGTCTCTAGTTTTTTTTGGTGAACTT GAATTGGATGTGCAAATGCTTCCATGGGTAAGAAAGACGAGAAGA CCCCGTGAAG | 92 |
| JF339 106 | Talochla mys gemmulat a | TGCTAAGGTAGCTAAATTATGGCCCATTAATTGTGGGTCCTGTGAA TGGTTTGACGAGTCCTCGACTGTCTCAAGATTACTTTGGTGAACTT GAATTAAATGTGCAAATGCTTTTATGGGTAAGAAAGACGAGAAGA CCCCGTGAAG | 93 |
| HM630 521 | Zygochla mys patagonic a | TGCTAAGGTAGCTAAATTATGGCCCATTAATTGTGGGTCCTGTGAA TGGTTTGACGAGTCCTCGACTGTCTCGAGATTACTTTGGTGAACTT GAATTAAATGTGCAAATGCTTTTATGGGTAAGAAAGACGAGAAGA CCCCGTGAAG | 94 |
| NC_02 7943 | Argopect en purpuratu s | TGCTAAGGTAGCTAAGTTATGGCCTAGTTAATTGTAGGCCCTGTG AATGGTTTGACGAGTTTTCTTCTGTCTCTAGCTTGTTTCAGTGAAC TTGAATTGGATGTGCAAATGCTTCCTTGCGAAAGAAAGACGAGAA GACCCCGTGAAG | 95 |
| NC_01 2977 | Argopect en irradians | TGCTAAGGTAGCTAAGTTATGGCCTAGTTAATTGTAGGCCCTGTG AATGGTTCGACGAGTTTTCTTCTGTCTCTAGCTTGTTTAAGTGAAC TTGAATTGGATGTGCAAATGCTTCCTTGGTAAAGAAAGACGAGAA GACCCCGTGAAG | 96 |
| NC_01 2138 | Azumape cten farreri | TGCTAAGGTAGCTAAATTATGGCCTATTAATTGTAGGTCCTGTGAA TGGTTTGACGAGTCCTCGACTGTCTCGAGGTTGTTTTGGTGAACT TGAATTGAATGTGCAAATGCTTTCATGGGAAAGAAAGACGAGAAG ACCCCGTGAAG | 97 |
| NC_00 9081 | Mizuhope cten yessoensi | TGCTAAGGTAGCTAAATTATGGCCCATTAATTGTGGGTCCTGTGAA TGGTTTGACGAGTCCTCTACTGTCTCAAGGTTGTTTTGGTGAACTT GAATTGAATGTGCAAATGCTTTCATACGAAAGAAAGACGAAGAAG ACCCCGTGAAG | 98 |
| NC_00 7234 | Placopect en magellani cus | TGCTAAGGTAGCTAAGTTATGGCCTATTAATTGTAGGTCTTGTGAA TGGTTTGACGAGCCTCCAACTGTCTCTAGGTTGTTTTGGTGAACTT GAGTTGAATGTGTAAATGCCTTCGTGGGTAAGAAAGACGAGAAGA CCCCGTGAAG | 99 |
| EU379 485 | Euvola ziczac | TGCTAAGGTAGCTAAATTATGGCCTATTAATTGTAGGTCCTGTGAA TGGTTTGACGAGTTTCCAACTGTCTCTAGTTTTTTTTGGTGAACTT GAATTGGATGTGCAAATGCTTCCATGGGTAAGAAAGACGAGAAGA CCCCGTGAAG | 100 |
| KU754 458 | Pecten sulcicosta tus | TGCTAAGGTAGCTAAATTATGGCCTATTAATTGTAGGTCCTGTGAA TGGTTTGACGAGTTTCCAACTGTCTCTAGTTTTTTTTGGTGAACTT GAATTGGATGTGCAAATGCTTCCATGGGTAAGAAAGACGAGAAGA CCCCGTGAAG | 101 |
| KR827 548 | Chlamys islandica | TGCTAAGGTAGCTAAATTATGGCCCATTAATTGTGGGTCCTGTGAA TGGTTTGACGAGTCCTCGACTGTCTCAAGGTTGTTTTGGTGAACTT GAATTGAATGTGTAAATGCTTTCATGAGAAAGAAAGACGAGAAGA CCCCGTGAAG | 102 |
| KT161 261 | Argopect en ventricos us | TGCTAAGGTAGCTAAGTTATGGCCTAGTTAATTGTAGGCCCTGTG AATGGTTTGACGAGTTTTCCTCTGTCTCTAGCTTGTTTTAGTGAAC TTGAATTGGATGTGCAAATGCTTCCTTGAGAAAGAAAGACGAGAA GACCCCGTGAAG | 103 |
| KT988 340 | Mimachla mys varia | TGCTAAGGTAGCTAAATTATGGCCTATTAATTGTAGGTCCTGTGAA TGGTTTGACGAGTCCCCTACTGTCTCGAGGTTGTTTTGGTGAAATT GAATTGAACGTGCAAATGCTTTCATGGGGAAGAAAGACGAGAAGA CCCCGTGAAG | 104 |
| KP900 978 | Amusium pleuronec tes | TGCTAAGGTAGCTAAATTATGGCCTATTAATTGTAGGTCCTGTGAA TGGTTTGACGAGTTTCCAACTGTCTCTAATTTTTTTTGGTGAACTT GAATTGGATGTGCAAATGCTTCCATGGGTAAGAAAGACGAGAAGA CCCCGTGAAG | 105 |
| NC_02 2416 | Mimachla mys sanguine a | TGCTAAGGTAGCTAAATTATGGCCCATTAATTGTGGGTCCTGTGAA TGGTTTGACGAGTCCTCGGCTGTCTCAAGGTTGTTTTGGTGAACT TGAATTGAATGTGCAAATGCTTTCATAGGTAAGAAAGACGAGAAG ACCCCGTGAAG | 106 |
| KP300 543 | Talochla mys dichroa | TGCAAAGGTAGCTAAATTATGGCCCATTAATTGTGGGTCCTGTGA ACGGTTTGACGAGTCCTCGACTGTCTCAAGATTACTTTGGTGAAC TTGAATTAAATGTGCAAATGCTTTTATGGGTAAGAAAGACGAGAAG ACCCCGTGAAG | 107 |
| KP300 549 | Mimachla mys gloriosa | TGCTAAGGTAGCTAAATTATGGCCCATTAATTGTGGGTCTTGCGAA CGGCTTGACGAGTTCCCTACTGTCTCAAAGTTGTTTTGGTGAACTT GAATTGGATGTGCAAATGCTTCCATGAGTAAGAAAGACGAGAAGA CCCCGTGAAG | 108 |
| JF339 118 | Mimachla mys cloacata | TGCTAAGGTAGCTGAATTCTGGCCCATTAAATGTGGGTCTTAGTG AATGGTTTGACGAGTCCTCGACTGTCTCAAGGTTGTTTTTTTGAAA TTGAATTAAATGTGCAAATGCATTTATAGGGAAGAAAGACGAGAAG ACCCCGTGAAG | 109 |
| JF339 117 | Mimachla mys asperrima | TGCTAAGGTAGCTAAATTTTGGCCCATTAATTGTGGGTCTTGTGAA TGGTTTGACGAGTTCCCTACTGTCTCAAAGTTGTTTTGGTGAACTT GAATTGGATGTGCAAATGCATCTGTAGGTAAGAAAGACGAGAAGA CCCCGTGAAG | 110 |
| KF982 786 | Annachla mys striatula | TGCTAAGGTAGCTAAATTATGGCCTATTAATTGTAGGTCCTGTGAA TGGTTTGACGAGTCTTCAACTGTCTCTGATTTGCTTTGGTGAAATT GAATTGGAAGTGCAAATGCTTCCGTAGGTAAGAAAGACGAGAAGA CCCCGTGAAG | 111 |
| KF982 788 | Decatope cten radula | TGCAAAGGTAGCTAAATTATGGCCTATTAATTGTAGGTCCTGTGAA TGGTTTGACGAGCTCCCGACTGTCTCTAGTTTGGTTTAGTGAACTT GAATTGGATGTGCAAATGCTTCCATGGGAAAGAAAGACGAGAAGA CCCCGTGAAG | 112 |
| KF982 787 | Bractechl amys vexillum | TGCAAAGGTAGCTAAATTACGGCCTATTAATTGTAGGTCCTGTGAA TGGTTTGACGAGCCTCCGGCTGTCTCTAATCTGTTTTGGTGAACTT GAATTGGACGTGCAAATGCTTCCTTGAAAAAGAAAGACGAGAAGA CCCCGTGAAG | 113 |
| EU379 445 | Aequipect en glyptus | TGCTAAGGTAGCTAAATTATGGCCTATTAATTGTAGGTCCTGTGAA TGGTTTGACGAGTTTTTAACTGTCTCTGGTTTGTTTTGGTGAATTT GAATTGGAAGTGCAAATGCTTTCGTGAGTGAGAAAGACGAGAAGA CCCCGTGAAG | 114 |
| KP300 554 | Scaeochl amys lemniscat a | TGCTAAGGTAGCTAAATTATGGCCTATTAATTGTAGGTCCTGTGAA TGGTTTGACGAGTCTTCAACTGTCTCAAAGTTATTTTGGTGAACTT GAATTAAATGTGCAAATGCATTTATAGGGAAGAAAGACGAGAAGA CCCCGTGAAG | 115 |
| FJ263 645 | Chlamys rubida | TGCTAAGGTAGCTAAATTATGGCCCATTAATTGTGGGTCCTGTGAA TGGTTTGACGAGTCCTCGACTGTCTCAAGGTTGTTTTGGTGAACTT GAATTGAATGTGCAAATGCTTTCATGAGAAAGAAAGACGAGAAGA CCCCGTGAAG | 116 |
| JF496 755 | Karneka mpia sulcata | TGCTAAGGTAGCTAAATTTTGGTCTATTAATTGTAGGTCTTGTGAA TGGTTTGACGAGTCTTCGACTGTCTCTAGGTTGTTTTGGTGAACTT GAATTGAATGTGTAAATGCTTTCGTGGGCGAGAAAGACGAGAAGA CCCCGTGAAG | 117 |
| FJ263 644 | Crassado ma gigantea | TGCTAAGGTAGCTAAATTATGGCCCATTAATTGTGGGTCCTGTGAA TGGTTTGACGAGTCCTCGACTGTCTCAAGGTTGTTTTGGTGAACTT GAATTGAACGTGCAAATGCTTTCATGGGAGAGAAAGACGAGAAGA CCCCGTGAAG | 118 |
| JF339 127 | Ylistrum balloti | TGCTAAGGTAGCTAAATTATGGCCTATTAATTGTAGGTCCTGTGAA TGGTTTGACGAGTCTTTAACTGTCTCTAGTTTGTTTTGGTGAAATT GAATTGGATGTGCAAATGCTTCCATAGGTGAGAAAGACGAGAAGA CCCCGTGAAG | 119 |
| AF122 977 | Tridacna mbalavua na | AGTAGCGTGATAAGTCGGCCTTTAATTGGGGTCTGGTATGAAGGG GTTGACGTCGGAGAAGCTGTCTCAAAAGAATTTTGTGAAATTTACT TTCTAGTGAAAAGGCTAGAATAATATTAAAAGACGAGAAGACCCTG TCGAGC | 120 |
| AB751 362 | Siliqua alta | GGTAGCGTAATAAATTGTCTCTTAATTGGAGAAGGGTATGAATGGT TTGACGTAAGGGTTTCTGTCTCAACAATAGTAATGTGAAGTTTTCT TTTAAGTCAAAAGGCTTAAATTATATTAAAAGACGAGAAGACCCTG TCGAGC | 121 |
| AB751 329 | Megangul us zyonoens is | GGTAGCATAATAGGTCGTCCTTTAATTGGGGGAAAGTATGAATGG TCGGATGTTGGAAATTCTTTATTAAGAGTAGGTGTTAAATTTTACTT TTTAGTGCAAAGGCTAAGATAATATTATTAGACGAGAAGACCCTGT TGAGC | 122 |
| AB751 328 | Megangul us venulosu s | GGTAGCATAATAGGTCGTCCTTTAATTGGGGGAAAGTATGAATGG TCGGATGTTGGAAATCCTTTATTAAGAGTAGGTGTTAAATTTTACTT TTTAGTGCAAAGGCTAAAATTAGATTATTAGACGAGAAGACCCTGT TGAGC | 123 |
| AB751 311 | Donax faba | GCGTTAAAGTAGCATAATAGGTCGTCTTTTAATTGGAGAATGGAAT GAATGGAGTGACGATGGAAAATCTTTATTAGAAATATAAGTTGAAG TTTATTTTTAAGTGAAAAGGCTTAAATTAAGGTGTTAGACGAGAAG ACCCTATTGAGC | 124 |
| AB751 310 | Donax cuneatus | GCGTTAAGGTAGCATAATAGCTCGTCTTTTAATTGGAGGATGGAAT GAATGGAGTGACGATGGAAAGTCTTTATTAAAAACACTGATCGAA GTTTACTTTTAAGTGAAAAGGCTTAAATGGAGTTGTTAGACGAGAA GACCCTGTTGAGC | 125 |
| AB751 309 | Donax kiusiuensi s | GCGTTAAAGTAGCATAATAAATTGTCTCTTAATTAGAGGATAGTAT GAAAGGTTGGATGTTGGAGAAGCTTTATTGGAAGCATTAATTGAA GTTTACATATAAGTGAAAAGGCTTATATGAGATTGTTAGACGAGAA GACCCTGTTGAGC | 126 |
| DQ160 001 | Mactra quadrang ularis | GCAATAAATTGTCCTTTAATTGGGGAAATGAATGAAGGGGTTGAC GAGGGGGAAGCTGTCTTTTTAATATAGTTCGAAGTTTTCTTTTATG TGAAAAGACTTAGATTTTACAATAAGAAGAGAAGACCCCGGCGAG C | 127 |
| AJ548 775 | Ensis ensis | CTTAATTGGAGAAGGGTATGAATGGTTTGACGTGGTAGTTTCTGT CTCAATGGTAATATTATGAAGTTTTCTTTTGAGTTAAAAGTCTCAAA TTTAGATAAAAGACGAGAAGACCCTGTCGAGC | 128 |
| AM085 110 | Chamele a gallina | GTGCTAAGGTAGCGTGATAAGTTGTCTTTTAATTGGAGAATGGTAT GAATGGTTTAACGTAGAATAACTGTCTTTGGAAAATAAAATTAAGT TTCCATGTAAGTGAAAAGGCTTATATTTTTGTAAAAGACGAGAAGA CCCTGTCGAGC | 129 |
| AJ548 774 | Spisula subtrunca ta | GGTAGCGCAGTCACTCGTCTTTTAATTGGAGAAAGGTATGAATGG TTTGACGAGGGAAAATCTGTCTCAAGGATATGGGCTAAAGTTTTCT TTTAAGTGAAAAGACTTAAATTTTTAAATAAGAAGAGAAGACCCCG GCGAGC | 130 |
| AJ548 772 | Callista chione | GTTTAACGGCCGCAGTTGTCCTGTGCTAAGGTAGCATAATGATTA GCTCTTTAATTGGGGGAAAGTATGAATGGTTTGACGTGGAGATAC TGTCTTAAATATAATAAATAGAAATTTTTTTTCAAGTGAAAAAACCT GAATTTTTG | 131 |
| AJ294 950 | Polititape s rhomboid es | TGTACTAAGGTAGCGCGGTAATTTGTCCCTTAATTGGGGGATGGT ATGAAAGGCTTGTCTTAAGTTTTTTGTCTCAAAAAGACAAAGTGAA GTTTCCTTTTAGATGAAAAGGTCTAAATAAAAATAAAAGACGAGAA GACCCTGTCGAGC | 132 |
| AJ417 845 | Venerupi s corrugata | GGTAGCGCGATAATTTGTCTCTTAATTGGAGAATGGTATGAAAGG TTTGACGCAGATCAGTTGTCTCTTAGAAAAAAGGTGAAGTTTCCTT TTAAATGAGAAGGTTTAAATTTGAATAAAAGACGAGAAGACCCTGC CGAG | 133 |
| AJ294 950 | Polititape s aureus | TGTACTAAGGTAGCGCGATAATTTGTCTCTTAATTGGAGAATGGTA TGAAAGGTTTGACGCAGATTGATTGTCTCTAGAAAAGAAAATGAAG TTTCCTTTTAAATGAAAAGGTTTAAATTGTAATAAAAGACGAGAAGA CCCTGCCGAGC | 134 |
| DQ459 295 | Venus crebrisulc a | GTGCTAAGGTAGCGTGATAAGTTGTCCTTTGATTGGGGAATGGTA TGAAAGGTTTGACGTGGAATAACTGTCTTTAGAAAATTAAACTAAG TTTCCATTTAAGTGAAAAGGCTTATATTTTTGTAAAAGACGAGAAG ACCCTGTCGAGC | 135 |
| DQ459 280 | Mercenari a campechi ensis | GGTAGCGTGATAAGTTGTCTTTTGATTGGAGAATGGAATGAAGGG TTTGACGTAGAATTTCTTTTTTTGTAGAAGATACTAAAATTTCCTTC TAAGTGAAAAGGCTTAGGTTTTTGAAAAAGACGAGAAGACCCTGT CGAAC | 136 |
| NC_05 1506 | Antigona lamellaris | GTGCTAAGGTAGCGTGATAGGTTGTCTCTTAATTGGAGAATGGTA TGAAGGGTCTCACGTGGGGTTTCTTTTTTTAATTAATAAAATGAAG TTTCTTTTTAAGTGAAAAAGCTTAAGTTTTTGTAAAAGACGAGAAGA CCCTGTCGAGC | 137 |
| DQ459 260 | Ameghin omya antiqua | GTGCTAAGGTAGCGTGATAAGTTGTCCTTTGATTGGGGACTGGTA TGAATGGCTTGACGTAGAATGACTATTTTTATAGAATAAAGTAAAC TTTCCTTCTGAGTGAAAAGGCTTAGGTTTTTGTAAAAGACGAGAAG ACCCTGTCGAGC | 138 |
| DQ356 382 | Callista erycina | GTTTAACGGCCGCAGTTGTCCTGTGCTAAGGTAGCATAATAATTA GCTCTTTAATTGGGGGAAAGTATGAATGGTTTGACGTGGAAATAC TGTCTTTAATGTAATTAAGAGAAATTTTTTTATAGGTGAAAAAACCT AAATATTTGTAAGA | 139 |
| DQ356 381 | Venerupi s aspera | GGTAGCGCGATAATTTGTCTCTTAATTGGAGAATGGTATGAAAGG TTTGTCATAGAGCGGTTGTCTCTAAAAAATTAAGTGAAATTTCTTTT TAAATGAAAAGGTTTAAATAAAAATAAAAGACGAGAAGACCCTGTC GAGC | 140 |
| DQ356 380 | Paphia philippian a | GGTAGCGTGATAATTTGTCCTTTAATTGGGGAATGGTATGAAAGG TTTGACGTGGATGAGTTGTCTCTAGAAAAATTATTGAAGTTTCCTT TTAAATGAAAAGGTTTAAATTTTTGTAAAAGACGAGAAGACCCTGG CGAG | 141 |
| JF808 192 | Venus casina | GTGCTAAGGTAGCGTGGTAAGTTGTCTTTTAATTGGAGAATGGTAT GAATGGTTTGACGTAGAATAACTGTCTTTGGGGAATAGAATTAAGT TTCCTTTTAAGTGAAAAGGCTTATATTTTTGTAAAAGACGAGAAGA CCCTGTCGAGC | 142 |
| HF970 514 | Ensis terranove nsis | CTTAATTGGAGAAGGGTATGAATGGTTTGACGTGGTAGTTTCTGT CTCAATGGTAATATTATGAAGTTTTCTTTTGAGTTAAAAGTCTCAAA TTTAGATAAAAGACGAGAAGACCCTGTCGAGC | 143 |
| GQ166 567 | Mactra stultorum | GGTAGCGCAATCAATTGTCCTTTAATTGGGGAAAGGAATGAAGGG GTTGACGAGGGTGAGCTGTCTCTGTAGTATAGCTTAAACTTTTCTT TTAAGTGAAAAGACTTAAATTTTAAAATAAGACGAGAAGACCCCGG CGAGC | 144 |
| HF970 473 | Ensis macha | CTTAATTGGAGAAGGGTATGAATGGTTTGACGTGGTAGTTTCTGT CTCAATGATAGTATTACGAAGTTTTCCTTTGAGTTAAAAGTCTCAAA TAAATGTAAAAGACGAGAAGACCCTGTCGAGC | 145 |
| EU169 034 | Siliqua minima | GGTAGCGTAATAAATTGTCTCTTAATTGGAGAAGGGTATGAATGGT TTGACGTAGGAGTTTCTGTCTCAACAATAGTAATGCGAAGTTTTCC TTTAAGTAAAAAGGCTTAAATATTATTAAAAGACGAGAAGACCCTG TCGAGC | 146 |
| GQ166 561 | Ensis leei | CTTAATTGGAGAAGGGTATGAATGGTTTGACGTGGTAGTTTCTGT CTCAATGGTAATATTATGAAGTTTTCTTTTGAGTTAAAAGTCTCAAA TTTAGATAAAAGACGAGAAGACCCTGTCGAGC | 147 |
| DQ459 296 | Polititape s durus | GGTAGCGCGATAATTTGTCTCTTAATTGGAGAATGGAATGAATGG TTTGTCGCATGTTGGTGTCTCTGGGAAAATGAAGTTTCCTTTTAAT TGAAAAGGTTTAAATTTTAATAAAAGACGAGAAGACCCTGTGAGA | 148 |
| KP052 743 | Cerastod erma glaucum | TTGGCCTTTAATTGGGGTCCGGTATGAATGGGTTGACGATAGAAG CTCTGTCTCGTTAGAATGTGTTGAAATTTACATTTCGGTGAAAAGA CCGAACCGCTTCTAAAAGACGAGAAGACCCCGTCGAG | 149 |
| KF611 752 | Donax longissim us | GTACTAAGGTAGCGCTACAATTTGCCCTTTAAATGGGGGCTGGTA TGAATGGGTTCACGTGAGAGTTGCTGTGTCACAAAAATTTATTGAA ATTAACTTTTAGGTGAAGAGGCCTAGCTGAGTTTAAAGGACGACA AGACCCTAGAAGC | 150 |
| KC429 308 | Solen vaginoide s | CTTAATTGGGGGCTAGTATGAAGGGTTTGACGTAAAAAAAGTTGT CTCTGAGATAATATTGTGAAGTTTTTTTCTGGTGAAAAATCTAGG ATTTAATTAAAAGACGAGAAGACCCTGTCGAGC | 151 |
| KC429 301 | Venus verrucosa | GTGCTAAGGTAGCGTGATAAGTTGTCTTTTAATTGGAGAATGGTAT GAATGGTTTGACGTAGAATAACTGTCTTTGGAAAATTAAACTAAGT TTCCTTTTAAGTGAAAAGGCTTATATTTTTGTAAAAGACGAGAAGA CCCTGTCGAGC | 152 |
| JN969 934 | Ezocallist a brevisiph onata | ATTTAAAGGCTGCAGTTATCTGTACAAAGGTAGCGCAATAAGTAG CCCCTTAATTGGGGGATGGTATGAATGGTTTGACGTGGAGATGCT GTTTCTAGTGTAGTTTAAATGAAATTTCTTTCTAGGTGAAAAAGCCT AGGTTTTTG | 153 |
| KR422 892 | Procardiu m indicum | AGTAGCGTAATAATTTGTCCCTTAATTAGGGTCTGTATGAACGGGT TGACGTGGAAAAACTGTCTTGAGGAAATAGTTTGAAATTTTCTTTT TAGTGAAAAGCCTGAAATAGTATTAAAAGACGAGAAGACCCCGTC GAGC | 154 |
| KR422 893 | Cardium maxicost atum | AGTAGCGTAATAATTTGTCCCTTAATTAGGGTCTGTATGAAAGGGT TGACGTGGAAAAACTGTCTTGAAAAAATAATCTAAAATTTTCTTTTT AGTGAAAAGCCTGAAATGTGCTTAAAAGACGAGAAGACCCCGTCG AGC | 155 |
| KR422 891 | Cardium costatum | AGTAGCGTAATAATTTGTCCCTTAATTAGGGTCTGTATGAAAGGGT TGACGTGGAAGAACTGTCTTGAAAAATAATCTAAAATTTTCTTTTT AGTGAAAAGCCTAAGATATATTTAAAAGACGAGAAGACCCCGTCG AGC | 156 |
| KR422 879 | Acanthoc ardia paucicost ata | TTGGCCTTTAATTGGGGTCCAGTATGAATGGGTTGACGATAGAAT GGCTGTCTTATAGGGGGGCCTTAGAAATTTTCTTTTTGGTGAAAAG ACCTAAATAAGTTTAATAGACGAGAAGACCCCGTCGAG | 157 |
| KR422 878 | Acanthoc ardia echinata | TTGGCCTTTAATTGGGGTCCAGTATGAATGGGTTGACGATAGAAT AGCTGTCTTACAGGGGTTCATTAGAAATTTTCTTTTTGGTGAAAAG ACCTGATTAAATTTAATAGACGAGAAGACCCCGTCGAG | 158 |
| KR422 877 | Acanthoc ardia aculeata | TTGGCCTTTAATTGGGGTCCAGTATGAATGGGTTGACGATAGAAT AGCTGTCTTATAGGGGCCCATTAGAAATTTTCTTTTTGGTGAAAAG ACCTGATTAAACTTAATAGACGAGAAGACCCCGTCGAG | 159 |
| KP055 816 | Ruditape s philippina rum | GGTAGCGCGATAATTTGTCTCTTAATTAGAGAATGGTATGAAGGG TTAGACGTGGGATTATTGTCTCTAAAAAATACAGTTAAGTTTCCTTT TAAATGAAAAGGTTTAAATTTATTAAAAGACGAGAAGACCCTGTCG AGC | 160 |
| KR422 901 | Corculum cardissa | AGTAGCATAATAATTTGCCCTTTAATTGGGGGATAGTATGAATGGG TTGACGATGGAAAGACTGTCTCCAGAAGAGTAATTGAAATTTTCTT CCCAGTGAAAAGCCTGGGATTAGGTTGAAAGACGAGAAGACCCC GTCGAGC | 161 |
| KX713 257 | Spisula solida | GGTAGCGCAATCAATCGTCTCTTAATTGGAGAAAGGTATGAATGG TTTGACGAGGGAAAAACTGTCTCAAATATAGATTTTGAAGTTTTCT TTTAAGTGAAAAGACTTAAATTTTTAAATAAGAAGAGAAGACCCCG GTGAGC | 162 |
| NC_04 6518 | Scrobicul aria plana | GGTAGCGCGATAGGTCGCTCTTTAATTGGGAGAGAGTATGAATGG TCGGATGTTGGAAAGCCTTTATTAAAAGTAATTATTGAAATTTACCT TTTAGTGCAAAGGCTAAAATGATTTTATTAGACGAGAAGACCCTGT TGAGC | 163 |
| KX713 231 | Mactra violacea | GGTAGCGCAGTCAATTGTCCTTTAATTGGGGAAAGGAATGAAAGG GTTGACGAGGGGGAGGCTGTCTTTGTAATATAATTTGAAATTTTCT TTTACGTGAAAAGACTTAAATTTTAAAATAAGAAGAGAAGACCCCG GCGAGC | 164 |
| KX713 203 | Chamele a striatula | GTGCTAAGGTAGCGCGGTAAGTTGTCTTTTAATTGGAGAATGGTA TGAATGGTTTAACGTAGAATAACTGTCTTTGAGGAATAGAGTTAAA TTTCCATTTAAGTGAAAAGGCTTATATTTTTGTAAAAGACGAGAAG ACCCTATCGAAC | 165 |
| KX713 217 | Ensis siliqua | CTTAATTGGAGAAGGGTATGAATGGTTTGACGTGGTAGTTTCTGT CTCAATAATAGTATTGCGAAATTTTCATTTAAGTTAAAAGTCTTAAA TACAGATAAAAGACGAGAAGACCCTGTCGAGC | 166 |
| KR422 986 | Serripes groenland icus | AGTAGCGTAATAATTTGGCCCTTAATTAGGGTCTGGAATGAATGG GTTGACGTGGGAGGGCTGTCTCAAAGGGATTACTTAAAATTTTCTT CCTAGTGAAAAGCCTGGGATACTAGTAAAAGACGAGAAGACCCCG TCGAGC | 167 |
| MN068 431 | Tridacna elongatis sima | AGTAGCGTGATAAGTCGGCCTTTAATTGGGGTCTGGTATGAAGGG GTTGACGTCGGAAAAGCTGTCTCGGAAAAACTAAGTGAAATTTAC TTTTAAGTGAAAAGGCTTAAATAAGGATAAAAGACGAGAAGGCCC TGTCGAGC | 168 |
| MN068 543 | Tridacna rosewater i | AGTAGCGTGATAAGTCGGCCTTTAATTGGGGTCTGGTATGAAGGG GTTGACGTCGGAAAAGCTGTCTCAGAAAAACTAAGTGAAATTTACT TTTAAGTGAAAAGGCTTAAATGGGGGTAAAAGACGAGAAGGCCCT GTCGAGC | 169 |
| NC_01 6174 | Meretrix lamarckii | TACCGCAGGGATAACAGCGTAATTTTTTTCCGTAAGTTGTATTGGG AAAAAAGTTTACAACCTCGATGTTGAATTAAGAAAACTTTGTGGCG CAGACGCCACAGTGAGTAAAACTGTTCGTTTTTTTAATCTCTTACAT GA | 170 |
| NC_01 4809 | Meretrix lusoria | TACCGCAGGGATAACAGCGTAATTTTTTTTTGTAATTGTATCGATA AAAGAGTTTACGACCTCGATGTTGAATTAAGAAACCTTTATGGCGC AGGAGCTATAGGAGGAAAACTGTTCGTTTTTTAATTTCTTACATGA | 171 |
| NC_01 4579 | Paphia euglypta | GGTAGCGCGATAATTTGTCTCTTAATTGGAGAATGGTATGAAAGG TTTGACGTAGACCTATTGTCTCTAAAGAAAAAAATGAAGTTTCCTTT TAAATGAAAAGGTTTAAATTTTTGTAAAAGACGAGAAGACCCTGCC GAGC | 172 |
| NC_01 3188 | Meretrix meretrix | TACCGCAGGGATAACAGCGTAATTTTTTTCTGTAATTGTATCGAGA AAAGAGTTTACGACCTCGATGTTGAATTAAGAAACCTTTATGGCGC AGGAGCTATAGGAGGAAAACTGTTCGTTTTTTAATTTCTTACATGA | 173 |
| NC_01 2767 | Meretrix petechiali s | TACCGCAGGGATAACAGCGTAATTTTTTTCTGTAATTGTATCGAGA AAAGAGTTTACGACCTCGATGTTGAATTAAGAAACCTTTATGGCGC AGGAGCTATAGGAGGAAAACTGTTCGTTTTTTAATTTCTTACATGA | 174 |
| NC_00 8452 | Acanthoc ardia tuberculat a | TTGGCCTTTAATTGGGGTCCAGTATGAATGGGTTGACGATGGAAT AGCTGTCTTATAGGGGCTCATTAGAAATTTTCTTTTTGGTGAAAAG ACCTAATTAAACTTAATAGACGAGAAGACCCCGTCGAG | 175 |
| LT630 276 | Tridacna maxima | AGTAGCGTGATAAGTCGGCCTTTAATTGGGGTCTGGTATGAAAGG GTTGACGTCGGAAAAGCTGTCTCAGAAAGATTAAATGAAATTTACT TTTAAGTGAAAAGGCTTAGATGGGAGTAAAAGACGAGAAGGCCCT GTCGAGC | 176 |
| NC_02 3384 | Lutraria rhynchae na | TGTAGCGTAATAATTAGCCTTTTAAAAGGAGGGAGGAATGAATGG TTTGACGAGAGACCTGCTGTCTCGGAAGTAAATTCTAAAGTTTTCT TTTAAGTGAAAAGACTTAGATTTTTAAATAAGAAGAGAAGACCCCG GTGAGC | 177 |
| NC_02 2924 | Meretrix lyrata | TACCGCAGGGATAACAGCGTAATTTTTTTTTGTAAATTTTATTGGAA AAAAAGTTTACGACCTCGATGTTGAATTAAGAAAACTTTATGGCTT AGGCGCTATAATGAGTAAAACTGTTCGTTTTTTAATTTCTTACATGA | 178 |
| NC_02 2709 | Arctica islandica | GGTAGCGTAATCAGTTGTCCTTTAATTGGGGAAAGGAATGAATGG TTTGACGAAAGGGTAGCTGTTTCAGAGGTAGATAAGGAAGTTTTC TTTCAGGTGAAAAGACCTGAGTTTTTGTAAAAGACGAGAAGACCC CGTCGAAC | 179 |
| NC_01 7616 | Solen strictus | CTTAATTGGGGGCTAGAATGAAGGGTTTGACGTAAAAAAATTTGTC TCCAAAATAGTAGTATGAAGTTTTCTTTTCAGTGAAAAGCCTGAAA TTGTGATAAAAGACGAGAAGACCCTGTCGAGC | 180 |
| NC_01 6891 | Paratape s undulatus | GGTAGCGCGATAATTTGTCTCTTAATTGGAGAATGGTATGAAAGG TTTGTCGCAGACTGGTTGTCTCTGAAGAAATAGATGAAGTTTCCTT TTAAATGAAAAGGTTTAAATTTTTGTAAAAGACGAGAAGACCCTGC CGAG | 181 |
| NC_01 6890 | Paratape s textilis | GGTAGCGCGATAATTTGTCTCTTAATTGGAGAATGGTATGAAAGG TTTGTCGCAGATTAATTGTCTCTAAAAAAAACAATGAAGTTTCCTTT CAAATGAAAAGGTTTAAATTTTTGTAAAAGACGAGAAGACCCTGTC GAG | 182 |
| NC_01 6889 | Paphia amabilis | GGTAGCGCGATAATTTGTCTCTTAATTGGAGAATGGTATGAAAGG TTTGACGTAGATCGATTGTCTCTATAAAAAAAAAATGAAGTTTCCTT TTAAATGAAAAGGTTTAAATTTTTGTAAAAGACGAGAAGACCCTGC CGAGC | 183 |
| NC_01 6665 | Solen grandis | CTTAATTGGGGGCTAGAATGAAGGGTTTGACGTAAAAAAGTTTGT CTCCAAAATAAAAGTATGAAGTTTTCTTTTCAGTGAAAAGTCTGATA TAATGGTAAAAGACGAGAAGACCCTGTCGAGC | 184 |
| NC_03 6766 | Lutraria maxima | TGTAGCGTAATCATTAGCCTTTTAAAAGGAGGGAGGAATGAATGG TTTGACGAGGGACCTGCTGTCTCAGGAGTAATTTCTAAAGTTTTCT TTTAAGTGAAAAGACTTAAATTTTTAAATAAGAAGAGAAGACCCCG GTGAGC | 185 |
| NC_03 5987 | Donax vittatus | GTATTGAGGTAGCATAATAATTAGTCTTTTAATTGGGGGATGGAAT GAATGGTTGAATGATGGGGTGTCTTTATTAGTAGTATAAATTCGAA GTTTTCTTTTAAGTGAAAAGACTTAAATTTTTTTGTTAGACGATAAG ACCCTATTGAGC | 186 |
| NC_03 5986 | Donax variegatu s | GCGTTACGGTAGCATAATAAGTTGTCTTTTAATTGGGGGATAGTAT GAATGGTTTGATGATGGGGTAGCTTTATTAATAATATACATGTGAA GTTTACTTTTAAGTGAAAAGGCTTAAGTTGTTTTGTTAGACGATGA GACCCTATCGAGC | 187 |
| NC_03 5985 | Donax trunculus | GTGTTAAGGTAGCATAATAAATAGTCTCTTAATTGGGGGATAGAAT GAATGGTTGAATGATGGGATAGCTTTATTAGTAATATAAATATGAA GTTTACTTTTAAGTGAAAAGGCTTAAATTTTATTGTTAGACGATAAG ACCCTATTGAGC | 188 |
| NC_03 5984 | Donax semistriat us | GTGTTAAGGTAGCATAATAATTAGTCTTTTAATTGGGGGATGGAAT GAATGGTTGAATGATGGGGTGTCTTTATTAGTAGTATAAATTTGAA GTTTTCTTTTAAGTGAAAAGGCTTAAATTTTTTTGTTAGACGATAAG ACCCTATTGAGC | 189 |
| NC_03 5757 | Ruditape s decussat us | GGTAGCGCGATAATTTGTCTTTTAATTGAAGAATGGTAGAAAGGTT GTCGCAAGTTGCTTGTCTCTGAAAAAGAAAGCGAAGTTTCCTTTTA AATGAAAAGGTTTAAGTAAAGATAAAAGACGAGAAGACCCTATTGA GC | 190 |
| NC_03 5728 | Cerastod erma edule | TTGGCCTTTAATTGGGGTCCGGTATGAATGGGTTGACGATAGAAA AGCTGTCTCATTGGAATATATTGAACTTTTCTTCTCGGTGAAAAGA CCGCGCTTCTGTTAAAAGACGAGAAGACCCCGTCGAG | 191 |
| NC_02 6558 | Tridacna squamos a | AGTAGCGTGATAAGTCGGCCTTTAATTGGGGTCTGGTATGAAAGG GTTGACGTCGGAAAAACTGTCTCGGAAGAACTAAGTGAAATTTAC TTTTAAGTGAAAAGGCTTAAATTAAAGTAAAAGACGAGAAGACCCT GTCGAGC | 192 |
| NC_03 1332 | Ruditape s philippina rum | GGTAGCGCGATAATTTGTCTCTTAATTAGAGAATGGTATGAAGGG TTAGACGTGGGATTATTGTCTCTAAAAAAKAKAGTTAAGTTTCCTTT TAAATGAAAAGGTTTAAATTTATTAAAAGACGAGAAGACCCTGTCG AGC | 193 |
| NC_02 5510 | Mactra chinensis | GGTAGCGCAATAAATTGTCCTTTAATTGGGGAAATGAATGAAAGG GTTGACGAGGGGGAAGCTATCTTTTTGGTATAGTTTGAAGTTTTCT TTTATGTGAAAAGACTTAGATTTTATAATTAGAAGAGAAGACCCCG GCGAGC | 194 |
| NC_03 9945 | Tridacna derasa | AGTAGCGTGATAAGTCGGCCTTTAATTGGGGTCTGGTATGAAGGG GTTGACGTCGGAGAAACTGTCTCGGAAAAATATGGTGAAATTTAC TTTTAAGTGAAAAGGCTTAAATAAAAGTAAAAGACGAGAAGGCCCT GTCGAGC | 195 |
| NC_04 8487 | Mercenari a mercenari a | GGTAGCGTGATAAGTTGTCTTTTAATTGGAGAATGGAATGAAGGG TTTGACGTGAAATTTCTTTTTTTATAAAAGGTACTAAAGTTTCCTTC TAAGTGAAAAGGCTTAGGTTTTTGAAAAAGACGAGAAGACCCTGT CGAAC | 196 |
| NC_05 0683 | Tridacna gigas | AGTAGCGTGATAAGTTGGCCTTTAATTGGGGTCTGGTATGAAGGG GTTGACGTCGGAGAAACTGTCTCGGAAAGATAAAATGAAATTTAC GTCCTAGTGAAAAGGCTGGGATGGCTGTAAAAGACGAGAAGACC CTGTCGAGC | 197 |
| NC_02 1375 | Mactra antiquata | GGTAGCGCAGTCAATTGTCCTTTAATTGGGGAAAGGAATGAAAGG GTTGACGAGGGGGAGGCTGTCTTTGTAATATAATTTGAAATTTTCT TTTACGTGAAAAGACTTAAATTTTAAAATAAGAAGAGAAGACCCCG GCGAGC | 198 |
| NC_04 1247 | Helix pomatia | TGACTGTGCAAAGGTAGCATAATCAGTTGGCTTTTAATTAGAGTCT GGTATGAACGAAAATATGGGTAGTAACTGTCTTAGCACATTTTTTC GAACTTATTAACCAAGTGCAAATGCTTGTAATTTAACAATAGACGA GAAGACCCTAGAAACTT | 199 |
| NC_02 4601 | Achatina fulica | TGACTGTGCAAAGGTAGCATAATAATTTGTCCTCTAATTAAGGTCT GGAATGAAGGGGGACACAGGGGAGAGCTGTCTCCAATAAGGTTA ATTTAACTTTCTTATCAGGTGAAAATTCCTGAGCCTCCGATGAAAG ACGAGAAGACCCTTAGAGTTT | 200 |
| NC_02 1747 | Helix aspersa | TGACTGTGCAAAGGTAGCATAATCAGTTGGCCTATAATTGAGGTC TGGTATGAACGAATAATTGGGTAGTAACTGTCTTAATGTATTCTTA CAAATTTAGTAAGCAAGTGAAAATACTTGCTAAATTATAAAAGACG AGAAGACCCTAGAAACTT | 201 |
| KT211 748 | Helix thessalica | TGACTGTGCAAAGGTAGCATAATCAGTTGGCTTTTAATTAGAGTCT GGTATGAACGAAAATATGGGTAGTAACTGTCTTAGCACATTTCTTT GAACTTGTTAAACAAGTGCAAATGCTTGTAATTAAAAAATAGACGA GAAGACCCTAGAAACTT | 202 |
| KR705 016 | Helix pomatia | TGACTGTGCAAAGGTAGCATAATCAGTTGGCTTTTAATTAGAGTCT GGTATGAACGAAAATATGGGTAGTAACTGTCTTAGCATATTTTTTC GAACTTATTAAACAAGTGCAAATGCTTGTAATTTAACAATAGACGA GAAGACCCTAGAAACTT | 203 |
| KR705 014 | Helix lucorum | TGACTGTGCAAAGGTAGCATAATCAGTTGGCTTTTAATTGGAGTCT GGTATGAACGAACACATGGGTGGCGACTGTCTAAATATATTTTTCC TAACTTTTTAATCAAGTGAAAATACTTGTGCTAATAAAAAAGACGA GAAGACCCTAGAAACTT | 204 |
| KR705 012 | Helix nicaeensi s | TGACTGTGCAAAGGTAGCATAATCAGTTGGCTTTTAATTGGAGTCT GGCATGAACGAAAGCATGGGTGGCGACTGTCTTAATATATTTTTTC TAATTTTTTAATCAAGTGAAAATACTTGTGTAAAATAAAAAAGACGA GAAGACCCTAGAAACTT | 205 |
| JQ619 236 | Achatina reticulata | TGACTGTGCAAAGGTAGCATAATAATTTGTCCTTTAATTTAGGTCT GGAATGAATGAGAACACAGGGGAGAGCTGTCTCTAGWARGGTTA ACTTAACTTCCTTATCAGGTGAAAATTCCTGAGCTTCTTTGGAAAG ACGAGAAGACCCTTAGAGTTT | 206 |
| EU912 833 | Helix aspersa maxima | GACTGTGCAAAGGTAGCATAATCAGTTGGCCTATAATTGAGGTCT GGAATGAACGAAAAATAGGGTAGCAACTGTCTTAGTATATTTTTTT AAACTTAGTTAGCAAGTGAAAATACTTGCAAATATACAAAAGACGA GAAGACCCTAGAAACTT | 207 |
| GQ402 391 | Helix aperta | GTCTGGTATGAACGAACATTTGGGTGACAACTGTCTAAATTAATTT TTCTAAACTTAGTTAGCAAGTGCAAATACTTGTATGTATATAAAAGA CGAGAAGACCCTAGAAACTT | 208 |
| KR705 013 | Helix albescen s | TGACTGTGCAAAGGTAGCATAATCAGTTGACTCTTAATTAGGGTCT GGTATGAAAGAAAATATGGGTAGTAACTGTCTTAATATATTTATTTG AAATTAATAAGCAAGTGCAAATACTTGCATACATAAAAAAGACGAG AAGACCCTAGAAACTT | 209 |
| MH130 070 | Tyrrhenar ia ceratina | TGACTGTGCAAAGGTAGCATAATCAGTTGGCTTTTAATTAGAGTCT GGTATGAATGAAAATATGGGTGGTAACTGTCTCTGACATATTAATT TGAACTTTTTTTAGCAAGTGCAAATACTTGCGAGGGTAAAAAGAC GAGAAGACCCTAGAAACTT | 210 |
| MF564 117 | Helix vladika | TGACTGTGCAAAGGTAGCATAATCAGTTGGCTTTTAATTGGAGTCT GGCATGAATGAAAATATGGGTAGTAACTGTCTTAGCATATTATTTG AACTTATTAAACAAGTGCAAATGCTTGTAATTACACAATAGACGAG AAGACCCTAGAAACTT | 211 |
| KF246 957 | Pleurodo nte discolor | TGACTGTGCAAAGGTAGCATAATCAGTTGGCTTATAATTGAAGTCT TGTATGAAGGAATAAATGGGTTACAACTGTCTCTACAGATAAATAG CGAATTTACTTAATAAGTGAAAATACTTATAGTTTTTTAAAAGACGA GAAGACCCTAGAAATTT | 212 |
| KF246 963 | Pleurodo nte lychnuch us | TGACTGTGCAAAGGTAGCATAATCAGTTGGCTTATAATTGAAGTCT AGTATGAATGAAGGAATGGGTGAGTCCTGTCTCCAGAAAAGAGGT GAACTTACCTAACAAGTGAAAATGCTTGTACAAAGACAAAAGACGA GAAGACCCTAGAAATTT | 213 |
| GQ402 402 | Erctella mazzullii | CATATTGAGGTCTGGTATGATGAAATTTTGGGTAGAAACTGTCTTA ATATATTTTTTATAATTTAATATGCAAGTGAAAATACTTGCTTTAATA TAAAAGACGAGAAGACCCTAGAAACTT | 214 |
| GQ402 397 | Erctella cephalae ditana | GAAGTCTGGTATGAATGAACTTTGGGTAGTAACTGTCTTAATATAT TTTAAAAAATTTAATATGTAAGTGAAAATACTTACTTTAATATAAAAG ACGAGAAGACCCTAGAAACTT | 215 |
| KF246 958 | Pleurodo nte formosa | TGACTGTGCAAAGGTAGCATAATCAGTTGGCTTATAATTGAAGTCT GGTATGAAGGAATAAATGGGTAGTTTCTGTCTTTAGGGGAACGAC GAACTTACTCAGCAAGTGAAAATACTTGTATATTAACAAAAGACGA GAAGACCCTAGAAATTT | 216 |
| KR705 018 | Helix christophi | TGACTGTGCAAAGGTAGCATAATCAGTTGACTCTTAATTGGGGCC TGGGATGAATGAAATAATGGGCGGCAGCTTTCTCAGATATATTTCT ATAAACTTGGTTAGTAGGTGCAAATACCTCTATGTAGATAAAAGAC GAGAAGACCCTAGAAGCTT | 217 |
| KR705 019 | Helix nordman ni | TGACTGTGCAAAGGTAGCATAATCAGTTGGCTTTTAATTGGAGTCT GGGATGAATGAAATAATGGGCGGCAACTTTCTCAAATATATTATTA TGAATTTGGTTAGTAGGTGCAAATACCTACATAAAAACAAAAGACG AGAAGACCCTAGAAGCTT | 218 |
| KF246 965 | Pleurodo nte nucleola | TGACTGTGCAAAGGTAGCATAATCAGTTGTCTTTTAATTGAAGTCT GGCATGAATGAATAAATGGGTAGGAACTGTCTCAAGAAAGAAACA AAAATTTACTTAGTAAGTGAAAATACTTATGTTTTGACAAAAGACGA GAAGACCCTAGAAATTT | 219 |
| KF246 967 | Pleurodo nte parilis | TGACTGTGCAAAGGTAGCATAATCAGTTGGCTTTTAATTGAAGTCT AGTATGAAGGAACAAATGGGTGAAAGCTGTCTTTAGTAGAAAACA AGGAACTTACTTAGTAAGTGAAAATACTTACAGTTAGATAATAGAC GAGAAGACCCTAGAAATTT | 220 |
| KF246 969 | Gonosto mopsis auridens | TGACTGTGCAAAGGTAGCATAATCAGTTGGCTTATAATTGAAGTCT AGTATGAAAGAATAAAATGGGCGGCGCTGTCTTCTTAAGGTATC TAGAACTTACTTAGTAAGTGAAAATACTTACATATAAATAAAAGACG AGAAGACCCTTGAAGTTT | 221 |
| KF246 970 | Caracolu s caracollu s | TGACTGTGCAAAGGTAGCATAATAATTTGGCTTTTAATTGGAGTCT GGAATGAATGGGTACATGGATATAGGCTGTCTTATACTAATTACTT ATAACTTTTTTAATTAAGTGAAAATACTTAAAGAAATATAATAGACG AGAAGACCCTAGAAATTT | 222 |
| KF246 999 | Lacteolun a selenina | TGACTGTGCAAAGGTAGCATAATAAATTGGCTTATAATTGAAGTCG GGAATGAATGGATTAATGAATATAAACTGTCTTATCTAAATTTTAAT TAATTTTCTTAGTAAGTGAAAATTCTTACAATGTGTTTTATTAGACG AGAAGACCCTTGAAATTT | 223 |
| KF247 012 | Cernuella cisalpine | TGACTGTGCTAAGGTAGCATAATCATTTGGCTTATAATTGAAGTCT GGCATGAAAGAATTTATGGGTAACAACTGTCTCCTGTAATTATAGA TGAACTTACTCAATAAGTGAAAATGCTTATATAATCACAATAGACG AGAAGACCCTAGAAATTT | 224 |
| KF247 015 | Cochlicell a acuta | TGACTGTGCAAAGGTAGCATAATCAATTGGCTTGTAATTTAAGTCT GGTATGAATGTAGTTTTAGGCTGAAACTGTCTCTTTATATACATTG AAATTTACTTAGCCGGTGAAAATGCCGGCAGTTAAATAATAGACGA GAAGACCCTAGAAATTT | 225 |
| KF247 017 | Disculella maderens is | TGACTGTGCAAAGGTAGCATAATCATTTGGCTTATAATTGAAGTCT AGTATGAATGAATTAATGGGTAAAAGCTGTCTCTTTATAATTAAGTA GAAATTACTTAGAGGGTGAAAATTCTCTCAATATCTTAATAGACGA GAAGACCCTAGAAATTT | 226 |
| KF247 020 | Dialeuca nemoraloi des | TGACTGTGCAAAGGTAGCATAATAAATTGGCTTATAATTGAAGTCT TGAATGAAAGGTAACACGAGTAAAATCTGTCTTTTAGAAATATTTTT AATTTACTTATTAAGTGAAAATTCTTAATTTAAAAAATTAGACGAGA AGACCCTAGAAATTT | 227 |
| KF247 021 | Monadeni a fidelis | TGACTGTGCAAAGGTAGCATAATCAGTTGGCCTTTAATTGGGGTC TGGAATGAACGAAGATTTGGGTAATAGCTGTCTTTATATGGTAAGC AAAACTTGCTTAGTAAGTGAAAATTCTTACATGATAAGAAAGACGA GAAGACCCTAGAAATTT | 228 |
| NC_00 1816 | Cepaea nemoralis | TGACTGTGCAAAGGTAGCATAATAATTTGGCTTATAATTGAAGTCT GGCTCGAAAGAATTAATGGGAAGCAGCTGTCTCCAGGGGTACTA GTACAAAATTAGTAAGTAAGTGAAAATACTTGCGGGAAGAAAATAG ACGAGAAGACCCTAGAAGCTT | 229 |
| KF247 040 | Sphincter ochila candidissi ma | TGACTGTGCTAAGGTAGCATAATCAATTGACTCATAATTGGAGTCT AGCATGAATGAAAGAATGGGTAAACACTGTCTCTGTCTTACCGATT GAAATTACTAAGCAGGTGAAAATACCTGTATTCAAATAATAGACGA GAAGACCCTAGAAGACC | 230 |
| KF247 041 | Microphy sula ingersolli | TAACTGTGCAAAGGTAGCATAATAATTTGGCTTATAATTGAAGTCT AGAATGAAAGGAAGCATGGGCGAATTCTGTTTCTTAATTTTTTTTT GAAATTAATAATAAGGTGAAAATTCCTTTTAAATTCTATTAGACGAG AAGACCCTACAGAGCT | 231 |
| KF247 043 | Helicodon ta obvoluta | TGACTGTGCAAAGGTAGCATAATAATTTGGCTTATAATTGAAGTCT GGTATGAATGAATATATGGGTACCAACTGTCTCCACTATATAGCAT GAATTTACTGATTGGGTGCAAATTCCCATCGCAAGATAATAGACGA GAAGACCCTAGAAATTT | 232 |
| NC_03 0723 | Cernuella virgata | TGACTGTGCTAAGGTAGCATAATCATTTGGCTTATAATTGAAGTCT GGCATGAAGGAATTTATGGGTAACAACTGTCTCCTATAACTGTAAT GAACTTGCTAAATAAGTGAAAATACTTATGTAACCATAATAGACGA GAAGACCCTAGAAATTT | 233 |
| AF110 075 | Loligo forbesii | GGGACGAGAAGACCCTATTGAGCTTATAATTTTATTATTATTATTAT TTGTAATTGGTGTAATAATATATAAATTTTAATTGGGGTGATTAAGG AATAAAAATTAATTAATATGATTAAATAACTTCCTTAGAAATTATATT GTTAAATAAAGTAACCTATARTTATTATAAAAATTTATAATCAGTATT GCTTATAATATAGTTACCATAGGGATAACAGCGTAAT | 234 |
| AB270 953 | Nototodar us sloanii | GGGACGAGAAGACCCTAATGAGCTTATAATTTTATTGTTATGGTTT AATATATTTAAATGATAATATATAAATTTTAATTGGGGTGATTAAGG AATAAATTATGATTAATAACTTCCTTAGGTTAAATATATATTGATTAA GTAACCAATAAATATATTGCTTATAATATAGTTACCATAGGGATAAC AGCGTAAT | 235 |
| AB193 802 | Sepia lorigera | GGGACGAGAAGACCCTATTGAGCTTTAAATTTGAAGTATTGTATTT ATTAATATAATATTTAAAAATTTTAATTGGGGTGATTAAGGAATAAA AATTATAATATATTAAAAATTTAACTTCCTTAAATAATATATTGTTAA ATAAAGTAACCAATAATATTGCTTATATAAAGTTACCATAGGGATAA CAGCGTAAT | 236 |
| AB193 801 | Sepia pardex | GGGACGAGAAGACCCTATTGAGCTTTAAATTTGGAATATTATATTT GTTAGTATAGTATTTAAAAAATTTTAATTGGGGTGATTAAGGAATAA AATTATAAGTATATTAAAATTTAACTTCCTTAAATAATATATTATTAA ATAAAGTAACCAATAATATTGCTTATATAAAGTTACCATAGGGATAA CAGCGTAAT | 237 |
| AB192 323 | Sepia kobiensis | GGGACGAGAAGACCCTATTGAGCTTTAAATTTGGGAATATTATATT TATTAAGTATAATGTTTGAAAAATTTTAATTGGGGTGATTAAGGAAT AAAATAAAATATATTAAAAATTTAACTTCCTTAGATAATATATTGTTA AATAAAGTAACCAATAATATTGCTTATATAAAGTTACCATAGGGATA ACAGCGTAAT | 238 |
| AB191 138 | Rossia pacifica | GGACGAGAAAGACCCTATTGAGCTTGTAATTTTATTTTATTGTTTAT TAAGGATAGTAAAGTATAAATTTTAATTGGGGTGATTAAGGAATAA GATAATATAAGATAACTTCCTTAAAGGTTAGTATAGGTGTATGAAG TAACCAATAAATTATATTGCTTATATTATAGTTACCATAGGGATAAC AGCGTAAT | 239 |
| AB191 136 | Berryteut his magister | GGGACGAGAAGACCCTATTGAGCTTATAATTTTATTGCTACTAAAT TATTATTAGTGTAGTAGTATATAAATTTTAATTGGGGTGATTAAGGA ATAATTTAGTTTAGTAACTTCCTTAAATTTACATATTGGTGGATAAA AGTAACCCATAAAGTATTGCTTATAATATAGTACCATAGGGATAAC AGCCGAAT | 240 |
| AJ252 763 | Eledone massyae | GGAACGAGAAGACCCTATTGAGCTTAATATTAAAATATAAATTTTG TTAGTAATAAATTAATAAAGTTTATTAATTTTAAATTTTGATTGGGGT GATCGAGGAATATAAATAATTAAATTTAACTTCCTAATATTATATAT TTATAAATAAAGAAACCAAATATTTTGCTTAGAAGGAAGTTACCATA GGGATTACAGCGTGAT | 241 |
| AF369 957 | Sepia robsoni | GGGACGAGAAGACCCTATTGAGCTTAAATTTAATTACATAATTAAA TTATGTTTTGATAAATTTTAATTGGGGTGATTAAGGAATAAAAATAT TATATAGTAACTTCCTTAATAGTTAATACTGTTGGGTTAATTAACCA ATAATATTGCTTATAATAAAGTTACCATAGGGATAACAGCGTAAT | 242 |
| AF110 081 | Loligo reynaudii | GGGACGAGAAGACCCTACTGAGCTTGTAATTTTATTATTACTGCTA TTTGTAATTGGGGCAGTAATATATAAATTTTAATTGGGGTGATTAA GGAATAAAATAAATATATCTTAGTAACTTCCTTATAAAGTATATTGT TAGATGAAGTAACCAATTAAAGTTACATGATATTGTAATAAATATTG CTTATAATATATAGTTACCATAGGGATCACAGCGTAAT | 243 |
| AF110 079 | Doryteuth is (Amerigo) pealeii | GGGACGAGAAGACCCTATTGAGCTTATAATTTATTATTGTCAGTA TTGTTAATTTTTATTGGCACTATATAAATTTTAATTGGGGTGATTAA GGAATAAGGTTTATATATTTCAGTAACTTCCTTAGTAGTAGATAAAT TGTTAGATAAAGTAACCAATATTATTAGATTTATTGCTTATACTATA GTTACCATAGGGATAACAGCGTAAT | 244 |
| AF110 076 | Doryteuth is (Amerigo) gahi | GGGACGAGAAGACCCTATTGAGCTTATAATTTATTATTACCAATA TTATTAATTGTTAGTGGTAATATATAAATTTTAATTGGGGTGATTAA GGACATAAAAAGTTTATTATTTAGTAACTTCCTTAGTAAATATAAAT TGTTAAATAAAGTAACCAATATAAATAAATATTATTGCTTATATTATA GTTACCATAGGGATAACAGCGTAAT | 245 |
| AY293 671 | Sepiola rondeletii | GGGACGAGAAGACCCTATTGAGCTTATAATTTTATTATTATTATTAT ATGTAATAATAAATGAATTTTAATTGGGGTGATTAAGGAATAAATTA GTATTAGTTAGTAACTTCCTTAAATAGATAAATAGATGTATAAATTA ACCAATAATTATATTGCTTTTATTAAAGTTACCATAGGGATAACAGC GTAAT | 246 |
| AY293 670 | Sepiola robusta | GGGACGAGAAGACCCTATTGAGCTTGTAATTTTATTAGTATTATTA ATATAATATTTAATAAATTTTAATTGGGGTGATTAAGGAATAAATTA ATATTATTAGTAACTTCCTTAAGTAATAGGATAGGTGTATAAATTAA CCAATAATTGTATTGCTTTTATCAAAGTTACCATAGGGATAACAGC GTAAT | 247 |
| AY293 669 | Sepiola intermedi a | GGGACGAGAAGACCCTATTGAGCTTATAATTTTATTAGTATTATTA ATATAATATTTAATAAATTTTAATTGGGGTGATTAAGGAATAAATTA ATATTATTAGTAACTTCCTTAAGTAATAGGATAGGTGTATAAATTAA CCAATAATTGTATTGCTTTTATCAAAGTTACCATAGGGATAACAGC GTAAT | 248 |
| AY293 668 | Adinaefiol a ligulata | GGGACGAGAAGACCCTATTGAGCTTTTAATTTTGATATATTATATTT ATATAATGTTGAATAAATTTTAATTGGGGTGATTAAGGAATAAATAT AATTAATTAGTATCTTCCTTAAAATAATAGTATAGATATATAAATTAA CCAATAATTATATTGCTTATATTAGAGTTACCATAGGGATAACAGC GTAAT | 249 |
| AM088 007 | Sepia smithi | GGGACGAGAAGACCCTATTGAGCTTAAATTTAATTTTATTAATTAT GATATTAGTAAATAATAAATTTTAATTGGGGTGATTAAGGAATAAAT ATAATAATAAGTAATAACTTCCTTAAATTATTAAACTGTTAGGTGAA GTAACCAATAATATTGCTTATATTAAAGTTACCATAGGGATAACAG CGTAAT | 250 |
| AM088 005 | Sepia elliptica | GGGNCGAGAAGACCCTATTGAGCTTAAATTAATNGTATATTAAATT AAGATTAATATATTAATAATTTTAATTGGGGTGATTAAGGAATAAAA AATATTGTAAAATGATAACTTCCTTAAATTTAATACTGTTAGGTAGA GTAACCAATATTTATTGCTTATATTAAAGTTACCATAGGGATAACAG CGTAAT | 251 |
| AJ252 765 | Eledone palari | AGGGACAAAAGACCCTATTGAGCTTTTATTATTTTTTAAATTATTAT TAATAAGAAATTTATGCAAATAATTTGATTGGGGTGATCAAGGAAT AAAGAAATAACTTCCTTAAATATTAAGGTTTAATAGGAAAATAAACC AAATTTTTGCTTTTATGATGAGTTACCATAGGGATAACAGGGTAAT | 252 |
| AJ252 764 | Eledone moschata | GGGACGAGAAGACCCTATTGAGCTTAATGTTAAATTATAAATAATT TAATGATCTTATTATGTTTTACATTATTTATAAAATTTAAATTTTGAT TGGGGTGATCAAGGAATAAAAGTAGTGTAGTGTTTATAACTTCCTT ATAGGATTTATGAGTTATTAATGGTATAATAAACCAAATATTTTGCT TATAAGTAAGTTACCATAGGGATAACAGCGTAAT | 253 |
| EU735 193 | Sepiola affinis | GGGACGAGAAGACCCTATTGAGCTTATAATTTTATTAGTATTATTA ATATAATATTTAATAAATTTTAATTGGGGTGATTAAGGAATAAATTA ATATTATTAGTAACTTCCTTAAGTAATAGGATAGGTGTATAAATTAA CCAATAATTGTATTGCTTTTATCAAAGTTACCATAGGGATAACAGC GTAAT | 254 |
| EU735 192 | Rossia palpebros a | GGGACGAGAAGACCCTACTGAGCTTGTAATTTTATTTTATTATTTTT AGGTAGTAGGGTATAAATTTTAATTGGGGTGATTAAGGAATAAAGT GTATTAGGGTAACTTCCTTAAGTGATAGTATATGTGTATTAAGTAA CCAATAAATTATATTGCTTATATTATAGTTACCATAGGGATAACAGC GTAAT | 255 |
| AY681 045 | Gonatus madokai | GGGACGAGAAGACCCTATTGAGCTTATAATTTTATCATTATTAGGT TTATTATTAATATAGTGATGTATAAATTTTAATTGGGGTGATTAAGG AATAAATTTAATTTAGTAACTTCCTTAAGTTTTATATTGTTGAATAAA GTAACCAATAAATTATTGCTTATAATATAGTTACCATAGGGATAACA GCGTAAT | 256 |
| AY681 038 | Gonatus kamtscha ticus | GGGACGAGAAGACCCTATTGAGCTTATAATTTTATCATTATTAGAT TTATTATTAATATAGTGATGTATAAATTTTAATTGGGGTGATTAAGG AATAAATTTAATTTAGTAACTTCCTTAAGTTCTATATTGTTGAATAAA GTAACCAATAAATTATTGCTTATAATATAGTTACCATAGGGATAACA GCGTAAT | 257 |
| AY616 973 | Eledone cirrhosa | GGGACGAGAAGACCCTATTGAGCTTGATATTAAAATATAAATTTTT TTTAAAGTTAATATAATTATGTTATGGGTTTAAATTTTGATTGGGGT GATCAAGGAATAAAAAAGATTAATGTATATATATATTAACTTCCTTA TATATAAATATTTATAGATAAATAAACCGAGAGTTTTGCTTAAAAGT AAGTTACCATAGGGATAACAGCGTAAT | 258 |
| AY377 630S | Sepia elegans | GGGAAGAGAAGACCCTATTGAGCTTTAATTTTAATAGGTTATAATA AATATTATAGTAAAATAGAAATTTTAATTGGGGTGATTAAGGAATAA AAAAGTAACTTCCTTAAAAATAAAATTGTTAGGTTAAGTAACCAATT GTATTGCTTATATAAAGTTACCATAGGGATAACAGCGTAAT | 259 |
| AY293 676 | Rossia bipillata | GGGACGAGAAGACCCTAGTGAGCTTATAATTTTATTTTGTTGTTAG TAAATAGTAGAGATGAAATTTTAATTGGGGTGATTAAGGAATAAAA TATGTTTATATAACTTCCTTAGATTTGGTATAGGTATATAAAGTAAC CAATAAATATATTGCTTATATTATAGTTACCATAGGGATAACAGCGT AAT | 260 |
| AY293 672 | Sepiola atlantica | GGGACGAGAAGACCCTATTGAGCTTATAATTTTATTATTATTATAT GTAATAATAAATGAATTTTAATTGGGGTGATTAAGGAATAATTAGTA TTAGTTAGTAACTTCCTTAAATAGATAAATAGATGTATAAATTAACC AATAATTATATTGCTTTTATTAAAGTTACCATAGGGATAACAGCGTA AT | 261 |
| KF266 732 | Lolligunc ula (Lolligunc ula) panamen sis | GGGACGAGAAGACCCTATTGAGCTTGTAATTTTATTGTTATTATTA TATTAATTGTTAATGGTAATATATAAATTTTAATTGGGGTGATTAAG GAATAAAATTTTATATTAGTAACTTCCTTAATAAGATGATTGTTAGA TAAAGTAACCAATATTATTAAATATTATTGCTTATATTGTAGTTACC ATAGGGATAACAGCGTAAT | 262 |
| KC792 312 | Octopus maya | GGGACGAAAAGACCCTATTGAGCTTATATTATTTTGTAAATTATAT GATTGTTGTTTATTGGAATTAATTTTGATTGGGGTGATCAAGGAAT AAATTATTATTAAAATTAACTTCCTTAATTAATTATTTTATGGAGAAA TAAACCAAATTTTTTTGCTTAGATGATAAGTTACCATAGGGATAACA GCGTAAT | 263 |
| HQ733 953 | Illex illecebros us | GGGACGAGAAGACCCTACTGAGCTTATAATTTTATTATCACATATA TTTATGTATAATGTGCTATATAAATTTTAATTGGGGTGATTAAGGAA TAATATAGCTATTTTATAACTTCCTTAGATATTATATTGTTGAATTAA GTAACCGATTAATTAATAGAATTGTATTATGTATTGCTTATAATATA GTTACCATAGGGATAACAGCGTAAT | 264 |
| GQ412 305 | Nototodar us gouldi | GGGACGAGAAGACCCTAATGAGCTTATAATTTTATTGTTGTGGTTT AATATATTTAAATGATAATATATAAATTTTAATTGGGGTGATTAAGG AATAAATTATGATTAATAACTTCCTTAGGCTGAATATATATTGATTA AGTAACCAATAAATATATTGCTTATAATATAGTTACCATAGGGATAA CAGCGTAAT | 265 |
| EU735 264 | Gonatops is octopedat us | GGGACGAGAAGACCCTATTGAGCTTATAATTTTATCATTATTAGAT TTATTATTAGTGTAGTGTGTATAAATTTTAATTGGGGTGATTAAGGA ATAATTTAATTTATTAACTTCCTTAAGTTACATATTGTTGAATAAAGT AACCAATAAATTATTGCTTATAATATAGTTACCATAGGGATAACAG CGTAAT | 266 |
| EU735 218 | coindetii | GGGACGAGAAGACCCTAATGAGCTTATAATTTATTATTATTATTTA ATATATTTAAATAATAATATATAAATTTTAATTGGGGTGATTAAGGA ATAAAGAATTATATAATAACTTCCTTAGATTAAATATATATAGATAAA GTAACCAATAATATATTGCTTATAATATAGTTACCATAGGGATAACA GCGTAA | 267 |
| EU735 238 | Berryteut his anonychu s | GGGACGAGAAGACCCTAATGAGCTTATAATTTTATTGTTGTTAAAT TTATTATTAGTAAAGCAGCATATAAATTTTAATTGGGGTGATTAAGG AATAAATTAATTTAGTAACTTCCTTATATTTAAATATTGTTAAATAAA GTAACCAATAAATTATTGATTATAATATAGTTACCATAGGGATAACA GCGTAAT | 268 |
| EU735 210 | Gonatus fabricii | GGGACGAGAAGACCCTATTGAGCTTATAATTTTATCATTATTAGAT TTATTATTAGTGTAATGTTGTATAAATTTTAATTGGGGTGATTAAGG AATAATATTAATTTAGTAACTTCCTTAAGTTATATATTGTTGAATAAA GTAACCAATTAAATTATTGCTTATAATATAGTTACCATAGGGATAAC AGCGTAAT | 269 |
| KR259 947 | Lusepiola birostrata | GGGACGAGAAGACCCTATTGAGCTTGTAATTTTATTTTATTGTTTA TTAGGATAGTAAAGTATAAATTTTAATTGGGGTGATTAAGGAATAA GATAATATAAGATAACTTCCTTAAAGGTTAGTATAGGTGTATGAAG TAACCAATAAATTATATTGCTTATATTATAGTTACCATAGGGATAAC AGCGTAAT | 270 |
| KJ605 235 | Octopus tetricus | GGACGAAAANGACCCTATTGAGCTTTTGTTATTATATAAATATACA TATAAATGTATAATTTATTTATAAATAACTTTGGTTGGGGTGATCAA GGAATAAAAATAATTTAAAATGTTGTGTTAACTTCCTTAATTTTGAG TTTAATTGAAGAATAAACCAAGTTTTTTGCTTAGAAGATAAGTTACC ATAGGGATAACAGCGTAAT | 271 |
| KF854 042 | Uroteuthi s (Photololi go) sibogae | GGGACGAGAAGACCCTATTGAGCTTGTAATTCTGTTATTATTATGT TTATTAAATATCATTAATAGTATATGAATTTTAATTGGGGTGATTAA GGAATAAATAATTTATAATATATAACTTCCTTAGAGTATTATATTGTT GAATGAATTAACCGATATAATTAACACAGGTTGTGTTGTGTATTGC TTATAATATAGTTACCATAGGGATAACAGCGTAAT | 272 |
| KF854 023 | Doryteuth is (Doryteut his) pleii | GGGACGAGAAGACCCTATTGAGCTTATAATTTTATTGTTGTCAGTG AGTATTAATTTCACTGTTGGTATATAAATTTTAATTGGGGTGATTAA GGAATAAAGTTTATATATTAATAACTTCCTTAGAGTTAATATTATTG TTAGATAAAGTAACCAATAAATTGTTAAGATTATTGCTTATACTATA GTTACCATAGGGATAACAGCGTAAT | 273 |
| KF854 021 | Doryteuth is sanpaule nsis | GGGACGAGAAGACCCTATTGAGCTTATAATTTTATTGTCATCAAGA TTGTTATGTAACTGGTGACATATAAATTTTAATTGGGGTGATTGAG GAATAAAGTTTTATTGTGGAAGTAACTTCCTTAGTAATGCATATTGT TAAATGAAGTAACCAATAATTAATTACTATTGCTTATACTATAGTTA CCATAGGGATAACAGCGTAAT | 274 |
| KF854 020 | Doryteuth is (Amerigo) suriname nsis | GGGACGAGAAGACCCTATTGAGCTTATAATTTTATTACCGTTAGTA GTGATTAAAATTTTACTAAGGGTATATAAATTTTAATTGGGGTGATT AAGGAATAAAAGCTTATGTATTAATAACTTCCTTAGAAATAAATAAA AAATTGTTAGATAAAGTAACCAATAAATTGTTAAAATTATTGCTTAT ACTATAGTTACCATAGGGATAACAGCGTAAT | 275 |
| KF373 761 | Octopus hubbsoru m | GGGACGAAAAGACCCTATTGAGCTTGTATTATTTAACAAATTATAT GATTGTTATTTGTTTGAATTAATTTTGATTGGGGTGATCAAGGAATA AATTTTATTTAGTTTAACTTCCTTAATTAATTGTTTTATAGAAGAATA AACCAAAGTTTTTGCTTAGAAGATAAGTTACCATAGGGATAACAGC GTAAT | 276 |
| MG01 0490 | Macrotrito pus defilippi | GGGACGAAAAGACCCTATTGAGCTTGTGTTAATAAATAAATACTAN NNTATATATATATANNNNNNTATATATATATATATTANNTTTGTGAG TTTTAAATTTGGTTGGGGTGATCAAGGAATAAAATTAAAATAAAAN NNNNNNNNNNGTAACTTCCTTAAAAATNNNAATATTAATGGGTTA ATAAACCAAGTTTTTTGCTTATAAGGTAAGTTACCATAGGGATAAC AGCGTAAT | 277 |
| MF040 832 | Octopus insularis | GGGACGAAAAGACCCTATTGAGCTTATATTATTTTATAAATTATATG ATTGTTGTTTATTTAGAATAATTTTGATTGGGGTGATCAAGGAATAA GATTGTTTAAATTAACTTCCTTAATTAATTTGTTTTGTGGAAAAATA AACCAAATTTTTTGCTTAGAAGATAAGTTACCATAGGGATAACAGC GTAAT | 278 |
| LC121 083 | Loliolus (Nipponol oligo) sumatren sis | GGGACGAGAAGACCCTATTGAGCTTATAATTTTATTACTATTATGT TTGTTAAATTCATCATTGTAGTATATAAATTTTAATTGGGGTGATTA AGGAATAAGTTTTTTATAATGTAACTTCCTTAGATTAATATATTGTT AGATTAAGTAACCGATGTGATTAACATAATATATGTTATGTATTGCT TATAATATAGTTACCATAGGGATAACAGCGTAAT | 279 |
| LC121 068 | Sepia stellifera | GGGACGAGAAGACCCTATTGAGCTTAAATTTAAAAATATTAAATAA TTTAATATTTTGATAAATTTTAATTGGGGTGATTAAGGAATAAAATA ATAGGTATTATTAAATATAGTAACTTCCTTAAATTTAATACTGTTAG GTTAAGTAACCAATGTAATATTGCTTATAATAAAGTTACCATAGGG ATAACAGCGTAAT | 280 |
| LC121 066 | Sepia recurviros tra | GGGACGAGAAGACCCTATTGAGCTTGAATTTAATTATATTATTTTA ATAATATTTTGATAAATTTTAATTGGGGTGATTAAGGAATAAAAATT TATATATATTATGTAGTAACTTCCTTAATTATTATACTGTTAGGTTAA GTAACCAATAATATTGCTTATAATAAAGTTACCATAGGGATAACAG CGTAAT | 281 |
| LC121 063 | Sepia madokai | GGGACGAGAAGACCCTATTGAGCTTTAATTTAATTATATAATATAG TTTGTATTTTGTTAAATTTTAATTGGGGTGATTAAGGAATAAGATTA GTATTAATTAGTAACTTCCTTAAATTTTATTGTTACTGTTGGGTGAA GTAACCAATATTATTGCTTATAATAAAGTTACCATAGGGATAACAG CGTAAT | 282 |
| LC121 061 | Sepia kobiensis | GGGACGAGAAGACCCTATTGAGCTTAAATTTAAAAATATTAAATAA TTTAATATTTTGATAAATTTTAATTGGGGTGATTAAGGAATAAAATA ATAGGTATTATTAAATATAGTAACTTCCTTAAATTTAATACTGTTAG GTTAAGTAACCAATGTAATATTGCTTATAATAAAGTTACCATAGGG ATAACAGCGTAAT | 283 |
| NC_02 9702 | Amphioct opus aegina | GGGACGAAAAGACCCTATTGAGCTTTATATTATACTTTAAAATTATT TATTGTTTTTATTTAAATATAATTTTGGTTGGGGTGATCAAGGAATA AATAATTAATTTTAACTTCCTTAATTTGTTTATAGTTATATGAATAAA CCAAATTTTTTTGCTTAGAAGATAAGTTACCATAGGGATAACAGCGT AAT | 284 |
| NC_00 7895 | Sepia officinalis | GGGACGAGAAGACCCTAATGAGCTTAAATTTTATTTTATTATATATA TTTTATAGTAAAAAAGAAGTTTTAGTTGGGGTGATTAAGGAATAAA AAAACGAATGATGTAACTTCCTTAAATATAAAATTGTTAAATTAAGT AACCAATTTATATTGCTTATAATAGAGTTACCATAGGGATAACAGC GTAAT | 285 |
| NC_00 7894 | Sepioteut his lessonian a | GGGACGAGAAGACCCTATTGAGCTTGTAGTTTTTATATAACTATAT TATTAATTTATATAGTTATTTATAAACTTTAGTTGGGGTGACTAAGG AATAATATAAGTAACTTCCTTAGAGTAAAGATTGTTAAATAAAGTAA CCAAATTTAATATTGCTTATAATATAGTTACCATAGGGATAACAGC GTAAT | 286 |
| NC_00 6354 | Todarode s pacificus | GGGACGAGAAGACCCTAATGAGCTTATAATTTTATTATTGTGATTT AATATATTTAAATGATAATATATAAATTTTAATTGGGGTGATTAAGG AATAATTTATAAGTAGTAACTTCCTTAGATTAAATATATATTGATTAA GTAACCAATAAATATATTGCTTATAATATAGTTACCATAGGGATAAC AGCGTAAT | 287 |
| NC_00 6353 | Octopus vulgaris | GGGACGAAAAGACCCTATTGAGCTTTTGTTATTATATAGATATATA TATGTATATATATATGATTTATTTGTAAATAACTTTGGTTGGGGTGA TCAAGGAATAAAAATTAATTTAAAGTGTTTTATTAACTTCCTTAATTT TAAATTTAGTTGAAGAATAAACCAAGTTTTTTGCTTAGAAGATAAGT TACCATAGGGATAACAGCGTAAT | 288 |
| NC_00 2507 | Heterololi go bleekeri | GGACGAGAAGACCCTATATTTAATTCTTATTATTTTTTTAGATTTAT TTTAATTGGGGTGATTAAGGAATAAAGTATATTAGTTAGTAACTTC CTTAGTAGGATATATTGTTGAATAAAGTAACCGATATTACGTATAG AACCGTATATTGCTTATAATATAGTTACCATAGGGATAACAGCGTA AT | 289 |
| MT712 046 | Octopus sinensis | GGGACGAAAAGACCCTATTGAGCTTTTGTTATTATATAGATATATA TATATATATATATATGATTTATTTGTAAATAACTTTGGTTGGGGTGA TCAAGGAATAAAAATTAATTTAAAGTGTTTTATTAACTTCCTTAATTT TAAATTTAGTTGAAGAATAAACCAAGTTTTTTGCTTAGAAGATAAGT TACCATAGGGATAACAGCGTAAT | 290 |
| MT025 991 | Octopus american us | GGGACGAAAAGACCCTATTGAGCTTTTGTTGTTATATAGATATATA TAAATATATAATTTATTTGTAAATAACTTTGGTTGGGGTGATCAAGG AATAAAATTAATTTAAAGTGTTATATTAACTTCCTTAATTTTGAATTT AGTTGAAGAATAAACCAAGTTTTTTGCTTAGAAGATAAGTTACCAT AGGGATAACAGCGTAAT | 291 |
| MG54 8981 | Narrowte uthis nesisi | GGGACGAGAAGACCCTATTGAGCTTATAATTTTACTGTTGTTTATA AGTATTTATTTGATGATGGATAAATTTTAATTGGGGTGATTGAGGA ATAAAATATATTAAATAGTAACTTCCTTAGGTTAAATGTTGTTGGAT AAAGTAACCAATATATTATTGCTTATAATATAGTTACCATAGGGATA ACAGCGTAAT | 292 |
| NC_02 0348 | Ommastr ephes bartramii | GGGACGAGAAGACCCTAATGAGCTTATAATTTTATTATCATTATTT AAAACATTTAGGTGGTGGTATATAAATTTTAATTGGGGTGATTAAG GAATAAATTGTGTATAGTAACTTCCTTAAATTAGGTATTTGTAGATA AAGTAACCAATGTAATATTGCTTATAGTATAGTTACCATAGGGATA ACAGCGTAAT | 293 |
| NC_01 7749 | Sepiella japonica | GGGACGAGAAGACCCTAATGAGCTTAAATTTTATATTATTGTTATA TATGTTTTATAATTAATTGGAAATTTTAATTGGGGTGATTAAGGAAT AATATTAAGATAAATAACTTCCTTATATAATAATAAATTGTTGAATTA AGTAACCAATATTATTGCTTTTAATAAAGTTACCATAGGGATAACA GCGTAAT | 294 |
| NC_01 7746 | Uroteuthi s (Photololi go) edulis | GGGACGAGAAGACCCTATTGAGCTTATAATTTATTAATATTTTATA AATATTTATGTTGTGTTATATAAATTTTAATTGGGGTGATTAAGGAA TAATTTTATTGATTTTATAGTGTAACTTCCTTAGAAAACATATTGTTA AATTAAGTAACCGATTTATATATATAGGTTTATATTGGGTATTGCTTAT AATATAGTTACCATAGGGATAACAGCGTAAT | 295 |
| NC_01 2840 | Doryteuth is (Amerigo) opalesce ns | GGGACGAGAAGACCCTATTGAGCTTGTAATTTTATTATTATTAGTA TTATTTATTCTTATTGGTAGTATATAAATTTTAATTGGGGTGATTAA GGAATAAGGTTTGTAGTTTTAGTAACTTCCTTATTAATAATGATTGT TAAATAAAGTAACCAATATTATTGCTTATACTATAGTTACCATAGGG ATAACAGCGTAAT | 296 |
| NC_01 1581 | Architeut his dux | GGGACGAGAAGACCCTATTGAGCTTATAATTTTATTGTTGTTTTGT AATTATTTATGTGATGATAGATAAATTTTAATTGGGGTGATTGAGG AATAAGGTAATAGTAGTAACTTCCTTAGAAATAAATGTTGTTGGGT GAAGTAACCAATATATTATTGCTTATAATATAGTTACCATAGGGATA ACAGCGTAAT | 297 |
| NC_00 9734 | Dosidicus gigas | GGGACGAGAAGACCCTAATGAGCTTGTAATTTTATTATTGTTATTT AAAATATTTAGATAGCAATATATAAATTTTAATTGGGGTGATTAAGG AATAAGTTGAATATAGTAACTTCCTTAAATTGAATATTTATAGATAA AGTAACCAATATAATATTGCTTATAATATAGTTACCATAGGGATAAC AGCGTAAT | 298 |
| NC_00 9690 | Sepia esculenta | GGGACGAGAAGACCCTATTGAGCTTAAATTTAAAAGTACTATAGTA ATTTTTTTAATATTTTGATAAATTTTAATTGGGGTGATTAAGGAATA AAAGGATAATAAATTGGTAACTTCCTTAAACTTAATACTATTAGGTT AAGTAACCAATAATATTGCTTATAATAAAGTTACCATAGGGATAAC AGCGTAAT | 299 |
| NC_00 7896 | Amphioct opus fangsiao | GGGACGAAAAGACCCTATTGAGCTTTATATTATTGTTTAAATTTATT ATGCTGTTTTTATTTAAAATAATTTTGGTTGGGGTGATCAAGGAATA AAATATTAAATTTATTAACTTCCTTATTATATTTAAATATCTATATTAA TAAACCAAACTTTTTGCTTAGAAGATAAGTTACCATAGGGATAACA GCGTAAT | 300 |
| X7958 5 | Loligo vulgaris | GGGACGAGAAGACCCTACTGAGCTTATAATTTTATTATTATTGTTA TTTTTATTGGAGCAGTAATATATAAATTTTAATTGGGGTGATTAAGG AATAAATAAATATATTTAGTAACTTCCTTATAAAGAATATTGTTAGAT GAAGTAACCAATAAAGTTACATGATTATTGTAATAAATATTGCTTAT AATATAGTTACCATAGGGATAACAGSGTAAT | 301 |
| NC_04 4093 | Octopus mimus | GGGACGAAAAGACCCTATTGAGCTTGTATTATTTAACAAATTATAT GATTGTTATTTGTTTGAATTAATTTTGATTGGGGTGATCAAGGAATA AATTTTATTTAGTTTAACTTCCTTAGTTAATTGTTTTATAGAAGAATA AACCAAAGTTTTTGCTTAGAAGATAAGTTACCATAGGGATAACAGC GTAAT | 302 |
| NC_02 9747 | Octopus conispadi ceus | GGGACGAGAAGACCCTATTGAGCTTTATATTAGTTATAATAAATAA TAAAAATATAATTTATTTGGTTAAATTAGTATTATATTAATAATATTT ATTATGTTTAATTTTGATTGGGGTGATCAAGGAATAAAGTGGATAT | 303 |
| | | TATTTAACTTCCTTAAAATTTAATTTTTTTAGAATAATTAACCAATGA TATTGCTTATATGATAAGTTACCATAGGGATAACAGCGTAAT | |
| NC_02 9723 | Octopus bimaculoi des | GGGACGAAAAGACCCTATTGAGCTTATATTATTTATTAAATTATATA GTTATAGTTTATTATAAATTAATTTTGATTGGGGTGATCAAGGAATA AATTTTATTATTATATAACTTCCTTAGTTAAAATAGTTTTTGGAAAAA TAAACCAAGTTTTTTGCTTAGAAGATAAGTTACCATAGGGATAACA GCGTAAT | 304 |
| NC_02 8189 | Uroteuthi s (Photololi go) chinensis | GGGACGAGAAGACCCTACTGAGCTTATAATTTTATTATCACATATA TTTATGTATAATGTGCTATATAAATTTTAATTGGGGTGATTAAGGAA TAATATAGCTATTTTATAACTTCCTTAGATATTATATTGTTGAATTAA GTAACCGATTAATTAATAGAATTGTATTATGTATTGCTTATAATATA GTTACCATAGGGATAACAGCGTAAT | 305 |
| NC_02 7729 | Uroteuthi s (Photololi go) duvaucelii | GGGACGAGAAGACCCTATTGAGCTTATAATTTTATTACTATTATGG TTATTGAATTTATCATTGTAGTATATAAATTTTAATTGGGGTGATTA AGGAATAAATTTTTATAATGTAACTTCCTTAGATTATTATATTGTTAA ATTAAGTAACCGATGTGATTAGCACAATGTGTGTTATGTATTGCTT ATAATATAGTTACCATAGGGATAACAGCGTAAT | 306 |
| NC_02 6908 | argentinu s | GGGACGAGAAGACCCTAATGAGCTTATAATTTTATTATTATTATTTA ATATATTTAAATAATAATATATAAATTTTAATTGGGGTGATTAAGGA ATAAAAGAATTATATAATAACTTCCTTAGATTAAATATATATATATAT AAAGTAACCAATAATATATTGCTTATAATATAGTTACCATAGGGATA ACAGCGTAAT | 307 |
| NC_02 2959 | Sepia aculeata | GGGACGAGAAGACCCTATTGAGCTTGAATTTAATTGTATTATTTTA TAATATTTTAATAAATTTTAATTGGGGTGATTAAGGAATAAAAATT TTATATATTGTATAGTAACTTCCTTAATATGATTGTACTGTTAGGTT AAGTAACCAATAATATTGCTTATAATAGAGTTACCATAGGGATAAC AGCGTAAT | 308 |
| NC_02 2693 | Sepiella inermis | GGGACGAGAAGACCCTAATGAGCTTAAATTTTATATTATTATTAAT ATATAATTATAATAATTGGAAATTTTAATTGGGGTGATTAAGGAATA ATATTAAAATAAATAACTTCCTTATATATTAATAAATTGTTGAATTAA GTAACCAATAATATTGCTTTTAATAAAGTTACCATAGGGATAACAG CGTAAT | 309 |
| NC_02 2468 | Sepia lycidas | GGGACGAGAAGACCCTATTGAGCTTAAATTTAATTACATAATTTAA TTATGTTTTGATAAATTTTAATTGGGGTGATTAAGGAATAAAATTAT TATATAGTAACTTCCTTAATAGTTAATACTGTTGGGTTAAATAACCA ATAATATTGCATATAATAAAGTTACCATAGGGATAACAGCGTAAT | 310 |
| NC_02 2467 | Sepia latimanus | GGGACGAGAAGACCCTAATGAGCTTTTAATTTAATAGTATTTAATG TAAAATTGAATGTAAAGTTGTATAAATTTTAATTGGGGTGATTAAGG AATAAAATTATTTTAAATAATAACTTCCTTAAATGTAGTATTGTTAGA TTAATTAACCAATATTATTGCTTATAATAGAGTTACCATAGGGATAA CAGCGTAAT | 311 |
| NC_02 2466 | Sepia apama | GGGACGAGAAGACCCTAATGAGCTTAAATTTAATAGTGCTAAAATT ATAAGTTAATAGGATTATGAAAATTTTAATTGGGGTGATTAAGGGA TAATTTATTAAATAATAACTTCCTTAAATATAATATTGTTGAATTAAT TAACCAATTTATATTGCTTATAGTATAGTTACCATAGGGATAACAG CGTAAT | 312 |
| NC_02 1146 | Sepia pharaonis | GGGACGAGAAGACCCTATTGAGCTTAAATTTAGTAATATTATGTTT AGTAGTATTTAATAAATTTTAATTGGGGTGATTAAGGAATAAAATTA TATATTGAATAATAACTTCCTTAATTGTGTAATACTGTTAGGTTAAG TAACCAATAATATTGCTTATAATAAAGTTACCATAGGGATAACAGC GTAAT | 313 |
| NC_02 8034 | Loliolus (Nipponol oligo) beka | GGGACGAGAAGACCCTATTGAGCTTATAATTTTTGTTAGTGCTTTT ATTAATATTTATAATTGTATTATATAAATTTTAATTGGGGTGATTAAG GAATAACTGATTGGAAAGTAACTTCCTTAGATGTGTGTATTGTTAG ATTAAGTAACCGATTAATTAATATATTGTATTAGATATTGCTTATAAT ATAGTTACCATAGGGATAACAGCGTAAT | 314 |
| AF110 072 | Alloteuthi s subulata | GGGACGAGAAGACCCTATTGAGCTTATAATTTTATTGTTATTATATT TATATTAATATAGTAATTTATAAATTTTAATTGGGGTGATTAAGGAA TAAAGATAAATAATTAAAGTAACTTCCTTAAAATAAAGTATTGTTAA ATAAAGTAACCGATAAATTAGTATTAAAATAATACTATATATTGCTT ATATTATAGTTACCATAGGGATAACAGCGTAAT | 315 |
| AB270 952 | Nototodar us hawaiiens is | GGGACGAGAAGACCCTAATGAGCTTATAATTTTATTATTATGATTT GATATATTTGAATGATAATATATAAATTTTAATTGGGGTGATTAAGG AATAAGGTTTTATATTAATAACTTCCTTAAATTAAATATATATGTATT AAGTAACCAATAAATATATTGCTTATAATATAGTTACCATAGGGATA ACAGCGTAAT | 316 |
| X7957 8 | Sepia orbignyan a | GGGAAGAGAAGACCCTATTGAGCTTTAATTTAAAAGACTATTAAAA AATTATAGTTAATAAAAATTTTAATTGGGGTGATTAAGGAATAAAAA AGGCAACTTCCTTAAAATTAAAAGTTGTTAGGTTAAGTAACCAATA ATATTGCTTTTATAAAGTTACCATAGGGATAACAGCGTAAT | 317 |
| X7958 6 | Sepia papuensi s | RGGACGAGAAGACCCTATGAGCTTAAATTTATTAATTTTTATTAGA ATTTATAAAAATTTATAAAATTTTAATTGGGGTGATTAAGGAATAAG TGTTTAAATAGTAACTTCCTTAGATATAATTTTGTTAGATTAATTAAC CAATTAATATTGCTTATAAAATAGTTACCGTAGGGATAACAGCGTA AT | 318 |
| X7957 7 | Rossia macroso ma | GGGACGAGAAGACCCTATTGAGCTTATAATTTTATTTTGTTGTTTA TTGAAATAGTAGGGTATAAATTTTAATTGGGGTGATTAAGGAATAA GGTGATAGATAACTTCCTTAGGTAATAATGTAGATGTATGAAGTAA CCAATAATGCATTGCTTATATTATAGTTACCATAGGGATAACAGCG TAAT | 319 |
| AF110 084 | Lolligunc ula (Lolligunc ula) brevis | GGGACGAGAAGACCCTATTGAGCTTGTAATTTTATTAGTTACCAGT TAATATTATATAAATATGGTAACTTATAAATTTTAATTGGGGTGATT AAGGAATAAATGTTTAGTTAGTAACTTCCTTAGATTAATAAATAAAT TGTTAAATGAAGTAACCAATAAAATTTAATGTATAAATATTGCTTAT AATATAGTTACCATAGGGATAACAGCGTAAT | 320 |
| EU735 243 | Lolligunc ula (Loliolops is) diomedea e | GGGACGAGAAGACCCTATTGAGCTTATAATTTTATTAATTATCAAT ATTTATATATGTATTGATTTGGTAATTTATAAATTTTAATTGGGGTG ATTAAGGAATAAATTTTATTTATAGTAACTTCCTTAGATAAGTTAAT AAATGATTGTTAAATGAAGTAACCAATAAAAATTAGGTGATATTGCT TATAATATAGTTACCATAGGGATAACAGCGTAAT | 321 |
| AF110 085 | Afrololigo mercatori s | GGGACGAGAAGACCCTATTGAGCTTATAATTTTGTTATTATTATATT TATATTAATATAGTAATTTATAAATTTTAATTGGGGTGATTAAGGAA TAAAGATAAATATGTAAGTAACTTCCTTAATAAATTATATTGTTAAAT GAAGTAACCAATATAATATTAGTAATAATATATATTGCTTATAGTAT AGTTACCATAGGGATAACAGCGTAAT | 322 |
| NC_01 5896 | Octopus minor | GGGACGAGAAGACCCTATTGAGCTTTATTTATTAATATTTAAATATA AATATATAATTTATATTATGAAAATTATGTTGTATATATATATATATT TAATTGGATTTAATTTTGATTGGGGTGATCAAGGAATAAAGATAAA GTAACTTCCTTAAAATTAATGATTGTTTTTATAGAGTAATAAACCAA GTTTTTTGCTTATAAGATAAGTTACCATAGGGGTAACAGCGTAAT | 323 |
| NC_02 8547 | Octopus bimaculat us | GGGACGAAAAGACCCTATTGAGCTTATATTATTTATTAAATTATAG AGTTATAATTTATTATAAATTAATTTTGGTTGGGGTGATCAAGGAAT AAGTTTAATGATTATATAACTTCCTTAACTGAACTATTTTTTTGGAA AAATAAACCAAGTTTTTTTGCTTAGAAGATAAGTTACCATAGGGAT AACAGCGTAAT | 324 |
| NC_03 8213 | Octopus variabilis | GGGACGAGAAGACCCTATTGAGCTTTATTTATTAATATTTAAATATA AATATATAATTTATATTATGAAAATTATGTTGTATATATATATATATT TAATTGGATTTAATTTTGATTGGGGTGATCAAGGAATAAAGATAAA GTAACTTCCTTAAAATTAATGATTGTTTTTATAGAGTAATAAACCAA GTTTTTTGCTTATAAGATAAGTTACCATAGGGGTAACAGCGTAAT | 325 |
| AB191 115 | Octopus cyanea | GGGACGAAAAGACCCTATTGAGCTTTATATATTAATATATAAATTTA ATTGGTGATTTATAAAAAGTTTATTAATATTAATTTTGGTTGGGGTG ATCAAGGAATAAAAGGTAAATTGTATTTTAGATAACTTCCTTAGAAA AATAATTGATGGAGTAATAAACCAAGATTTTTGCTTATAAGATAAGT TACCATAGGGATAACAGCGTAAT | 326 |
| AB191 114 | Callistoct opus ornatus | GGGACGAGAAGACCCTATTGAGCTTGGTTATTAATAATTAAATATA AAATAAATTGTTTTTATGGATTTATTTAGTATGATAGTATATAAATAT ATTTAGTTAAATTAATTTTGATTGGGGTGATCAAGGAATAAAATATT TAAAAAGTAACTTCCTTAATTTTTATTAGAGAATTTTAATTGAGTAAT AAACCAAAGTTTTTGCTTATAAGATAAGTTACCATAGGGGTAACAG CGTAAT | 327 |
| MG99 9649 | Enterocto pus megalocy athus | GGGACGAGAAGACCCTATTGAGCTTTATATTAGTTATAATAAATAA TAAAATTTTATTTATTTGGTTAAATAATATTATGTTTATTATGTTTAAT TTTGATTGGGGTGATCAAGGAATAAGATAAGTATAATTTAACTTCC TTAAAAATAATTTTTTTATAGAATAATTAACCAATAATATTGCTTATA TGATAAGTTACCATAGGGATAACAGCGTAAT | 328 |
| AB191 107 | Enterocto pus dofleini | GGGACGAGAAGACCCTATTGAGCTTTATATTAGTTATAATAAATAA TAAAAATATAATTTATTTGGTTAAATTAGTATTATATTAATAATATTT ATTATGTTTAATTTTGATTGGGGTGATCAAGGAATAAAGTGGATAT TATTTAACTTCCTTAAAATTTAATTTTTTTAGAATAATTAACCAATGA TATTGCTTATATGATAAGTTACCATAGGGATAACAGCGTAAT | 329 |
| GQ226 031 | Sasakiop us salebrosu s | GGGACGAGAAGACCCTATTGAGCTTTATATTAGTTATAATAAATGG TAAAATATAATTTATTACGTTTATTGTGTTTAATTTTGATTGGGGTG ATCAAGGAATAAAATAAATATAATTTAACTTCCTTAGAAAAATTATT TTTTAGAATAATTAACCAATGATATTGCTTATATGATAAGTTACCAT AGGGATAACAGCGTAAT | 330 |
| AY545 105 | Octopus berrima | GGGACGAGAAGACCCTATTGAGCTTAATATTAATATTTAAATATAA AAATTTTTAGTTTTTATTTTATATGAATGTTTATTGAATGTAATTTTG ATTGGGGTGATCAAGGAATAAAAGTAAATATTAACTTCCTTAAGTT GTTGATTTTATTGAGTAATAAACCAAATTTTTTTGCTTATAAGATAAG TTACCATAGGGGTAACAGCGTAAT | 331 |
| NC_03 6351 | Amphioct opus marginatu s | GGGACGAAAAGACCCTATTGAGCTTTATATTATATTTTAAATATAAT TTGTTTTGTATTTATTTAAATATAATTTTGGTTGGGGTGATCAAGGA ATAAAGTATATTTGTATTAACTTCCTTAGATATTATATATAAATAAAT TAATAAACCAAATTTATTGCTTAGAAGATAAGTTACCATAGGGATA ACAGCGTAAT | 332 |
| AJ311 110 | Octopus maorum | GGGACGAGAAGACCCTATTGAGCTTGTATATTAATATTTAAATGTT AGATGGTTTATTTATTATATTGTTAATAAATGAATAGATTTTTGTTGA TTAATAAGTATGGATGTTTAATTTAATTAATTTTGATTGGGGTGATC AAGGAATAAAAGAAAAGAAATTTAATAACTTCCTTAAGTTTTGAAAT GTTTATTGAATAATAACCAAATTTTTTTGCTTATAAGATAAGTTACC ATAGGGGTAACAGCGTAAT | 333 |
| MK450 541 | Octopus fitchi | GGGACGAGAAGACCCTATTGAGCTTAGAATTATTATTTAATTAATG TGATAATTTATTTATTAGTTTATTGATATTTATTTATGTAATTTTGATT GGGGTGATCAAGGAATAAAAGTTTATAATAACTTCCTTAGGATTTT GTAATTTATAGATTAATAAACCATAATTTTGCTTAGAAGATAAGTTA CCATAGGGATAACAGCGTAAT | 334 |
| NC_04 9899 | Amphioct opus neglectus | GGGACGAAAAGACCCTATTGAGCTTTATATTATATTTTAAATTTGAT TTTATATTTATTTACATATAATTTTGGTTGGGGTGATCAAGGAATAA AATGTATTTATATTAACTTCCTTAAATGGTGATATATATAAATGAAT AAACCAAATTTTTTGCTTAGAAGATAAGTTACCATAGGGATAACAG CGTAAT | 335 |
| NC_02 6724 | Loliolus (Nipponol oligo) uyii | GGGACGAGAAGACCCTATTGAGCTTATAATTTTATTAATATTTTATT GTTATTTATAATTATATTATATAAATTTTAATTGGGGTGATTGAGGA ATAATTAATTAAATAGTAACTTCCTTAGGTATTTATATTGTTGAATTA AGTAACCGATTAATTAATATGTTTTATACAAGTATTGCTTATAATAT AGTTACCATAGGGATAACAGCGTAAT | 336 |
| NC_03 0208 | Loliolus (Nipponol oligo) japonica | GGGACGAGAAGACCCTATTGAGCTTATAATTTTATTGATATTTTTAT TATTATTTATAATTATATTATATAAATTTTAATTGGGGTGATTAAGGA ATAATTAGTTAAAAAATAACTTCCTTAAGTGTAATATATTGTTGAAT TAAGTAACCGATTAATTAATATTTTTATATTAGTATTGCTTATAATAT AGTTACCATAGGGATAACAGCGTAAT | 337 |
| NC_01 6423 | Bathyteut his abyssicol a | GGGACGAGAAGACCCTATTGAGCTTGTAATTTTATTATTATTATATT AGTAGTATTGATATAGTAATATATAATTTTAGTTGGGGTGATTAAG GAATAAATTGTATAGTAACTTCCTTAGGTTATATATTTTATTATGAA GTAACCAATATAGTATTGCTTATATTATAGTTACCATAGGGATAACA GCGTAAT | 338 |
| NC_01 6425 | Semirossi a patagonic a | GGGACGAGAAGACCCTACTGAGCTTGTAATTTTATTTTATTATTTA TTGTAATAGTAATGTATAAATTTTAATTGGGGTGATTAAGGAATAAA TATAATATTAAAGTAACTTCCTTAAATAAAAATATAGATATATAAAGT AACTAATAATTATATTACTTATATTATAGTTACCATAGGGATAACAG CGTAAT | 339 |
| NC_02 3257 | Cistopus taiwanicu s | GGGACGAAAAGACCCTATTGAGCTTATATTAATATTTATAGTATTAT AAGTATATATATATATATATATATATATATATATATATATATATATA GAGTATATGTTATAAATATATTAATTTTGGTTGGGGTGATCAAGGA ATAAAGTATAGAAAAATTTTTATATAACTTCCTTAATTTTTAATTTTA TTGATTAATAAACCAATATTTTTGCTTATATGATAAGTTACCATAGG GATAACAGCGTAAT | 340 |
| NC_01 0636 | Sthenote uthis oualanien sis | GGGACGAGAAGACCCTAATGAGCTTATAATTTTATTATTGTTGTGC AAAACATTTGGTCAATAATATATAAATTTTAATTGGGGTGATTGAG GAATAAATTAGATAAAGTAACTTCCTTAAATTAAATATTTATAGATA AAGTAACCAATATAATATTGCTTATAATATAGTTACCATAGGGATAA CAGCGTAAT | 341 |
| NC_00 7893 | Wataseni a scintillans | GGGACGAGAAGACCCTAATGAGCTTTAATTTTATTAATTATTTATTT TAACAAATTTGTAGTTATATAAATTTTAATTGGGGTGATTAAGGAGT AAATTTAAGTAACCTCCTTATTTTTATTACTGATAAGTAAAATAACC AATTTTGTTGCTTATACTAGAGTTACCATAGGGATAACAGCGTAAT | 342 |
| EU735 265 | Gonatops is okutanii | GGGACGAGAAGACCCTATTGAGCTTATAATTTTATCATTATTAGAC TTATTATTAGTGTAGTAGTGTATAAATTTTAATTGGGGTGATTAAGG AATAAATTTAATTTAGTAACTTCCTTAAGTTTTATATTGTTAAATAAA GTAACCAATAAGTTATTGCTTATAATATAGTTACCATAGGGATAAC AGCGTAAT | 343 |
| AY293 656 | Uroteuthi s (Aestuari olus) noctiluca | GGGACGAGAAGACCCTATTGAGCTTATAATTTTATTAGTGCTTAGT TTAAATACAAATGCATATATATAAATTTTAATTGGGGTGATTAAGGA ATAAAAATAATAAATTATAAGTAACTTCCTTAGAAGTATTAGTATTG ATAAATGAAGTAACCAATAAATAAACATATATTGCTTATAACATAGT TACCATAGGGATAACAGCGTAAT | 344 |
| AB675 085 | Sepioteut his australis | GGGACGAGAAGACCCTAATGAGCTTATAATTGTATTATTATTATAA TTAGATGTTTATAATAGTTTATAAATTTTAATTGGGGTGATTAAGGA ATATATGTACACAACTTCCTTAAATATAATATTGTTGGATGGAGTAA CCAATAGATTATTGCTTATAATAAAGTTACCATAGGGATAACAGCG TAAT | 345 |
| AF110 090 | Sepioteut his sepioidea | GGGACGAGAAGACCCTATTGAGCTTGAATTTTATAATAATTATGTA TATTAATAGCAGTAATTATATATAAATTTTAATTGGGGTGATTGAGG AATAATTTTTATGAAGTAACTTCCTTAAAATAAATATTGTTAAATTAA GTACCCGATTAATAGTATATTGCTTGTAATATAGTTACCATAGGGA TAACAGCGTAAT | 346 |
| AJ311 108 | Amphioct opus kagoshim ensis | GCGGTAWTATAACTGTACTAAGGTAGCATAATAATTTGCCTTATAA ATTGGGGCTAGAATGAATGGTTTGACGAAAATTATACTGTCTCTAT TTTATTCATTAGAAGTTAATTTTTATAGTGAGAAAGCTTGGATGATT TAAAGGGACGAAAAGACCCTATTGAGCTTTATATTATATTTTAAATA TAATTTTTTGTGTTTATATAAATATAATTTTGGTTGGGGTGATCAAG GAATAAGATATATCTATGTTAACTTCCTTAAATATATATATAAATTAA TAAACCAAATTTATTGCTTRGAAGATAAGTTACCATAGGGATAACA GCGTAAT | 347 |
| LC121 044 | Amphioct opus membran aceus | GGGACGAAAAGACCCTATTGAGCTTTATATTATATTTTAAATTTGAT TTTTTATTTATTTAATTATATATAATTTTGGTTGGGGTGATCAAGGA ATAAAATGTATTTTATGTTAACTTCCTTAAATGGTAATATTTATAAAT GAATAAACCAAATTTTTTGCTTAGAAGATAAGTTACCATAGGGATA ACAGCGTAAT | 348 |
| LC121 036 | Amphioct opus exannulat us | GGGACGAAAAGACCCTATTGAGCTTTATATTATTATTTAAATTTATT ATATTAATTTTTATTTTAAATAATTTTGGTTGGGGTGATCAAGGAAT AAAATATTAAATTTATTAACTTCCTTATATATTTAAATATATATATATT AATAAACCAAATTTTTTGCTTAAAAGATAAGTTACCATAGGGATAAC AGCGTAAT | 349 |
| LC121 047 | Amphioct opus rex | GGGACGAAAAGACCCTATTGAGCTTTATATTATTATTTAAATTTATT ATATTAATTTTTATTTTAAATAATTTTGGTTGGGGTGATCAAGGAAT AAAATATTAAATTTATTAACTTCCTTATATATTTAAATATATATATATA TTAATAAACCAAATTTTTTTGCTTAAAAGATAAGTTACCATAGGGATA ACAGCGTAAT | 350 |
| AB192 324 | Sepia peterseni | GGGACGAGAAGACCCTATTGAGCTTTAAATTTGGAAATATTATATT TATTAAATATAATGNTTGAAAAATTTTAATTGGGGTGATTAAGGAAT AAAAGAAATAAATATATTAAAATTTAACTTCCTTAAATAATATATTGT TAAATAAAGTAACCAATAATATTGCTTATATAAAGTTACCATAGGGA TAACAGCGTAAT | 351 |
| AB058 620 | Varuna litterata | TTGAAGTGACGATAAGACCCTATAAAACTTTATATAAACATATTATT TGATTGAATTTAAAATAAAAATTTAATAATAAAATTTATTTTGTTGGG GCGATAAAAGTAAAATTTTTATTAACTGCTTTAAGTTTTGAATACAT TTATGTTTGATTAAAATTTTTAGATGATCCTAAATAAAGATTAAAAG TTTAAGTTACTTTAGGGATAACAGCGTTAT | 352 |
| AF107 616 | Hemisquil la ensigera | GGGGGACGATAAGACCCTATAAAGCTTTATGATAAGGCCTTTTTG TAAAGAATTTCTATTGTGAAATTACTAGAAGGTTTATATCATTTTAC TGGGGCGGTAAAAGTATATAATTGAATAACTGCTTTTATTATTGAA AACAATTATAATTGAAATACAAAATGATCCATTATTAATGATTATAA AAACAAGTTACTTTAGGGATAACAGCGTAAT | 353 |
| AF107 615 | Gonodact ylus smithii | GGAGGACGATAAGACCCTATAAAGCTTTATAATATATTTTCTGTTA TATAAATTGTTAATTGTTAACTTATTAAAGAAATAATATTATTTTACT GGGGCGGTAAAAGTATATAATTTAGGGTAACTGCTTTTGTTATTAT AAACAATGATAATTGAATGATATATTGATCCATTATTAGTGAGCATA AGGACAAGTTACTTTAGGGATAACAGCGTAAT | 354 |
| AF107 611 | Pullosquil la thomassi ni | AAGGGACGATAAGACCCTATAAAGCTTTATAACATAATCTTTTTGC GCAAAATATCTAATGTAAAACATTAAAAGAATCATGTTATTTTACTG GGGCGGTAAAAGTATATTAAAAATAACTGCTTTTGTTTTATAAAACA ATAATAATTGGTACACAGATTGATCCATTAATAATGATCATAAAAAC AAGTTACTTTAGGGATAACAGCGTAAT | 355 |
| AF107 609 | Chorisquil la trigibbosa | GAGGGACGATAAGACCCTATAAAGCTTTATGATATTTTCTTTTTATA TTAGTTAGTAAAATGTAGTTTATTAAAAGAAAAATATTATTTTACTG GGGCGGTAGAAGTATATTTTTGATTAACTGCTTCTGTTTTGTAAGT CAATTATAATTGAAATATGAATTGATCCATTATTAATGATCATAAGA ACAAGTTACTTTAGGGATAACAGCGTAAT | 356 |
| MT335 860 | Chionoec etes opilio | GGGGGACGATAAGACCCTATAAAGCTTTACATATAAGTAGAGTTTA CTTAATTAAAAAGTAAAAGTTTAATCTAATTAATGTGTTTTGTTGGG GCGACATAAATATAATTTATATTAACTGTTTATAAATTAATACAATAA TAAATGATTTTAATGTAGTTGATCCTTATTAAAGATTAAAAGACTAA GTTACTTTAGGGATAACAGCGTTAT | 357 |
| AB220 027 | Chionoec etes opilio | AGGGGACGATAAGACCCTATAAAGCTTTACATATATTATAAGTTTG ATTAAATTAAAATATAAAAATCTAAATTATATATTATGTTTTGTTGGG GCGACAAAAATATAAGTAATAATAACTGTTTAAGAAAAAAACAATAA TAAATGAATAATTAAATGATCCTTGGTTAAAGATTAAAGATCAAGTT ACTTTAGGGATAACAGCGTTAT | 358 |
| AB188 686 | Telmessu s acutidens | AGGGGACGATAAGACCCTATAAAGCTTTACATATATTATAAATTTG ATTAAATTAAAATCTAAAAATCTAAATTATATATTATGTTTTGTTGGG GCGACAAAAATATAAGTAATAATAACTGTTTAATAAAAAAACAATAA TAAATGAATAATTAAATGATCCTTATTTAAAGATTAAAGATCAAGTT ACTTTAGGGATAACAGCGTTAT | 359 |
| AB236 927 | Lithodes aequispin us | CAAAAAGACGATAAGACCCTATAAATCTTTACAATAAATATATTTTA TATTTTAGCTTATAAGTGTATAAGAAATAAAAATATAGGTTTGTTGC GCTGGGGCGGCGTAGATATATAAATAAACTGTCTATAATTTAAATA CAATAATCATTGCTTAATACAAATTGATCCTTAAATAGATTAAAAGA TTAAGATACTTTAGGGATAACAGCGTTAT | 360 |
| AB248 090 | Panulirus echinatus | GGGGGACGATAAGACCCTATAAATCTTGATAATAAGACTAATAACT TCATATATTAGTTGTGAGTCTGAGGTTAATACTTTTATTATTTTGTT GGGGCGACAGAAGTATAATTTTAGTAACTGCTTTGGCTTAATAGTT AATTATTTTTATAATGTTTATTGGTTAAGATCCTTCTGTGAGGATGC CAGATTAAGTTACTTTAGGGATAACAGCGTAAT | 361 |
| AF192 870 | Jasus paulensis | GGGGGACGATAAGACCCTATAAATCTTTATGNTAGTTAGTAATAAC ATTAAAAATTAATTTNACATTTTTTTGTTAGTAGTTTTAGCATTTTGC TGGGGCGGCAGGGGTATAATTTAATAACTGCTCTAGAGTTGTAGT TAATTATGTTTAAATTATTGGTAGTTGATCCTTTAGTGGAGATTATA GATTAAGTTACTTTAGGGATAACAGCGCAAT | 362 |
| AF192 869 | Jasus caveorum | GGGGGACGATAAGACCCTATAAATCTTTATGTTAGTTAGTAATAAC ATTAAAAATTAATTTTACATTTTTTTGTTAGTAGTTTTAGCATTTTGC TGGGGCGGCAGGGGTATAATTTAATAACTGCTCTAGAGTTGTAGT TAATTATGTTTAAATTATTGGTAGCTGATCCTTTAGTGGAGATTATA GATTAAGTTACTTTAGGGATAACAGCGCAAT | 363 |
| AF175 246 | Parastac us pilimanus | AAGGGACGATAAGACCCTATAAAGCTTCACATTAAGTATTTAAAAT TTTAAGTGAGTAATAAAATTTTATTAGATGTAATATGTTTTGTTGGG GCGACAGGAATATAAGAGATTTAACTGTTCTAGATAAGTTAATCAA AAATACTTGATCTTTAAGGACTTTTTATTAAAGTATTAGACTAAGTT ACTTTAGGGATAACAGCGTTAT | 364 |
| MG55 1495 | Parastac us brasiliensi s | AAGGGACGATAAGACCCTATAAAGCTTCACATTAAGTCTTTAAAAT TTTAATTAAGTAATAAAATTTTATTAAATGCAATATGTTTTGTTGGG GCGACAGGAGTATAAGAAATTTAACTGCTCTAAATAATAAGTCAAA GATACTTGATCTTTGGGGACTTTTTATTAAAGTGTTAGATTAAGTTA CTTTAGGGATAACAGCGTAAT | 365 |
| AF175 243 | Parastac us defossus | AAGGGACGATAAGACCCTATAAAGCTTTACATTAAGCTTTTAAAAT TTTAATTAAGTAATAAAATTTTATTAAATGCAATATGTTTTGTTGGG GCGACAGGAATATAAGAAATTTAACTGTTCTAAATAATAAATCAAA GATACTTGATCTTTGGGGACTTTTTTGTTAAAGTGTTAGATTAAGTTA CTTTAGGGATAACAGCGTTAT | 366 |
| AF175 233 | Parastac us nicoleti | AAGGGACGATAAGACCCTATAAAGCTTTACATTCAACTTTTTAGGA TTATTAATTAAGTAATAAAAGTTTGTTAGGTATAATATGTTTTGTTG GGGCGACAGGAATAAAAGAGATTTAACTGTTCTAAATAATAAATCA GAAATATTTGATTTTTAATGATTTTTTATTAAAATATTAGATTAAGTT ACTTTAGGGATAACAGCGTTAT | 367 |
| AF133 678 | Gonodact ylus graphuru s | GAGGGACGATAAGACCCTATAAAGCTTTATAGTAGTTCCTTTTTAT TCAAATTATAAAATGTTAACCTTTTGAAAGATTACTACTATTTTACT GGGGCGGTAGAAGTATATTTATGATTAACTGCTTTTGTTTTGACAA ACAATAATAATTGAATATTTATGTGATCCATTATTAATGATCAAAAG TACAAGTTACTTTAGGGATAACAGCGTAAT | 368 |
| AF192 873 | Jasus lalandii | GGGGGACGATAAGACCCTATAAATCTTTATACTAGTTAGTAATAAC ATTAAAGATTAATTTTACATTTTTTTGTTAGTAGTTTTAGTATTTTGC TGGGGCGGCAGGGGTATAATTTAATAACTGCTCTAGAGTTGTAGT TAATTATGTTTAAATTGTTGGTAGTTGATCCTTTAGTGGAGATCATA GATTAAGTTACTTTAGGGATAACAGCGCAAT | 369 |
| AF192 872 | Jasus paulensis | GGGGGACGATAAGACCCTATAAATCTTTATGTTAGTTAGTAATAAC ATTAAAAATTAATTTTACATTTTTTTGTTAGTAGTTTTAGCATTTTGC TGGGGCGGCAGGGGTATAATTTAATAACTGCTCTAGAGTTGTAGT | 370 |
| | | TAATTATGTTTAAATTATTGGTAGTTGATCCTTTAGTGGAGATTATA GATTAAGTTACTTTAGGGATAACAGCGCAAT | |
| AF425 333 | Lopholith odes mandtii | AAGACGATGAGACCCTATAAATCTTTACAATAAATATATTTTATATT TTAGCTTATAAGTATATAAAAAATAAAATATAGACTTGTTGCGCTGG GGCGGCGTAGATATATAAATAAACTGTCTATAGTTTAAATACAATA ATCATTGCTTAATACAAATTGATCCTTAAATAGATTAAAAGATTAAG ATACTTTAGGGATAACAGCGTTAT | 371 |
| AF425 331 | Lithodes santolla | CAAAAAGACGATAAGACCCTATAAATCTTTACAATAAATATATTTTA TATTTTAACTTATAAGTGTATAAGAAATAAAAATATAGATTTGTTTC GCTGGGGCGGCGTAGATATATAAATAAACTGTCTATAATTTAAATA CAGTAATTATTGCTTAATATAAATTGATCCTTAAATAGATTAAAAGA TTAAGATACTTTAGGGATAACAGCGTTAT | 372 |
| AF425 330 | Lithodes maja | CAAAAAGACGATAAGACCCTATAAATCTTTACAATAAATATATTTTA TATTTTAGCTTATAAGTGTATAAAAAATAAAAATATAGATTTGTTGC GCTGGGGCGGCGTAGATATATAAATAAACTGTCTATAATTTAAATA CAATAATCATTGTTTAATATAGATTGATCCTTAAATAGATTAAAAGA TTAAGATACTTTAGGGATAACAGCGTTAT | 373 |
| AF337 979 | Jasus edwardsii | GGGGGACGATAAGACCCTATAAATCTTTATACTAGTTAGTAATAAC ATTAAAGATTAATTTTACATTTTTTTGTTAGTAGTTTTAGTATTTTGC TGGGGCGGCAGGGGTATAATTTAATAACTGCTCTAGAGTTGTAGT TAATTATGTTTAAATTGTTGGTAGTTGATCCTTTAGTGGAGATTATA GATTAAGTTACTTTAGGGATAACAGCGCAAT | 374 |
| AF337 976 | Panulirus regius | GGGGGACGATAAGACCCTATAAATCTTTATATTTCGGTCAGTAAAT TTTAGAGATTAGTTATACATTTAGTGTTATTGAACTCAAATATTTTG TTGGGGCGACAGGAGTATAATAAGTCACTGCTTTGTTTATAAGTTA ATTATGTTTATTTTGAAAACCAATATTGATCCTTTCTAGAGAAATCA GACTAAGTTACTTTAGGGATAACAGCGTAAT | 375 |
| AF337 973 | Panulirus pascuens is | GGGGGACGATAAGACCCTATAAATCTTGATAACCAAAGTAATAATT TTATGTAGTAGTCATAAGCTTAAATTTAATACTTTTGTTATTTTGTTG GGGCTACAGGGGGTATAATTAATAACTGCGGTTGGGAAAAATAAT TAATTATTTTTATTGTGTTGTGTTTAAGATCCTTCTTTGAAGATACA GGTCAAGTTACTTTAGGGATAACAGCGTAAT | 376 |
| AF337 969 | Panulirus laevicaud a | GGGGGACGATAAGACCCTATAAATCTTTATATTTTAGTCGATAAGT TTTAGGAATTAGTTACACACTTAGTATCATTGGACTAAAATATTTTG TTGGGGCGACAGAAGTATAATAAGTAACTGCTTTGTTTATAAATTA ATTATTTTTATTATGAGAGCTGATATAGATCCTCTTTAGAGAAATCA GATTAAGTTACTTTAGGGATAACAGCGTAAT | 377 |
| AF337 964 | Panulirus gracilis | GGGGGACGATAAGACCCTATAAATCTTTATGTTTTGGTTAATAAAT TTTAGAAATTAGTTATATATTTAGTATTATTAGGCTAAAATATTTTGT TGGGGCGACAGGAGTATAATAAGTAACTGCTTCGGTTTGTAAATT AATTATTTTTATATTGGAAATGTAATATTGATCCTTTTTAGAGAAAT CAGACTAAGTTACTTTAGGGATAACAGCGTAAT | 378 |
| AF337 963 | Panulirus guttatus | GGGGGACGATAAGACCCTATAAATCTTGATAGAGAAGATTATAAC CTTAGAAAGTAGTTACAGTTCTCGGGTTAAGACATTTTCTATTTTGT TGGGGCGACAGGAGTATAATTAGTAACTGCTTTGGGCTTAATATG TAATTATCGCTATTGTGGGTATTATGAGAGATCCTTCTTTGAAGAT GTCAGACTAAGTTACTTTAGGGATAACAGCGTAAT | 379 |
| AF337 960 | Panulirus inflatus | GGGGGACGATAAGACCCTATAAATCTTTATGTTTTAACTAATAAAT TTTAGGGATTAGTTATATACCTAGTATTATTAGTTGAAATATTTTGT TGGGGCGACAGGAGTATAATAAGTAACTGCTTTGTTTATAAAATTA ATTGTTTTTATTGTGGAAATTGGTATGATCCTCTTTAGAGAAATCAG ACTAAGTTACTTTAGGGATAACAGCGTAAT | 380 |
| AF339 156 | Panulirus femoristri ga | GGGGGACGATAAGACCTATAAATCTTGATAACCAAAGTAATAATTT TTATGTAGTAGTCATAAGCTTTAAATTTAATACTTTTGTTATTTTGTT GGGGCGACAGGGGTATAATTAGTAACTGGCTTTGAGTAAAGAATT AATTATTTTTATTGTGTTGTGTTTGAGATCCTTCTTTGAAGATATCA GATCAAGTTACTTTAGGGATAACAGGTAATC | 381 |
| AY227 446 | Chionoec etes bairdi | GGGGGACGATAAGACCCTATAAAGCTTTACATATAAGTAGAGTTTA CTTAATTAAAAAGTAAAAGTTTAATCTAATTAATGTGTTTTGTTGGG GCGACATAAATATAATTTATATTAACTGTTTATAAATTAATACAATAA TAAATGATTTTAATGTAGTTGATCCTTATTAAAGATTAAAAGACTAA GTTACTTTAGGGATAACAGCGTTAT | 382 |
| AF425 339 | Paralomis granulosa | AAAAGACGATAAGACCCTATAAATCTTTACAAGAAATATATTTTATG TTTTAGCTTATAAGTAATATAAGAAATAAAAATATAAGTTTGTTGCG CTGGGGCGGCGTAGATATATAAATAAACTGTCTATGATTTAAATAC AGTAATTATTGCTTAATACAAATTGATCCTTAAATAGATTAAAAGAT TAAGATACTTTAGGGATAACAGCGTTAT | 383 |
| AF502 949 | Scyllarus arctus | GGGGGACGATAAGACCCTATAAATCTTTATACTCGGCTTTATTGAG CAAAATACTAGAATAATTTTTGTTCGATAGGTTTGAGTATTTTGTTG GGGCGACAGGAGTATAAAATGTAACTGCTTTAAAATAATAGTTAAT TGATGTTTAGTTAAGCGATCCTTTAATAAAGATGTTAGTTTAAGTTA CTTTAGGGATAACAGCGTAAT | 384 |
| AF502 955 | Palinurus elephas | AGGGGACGATAAGACCCTATAAATCTTTATATTTGTCATAATGATA TTAAGTATTATAATTGAAAATTTAGTTTTATTGGGATAGAATATTTT GTTGGGGCGACAGGAGTATAAAAAAGTAACTGCTTTGTAAGATGA GTTAATAATTTTTAATGTGTGATATAGTTGATCCTTCAGTGAGGATT TCAGAATAAGTTACTTTAGGGATAACAGCGTAAT | 385 |
| AJ784 020 | Episesar ma mederi | AAAAGACGATAAGACCCTATAAAACTTAATATAAGTTAATTGTTTAG TAGAATTTTTAATAATAAATATTTAATAATTAGTTTTTATTTTATTGG GGTGATAATGGTAAAATGATTATTAACTGTTGATTATTTTATAACAA AAATTATTGAATAAATATATAATAAATGATCCTTGTTAAAGATTAAA AGTTTAAGTTACTTTAGGGATAACAGCGTTAT | 386 |
| NC_03 3504 | Austropot amobius torrentiu m | GGGGGACGATAAGACCCTATAAAACTTTATATTTTGAAATAATAAT TAATTTTGTGTGGGAGATTTATTTTTAAGTATTTTATTGGGGTGATA AGGATAAAATAATAAATAACTGTCTTTTTTTATTACAGTAATATTTG AGCAAATGAACCTAGTAAGGGAAAGAAGATTAAGTTACTTTAGGG ATAACAGCGTAAT | 387 |
| AM410 525 | Cycloach elous granulatu s | AGGGGACGATAAGACCCTATAAAGCTTTATAAATTCAGGAATTTTA GATATTTTATATATAAATCTATTTTTTGATAATTTATTTGGTTGGGG CGACAAGGGTATAATTATAAATAACTGCCATTAATGTTTATACATAT ATTTTTGATTTAAATTTGATATAGTAGTTGATCCTTTTTAAGGATTTA AAGATCAAGTTACTTTAGGGATAACAGCGTAAT | 388 |
| AY151 824 | Eriocheir recta | AAGGGACGATAAGACCCTATAAAGCTTAATATAAAATTTTATTTAGT CAAATTTTACGTTATAAAGACTTTGTAAGTATGAGTTTATTTTATTG GGGCGATAAGAGTAAAATGATTGTTAACTGCTTAATATTAAATACA CTTATGAGTGATTAAATTTTTAGATGATCCTAATTAAAGATTAGAAG TTTAAGTTACTTTAGGGATAACAGCGTTAT | 389 |
| AY351 244 | Cervimun ida johni | AAGGGACGATAAGACCTTATAAATCTTTATATTAATTTGTTTTTTGT CTTTTAATAATGTAATGTTGATAATAAAATAAAGTATATTGTGTTGG GGTGATGAGAATATAAAATTAACTGTTCTAAATTAAACACAAATTTA TTTGTATAAAATAATAGATCCTTATTAAAGATTAAAAGGTTAAGTTA CTTTAGGGATAACAGCGTTAT | 390 |
| DQ388 058 | Achelous floridanus | GGGGGACGATAAGACCCTATAAAGCTTTATAAAAGTAGTAAGATC ACATATTTGAATATAAAAGCGATTATATAATCTTTTATTTTGTTGGG GCGACAGTAGTATAATTGAAAGTAACTGCTATTATGAAAACACATT GATTTATGATTATTTATTAATGGATCCCTTTTAAGGATTATAAAATC AAGTTACTTTAGGGATAACAGCGTTAT | 391 |
| DQ388 053 | Portunus sayi | GAGGGACGATAAGACCCTATAAAGCTTTATAAATTTAAAGATTTTA CATATTTTATCTATAATGGTAATCTCTGGAGAATTTATTGGGTTGG GGCAACAAAAGTATAATTTATCAATAACTGCTATAAAATAATACAATT ATCTTTGTGTGGGTAAATTAGTTGATCCCTGAAAAGGATTAAAAGA TCAAGTTACTTTAGGGATAACAGCGTAAT | 392 |
| DQ388 054 | Portunus anceps | GAGGGACGATAAGACCCTATAAAGCTTTATAAACTAAAAGAATTCA CAAATTTAGACATAAAGGTAATTTTCTAACTTCTTATTTTGTTGGGG CGACAGTAGATAACTAAAGTAACTGCTATTATAGGAGTACAATGAT TTTTGATTAGCCAGTAACAGATCCCTACTAAGGATTATAAAACCAA GTTACTTTAGGGATAACAGCGTTAT | 393 |
| DQ377 978 | Palinurus gilchristi | GGGGGACGATAAGACCCTATAAATCTTTATATTTATTATGATAACA TTAAATATTATAATTGAAAATTTAGTTTTACTGGAATAAAATATTTTG TTGGGGCGACAGGAGTATAAAAAATAACTGCTTTGTAATATGGGTT AATAATTTTTAATATGTAGTATAGTTGATCCTTCAGTGAGGATTTTA GAATAAGTTACTTTAGGGATAACAGCGTAAT | 394 |
| DQ377 977 | Palinurus mauritani cus | GGGGGACGATAAGACCCTATAAATCTTTATATTTATTATGATAACA TTAAATATTATAATTGAAAATTTAATTTTACTGTAATAGGATATTTTG TTGGGGCGACAGGAGTATAAAAAATAACTGCTTTGTAAGATGGGT TAATAATTTTTTAATGTGTAGTATATTTGATCCTTCAGTGAGGATTTT AGAATAAGTTACTTTAGGGATAACAGCGTAAT | 395 |
| DQ377 975 | Palinurus charlesto ni | GGGGGACGATAAGACCCTATAAATCTTTATATTTATTATGATAACA TTAAATATTATAATTGAAAATTTAGTTTTACTGGAATAAAATATTTTG TTGGGGCGACAGGAGTATAAAAAATAACTGCTTTATAAGATAGGTT AATAATTTTTAATATGTAGTATAGTTGATCCTTCAGTGAGGATTTTA GAATAAGTTACTTTAGGGATAACAGCGTAAT | 396 |
| AY947 836 | Pseudosq uilla ciliata | AGTGACGATAAGACCCTATGAAAGCTTTATGAGTGTGCTTCTTTTA TATGTAAAATGAATTTAATATTAACATATTAAAGAAATCACATTATTT TACTGGGGCGGTAAAAGTATAAATGATTAACTGCTTTTGTTATGCA AAACAATGATAATTGGTATGTAAATTGATCCATTAATAATGATTATA AAAACAAGTTACTTTAGGGATAACAGCGTAAT | 397 |
| AY351 259 | Pleuronc odes monodon | AAGGGGCGATAAGACCCTATAAATCTTTATATTAAATTGTTTTTAGT TTATTAATAATGTAATTTAAATAATAAGGCAAAATATATTGTGTTGG GGTGATGGGAATATAAAAAATTAACTGTTCTGAAATAAAAACAAAT TTATTTGATTTAATAATAGATCCTTTTTAAAGATTAAAAGTTTAAGTT ACTTTAGGGATAACAGCGCTAT | 398 |
| DQ388 060 | Portunus ventralis | AGGGGACGATAAGACCCTATAAAGCTTTATAAATCTATAAATTATT ACATATTTAAAATATAAAGGTAATCTTTTAGATATTTATTTGGTTGG GGCGACAGTGGTATAAATTAATTAACTGCTAATAAAAGTTACAATA ATTTTTGATTCTTAAGTTATAGATCCTCTCTAGGGATTAAAAAATCA AGTTACTTTAGGGATAACAGCGTTAT | 399 |
| DQ388 061 | Achelous spinicarp us | GGGGGACGATAAGACCCTATAAAGCTTTATAAATTTATAGACTTTA TATATTTAAAATAAAAATAATCTCTAAAACATTTATTTGGTTGGGGC GACAAAAGTATAATTTCAAATAACTGCTATTAAATTAACAATAATTT TTGTTTATGAATAATTGATCCTTCTATGAGGAGTAAAAGTTCAAGTT ACTTTAGGGATAACAGCGTCAT | 400 |
| DQ407 681 | Callinecte s toxotes | GAGGGACGATAAGACCCTATAAAGCTTTATAAATTTATTAATTTCG TTTATTTTTTATTATAATAATGATGATAAGAAAATTTATTTGGTTGGG GCAACCGAAGTATAATTTTAAATAACTGCTATAATTTAATACAATGA TTTTTGTTTTATATAATAATTGATCCTCTAAGAGATTAAAGACCAAG TTACTTTAGGGATAACAGCGTAAT | 401 |
| DQ407 680 | Callinecte s danae | GAGGGACGATAAGACCCTATAAAGCTTTATAAATTTATTAATTTCTT CTATTTTTTTATAATGAGGATGTTAAGAAGATTTATTTGGTTGGGG CAACCGAAGTATAATTAAAGATAACTGCTAATAGTTTATACAATGAT TTTTGTTTTAATAATAATTGATCCTTAAAAAGATTAAAGACCAAGTT ACTTTAGGGATAACAGCGTAAT | 402 |
| DQ407 679 | Callinecte s ornatus | GAGGGACGATAAGACCCTATAAAGCTTTATAAATTTATTGATTTCG TCTATTTTTCTTATAATAATGATATTGAGAAGATTTATTTGGTTGGG GCAACCGAAGTATAATTAAAGATAACTGCTACTATTTTATACAATG ATTTTTGTTTAAATGATAATTGATCCTTAAAAAGATTAAAGACCAAG TTACTTTAGGGATAACAGCGTAAT | 403 |
| DQ407 678 | Callinecte s marginatu s | GAGTGACGACAAGACCCTATAAAGCTTTATAAATTTATTTATTTCGT CTATTTTCTTTTATAATGATGATGTTAAGAAGGTTTATTTGGTTGGG GCAACCAGAGTATAATTAAAGATAACTGCTACAATTTAATACAATG ATTTTTGTTTATGAAATAATTGATCCTTGAAAAGATTAAAGATCAAG TTACTTTAGGGATAACAGCGTAAT | 404 |
| DQ407 677 | Callinecte s affinis | GAGGGACGATAAGACCCTATAAAGCTTTATAAATTTATTAATTTCG TTTATTTTCTATTATAATAATGATAATAAGAAGATTTATTTGGTTGG GGCAACCGAAGTATAAGTTTAAATAACTGCTATAATTTAATACAAT GATTTTTGTTTTATAAAATAATTGATCCTCGAAGAGATTAAAGACCA AGTTACTTTAGGGATAACAGCGTAAT | 405 |
| DQ407 673 | Callinecte s rathbuna e | GAGGGACGATAAGACCCTATAAAGCTTTATAAATTTATTAATTTCG TTTATTTTTTACTATAATGATGATAATAAGAAGATTTATTTGGTTGG GGCAACCGAAGTATAAGTTTAAATAACTGCTATAATTTAATACAAT GATTTTTGTTTTAAAAATAATTGATCCTCGAAGAGATTAAAGACCAA GTTACTTTAGGGATAACAGCGTAAT | 406 |
| DQ407 674 | Callinecte s bocourti | GAGGGACGATAAGACCCTATAAAGCTTTATAAATTTATTAATTTCG TTTATTTTCTATTATAATAATGATAATAAGAAGATTTATTTGGTTGG GGCAACCGAAGTATAAGTTTAAATAACTGCTATAATTTAATACAAT GATTTTTGTTTTAAAAAATAATTGATCCTCGAAGAGATTAAAGACCA AGTTACTTTAGGGATAACAGCGTAAT | 407 |
| DQ407 672 | Callinecte s similis | GAGGGACGATAAGACCCTATAAAGCTTTATAAATTTATTAATTTCTT CTATTTTTTTATAATGATGATGTTAAGAAGATTTATTTGGTTGGGGC AACCGAAGTATAATTAAAGATAACTGCTAATATTTTATACAATGATT TTTGTTTTAATAATAATTGATCCTTAAAAAGATTAAAGACCAAGTTA CTTTAGGGATAACAGCGTAAT | 408 |
| DQ407 671 | Callinecte s bellicosus | GAGGGACGATAAGACCCTATAAAGCTTTATAAATTTATTAATTTCG TCTATTTTTTTATAATGATGATGTTGAAAAGATTTATTTGGTTGGGG CAACCGAAGTATAATTTTTAATAACTGCTATAACTTAATACAATGAT TTTTGTTTGTTATTATAATTGATCCTTAAAAAGATTAAAGACCAAGT TACTTTAGGGATAACAGCGTAAT | 409 |
| DQ407 669 | Callinecte s arcuatus | GAGGGACGATAAGACCCTATAAAGCTTTATAAATTTATTAATTTTGT CTATTTTTCTTATAATGATGATGTTAAGAAGATTTATTTGGTTGGGG CAACCGAAGTATAATTAAAGATAACTGCTACTATTTTATACAATGAT TTTTGTTTTAATAATAATTGATCCTTAAAAAGATTAAAGACCAAGTT ACTTTAGGGATAACAGCGTAAT | 410 |
| EU186 116 | Metaneph rops armatus | GAGGGACGATAAGACCCTATAAAGCTTGATAATTTTATGTATAAAT AAATAAGTTGTTAGTGTTATATTGTTTATCTGTAAAATTATTTCGTT GGGGCGACGATAATATAATTTGTAACTGTTTGGGGGTTAGATTCA AATATGTTTGTGTGTTAATGATCCATTAATTGTTGATTAAAAATTTA AGTTACTTTAGGGATAACAGCGTTAT | 411 |
| EU186 114 | Metaneph rops moz ambicus | GAGGGACGATAAGACCCTATAAAGCTTGATAATTTTATATGTAAAT AAATAAGTTGTTAGTGTTATATTGTTTACTTACAAAATTATTTCGTT GGGGCGACGATAATATAATTTGTAACTGTTTGAGGTTTAGATCCAA ATATATTTGTGTTTAAGTGATCCATTAATTGTTGATTAAAAATTTAA GTTACTTTAGGGATAACAGCGTTAT | 412 |
| EU186 115 | Metaneph rops japonicus | GAGGGACGATAAGACCCTATAAAGCTTGATAATTTTATATATAAAT AAATAAGTTGTTAGTGTTATATTGTTTATCTGTAAAATTATTTCGTT GGGGCGACGATAATATAATTTGTAACTGTTTGGGGGTTAGATTCA AATATGTTTGTGTGTTAATGATCCATTAATTGTTGATTAGAAATTTA AGTTACTTTAGGGATAACAGCGTTAT | 413 |
| EU186 113 | Metaneph rops anda manicus | GAGGGACGATAAGACCCTATAAAGCTTGATAATTTTATATGTAAAT AAATAAGTTGTTAGTGTTATATTGTTTATTTATAAAATTATTTCGTTG GGGCGACGATAATATAATTTGTAACTGTTTGAGGTTTAGATCCAAA TATATTTGTGTTTAAATGATCCATTAATTGTTGATTAAAAATTTAAGT TACTTTAGGGATAACAGCGTTAT | 414 |
| EU186 112 | Metaneph rops velut inus | GAGGGACGATAAGACCCTATAAAGCTTGATAATTTTATATGTAAAT AAATAAGTTGTTAGTGTTATATTGTTTATTTATAAAATTATTTCGTTG GGGCGACGATAATATAATTTGTAACTGTTTGAGGTTTAGATCCAAA TATATTTGTGTTTAAATGATCCATTAATTGTTGATTAAAAATTTAAGT TACTTTAGGGAAAACAGCGTTAT | 415 |
| EU186 111 | Metaneph rops saga miensis | GAGGGACGATAAGACCCTATAAAGCTTGATAATTTTATATGTAAAT AAATAAGTTGTTAGTGTTATATTGTTTATTTATAAAATTATTTCGTTG GGGCGACGATAATATAATTTGTAACTGTTTGAGGTTTAGATCCAAA TATATTTGTGTTTAAATGATCCATTAATTGTTGATTAAAAATTTAAGT TACTTTAGGGATAACAGCGTTAT | 416 |
| EU186 109 | Metaneph rops bing hami | AAGGGACGATAAGACCCTATAAAGCTTAATAATTTTTGTAAGTAAA ATAAATAAATTGTTAGTGATATGCTGCTTACTTACTAAATTATTTCG TTGGGGTGACGAGGATATAATTTTGTAACTGTTTTAGTTTAAATTC AGGGATACTTGTACTCAATGATCCATTTATTTGATGATTAAAAATTT AAGTTACTTTAGGGATAACAGCGTTAT | 417 |
| EF599 137 | Parastac us pugnax | AAGGGACGATAAGACCCTATAAAGCTTTACATTAAGCGTTTAAAAT TTTAATTAAGTAATAAAATTTTATTAAATGTAATATGTTTTGTTGGG GCGACAAGAATATAAAAAATTTAACTGTTCTAGATAATAAGTCAGA AATATCTGATCTTTGAGGACTTTTTATTAAAGTATTAGATTAAGTTA CTTTAGGGATAACAGCGTTAT | 418 |
| EF060 259 | Paraneph rops zealandic us | GAGGGACGATAAGACCCTATAAAGCTTTACATTGAATTTATTAAAA AGTAATTTAAATAATAAAAGTTTATTAGTAAGGAGATGTTTTGTTGG GGTGACAAGAATATAATAAATATAACTGTTCTTTTTTAGTTCAAAGA TATTTGAAAAGGAGATCCTTAATAAAGATGTTAGAGTAAGTTACTTT AGGGATAACAGCCGTAA | 419 |
| DQ407 682 | Callinecte s exasperat us | GAGGGACGATAAGACCCTATAAAGCTTTATAAATTTATTAATTTCG TTTATTTTTTTATAATGATGATGTTAAGAAAGTTTATTTGGTTGGGG CAACCTAAGTATAATTAAAGATAACTGCTACGATTTTATACAATGAT TTTTGTTTTAATAGTAATTGATCCTTGAAAAGATTAAAGACCAAGTT ACTTTAGGGATAACAGCGTAAT | 420 |
| FJ174 903 | Palinurus barbarae | GGGGGACGATAAGACCCTATAAATCTTTATATTTATTATGATAACA TTAAATATTATAATTGAAAATTTAGTTTTACTGGAATAAAATATTTTG TTGGGGCGACAGGAGTATAAAAAAATAACTGCTTTGTAATATGGGTT AATAATTTTTAATATGTAGTATAGTTGATCCTTCAGTGAGGATTTTA GAATAAGTTACTTTAGGGATAACAGCGTAAT | 421 |
| NC_02 2736 | Sagmaria sus verreauxi | GGGGGACGATAAGACCCTATAAATCTTTACTCTGGAGACAATAAC ATTGAAGATTACTGCTAATAATTCCGTGTTATTGTTTTTAGTGTTTT GTTGGGGCGACAGGAGTATAAATAAATAACTGCTTTAAACGTATA GTTAATTATAGTTAAGTTGTTGATAAGTGATCCTTTAGTATGGATTA TAGATTAAGTTACTTTAGGGATAACAGCGTAAT | 422 |
| EU882 877 | Metaneph rops rubel lus | AAGGGACGATAAGACCCTGTAAAGCTTAATAATTCTACGAGTGGA TAAATAAATTGTTTAGTGGCAATGTTACTCACTTGTTAAATTATTTC GTTGGGGTGACGAGGATATAATTTGTAACTGTCTAAATTTAAAGTC AAGGATGCTTGTATTCGATGATCCATAATTGGTGATTAAAAATTTA AGTTACTTTAGGGATAACAGCGTTAT | 423 |
| EU186 128 | Metaneph rops chall engeri | GAGGGACGATAAGACCCTATAAAGCTTGATAATTTTATATATAATT AAATAAATTATTAGTATTATATTATTTATATATTAAATTATTTCGTTG GGGCGACGATAATATAATTTGTAACTGTTTGAAATTTTAATTCAGA AATATTTGTTTTTAATGATCCTTTTATTATTGATTAAAAATTTAAGTT ACTTTAGGGATAACAGCGTTAT | 424 |
| EU186 125 | Metaneph rops neptunus | GAGGGACGATAAGACCCTATAAAGCTTAATAATTTTGTATATAGAT AAATAAGTTGTTAGTATTATATTGTCTATTTATTAAATTATTTCGTTG GGGCGACGATAATATAATTTGTAACTGTTTGAAATATTTAATTCAG AAGTATTTGTATTTAATGATCCTTTTGTTGTTGATTAAAAATTTAAGT TACTTTAGGGATAACAGCGTTAT | 425 |
| EU186 124 | Metaneph rops austr aliensis | AAGGGACGATAAGACCCTATAAAGCTTGATAATTTTATATATAAGT AAATAAGTTGTTAGTATTATATTGTTTATATATAAAATTATTTCGTTG GGGCGACGATAATATAATTTGTAACTGTTTAATGTTTTGATTCAAAT ATATTTGTGTTTAATGATCCATTAATTGTTGATTAAAAATTTAAGTTA CTTTAGGGATAACAGCGTTAT | 426 |
| EU186 123 | Metaneph rops araf urensis | AAGGGACGATAAGACCCTATAAAGCTTGATAATTTTATATATAAGT AAATAAGTTGTTAGTGTTATATTGTTTATATATAAAATTATTTCGTTG GGGCGACGATAATATAATTTGTAACTGTTTAATGTTTTGATTCAAAT ATATTTGTGTTTAATGATCCATTAATTGTTGATTAAAAATTTAAGTTA CTTTAGGGATAACAGCGTTAT | 427 |
| EU186 121 | Metaneph rops bosc hmai | GAGGGACGATAAGACCCTATAAAGCTTGATAATTTTATATATAAAT AAATAGGTTGTTAGTGTTATATTGTCTATTTATAAAATTATTTCGTT GGGGCGACGATGATATAATTTGTAACTGTTTAAGGTTTTGATTCAA ATATATTTGTATTTAATGATCCATTTTTTGTTGATTAAAAATTTAAGTT ACTTTAGGGATAACAGCGTTAT | 428 |
| EU186 117 | Metaneph rops form osanus | GAGGGACGATAAGACCCTATAAAGCTTGATAATTTTATATATAAAT AAATAAGTTGTTAGTGTTATATCGTTTATTTATAAAATTATTTCGTTG GGGCGACGATAATATAATTTGTAACTGTTTAGGTTTAGATTCAAAT ATATTTGTGTTTTAATGATCCATTAATTGTTGATTAAAAATTTAAGTT ACTTTAGGGATAACAGCGTAAT | 429 |
| EU186 118 | Metaneph rops sine nsis | GAGGGACGATAAGACCCTATAAAGCTAGATAATTTTGTATTTAAAT AAATAAGTTGTTAGTGTTATATCATTTATTTATAAAATTATTTCGTTG GGGCGACGATAATATAATTTGTAACTGTTTAGGTTTAGATTCAAAT ATATTTGTGTTTTTATGATCCATTAATTGTTGATTAAAAATTTAAGTTA CTTTAGGGATAACAGCGTTAT | 430 |
| FJ462 647 | Lithodes f erox | CAAAAAGACGATAAGACCCTATAAATCTTAACAATAAATATATTTTA TATTTTAGTTTATAAGTGTATAAGGAATAAAAATATAGGTTTGTTAC GCTGGGGCGGCGTAGATATATAAATAAACTGTCTATAGTTTAAATA CAATAATCATTGCTTAATACAAATTGATCCTTAAATAGATTAAAAGA TTAAGATACTTTAGGGATAACAGCGTTAT | 431 |
| FJ224 280 | Oratosqui llina interru pta | GAGGGACGATAAGACCCTATAAAGCTTTATGTTACATTTCTTTTTT ATAAATTGTATTGTTATTAACTTATTTTAGAATTTGTATCATTTTACT GGGGCGGTAAAAGTATAATAAAGATTAACTGCTTTTGTTTTGAATA ACAATTTTAGTTGGAAATAAATTGATCCATTATTAATGATCATAAGA ACAAGTTACTTTAGGGATAACAGCGTAAT | 432 |
| FJ224 282 | Odontoda ctylus japonicus | GAGGGACGATAAGACCCTATAAAGCTTTATAGTGTAATTATTTAAT ATGAAATATATTAACTTGAAATTATTTAATAAAAAGCACTATTTTACT GGGGCGGTAAAAGTATAACTTAAACTAACTGCTTTTATTTTTAAAA ACAATTATAATTGAATATAAATTGATCCATTATTAATGATCACAAGA ACAAGTTACTTTAGGGATAACAGCGTTAT | 433 |
| FJ224 271 | Miyakella nepa | GAGGGACGATAAGACCCTATAAAGCTTTATGGTATTTTTCTTTTTT ATAAATTGTATTGTTATTAACTTATTTTAGAATTTTTATCATTTTACT GGGGCGGTAAAAGTATAATTAGATTAACTGCTTTTGTTTTAAATAA CAATTTTAGTTGGAAATAAATTGATCCATTATTAATGATCATAAGAA CAAGTTACTTTAGGGATAACAGCGTAAT | 434 |
| FJ224 263 | Erugosqu illa woodmas oni | GAGGGACGATAAGACCCTATAAAGCTTTATGATACTTTTCTTTTTT ACAAATTATCTCGTTATTAACTTATTTTAGAATTAATATCATTTTACT GGGGCGGTAAAAGTATAATAAAAAGATTAACTGCTTTTATTTTGTT GAACAATGTTAATTGGAAATAAACTGATCCATTATTAATGATCATAA GAACAAGTTACTTTAGGGATAACAGCGTAAT | 435 |
| FJ224 255 | Clorida decorata | AAGGGACGATAAGACCCTATAAAGCTTTATAATATATTTCTTTTACA TAAATTGTTTTGTTATTAACGTGTTTTAGATTATATATTATTTTACTG GGGCGGTAAAAGTATAATAAAAGATTAACTGCTTTTATTTTGTGTA ACAATTGTAGTTGGAAATAAATTGATCCATTATTAATGATTATAAAA ACAAGTTACTTTAGGGATAACAGCATAAT | 436 |
| FJ224 261 | Dictyosqu illa foveolata | GAGGGACGATAAGACCCTATAAAGCTTTATGGTATTCTTCTTTTTT ATAAATTGTTTTGTTATTAACTTATTTTAGAATTTTTATCATTTTACT GGGGCGGTAAAAGTATAATAAAAGATTAACTGCTTTTATTTTGTGT AACAATTTTATTTGGAAATAAATTGATCCATTATTAATGATTATAAG AACAAGTTACTTTAGGGATAACAGCGTTAT | 437 |
| FJ224 251 | Anchisqui lla fasciata | GAGGGACGATAAGACCCTATAAAGCTTTATAGTATATTTCTTTTGT ATAAATTATTTTGTTACTAACATATTTTAGAACATATATTATTTTACT GGGGCGGTAAAAGTATAATAAAGACTAACTGCTTTTATTTTGAATA ACAATTGTAGTTGGAAATAAATTGATCCATTATTAATGATTATAAAA ACAAGTTACTTTAGGGATAACAGCGTAAT | 438 |
| FJ174 906 | Scylla ride s herklotsii | GAGGGACGATAAGACCCTATAAATCTTTATATTTTGGGTTATAAGG TTAAAAATTAGGTTTTCATTTAATTTTATAAAGTTAAATATTTTGTTG GGGAGACAGAGGTATAAAGAAAGATAACTGCCTTAAAATTTATAGT TAAGAATAGTTAAGATAGAAAGTGATCCTTTAATAAGGAAATTAGA ATAAGTTACTTTAGGGATAACAGCGTAAT | 439 |
| FJ174 904 | Palinurus delagoae | GGGGGACGATAAGACCCTATAAATCTTTATATTTATTATGATAACA TTAAATATTATAATTGAAAATTTAGTTTTACTGGAATAAAATATTTTG TTGGGGCGACAGGAGTATAAAAAATAACTGCTTTGTAATATGGGTT AATAATTTTTAATATGTAGTATAGTTGATCCTTCAGTGAGGATTTTA GAATAAGTTACTTTAGGGATAACAGCGTAAT | 440 |
| GU727 618 | Astacus astacus | TAAAGGGACGATAAGACCCTATAAAACTTTATATTTAAAAATAATTG CTATTATTATTTAAAGAGTTTATTTTTAAATATTTTATTGGGGTGATA AGGATAAAATATAAGACAACTGTCTTTTATTTTAACAATAATTTTTG AGTAAATGATCTTAATAAAGGAATAAAAGATTAAGTTACTTTAGGG ATAACAGCGTAAT | 441 |
| FM208 780 | Portunus hastatus | GGGGGACGATAAGACCCTATAAAGCTTTATAAATCCATAAATTATT ATATATTTAAGATGATAAAAGTAATCTTTTAGATATTTATTTGGTTG GGGCGACAGTGGTATAAATCAATTAACTGCTAATAAAAATTACAAT AATTTTTGATTTCTAAGTTATAGATCCTCTTTAGGGATTAAAAATC AAGTTACTTTAGGGATAACAGCGTTAT | 442 |
| FM208 751 | Achelous ordwayi | GGGGGACGATAAGACCCTATAAAGCTTTATAAATCTACAAATTTTA TATATTTGTGAATAAAAATAATTTTTCTAATATTTATTTGGTTGGGG CGACAAAAGTGTAATTAAAATAACCGCTAATAATTTGTTACAATGAT TTTTGTTTGGCGAAGTTAATGGATCCCTTTTAAGGATTAAAAAATC AAGTTACTTTAGGGATAACAGCGTTAT | 443 |
| FM208 763 | Carcinus maenas | GGGGGACGATAAGACCCTATAAAACTTTATATATGCAACAATACAG TTGAATTAAAGCATAAAAACTTATTCGATAAAATATATTTAGTTGGG GCGACTGAGGTATAATTTATAGTAACTGATTAAAAGCAAGACAAAT AATATTTGATTAACTTTAATTGATCCTTTTTTAAAGATTAAAAGATTAA GTTACTTTAGGGATAACAGCGTAAT | 444 |
| FM208 752 | Portunus inaequali s | GGGGGACGATAAGACCCTATAAAGCTTTATAAATCTATAAATTATT ATATATTTAAAATAATAAAAGTAATTTTTTAGATATTTATTTGGTTGG GGCGACAGTGGTATAAATTAATTAACTGCTAATAAAAATTACAATA ATTTTTGATTTTTTAAGTCATAGATCCTCTATAGGGATTAAAAAATCA AGTTACTTTAGGGATAACAGCGTTAT | 445 |
| FJ965 952 | Astacoide s madagas cariensis | AAGGGACGATAAGACCCTATAAAACTTTATATTAGAATTATTTAAAA ACTATTTAGATAGAAAGGTAAGTAAGTAGTAAAATATTTTGTTGGG GCGACAAGAATATAAGAATATAACTGTTCTTAAGTATAATCAAAAAT TTTTGATTTAATGATCCTAGGTAAAGATATTAGAATAAGTTACTTTA GGGATAACAGCGTAAT | 446 |
| FM207 657 | Erimacru s isenbecki | AGGGGACGATAAGACCCTATAAAGCTTTACATACATTATAAGTTTG ATTTAAGCAAAATTTAAAAATCTAAATTATATATTATGTTTTGTTGG GGCGACAAAAATATAAGTAATAATAACTGTTTAATAAAAAAACAATA ATAAATGGACAATTAAATGATCCTTGTTTAAAGATTAAAGACCAAG TTACTTTAGGGATAACAGCGTTAT | 447 |
| FJ871 141 | Hemisquil la australien sis | GGGGGACGATAAGACCCTATAAAGCTTTATGATAAGGCCTTTTTG TAAAGAATTTCTATTGTGAAATTACTAAGGGGGTTTATATTATTTTA CTGGGGCGGTAAAAGTATATAATTGAATAACTGCTTTTATTATTGA | 448 |
| | | AAACAATTATAATTGAAATACAAAATGATCCATTATTAATGATTATA AAAACAAGTTACTTTAGGGATAACAGCGTAAT | |
| FJ871 139 | Austrosq uilla tsangi | AAGGGACGATAAGACCCTATAAAGCTTTATAGTGTAACTCTTTTTT ACTAGTTTTACTAATATTAAAATATTAAAGAAATAATACTATTTTACT GGGGCGGTGAGAGTATATAAAAATAACTGCTTTTATTTTATTAAAC AATAATAATTGAAAAATTAATTGATCCATTTCTAATGATTATAAGAA CAAGTTACTTTAGGGATAACAGCGTAAT | 449 |
| FJ462 648 | Lithodes confunde ns | CAAAAAGACGATAAGACCCTATAAATCTTTACAATAAATATATTTTA TATTTTAACTTATAAGTGTATAAGAAATAAAAATATAGATTTGTTTC GCTGGGGCGGCGTAGATATATAAATAAACTGTCTATAATTTAAATA CAGTAATTATTGCTTAATATAAATTGATCCTTAAATAGATTAAAAGA TTAAGATACTTTAGGGATAACAGCGTTAT | 450 |
| HM138 821 | Fallosquill a fallax | GAGGGACGATAAGACCCTATAAAGCTTTATGGTATACCTCTTTTAT AAAAATTGTTCTTTATTAACTTATTTTAGATTGTGTATTATTTTACTG GGGCGGTAAAAGTATAATTAAGAGTAACTGCTTTTATTTTGTATGA CAATTATAGTTGAAAATAAATTGATCCATTATTAATGATTACAAAAA CAAGTTACTTTAGGGATAACAGCGTAAT | 451 |
| HM138 820 | Echinosq uilla guerinii | GAGGGACGATAAGACCCTATAAAGCTTTATGGTAGTTTCTTTTTAT ATAGATTGGTAAAATTTAATTTATTAAAAGAAAAATATCATTCTACT GGGGCGGTAAAAGTATATCTTTGGTTAACTGCTTTTGTTTTGTAAG TCAATAATAGTTGAAATATTAATTGATCCATTATTAATGATCATAAG AACAAGTTACTTTAGGGATAACAGCGTAAT | 452 |
| HM138 819 | Coronis scolopen dra | GAGGGACGATAAGACCCTATAAAGCTTTATAATACTATCTTTTCAT ATTAATTATCTGGTTATTAACGTACTAAAAGAAAAGTATTATTTTAC TGGGGCGGTAAAAGTATATAAAAGAATAACTGCTTTTGTTTTACAA AACAACTATATTTGAAAAATAAATTGATCCATTATTAATGATCACAA GAACAAGTTACTTTAGGGATAACAGCGTAAT | 453 |
| HM138 818 | Chorisquil la tweediei | GAGGGACGATAAGACCCTATAAAGCTTTATGATATTTTCTTTTTATA TTAGTTAGTAAAATGTAGTTTATTAAAAGAAAAATATTATTTTACTG GGGCGGTAGAAGTATATTTTTGATTAACTGCTTCTGTTTTGTAAGT CAATTATAATTGAAATATGAATTGATCCATTATTAATGATCATAAGG ACAAGTTACTTTAGGGATAACAGCGTAAT | 454 |
| HM138 817 | Chorisquil la hystrix | GAGGGACGATAAGACCCTATAAAGCTTTATAATTATTCTTTTTTATA TTAGTTAGTAGAATGTAGTTTATTAAAAAGAAATGATTATTTTACTG GGGCGGTAAAAGTATATTTTGATCAACTGCTTTTGTTTTGTAAATC AATTATAATTGAAATATAAATTGATCCATTATTAATGATCATAAGAA CAAGTTACTTTAGGGATAACAGCGTAAT | 455 |
| HM138 816 | Chorisquil la excavata | GAGGGACGATAAGACCCTATAAAGCTTTATGGTACTTCTTTTTTAT ATTAATTAGGAAAATGTAGTTTATTAAAAAGAAAATACTATTTTACT GGGGCGGTAAAAGTATATTTTTGATTAACTGCTTTTGTTTTGTAAG TCAATAATAATTGAAATATTAATTGATCCATTATTAATGATCACAAG AACAAGTTACTTTAGGGATAACAGCGTAAT | 456 |
| HM138 815 | Busquilla plantei | GAGGGACGATAAGACCCTATAAAGCTTTATGATATTTCTTTTTCTT ATAAATTTTCTTGTTATTAACTTATTTTAGAGCTAATATCATTTTACT GGGGCGGTAAAAGTATAATAAAAGATTAACTGCTTTTGTTTTGTAT AACAATCTTAGTTGGAAATAAATTGATCCATTATTAATGATCATAAG AACAAGTTACTTTAGGGATAACAGCGTAAT | 457 |
| HM138 814 | Alima pacifica | GAGGGACGATAAGACCCTATAAAGCTTTATAATACACGCCTTCTTT ATGAAATATAAAATTATCAAAATATAATAGAACTTGTATTATTTTACT GGGGCGGTAAAAGTATAATAAACTAACTGCTTTTGTCCTGTATAAC AATCTTAATTGAAAATAAATTGATCCATTATTAATGATCACAAGAAC AAGTTACTTTAGGGATAACAGCGTAAT | 458 |
| HM138 813 | Alima orientalis | GAGGGACGATAAGACCCTATAAAGCTTTATAATACAATTCTTTTTT ATAAATTATCGCGTTATTAACCTATCTTAGAATTCGTATTATTTTAC TGGGGCGGTAAAAGTATATTAAAGACTAACTGCTTTTATTTTGTGT AACAATTTTAGTTGGAAATAAATTGATCCATTATTAATGATCACAAG AACAAGTTACTTTAGGGATAACAGCGTAAT | 459 |
| HM138 812 | Alachosq uilla vicina | AAATGACGATAAGACCCTATAAAGCTTTATAATTTTCTCTTTCTATG TAAAGTATAAGAATATTAAAACATTGAAAAGAGTAAATTATTTTACT GGGGCGGTAAAAGTATAAAAAATAACTGCTTTTATTTTGAATAACA ACTATTATTGGAATTTGAATTGATCCATTAATAATGATCACAAGAAC AAGTTACTTTAGGGATAACAGCGTAAT | 460 |
| HM138 822 | Gonodact ylellus espinosu s | GAGTGACGATAAGACCCTATAAAGCTTTATGGTATACCTTTTTTAT ACAAATTACATATTATTAACCTATTAAAAAGTAATATCATTTTACTG GGGCGGTAAAAGTATATTCGCTTAACTGCTTTTATTATTTAAAACA ATAATAATTGAACAATCCATTGATCCATTAATAATGATCATAAGGCC AAGTTACTTTAGGGATAACAGCGTAAT | 461 |
| HM138 823 | Gonodact ylellus affinis | GAGGGACGATAAGACCCTATAAAGCTTTATAATACATTTCTTTTAC ATAAATTATTAATTATTAACTTGTTAAAGAAATAATATTATTTTACTG GGGCGGTAAAAGTATATTTTATTTGAACAACTGCTTTTGTTTTAAAA AACAATTATAATTGAACAACTCATTGATCCATTAATAATGATCATAA GGCCAAGTTACTTTAGGGATAACAGCGTAAT | 462 |
| HM138 833 | Kempella mikado | GAGGGACGATAAGACCCTATAAAGCTTTATAATATTTTTCTTTCTTA TAAATTATATTGTTATTAACATATTTTAGAATTTGTATTATTTTACTG GGGCGGTAAAAGTATAATAAAATAACTGCTTTTATTTTGTATAACAA TCTTAATTGGTAATAAATTGATCCATTATTAATGATTATAAGAACAA GTTACTTTAGGGATAACAGCGTAAT | 463 |
| HM138 832 | Hemisquil la californie nsis | GGGGGACGATAAGACCCTATAAAGCTTTATGATAAGGCCTTTTTG TAAAGAATTTCTATTGTGAAATTACTAGGGGGTTTATATCATTTTAC TGGGGCGGTAAAAGTATATAATTGAATAACTGCTTTTATTATTGAA AACAATTATAATTGAAATACAAAATGATCCATTATTAATGATTATAA AAACAAGTTACTTTAGGGATAACAGCGTAAT | 464 |
| HM138 831 | Haptosqu illa trispinosa | GAGGGACGATAAGACCCTATAAAGCTTTATGATAGTTTCTTTTTAT AATAATTAGTAAAATGTAATTTATTAAAAGAAATATATTATTTTACTG GGGCGGTAAAAGTATATTTTTGATTAACTGCTTTTGTTTTGCAAAA CAATAATAATTGAATTATAAATTGATCCATTATTAATGATCATAAGA ACAAGTTACTTTAGGGATAACAGCGTAAT | 465 |
| HM138 830 | Haptosqu illa glyptocer cus | GAGTGACGATAAGACCCTATAAAGCTTTATGGTAGTTTCTTTTTAT AGTAATTAGTATAATGTAATTTATTAAAGGGAAATATATCATTTTAC TGGGGCGGTAAAAGTATATTTTTGATTAACTGCTTTTGTTTTGTAAA ACAATGATAATTGAATTATAAATTGATCCATTATTAATGATTACAAG AACAAGTTACTTTAGGGATAACAGCGTAAT | 466 |
| HM138 828 | Gonodact ylus platysom a | GAGGGACGATAAGACCCTATAAAGCTTTATGTTATATATTCTTTTAT GTAATCTATAATTATTAACTCATTGAAGAAATAATATCATTTTACTG GGGCGGTAAAAGTATATTTGAATAACTGCTTTTGTTATAAAAAACA ATAATAATTGAGTAATCCATTGATCCATTATTAATGATCATAAGACC AAGTTACTTTAGGGATAACAGCGTAAT | 467 |
| HM138 827 | Gonodact ylaceus falcatus | GAGGGACGATAAGACCCTATAAAGCTTTATAGTAATACCTTTTTAT TTAAAATATAAAATGTTAACTTGTTAAAAGAGTATTATTATTTTACTG GGGCGGTAAAAGTATATAATGATTAACTGCTTTTGTCTTACTAAAC AATAATAATTGAATAATTATGTGATCCATTATTAATGATCAAAAGTA CAAGTTACTTTAGGGATAACAGCGTAAT | 468 |
| HM138 825 | Gonodact ylus childi | GAGGGACGATAAGACCCTATAAAGCTTTATAATATAATTCTTTGAT ATGAAGTGTTACTTATTAATTTATTAAAGAAATACTATTATTTTACTG GGGCGGTAAAAGTATATATTTGGAGTAACTGCTTTTGTTATTTTAA ACAATAATAATTGAGTGTCATATTGATCCACTAATAGTGAATAAAA GAACAAGTTACTTTAGGGATAACAGCGTAAT | 469 |
| HM138 824 | Gonodact ylellus annularis | GAGGGACGATAAGACCCTATAAAGCTTTATAATACGTTTCTCTTAC ACAAATTATAAATTGTTAACTTGTTAAAGAATAAGTATTATTTTACT GGGGCGGTAAAAGTATACCATAGACTAACTGCTTTTGTTATTAAAA ACAATAATAATTGGATAATCGATTGATCCATTAATAATGATCATAAG GCCAAGTTACTTTAGGGATAACAGCGTAAT | 470 |
| HM138 842 | Odontoda ctylus scyllarus | GAGGGACGATAAGACCCTATAAAGCTTTATGATACAACTATTTCTT ATAAAATATCTTTTAAATCACTTAATAGAATGTATTATTTTACTGGG GCGGTAAAAGTATAATGTATTATTAACTGCTTTTGTTTTTAAAAACA ATCATAATTGGAAACAAATTGATCCATTATTAATGATCACAAGAACA AGTTACTTTAGGGATAACAGCGTTAT | 471 |
| HM138 841 | Odontoda ctylus latirostris | AAGGGACGATAAGACCCTATAAAGCTTTATAATAAAATCATCTTCT ATGGAATAAGTGTTATTAAATTAGCTGATGAGGTATATTATTTTACT GGGGCGGTAAAAGTATAAACGATTAACTGCTTTTGTTCTTAAAAAC AATTATAATTGAAAACATATTGATCCATTATTAATGATCACAAGAAC AAGTTACTTTAGGGATAACAGCGTTAT | 472 |
| HM138 840 | Odontoda ctylus havanens is | AAGGGACGATAAGACCCTATAAAGCTTTATGATAAAGTTATCCTCT ACGGAATAAACATTATTAAACTAGCTGATAACGCATATCATTTTACT GGGGCGGTAAAAGTATAATAAGATTAACTGCTTTTGTTTTTACAAA CAATTATAATTGAAAACAGATTGATCCATTATTAATGATCACAAGAA CAAGTTACTTTAGGGATAACAGCGTTAT | 473 |
| HM138 839 | Odontoda ctylus cultrifer | GAGGGACGATAAGACCCTATAAAGCTTTATATTACATATATTTTATA TAAAATGTTTATTATTAAATTATTTAATGAAATGTATTATTTTACTGG GGCGGTAAAAGTATATTTGAGTAACTGCTTTTGCTTTTAAAAACAA TCATAATTGAAAGTAAATTGATCCATTATTAATGATCACAAGAACAA GTTACTTTAGGGATAACAGCGTTAT | 474 |
| HM138 838 | Neogono dactylus oerstedii | GAGGGACGATAAGACCCTATAAAGCTTTATGGTAATATTTTTGTAT ATAAAATATTTCATGTAACTTATTAAAAAATAAACACCATTTTACTG GGGCGGTAAAAGTATATTTTTGATTAACTGCTTTTGTTGTTGAAAA CAGTTGTAGCTGAATAATAAATTGATCCATTATTAATGATCACAAGT ACAAGTTACTTTAGGGATAACAGCGTAAT | 475 |
| HM138 837 | Neogono dactylus bredini | GAGGGACGATAAGACCCTATAAAGCTTTATGGTAAAATTTTTATAT ATAAAATATTTCGTGTAACTTATTAAAAAGTAAATACCATTTTACTG GGGCGGTAAAAGTATATCTTGATTAACTGCTTTTATTATTAAAAACA GTTATAGCTGAATAATAAATTGATCCATTACTAATGTTCATAAGTTC AAGTTACTTTAGGGATAACAGCGTAAT | 476 |
| HM138 836 | Neogono dactylus bahiahon densis | GAGGGACGATAAGACCCTATAAAGCTTTATGGTAAAATTTTTGTAT ATAAATTATATAATATTAAATTATTAAAAAATAAATACCATTTTACTG GGGCGGTAAAAGTATATTTTTGATTAACTGCTTTTGTTATTAAAAAC AGTCGTAGCTGAATAATCAGTTGATCCATTATTAATGATCATAAGT TCAAGTTACTTTAGGGATAACAGCGTAAT | 477 |
| HM138 835 | Lysiosquil lina sulcata | AAGGGACGATAAGACCCTATAAAGCTTTATGATTACAATCTTTTAG TGTTGATTATTTTAATATTAAAGTATTAGAAAATTATATCATTTTACT GGGGCGGTAAAAGTATAAAGGAATAACTGCTTTTGTTGAGATTAAA CAACGGTAGTTGGAAAATAAATTGATCCATTAATAATGATCACAAG AACAAGTTACTTTAGGGATAACAGCGTAAT | 478 |
| HM138 854 | Squilla rugosa | GAGGGACGATAAGACCCTATAAAGCTTTATAATATTTCTTTTTTATA TAAATTGTGTTATTGTTAACTTATTTTAAAGATTTTATTATTTTACTG GGGCGGTAAAAGTATATTAAAGATTAACTGCTTTTGTTTTTTATAAC AATCGTAATTGGAAATAAATTGATCCATTATTAATGATCACAAGAAC AAGTTACTTTAGGGATAACAGCGTAAT | 479 |
| HM138 852 | Raoulser enea hieroglyp hica | GAGTGACGATAAGACCCTATAAAGCTTTATAGTACTGTTTTTCTTT ATAAATTAATTTAGTATTAACATGTCTAAAAATGTCTACTATTTTACT GGGGCGGTAAGAGTATAATAGGTTAACTGCTTTTTGTATGTACAAC AATGATACTTGAAATATAATTGATCCACGATTAGTGATTATAAAACC AAGTTACTTTAGGGATAACAGCGTAAT | 480 |
| HM138 851 | Raoulser enea oxyrhync ha | GAGTGACGATAAGACCCTATAAAGCTTTATAGTATGAGTTTTTTTT ATAAATTAACGTGATATTAACGTACTTAAGTTTTCTTACTATTTTACT GGGGCGGTAAAAGTATAATGGATTAACTGCTTTTTCTGTGTAAAAC AAGGATGTTTGAAATATAATTGATCCACAATTAGTGATTATAAAACC AAGTTACTTTAGGGATAACAGCGTAAT | 481 |
| HM138 845 | Pseudosq uillopsis marmorat a | GAGGGACGATAAGACCCTATAAAGCTTTATAGTGTTTTTTTTTTATA TAAATTGTGCTAATTTAGTTTATAAAAAAAAAACATACTATTTTACT GGGGCGGTAAAAGTATTTATTATTGAAAAACTGCTTTTGTTTTTAAA AACAAATATGATTGGAAATAAATTGATCCATTATTAATGATTATAAA AACAAGTTACTTTAGGGATAACAGCGTAAT | 482 |
| HM138 850 | Raoulser enea ornata | GAGTGACGATAAGACCCTATAAAGCTTTATAGTACTGTTTTTCTTT ATAAATTAATTTAGTATTAACATGTCTAAAAATGTCTACTATTTTACT GGGGCGGTAAGAGTATAATAGATTAACTGCTTTTTGTATGTACAAC AATGATACTTGAAATATAATTGATCCACGATTAGTGATTATAAAACC AAGTTACTTTAGGGATAACAGCGTAAT | 483 |
| HM138 849 | Raoulser enea komaii | GAGTGACGATAAGACCCTATAAAGCTTTATAGTATGAGTTTTTTTT ATAAATTAACGTGATATTAACGTACTTAAGTTTTCTTACTATTTTACT GGGGCGGTAAAAGTATAATGGATTAACTGCTTTTTCTGTGTAAAAC AAGGATGTTTGAAATATAATTGATCCACAATTAGTGATTATAAAACT AAGTTACTTTAGGGATAACAGCGTAAT | 484 |
| HM138 843 | Protosquil la folini | AAGGGACGATAAGACCCTATAAAGCTTTATAGTGGCCTCTTTTTAT ATAAATTGGTAAAATATAGTTTATTAAAAGAAGAATACTATTTTACT GGGGCGGTAAAAGTATATTTTTGATTAACTGCTTTTGTTTTGAATG TCAATAATAGTTGAGTAATAAGTTGATCCATTATTAATGATCACAAG AACAAGTTACTTTAGGGATAACAGCGTAAT | 485 |
| NC_04 1153 | Ibacus alticrenat us | AGGGGACGATAAGACCCTATAAATCTTTATATTTTAAATTTGTTAGT TTAAAAAATGGTTTAAATTTTGATTTAATAAATTAAAATATTTTGTTG GGGAGACAGGAGTATAAAAAGTAACTGCTTTATAATTTAAGTTAAT AATATTTAGTTAAGTGATCCTTTAATATGGATTTTAGATTAAGTTAC TTTAGGGATAACAGCATAAT | 486 |
| JN701 692 | Scylla ride s nodifer | GAGGGACGATAAGACCCTATAAATCTTTATATTTTGCCTTATAAGG TTAAAAATTAGGTTCTTATTTAATTTTATAAGGTTAAATATTTTGTTG GGGAGACAGAGGCATAAAAAAAGTAACTGCCTTAAAGTTTAAGTT AAAAATGTTTAAGTTGTTAAGTGATCCTTTAATAAGGAAATTAGAAT AAGTTACTTTAGGGATAACAGCGTAAT | 487 |
| MN817 127 | Scylla ride s haanii | GAGGGACGATAAGACCCTATAAATCTTTATATTTTGGCTTATAAGG TTAAAAATTAGGTTATAATTTAGTTTTATAAGGTTGAATATTTTGTTG GGGAGACAGGAGTATAAAAAGAAATAACTGCTTTAGAATTAGAGTT AAAGGTGTTTAAGTTTAAAAGTGATCCTTTAATAAGGAATGTAGAT TAAGTTACTTTAGGGATAACAGCGTAAT | 488 |
| JN701 689 | Scyllaride s brasiliensi s | GAGGGACGATAAGACCCTATAAATCTTTATATTTTGGTTTATACGG TTAAAAGTTAGGTTATTATTTAATTTTATAAGTTTGAATATTTTGTTG GGGAGACAGGGGCATAAAAGGAAATAACTGCCTTAAAGTTTAAGT TAAAAATGTTTAAATTATTAAGTGATCCTTTAATAAAGAATATAGAT TAAGTTACTTTAGGGATAACAGCGTAAT | 489 |
| NC_05 0686 | Taku spinosoc arinatus | GAGGGACGATAAGACCCTATAAAGCTTTATAGTGTAGTTTTTTTAT ATTAATTATATATTTTAGTTTATTAAAAAAGTAATACTATTTTACTGG GGCGGTAAAAGTATATTTTTGATTAACTGCTTTTGTTTTTAAAAACA ATAATAATTGGTTAATAGATTGATCCATTATTAATGATCACAAGAAC AAGTTACTTTAGGGATAACAGCGTAAT | 490 |
| JN566 222 | Jasus frontalis | GGGGGACGATAAGACCCTATAAATCTTTATACTAGTTAGTAATAAC ATTAAAGATTAATTTTATATTTTTTTGTTAGTAGTTTTAGTATTTTGC TGGGGCGGCAGGGGTATAATTCAATAACTGCTCTATAGTTGTAGT TAATTATGTTTAAATTGTTGGTAGTTGATCCTTTAGTGGAGATTATA GATTAAGTTACTTTAGGGATAACAGCGCAAT | 491 |
| HM637 974 | Menippe mercenari a | GAGGGACGATAAGACCCTATAAAGCTTTATATGTTATTTAAATTTA ATTGAATTGATTTATAAATTTTAAATTTAGAGTAATTTATTTTGCTGG GGCGGCATAAGTATAAGTTATATTAACTGCTTAATAATGATACAAT AATAGTTGAATTGTTGTTAAATGATCCTTTTTAAAGATTTAAGAACA AGTTACTTTAGGGATAACAGCGTTAT | 492 |
| JF737 171 | Procamb arus paeninsul anus | AAGGGACGATAAGACCCTATAAAACTTTATATTTATAATATAGTAGT TAGTTTTATTTAATAGTACTATTTTAGAGTATTTGGTTGGGGTGACA AGGATAAAATATAAAATAACTGTCTTTTTTTTTTACAGTAATGTTTG TTTTAATGATCCTAAAAGGGATTAAAGATTAAGTTACTTTAGGGATA ACAGCGTAAT | 493 |
| JQ229 876 | Puerulus sewelli | GGGGGACGATAAGACCCTATAAATCTTTATATTTGCCGTAGAAAA GAGTGTGTATTAGGGCTAAAGATATATTATTTTGTTGGGGCGATAT TTTGTTGGGGCGACAAGGGTATAATGAGTAACTGCTTTAAAAGTTT AATTAGATAATATCTAGTTTGAGAATAAGAGATCCTTTACTAAGGAT TGTTAAAATAAGTTACTTTAGGGATAACAGCGTAAT | 494 |
| JQ229 873 | Panulirus polyphag us | GGGGGACGATAAGACCCTATAAATCTTTATGTTTTATCTAGTGAAT TTCAGAGATTAGTTACATATTTGGTGTTACTACGGTAAAATATTTTG TTGGGGCGACAGGAGTATAACAAGTAACTGCTTTGGATATAGAAT TAATTATTTTTATAGTGAGAAGTTAAATTGATCCTCTTCTGGAGAAA TCAGACTAAGTTACTTTAGGGATAACAGCGTAAT | 495 |
| MN817 128 | Panulirus longipes | GGGGGACGATAAGACCCTATAAATCTTGATGGTCAAAATATTAATT TTTATGTAGTAGTTATAAGCTTAAATTTAATACTTTGACTATTTTGTT GGGGCGACAGGGGTATAATTAGTAACTGCTTTGAGTAAAGAATTA ATTATTTTTATTGTGTTGTGTTTGAGATCCTTCTTTGAAGATATCAG ATCAAGTTACTTTAGGGATAACAGCGTAAT | 496 |
| MT533 488 | Panulirus penicillatu s | GGGGGACGATAAGACCCTATAAATCTTTATGTTTTAACTAATAAAT TTTAGGGATTAGTTATATACCTAGTATTATTAGTTGAAATATTTTGT TGGGGCGACAGGAGTATAATAAGTAACTGCTTTGTTTATAAAATTA ATTGTTTTTATTGTGGAAATTGGTATGATCCTCTTTAGAGAAATCAG ACTAAGTTACTTTAGGGATAACAGCGTAAT | 497 |
| JN701 738 | Panulirus interru ptu s | GGGGGACGATAAGACCCTATAAATCTTTATAGCTTTGATTATAAGT TTTAGAGATTAGTTATAAGTCTAAAATTATTTTTTGAGCTATTTTGTT GGGGCGACAAAAGTATAACAAGTAACTGCTTTGGTTAATAAATTAA TTATTTTTATAATGTTATTTTAAAGATCCTTCTTTGAAGATGACAGA ATAAGTTACTTTAGGGATAACAGCGTAAT | 498 |
| JN701 739 | Panulirus marginatu s | GGGGGACGATAAGACCCTATAAATCTTGATAATTAAAGTAATAGTT TTTACGTAGTAGTTATAAGCTTAAATCTAATGCTTTGATTATTTTGT TGGGGCGACAAGGGTATAATAAATAACTGCCTTGAATAGATAGCT AATTATTTTTATTATGTTATGTTTAAGATCCTTCTTTGAAGATATCAG ATCAAGTTACTTTAGGGATAACAGCGTAAT | 499 |
| JN701 700 | Ibacus peronii | AGGGGACGATAAGACCCTATAAATCTTTATATTTAAAGTTTGTTAAT TTAAAAAGTAGATTAAAATTTTAATTTAATAAATTTAAATATTTTGTT GGGGAGACAGGAGTATAAAAAATAACTGCTTTAAAGTTTGAGTTAA TAATATTTAATTAAGTGATCCTTTACTATGGATTTTAGATTAAGTTA CTTTAGGGATAACAGCATAAT | 500 |
| JN701 698 | Ibacus chacei | AGGGGACGATAAGACCCTATAAATCTTTATATTTGGAGTTTGTTAA TTTGAAAAGTAAAATTAAAATTTTAATTTAATGAATTTAAATATTTTG TTGGGGAGACAGGAGTATAAAAAATAACTGCTTTAAAATTTGAGTT AATAATATTTAATTAAGTGATCCTTTATTATGGATTTTAGATTAAGTT ACTTTAGGGATAACAGCATAAT | 501 |
| KF220 508 | Charybdi s hellerii | GAGGGACGATAAGACCCTATAAAGCTTTATAAATTTGAAGATTTTG TGTATTTTTATATAAAAATGAATTTTGAAGATTTTATTTGGTTGGGG CGACAATGGTATAATGAGAATAACTGCTATTAAAATTAACAATTATA TTTGAATTATTAATAATTGATCCTTTATAAGGATTTTAAGATCAAGT TACTTTAGGGATAACAGCGTTAT | 502 |
| KC237 200 | Faxonella clypeata | AAGGGACGATAAGACCCTATAAAACTTTATATTTAATAATAGTAATT AATTTTATTTTAAGAGTATTATTTGAAAGTATTTGGTTGGGGTGACA AGGATAAAATATAAAATAACTGTCTTTTTTTTATTACAATAATATTTG GTTTAATGATCCTAAAATGGATTAAAGATTAAGTTACTTTAGGGATA ACAGCGTAAT | 503 |
| KC163 440 | Fallicamb arus kountzea e | AAGGGACGATAAGACCCTATAAAACTTTATATTTTATAAGTGGTAG TTAATTTTATTTTGTAATACTATTTAGGAGTATTTGGTTGGGGTGAC AAAGATAAAATAAAAAATAACTGTCTTTTTTATTTACAGTAATGTTT GATTTAATGATCCTTAAAAGGATTAAAGATTAAGTTACTTTAGGGAT AACAGCGTAAT | 504 |
| JX123 471 | Arenaeus mexicanu s | GAGGGACGATAAGACCCTATAAAGCTTTATAAGTGTAGGTATTTTG TATATTTTATGTTATAATAATAATTTGTTGAATACTTATTTGGTTGGG GCAACAAGAGTATAAAGAGAGTAACTGCTAAGATTTAAAACAATTA TATTTGATTAAAAAGTAATTGATCCCAAGAGGATTAAAAGATCAAG CTACTTTAGGGATAACAGCGTAAT | 505 |
| JX514 540 | Cambaru s tartarus | AAGGGACGATAAGACCCTATAAAACTTTATATTTAGGAGTAGAAGT TAGTTTTATTTTATTGGCTCTATTTAAAAGTATTTGGTTGGGGTGAC ATAAATAAAAAAATGAAATAATTGTTTTTATTTTTTACAATGATATTTG GGTTTGTGATCCTGAAGTGGATTGAAAGATTAAGTTACTTTAGGGA TAACAGCGTAAT | 506 |
| JQ407 454 | Chionoec etes tanneri | GGGGGACGATAAGACCCTATAAAGCTTTACATATAAGTAGAGTTTA CTTAATTAAAAAGTAAAAGTTTAATCTAATTAATGTGTTTTGTTGGG GCGACATAAATATAATTTATATTAACTGTTTATAAATTAATACAATAA TAAATGATTCTAATGTAATTGATCCTTATTAAAGATTAAAAGACTAA GTTACTTTAGGGATAACAGCGTTAT | 507 |
| JQ229 907 | Thenus unimacul atus | AGGGGACGATAAGACCCTATAAATCTTTATATTAATTTATTGTTAAG CTGGAAAATAGAATTAATTCGTGGGGTAACAATTTTAAATATTTTGT TGGGGAGACAGAAGTATAAAAGAATAACTACTTTGGATAAAGAGTT AATGATATTTAATGTAGAGACCCTTTCCTAAGGGTTATAGAACAAG TTACTTTAGGGATAACAGCGTAAT | 508 |
| JQ229 878 | Thenus indicus | GGGGGACGATAAGACCCTATAAATCTTTATATTAATTTGTTGTTAA ATTGGAAAATAGAATTAATTTCGGGCTTAACAATTTTAAATATTTTG TTGGGGAGACAGAAGTATAAATAAATAACTACTTTAAAGAGAAAGT TAATGATATTTAATGTAGAGATCCTTTACTAAGGATTGTAGAACAA GTTACTTTAGGGATAACAGCGTAAT | 509 |
| KM074 036 | Haptosqu illa hamifera | GAGTGACGATAAGACCCTATAAAGCTTTATAGTAGTGTCTTTTTAT AATAATTAGTAAAATGTAATTTATTGAAAGAAGTATACTATTTTACT GGGGCGGTAAAAGTATATTTTTTGATTAACTGCTTTTGTTTTGCAA GACAATGATAATTGAATTATGAATTGATCCATTATTAATGATTATAA GAACAAGTTACTTTAGGGATAACAGCGTAAT | 510 |
| KJ132 573 | Lithodes turritus | CAAAAAGACGATAAGACCCTATAAATCGTTACGCTGGGGCGGCGT AGATATATAAATAAACTGTCTATAATTTAGATACAATAATCATTGAA TTGATCCTTAAATAGATTAAAAGATTAAGATACTTTAGGGATAACA GCGTTAT | 511 |
| KF828 212 | Bouchard ina robisoni | AAGGGACGATAAGACCCTATAAAACTTTATATTTAAGAGTAGTAGT TAGTTTTGTTTAAGGAGTAGTATTTAAAAATATTTGGTTGGGGTGA CAAGGATAGAATAAAAAATAACTGTCTTTTTTTTTTACAGTAATATT TGGTTTAATGATCCTAAAAAGGATTAAAGATTAAGTTACTTTAGGG ATAACAGCGTAAT | 512 |
| KF828 201 | Trogloca mbarus maclanei | AAGGGACGATAAGACCCTATAAAACTTTATATTTATAAGGTAGTAA GTTAGTTTTATTTAAAGGTACTATGTTAGAATGTTTGGTTGGGGTG ACAAGGATAAAATATAAAATAACTGTCTTTTTTTTTTACAGTAATGT TTGGTTTAATGATTCTAAAAGGGATTAAAAGATTAAGTTACTTTAGG GATAACAGCGTAAT | 513 |
| KF828 192 | Hobbseu s yalobush ensis | AAGGGACGATAAGACCCTATAAAACTTTATATTTGAAAATAGTAGT TAATTTTGTTTAGTAAAGTAGTATTTAAAAGTATTTGGTTGGGGTGA CAAGGATAGAATATAAAATAACTGTCTTTTTTTTTTACAATAATATTT GTTTTGATGATCCTGAAATGGATTAAAGATTAAGTTACTTTAGGGA TAACAGCGTAAT | 514 |
| KF828 190 | Hobbseu s prominen s | AAGGGACGATAAGACCCTATAAAACTTTATATTTGAAAATAGTAGT TAATTTTATTTAATAAAGTATTATTTAAAAGTATTTGGTTGGGGTGA CAAGGATAGAATATAAAATAACTGTCTTTTTTTTTTTACAATAATATT TGTTTTAATGATCCTGAAATGGATTAAAGATTAAGTTACTTTAGGG ATAACAGCGTAAT | 515 |
| KF220 510 | Charybdi s lucifera | GAGGGACGATAAGACCCTATAAAGCTTTATAAATTTGAAGATTTTG TGTATTTTTATATAAAAATGAATTTTGAAGATTTTATTTGGTTGGGG CGACAATGGTATAATGAGAATAACTGCTATTAAAATTAACAATTATA TTTGAATTATTAATAATTGATCCTTTATAAGGATTTTAAGATCAAGT TACTTTAGGGATAACAGCGTTAT | 516 |
| KF828 189 | Hobbseu s petilus | AAGGGACGATAAGACCCTATAAAACTTTATATTTGAAAATAGTAGT TAATTTTATTTAATAAAGTATTATTTAAAAGTATTTGGTTGGGGTGA CAAGGATAGAATATAAAATAACTGTCTTTTTTTTTTACAATAATATTT GTTTTAATGATCCTGAAATGGATTAAAGATTAAGTTACTTTAGGGA TAACAGCGTAAT | 517 |
| KX237 946 | Faxonella creaseri | AAGGGACGATAAGACCCTATAAAACTTTATATTTAATAATAGTAATT AATTTTATTTTAAGAAGTATTATTTGAAAGTATTTGGTTGGGGTGAC AAGGATAAAATATAAAATAACTGTCTTTTTTTATTACAATAATATTTG GTTTAATGATCCTAAAATGGATTAAAGATTAAGTTACTTTAGGGATA ACAGCGTAAT | 518 |
| KU130 124 | Thranita danae | AAGGGACGATAAGACCCTATAAAGCTTTATAAGTTTAGAGATTTTA TATATTTTTCTATAGCAATAATTTCTTGAAAGCTTATTTGGTTGGGG CGACAATAGTATAATTGAAATAACTGCTTTAAAATAATACAAATATA TTTGAATTATAATTAGTTGATCCTTTATAAAGATTTAAGATCAAGTT ACTTTAGGGATAACAGCGTTAT | 519 |
| KT365 606 | Monomia petrea | AGGGGACGATAAGACCCTATAAAGCTTTATAAATTTAAAGATTTTA GAGATTTTATACCTAAATTTATTTTTTGATAGTTTATTTGGTTGGGG CGACAAAGGTATAATTATGGATAACTGCCATTAATGTTTATACATAT ATCTTTGATTTAAGGCTTAAATAATAATTGATCCTTTTTAAGGATTT AAAGATCAAGTTACTTTAGGGATAACAGCGTAAT | 520 |
| KT001 544 | Neogono dactylus wennerae | GAGGGACGATAAGACCCTATAAAGCTTTATGGTAGAATTTTTATAT ATAAATTATAAAATATCAACTTATTAGAAAATAAATACCATTATACT GGGGCGGTAAAAGTATATTTTTGATTAACTGCTTTTGTTGTTAAAA ACAGTTGTGGCTGAATAATCAGTTGATCCATTATTAATGATCATAA GTTCAAGTTACTTTAGGGATAACAGCGTAAT | 521 |
| KR026 905 | Xiphonect es pseudoha statoides | AGGGGACGATAAGACCCTATAAAACTTTATAAGTTTAAAGATCTCT ACTATTTTTTTTTATAAAGTTTTTTTTGATAGCTTATTTGGTTGGGG CGACAAAGGTATAATTATGAATAACTGCCATTAAAATTAATACATTT ATTGATGATTGTTGATAAATAGTTGATCCTTTTTTAAAGATTAAAAGA TCAAGTTACTTTAGGGATAACAGCGTAAT | 522 |
| KT001 545 | Gonodact ylellus viridis | GAGGGACGATAAGACCCTATAAAGCTTTATAATATATTTCTTTTATT TAAATTATAGTTTTTAACTCATTAAAGAAAATATATTATTTTACTGGG GCGGTAAAAGTATATTTTTGATTAACTGCTTTTGTTCTAACAAACAA TTATAATTGAATATCCATTGATCCATTATTAATGAGCATAAGACCAA GTTACTTTAGGGATAACAGCGTAAT | 523 |
| KT001 543 | Gonodact ylaceus ternatensi s | AAGTGACGATAAGACCCTATAAAGCTTTATAGTAGTTCCTTTCTAT TTAGATTATAAAATTTCAACTTTTTTAAAGGCTATTACTATTTTACTG GGGCGGTAAAAGTATATTATGATTAACTGCTTTTGTCTTGACAAAC AATAGTAATTGGATAATTATGTGATCCATTATTAATGATCAAAAGTA CAAGTTACTTTAGGGATAACAGCGTAAT | 524 |
| KR153 534 | Belosquill a laevis | ATGACGAACAAACCCATACTATAGCTTTATAATATGTTCTTCTTATG TAAGTTATCCCGCTTATTAAACTATTTTAAGAATCATATTATTTTAC CGGGGCGGTAATGGTATAATTTGTCGAGATTAACTGCTTTTCTTTT GTATGACATCTTTACTTGACAACTAATTGATCCATTATTAATGATCA CAAGAACAAGTTACTTTAGGGATAACAGCGTACT | 525 |
| KX238 053 | Procamb arus okaloosa e | AAGGGACGATAAGACCCTATAAAACTTTATATTTATAATATAGTAGT TAGTTTTATTTAATAGTACTATTTTAGAGTATTTGGTTGGGGTGACA AGGATAAAATATAAAATAACTGTCTTTTTTTTTTTACAATAATGTCT GTTTTAATGATCCTAAAAGGGATTAAAGATTAAGTTACTTTAGGGA TAACAGCGTAAT | 526 |
| KX238 050 | Procamb arus morrisi | AAGGGACGATAAGACCCTATAAAACTTTATATTTATAAGATATTAGT TAGTTTTATTTAAAAGTATTATATTAAAATATTTGGTTGGGGTGACA AGGATAGAATACAAAATAACTGTCTTTTTTTTTTACAGTAATGTTTG GTTTAATGATTCTAAAGGGATTAAAAGATTAAGTTACTTTAGGGAT AACAGCGTAAT | 527 |
| KX238 049 | Procamb arus milleri | AAGGGACGATAAGACCCTATAAAACTTTATATTTATAATATAGTAAT TAGTTTTATTTAATAATATTATATTAAAGTATTTGGTTGGGGTGACA AGGATATAATATAAAATAACTGTCTTTTTTTTTTACAGTAATATTTG GTTTAATGATCCTAGAAGGGATTAAAAGATTAAGTTACTTTAGGGA TAACAGCGTAAT | 528 |
| KX238 048 | Procamb arus mancus | AAGGGACGATAAGACCCTATAAAACTTTATATTTATAAAATAGTAG TTAGTTTTGTTTAGAGGTATTAATTTAGAGTATTTGGTTGGGGTGA CAAGGATAAAATATAAAATAACTGTCTTTTTTTATTACAGTAATGTT TGGTTTAATGATCCTAAAAGGGATTTAAGATTAAGTTACTTTAGGG ATAACAGCGTAAT | 529 |
| KX238 046 | Procamb arus lunzi | AAGGGACGATAAGACCCTATAAAACTTTATATTTATAAGTAGTAGT TAGTTTTATTTAAAAATATTATATTAAAATATTTGGTTGGGGTGACA AGGATAAAATATAAAATAACTGTTCTTTTTTTTTTTACAGTAATGTTT GATTTAATGATCCTAAAAAGGATTAAAAGATTAAGTTACTTTAGGG ATAACAGCGTAAT | 530 |
| KX237 948 | Hobbseu s cristatus | AAGGGACGATAAGACCCTATAAAACTTTATATTTGAGAATAGTAGT TAATTTTGTTTAGTAAAGTATTATTTAAAAGTATTTGGTTGGGGTGA CAAGGATAGAATATAAAATAACTGTCTTTTTTTTTTACAATAATATTT GTTTTAATGATCCTGGAATGGATTAAAGATTAAGTTACTTTAGGGA TAACAGCGTAAT | 531 |
| KX237 996 | Procamb arus acutissim us | AAGGGACGATAAGACCCTATAAAACTTTATATTTATAAGGTAGTAG TTAGTTTTATTTTAAAGTATTATTTTAGAGTATTTGGTTGGGGTGAC AAGGATAAAATATAAAATAACTGTCTTTTTTTTTTTACAGTAATGTTT GGTTTAATGATCCTAAAAGGGATTAAAGATTAAGTTACTTTAGGGA TAACAGCGTAAT | 532 |
| KX237 973 | Faxonius pagei | AAGGGACGATAAGACCCTATAAAACTTTATATTTAGAAGTAGAAGT TAATTTTATTTTAGTATTACTATTTAAAATATTTGGTTGGGGCGACG AGGATAAAAGGTTAAATAACTGTCTTTTTTTTTTTACAATAATATTT GGTTTAATGAGCCTAAAAGGGATTTAAGATTAAGTTACTTTAGGGA TAACAGCGTAAT | 533 |
| KY236 044 | Manningi a pilaensis | GAGGGACGATAAGACCCTATAAAGCTTTATAATAATTTTTTTTATA AAAATTATGGTATTATAACTTATTAAAAAATCATTATTATTTTACTGG GGCGGTAAAAGTATAATTTAGATTAACTGCTTTTAACATAAAAACC AATAATAATTGAGAACAATTTGATCCATTATTAATGATCATAAGAAC AAGTTACTTTAGGGATAACAGCGTAAT | 534 |
| KX279 350 | Pontastac us leptodact ylus | AGGGGACGATAAGACCCTATAAAACTTTATATTTTAAAATAATAATT AATTTTTATATAGAAGATTTATTTTTAAATATTTTATTGGGGTGATAA AGATAAAATGTGAGATAACTGTCTTTTTTTTTTTACAATTAATATTTG AGTAAAAGATCTTAACAAAGGAAATAAGAGATTAAGTTACTTTAGG GATAACAGCGTAAT | 535 |
| KX238 091 | Procamb arus zonangul us | AAGGGACGATAAGACCCTATAAAACTTTATATTTATAAGATAGTAG CTAGTTTTATTTAAAAGTATTATTTTGGAGTATTTGGTTGGGGTGAC AAGGATAAAATATAAAATAACTGTCTTTTTTTTTTTACAGTAATGTTT GGTTTAATGATCCTAAAAGGGATTAAAGATTAAGTTACTTTAGGGA TAACAGCGTAAT | 536 |
| KX238 089 | Procamb arus youngi | AGGGACGATAAGACCCTATAAAACTTTATATTTATAATATGGTAATT AATTTTATTTAAAAATATTATTTTAGAGTATTTGGTTGGGGTGACAA GGATAAAATGTTAAATAACTGTCTTTTTTTTTTTACAGTAATGTTTG GTTTAATGATCCTACAAAGGATTAAAGATTAAGTTACTTTAGGGAT AACAGCGTAAT | 537 |
| KX238 075 | Procamb arus seminola e | AAGGGACGATAAGACCCTATAAAACTTTATATTTATAAGGTAGTAG ATAGTTTTATTTATAAGTATTATATTAGAATATTTGGTTGGGGTGAC AAGGATAAAATATAAAATAACTGTCTTTTTTTTTTACAGTAATATTTG GTTTAATGATCCTAAAATGGATTAAAAGATTAAGTTACTTTAGGGAT AACAGCGTAAT | 538 |
| KX238 063 | Procamb arus pycnogon opodus | AGGGACGATAAGACCCTATAAAACTTTATATTTCTAACGTAGTAAT TAGTTTTATTTGAGAGTACTATATTAAAGTATTTGGTTGGGGTGAC AAGGATAAAATATAAAATAACTGTCTTTTTTTTTTACAGTAATATTTG GTTTAATGATCCTTAAAAGGATTAGAAGATTAAGTTACTTTAGGGA TAACAGCGTAAT | 539 |
| KX238 054 | Procamb arus orcinus | GAGGGACGATAAGACCCTATAAAACTTTATATTTATAAGGTAGTAA TTAGTTTTATTTAAAAGTATTATATTAGAATATTTGGTTGGGGTGAC AAGGATAAAATATAGAATAACTGTCTTTTTTTTTTACAGTAATATTT GGTTTAATGGGCCTGAAAAGGATTAAAAGATTAAGTTACTTTAGGG ATAACAGCGTAAT | 540 |
| KX238 055 | Procamb arus pallid us | AGGGACGATAAGACCCTATAAAACTTTATATTTATAAGGTAGTAAT TAGTTTTATTTAAAAGTATTATATTAGAATATTTGGTTGGGGTGACA AGGATAAAATACAGAATAACTGTCTTTTTTTTTTACAGTAATATTTG GTTTAATGGGCCTGAAAAGGATTAAAAGATTAAGTTACTTTAGGGA TAACAGCGTAAT | 541 |
| MH168 208 | Alima maxima | GGGGGACGATAAGACCCTATAAAGCTTTATATTACGTTTCTTCTTT ATAAAATATCCGGTTGTTAACTTATTTTAGAAATTGTATTATTTTACT GGGGCGGTAAAAGTATAAATAAGATTAACTGCTTTTGTCTTGCATA ACAATTTTAATTGGAAATAAATTGATCCATTAATAATGATCACAAGA ACAAGTTACTTTAGGGATAACAGCGTAAT | 542 |
| MF490 148 | Scylla ride s deceptor | GAGGGAYGATAAGACCCTGTAAATCTTTATATTTTGGCTGATAAGG TTAAAAGTTAGGTTAKTATTTAATTTTATAAGTTTGAATATTTTGTTG GGGAGACAGGGGYATAAAAGGAAATAACTGCCTTARAGTTTAAGT TAAAAATGTTTAAATTATTAAGTGATCCTTTAATAAGGAATATAGAC TAAGTTACTTTAGGGATAACAGCGTAAT | 543 |
| KY524 476 | Monomia argentata | AGGGGACGATAAGACCCTATAAAGCTTTATAAGTTTAGGAATTTTA AATATTTTGTAGATAAATTTATTTTCCAATATCTTATTTGGTTGGGG CGACAAAGGTATAATTATGAATAACTGCTATTATTAACAATACATCT ATCGTTGGCTAAAATTTATAATAATTGATCCTCTTTAGAGATTAAAA GATCAAGTTACTTTAGGGATAACAGCATAAT | 544 |
| KY524 461 | Xiphonect es pulchricri status | GAGGGACGATAAGACCCTATAAAGCTTTATATATTAAATGATTTTA TGGATTTAAAAAATAAAAATTATTCTGAAAAATTTATTTGGTTGGGG CGACAGAGGTATAATTTAGATAACTGCTAGGAATTTATACAATTAT TCTTGAATTATGTTTGTAAATGATCCTTGTTAGAGATTAAAAGATCA AGTTACTTTAGGGATAACAGCGTAAT | 545 |
| NC_04 2240 | Paralitho des platypus | AAAAGACGATAAGACCCTATAAATCTTTACAATAAATATATTTTATA TTTTAGCTTATAAGTAAATAAGAAATAAAAATATAGGTTTGTTACGC TGGGGCGGCGTAGATATATAAATAAACTGTCTATAGTTTAAATACA ATAATTATTGCTTAATACAAATTGATCCTTAAATAGATTAAAAGATT AAGATACTTTAGGGATAACAGCGTTAT | 546 |
| KY426 330 | Lopholith odes foraminat us | AAGACGATAAGACCCTATAAATCTTTACAATAAATATATTTTATATT TTAGCTTATAAGTGTATAAGAAATAAAAATATAGATTTGTTGCGCTG GGGCGGCGTAGATATATAAATAAACTGTCTATAATTTAAATACAAT AATCATTGTTTAATACAAATTGATCCTTAAATAGATTAAAAGATTAA GATACTTTAGGGATAACAGCGTTAT | 547 |
| KY236 047 | Faughnia formosae | GAGGGACGATAAGACCCTATAAAGCTTTATGATTCCTTTTTTTATT ATAAATTATATATTTTAGTTTACTAAAAAACAGGTATTATTTTACTGG GGCGGTAAAAGTATGTTTTTGGGTAACTGCTTTTATTTTTAAAAAC AATTATGTTTGGAAATAAAATGGTCCATTATTAATGATTATAAGAAC AAGTTACTTTAGGGATAACAGCGTAAT | 548 |
| KY236 046 | Faughnia profunda | GGGGGACGATAAGACCCTATAAAGCTTTATGGTAGTTTCTTTTAAT ATGAATTGTATGATTTCTAGTTTATTGAAGAAAAATTACTATTTTAC TGGGGCGGTAAAAGTATGCTTTCGGTTAACTGCTTTTGTTTTTAAA AACAAACATGGTTGGAAATAAATTGATCCATTATTAATGATTATAAG AACAAGTTACTTTAGGGATAACAGCGTAAT | 549 |
| KY236 045 | Bathysqui lla crassispin osa | GAGGGACGATAAGACCCTATAAAGCTTTATGGTGTACTACTTTGTT ACAAAATATAATAATTAAAACTTATTGAAGTAGTGGGCATTATTTTA CTGGGGCGGTAAAAGTATAAATAATCTGAGTAACTGCTTTTATTGT GCTAAACAATTATAATTGACTTTTAAATTGATCCATTACTAATGATC ACAAGAACAAGTTACTTTAGGGATAACAGCGTAAT | 550 |
| NC_00 6992 | Eriocheir sinensis | TAAAGGGACGATAAGACCCTATAAAGCTTAATATTAAATTTTATATA GTCAAATTTTAGGTTATAAAGATTTTGTAAATTGAATTTATTTTATTG GGGCGATAAGAGTAAAATGATTATTAACTGCTTAACATTAAGTACA CTTATGTGTGATTAAAATTTTAAATGATCCTAATTAAAGATTAAAAG TTTAAGTTACTTTAGGGATAACAGCGTTAT | 551 |
| NC_00 6916 | Harpiosq uilla harpax | GAGGGACGATAAGACCCTATAAAGCTTTATGACATATTTTTTTTTAT AAATTATTTTGTTATTAACTTAATATAAAATGTATGTCATTTTACTGG GGCGGTAAAAGTATAATTAAAGAATAACTGCTTTTGTTTTTAATAAC AATTATAGTTGGAAATGAATTGATCCATTATTAATGATCACAAGAAC AAGTTACTTTAGGGATAACAGCGTAAT | 552 |
| NC_00 6281 | Callinecte s sapidus | CCATAGGGACGATAAGACCCTATAAAGCTTTATAAATTTATTAATTT CGTTTATTTTTTATTATAATAATGATGATAAGAGAATTTATTTGGTT GGGGCAACCGAAGTATAATTTTAAATAACTGCTATAATTTAATACA ATGATTTTTGTCTTATACAATAATTGATCCTCAAAGAGATTAAAGAC CAAGTTACTTTAGGGATAACAGCGTAAT | 553 |
| NC_00 6081 | Squilla mantis | GAGGGACGATAAGACCCTATAAAGCTTTATAATACTTCTTTTTTATA TAAATTGTTCTATTATTAACTTATTTTAAAGATTGTATTATTTTACTG GGGCGGTAAAAGTATATTAAAGACTAACTGCTTTTATTTTTTATAAC AATTATGATTGAATGTAAATTGATCCATTATTAATGATCACAAGAAC AAGTTACTTTAGGGATAACAGCGTAAT | 554 |
| NC_00 5037 | Portunus tritubercul atus | GAGGGACGATAAGACCCTATAAAGCTTTATAAATTTAGAAATTTTA TATATTTTTTTAATAATGATAATCTTTTGAAAATTTATTGGGTTGGG GCAACAAAAGTATAATTATTAATAACTGCTAGAAAGTATTACAATTG TTTTTGTCATAAAGAAAATTAGTTGATCCCTGAAAGGGATTAAAAG ATCAAGTTACTTTAGGGATAACAGCGTAAT | 555 |
| NC_00 4251 | Panulirus japonicus | GGGGGACGATAAGACCCTATAAATCTTGATGATTAAGATAATAAAT TTTAGGCAGTAGTAATAAGTTTAAATTTAATGTCTTAATTATTTTGTT GGGGCGACAGGAGTATAAGAAGTAACTGCTTTGAAAAAATAATTA ATTATTTTTATGATGTTTTGTTTGTGATCCTTCTATGAAGATAATAG ATCAAGTTACTTTAGGGATAACAGCGTAAT | 556 |
| MN334 534 | Cancer pagurus | AGGGGACGATAAGACCCTATAAAGCTTTATATTTTAGCTATAATTT AATTAAATTAATTAGATCAAAGGTTTTAATAAGTTATTATATTTTTATT GGGGAGGTATAAGTATAATTTATGTAACTGCTTATAAGTTAGTACA CTGATAATTGATTAGAGTTTCAATGATCCTTTAAGATAAAGATTTAA GACTAAGTTACTTTAGGGATAACAGCGTTAT | 557 |
| MT750 295 | Chionoec etes japonicus | GGGGGACGATAAGACCCTATAAAGCTTTACATATAAGTAGAGTTTA CTTAATTAAAAAGTAAAAGTTTAATCTAATTAATGTGTTTTGTTGGG GCGACATAAATATAATTTATATTAACTGTTTATAATTTAATACAATAA TAAATGATTTTAATGTAGTTGATCCTTATTAAAGATTAAAAGACTAA GTTACTTTAGGGATAACAGCGTTAT | 558 |
| NC_01 2567 | Scylla tranqueb arica | GAGGGACGATAAGACCCTATAAAGCTTTATAAATTAAGTAAGTTAG AACAAATTTTATAGAATAAAGGTAAATTTAATATATTTATTTGGTTG GGGCGACAATGGTATAAATTAAATAACTGCTATTAAAATTAAACAA TTATATTTGATTAAAATTATAATTGATCCTTATTATGGATTAAAAGAT CAAGTTACTTTAGGGATAACAGCGTAAT | 559 |
| NC_01 2565 | Scylla serrata | CTAGAGGGACGATAAGACCCTATAAAGCTTCATAAGTTAAATAAAT TAGATGGATTTTTAGAATAAAAGTAAATTTAGTAAGCTTATTTAGTT GGGGCGACAAGGGTATAAAATTAAATAACTGCTATTAAGGTTAGA CAATTATATTTGGTTGGATTGTAATTGATCCTCGCTATGGATTAAAA GATTAAGTTACTTTAGGGATAACAGCGTAAT | 560 |
| NC_01 1598 | Eriocheir hepuensi s | AAGGGACGATAAGACCCTATAAAGCTTAATATTAAATTTTATATAGT CAAATTTTAAATTATAAAGATTTTGTAAATTGAATTTATTTTATTGGG GCGATAAGAGTAAAATGATTATTAACTGCTTAACATTAAGTACACT TATATGTGATTAAAATTTTAAATGATCCTAATTAAAGATTAAAAGTTT AAGTTACTTTAGGGATAACAGCGTTAT | 561 |
| NC_01 1597 | Eriocheir japonica | AAGGGACGATAAGACCCTATAAAGCTTAATATTAAATTTTATTTAGT CAAATTTTAGGTTATAAAGATTTTGTAAATTGAATTTATTTTATTGG GGCGATAAGAGTAAAATGATTATTAACTGCTTAATATTAAGTACAC TTATATGTGATTAAAATTTTAAATGATCCTAATTAAAGATTAAAAGTT TAAGTTACTTTAGGGATAACAGCGTTAT | 562 |
| NC_01 1243 | Cherax destructor | GGGGGACGATAAGACCCTATAAAGTTTGACATTAAATTAATTAAGG GTAATTTAGATTATAAAGTCTTATTATTATATAAGTGTTTAGTTGGG GCGACTAGGATATAAGTTATTTAACTGTTTCTTCATTCGAATCAAAA ATTTTTGATTTTATGATCCTTTTTAAGGGTACTAGAGTAAATTACTT TAGGGATAACAGCGTAAT | 563 |
| >NC_0 07444 | Squilla empusa | GAGGGACGATAAGACCCTATAAAGCTTTATGATATTTCTTTTTTGT ATAAAATATTTATTGTTAACATATTTAAAAGTTTATATTATTTTACTG GGGCGGTAAAAGTATATTAAAGACTAACTGCTTTTGTTTTTTATAA CAATTATAATTGAAAATAAATTGATCCATTATTAATGATCATAAGAA CAAGTTACTTTAGGGATAACAGCGTAAT | 564 |
| NC_00 7443 | Lysiosquil lina maculata | AAGGGACGATAAGACCCTATAAAGCTTTATGATGTATATTCGCTAA TGTTGATTTTCGTAATATTAAAGCGTTAGAGAATCATATCATTTTAC TGGGGCGGTAAAAGTATAAAAGAATAACTGCTTTTGTTGAAATTAA ACAATGATAGTTGGAGAATAAATTGATCCATTAATAATGATCATAA GAACAAGTTACTTTAGGGATAACAGCGTAAT | 565 |
| NC_00 7442 | Gonodact ylus chiragra | GAGGGACGATAAGACCCTATAAAGCTTTATAATGGATTCTTGTTAT ATAAATTATCAATTGTTAACATGTTAAAAGAATAATATTATTTTACTG GGGCGGTAAAAGTATATATCTGAGTAACTGCTTTTGTTTTTTAAAA CAGTGATGACTGACTTATTAATTGATCCACTAATAGTGAGCATAAG GACAAGTTACTTTAGGGATAACAGCGTAAT | 566 |
| NC_01 6015 | Panulirus homarus | GGGGGACGATAAGACCCTATAAATCTTTATGTTTTAACTAATAAAT TTTAGAGATTAGTTAAATATTTAGTGTTATTAAGTTAAATATTTTGTT GGGGCGACAGGAGTACAATAAGTAACTGCTTTGTTTATAAAGTTAA TTATTTTTATTATGGAAATTGTTATTGATCCTTTTTGGAGAAATCAG AATAAGTTACTTTAGGGATAACAGCGTAAT | 567 |
| NC_01 5607 | Homarus american us | TAAAGGGACGATAAGACCCTATAAAGCTTAATAATTTAGTATATAA TTAGATGAGTTGAAAGTTTAATATTATTTATATACTAAATTATTTCGT TGGGGCGACGATGATATAATTTGTAACTGTTTAAATTTAAAATACA GAGATATTTGTGTGTAATGATCCTTGTTGTTGATTAAAAATTTAAGT TACTTTAGGGATAACAGCGTTAT | 568 |
| NC_01 4854 | Panulirus ornatus | GGGGGACGATAAGACCCTATAAATCTTTATACTTTAACTAATAAAT TTTAAAGATTAGTTGCAAATTTAGTGTTATTAAGTTAAAATATTTTGT TGGGGCGACAGGAGTATAATAAGTAACTGCTTTGTTTATAAATTTA ATTATTTTTAATGTGTAAATTGTATTGATCCTCTGTTGGAGAAATCA GACTAAGTTACTTTAGGGATAACAGCGTAAT | 569 |
| NC_01 4342 | Oratosqui lla oratoria | GAGGGACGATAAGACCCTATAAAGCTTTATGATACTATTCTTTTTT ATAAATTGTTTTGTTATTAACTTATTTTAGAATTAGTATTATTTTACT GGGGCGGTAAAAGTATAATAAAAGAATAACTGCTTTTGTTTTGTAT AACAATTTTAGTTGGAAATAAATTGATCCATTATTAATGATCATAAG AACAAGTTACTTTAGGGATAACAGCGTAAT | 570 |
| NC_01 4339 | Panulirus stimpsoni | GGGGGACGATAAGACCCTATAAATCTTTATATTTTATTTAATAAATT TTAAAAATTAGTTATACATTTAGCGATATTAAGTCAAATATTTTGTT GGGGCGACAGGAGTATAATAAGTAACTGCTTTGTTTGTAAAGTTAA TTATTTTTATTGTGTAGAGTTAAATTGATCCTTTCTAGAGAAATCAG ATCAAGTTACTTTAGGGATAACAGCGTAAT | 571 |
| NC_01 3246 | Charybdi s japonica | GAGGGACGATAAGACCCTATAAAGCTTTATAAATTTAAAGATTTTA TATATTTTTATATAAAAATAAATTCCAGAGGATTTATTTTGTTGGGG CGACAATGGTATAATGAAAATAACTGCTATTAAAATTAACAATTATA TTTGTTTATGTAAATAATTGATCCTTTATAAGGATTTTAAGATCAAG TTACTTTAGGGATAACAGCGTTAT | 572 |
| NC_01 2572 | Scylla paramam osain | GAGGGACGATAAGACCCTATAAAGCTTTATAAGTTAAGTAAGTTAA ATTAATTTTTTAAAATAAAAATAAGCTTAAAAAATTTATTTGGTTGG GGCGACAATGGTATAAATTAAATAACTGCTATTAAAATTAAGCAAT TATATTTGATTAGAATTATAATTGATCCTTATTATGGATTAAAAGAT CAAGTTACTTTAGGGATAACAGCGTAAT | 573 |
| NC_01 2569 | Scylla olivacea | GAGGGACGATAAGACCCTATAAAGCTTTATAGATTGAGTGGATTA AACCAATTTTTTAGAATAAAAGTAAATTTGGAAGATTTATTTGGTTG GGGCGACAAATGTATAATGTTAAATAACTGCTATTAAATTTAAACA ATTATATTTGATAAAAATTTAAATGATCCTTAATAAAGATTAAAAGAT CAAGTTACTTTAGGGATAACAGCGTAAT | 574 |
| NC_02 2937 | Cherax quadricari natus | GAGGGACGATAAGACCCTATAAAGTTTATACATGAAGTTGGTTAA GAGTGATTTAAGGTGTTAAAGTTTATTATCAGCAGGGTGTTTAGTT GGGGCGACTAGGATATAAATTATATAACTGTTTTTTGTTTAAATCA GAGATATTTGTTCATATGATCCCTTTTTAGGATTAGAGAATAAATTA CTTTAGGGATAACAGCGTAAT | 575 |
| NC_02 2936 | Cherax cainii | AAGGGACGATAAGACCCTATAAAGTTTATACATCCAAATGATTGAA AATAATTAAGAAAATTAAAGTTTATTATTAGGGAGATGTTTAGTTGG GGCGACTAGGATATAAGTTAATTTAACTGTCCTAAATTAAAATCAA AGATATTTGAGTTTGTGATCCTTTTTTAGGATTAAAGAACAAGTTAC TTTAGGGATAACAGCGTAAT | 576 |
| NC_02 1458 | Paralitho des brevipes | CAAAAAGACGATAAGACCCTATAAATCTTTACAATAAATATATTTTA TATTTTATATTATAAGTGTATAATAAATAAAAATATAAATTTGTTGCG CTGGGGCGGCGTAGATATATAAATAAACTGTCTATAATTTAAATAC AATAATCATTGTTTAGTAAAATTGATCCTTAAATAGATTAAAAGATT AAGATACTTTAGGGATAACAGCGTTAT | 577 |
| NC_02 0029 | Paralitho des camtscha ticus | CAAAAAGACGATAAGACCCTATAAATCTTTACAATAAATATATTTTA TATTTTAGTTTATAAGTGAATGAGATATAAAAATATAGGTTTGTTGC GCTGGGGCGGCGTAGATATATAAATAAACTGTCTATAGTTTAAATA CAATAATCATTGCTTAATATAAATTGATCCTTAAATAGATTAAAAGA TTAAGATACTTTAGGGATAACAGCGTTAT | 578 |
| NC_02 0022 | Scylla ride s latus | GAGGGACGATAAGACCCTATAAATCTTTATATTTTGGGTTATAAGG TTAAAAATTAGGTTATCTTTTAATTTTATAAGGTTAAATATTTTGTTG GGGAGACAGAGGTATAAAAAGAGATAACTGCCTTAAAATTTATAGT TAAGAATAGTTAAGATTGAAAGTGATCCTTTAATAAGGAAAATAGA AGAAGTTACTTTAGGGATAACAGCGTAAT | 579 |
| NC_01 6926 | Procamb arus clarkii | GGAGGGACGATAAGACCCTATAAAACTTTATATTTATAATATAGTA GTTAGTTTTATTTAAGGGTATTATTTTAGAGTATTTGGTTGGGGTG ACAAGGATAAAATATTAAATAACTGTCTTTTTTTTTTACAGTGATGT TTGGTTTAATGATCCTAAAAGGGATTAAAGATTAAGTTACTTTAGG GATAACAGCGTAAT | 580 |
| NC_02 0021 | Procamb arus fallax | AAGGGACGATAAGACCCTATAAAACTTTATATTTATAAAGTAGTAG TTAGTTTTATTTAAAAGTATTATATTAGAATATTTGGTTGGGGTGAC AAGGATAAAATATAGAATAACTGTCTTTTTTTTTACAGCAATATTT GGTTTAATGATCCTAAAAAGGATTAAAAGATTAAGTTACTTTAGGG ATAACAGCGTGAT | 581 |
| NC_02 0020 | Homarus gammaru s | TAAAGGGACGATAAGACCCTATAAAGCTTAATAATTTAGTATATAA TTAGATGAGTTAAAAGTTTAATATTATTTATATACTGAATTATTTCGT TGGGGCGACGATGATATAATTTGTAACTGTTTAAATCTAAAATACA GAGATATTTGTGTGTAATGATCCTTGTTGTTGATTAAAAATTTAAGT TACTTTAGGGATAACAGCGTTAT | 582 |
| NC_02 4440 | Thenus orientalis | AGGGGACGATAAGACCCTATAAATCTTTATATTAATTTGTTGTTAAA TTGGAAAGTAGAATTAATTCCGGGTTTAATAATTTTGAATATTTTGT TGGGGAGACAGAAGTGTAAATAAATAACTACTTTAAAAAAAAAAGT TAATGATATTTAATGAGGAGATCCTTTACTAAGGATTGTAGAATAA GTTACTTTAGGGATAACAGCGTAAT | 583 |
| NC_02 4202 | Lithodes nintokuae | CAAAAAGACGATAAGACCCTATAAATCTTTACAATAAATATATTTTA TATTTTAGCTTATAAGTGTATAAGAAATAAAAATATAGATTTGTTGC GCTGGGGCGGCGTAGATATATAAATAAACTGTCTATAGTTTAAATA CAATAATCATTGCTTAATACAAATTGATCCTTAAATAGATTAAAAGA TTAAGATACTTTAGGGATAACAGCGTTAT | 584 |
| NC_02 3481 | Cherax cairnsens is | GGGGGACGATAAGACCCTATAAAGTTTGTACACTAAACTAGTTTA GAGTGATTTAGGTTTATAAAGTTTACTATTGGGGAGGTGTTTAGTT GGGGCGACTAGGATATAAGTTATCTAACTGTTTCTTTGTTGTAATC AGAAATATTTGAGTTTATGATCCTTTTTTAGGATTATAGAGTAAATT ACTTTAGGGATAACAGCGTAAT | 585 |
| NC_02 3480 | Cherax dispar | GGGGGACGATAAGACCCTATAAAGTTTGTACATTAAACTAATTAAA GGTGGTTTAGGTTTATAAAATTTGCCATTAGGGAAGTGTTTAGTTG GGGCGATTGGGATATAAGTTATCTAACTGTTTCTTTGTTTTAATCA AAAATATTTGAGTTTATGATCCTTTTTTAGGATTAAAGAGTAAATTA CTTTAGGGATAACAGCGTAAT | 586 |
| NC_02 3479 | Cherax quinquec arinatus | AAGGGACGATAAGACCCTGTAAAGTTTATACATTAAGTTAATTAAA AATAATTAAGGAGATTAAAATTTATTATTGGGTAGATGTTTAGTTGG GGCGACTAGGATATAATTTAATTATAACTGTCTTAGATTAAGGTCA AAGATATTTGAGTTTGTGATCCTTTCTTAGGATTCAAGAACAAGTT ACTTCAGGGATAACAGCGTAAT | 587 |
| NC_02 3478 | Cherax robustus | GAGGGACGATAAGACCCTATAAAGTTTTGTACATTAAACCAATTAA TAGGCGATTTAGGTTTGTAAGGTTTACTATTAGGTAGATGTTTGGT TGGGGCGACTAGGATATAAGTTATTTAACTGTTCTTTGTTAGAATC AAAGGTATTTGAGTTTATGATCCTTTTTAAGGGTTATAGAATAAATT ACTTTAGGGATAACAGCGTAAT | 588 |
| NC_02 2938 | Cherax monticola | GAGGGACGATAAGACCCTATAAAGTTTGTACACAGAGTTAATTAA GAGCGATTTGGAGTAATAAAATTTATTATTAGCAAGGTGTTTGGTT GGGGCGACTAGAATATAAGTTATATAACTGTTTTTTGTTTAAATCA GAGATATCTGTTTATATGATCCCTTTTTAGGATTAAAGAGTAAATTA CTTTAGGGATAACAGCGTAAT | 589 |
| NC_02 2939 | Cherax glaber | AAGGGACGATAAGACCCTATAAAGTTTATATACAGAACTAATTAAA GATAATTAGGAAGAATAAAATTTATTATTAGGTAGGTATTTAGTTGG GGCGACTAGGATATAAATTAATTTAACTGTTTTTAATTAAGGTCAAA GATATTTGAGTTCGTGATCCTTTTTTTAGGATTAAAGAATAAGTTACT TTAGGGATAACAGCGTAAT | 590 |
| NC_02 6224 | Cherax holthuisi | GAGGGACGATAAGACCCTATAAAGTTTGAACACCGGGTTTGTTAA GAGTGATTTAGAATAGTAAAGTTTGTTACTAGTAAAGTGTTTGGTT GGGGCGACTAGAATATAAGTTATATAACTGTTTTTTATTTGAATCA GAGATATCTGTTTATATGATCCCTTTTTAGGATTAAAGAGTAAATTA CTTTAGGGATAACAGCGTAAT | 591 |
| NC_02 6215 | Astacopsi s gouldi | GGGGGACGATAAGACCCTATAAAGTTTAACATAATGAGGATAAAA AATTAATTAGATTATAAAGTTTATTATCATAAATAATGTTTTGTTGG GGCGACAAGAATAAAAATAATTTAACTGTTCTTTTTTAGATACAAAA ATATTTGTGTAGGGTGATCTTTTTTTAGAAGTATTAGAGTAAATTACT TTAGGGATAACAGCGTAAT | 592 |
| NC_02 6209 | Portunus pelagicus | GAGGGACGATAAGACCCTATAAAGCTTTATAAATTTAAAGATTTTA TATATTTTTTATTATAATGATAATTTCTCAAAAATTTATTGGGTTGGG GCAACAAAAGTATAATTATCAATAACTGCTATAAATTACTACAATTA TTTTTGTTATTAAAGAGTTGTTGATCCTCGAAAGGGATTAAAAGAT CAAGTTACTTTAGGGATAACAGCGTAAT | 593 |
| NC_02 5957 | Paraneph rops planifrons | AAGGGACGATAAGACCCTATAAAGCTTTACATTGAATTTATTAAAA AGTAATTTAAATAATAAAAGTTTATTAGTAGGGAGATGTTTTGTTGG GGTGACAAGAATATAATAAATATAACTGTTCTTTTTTATTTCAAAAA TATTTGAATGGGAGATCCTTAATAAAGATGTTAGAGTAAGTTACTT TAGGGATAACAGCGTAAT | 594 |
| NC_02 5958 | Nephrops norvegicu s | TAAAGGGACGATAAGACCCTATAAAGCTTAATAATTTAATATATAA CCAGATAAATTAAAAGTTTAATATTCTTTATATATTAAATTATTTCGT TGGGGCGACGATGATATAATTTGTAACTGTTTAAATTTTAAATACA GAGATATTTGTGTGTAATGATCCTTTTTATTGATTAAAAATTTAAGT TACTTTAGGGATAACAGCGTTAT | 595 |
| NC_02 5581 | Ibacus ciliatus | AGGGGACGATAAGACCCTATAAATCTTTATATTTTATATTTATTAGT TTAAAAATTGGTATAAAATTTTGATTTAATAATTTAAAATATTTTGTT GGGGAGACAGGAGTATAATAAATAACTGCTTTAAAATTTAAGTTAA TAATATTTAATTGAGTGATCCTTTAATATGGATTTTAGATTAAGTTA CTTTAGGGATAACAGCGTAAT | 596 |
| NC_02 4632 | Charybdi s feriata | GAGGGACGATAAGACCCTATAAAGCTTTATAAATTTAAAAATCTTA TATATTTTTATATAAAAATAATTTTTGAGGAGTTTATTTGGTTGGGG CGACAGTGGTATAAGTAATGTAACTGCTATAGTGTTTAACAAAAAT ATTTGTTTAGTGTAAGTAATTGATCCTTTATAAGGATTTTAAGATCA AGTTACTTTAGGGATAACAGCGTTAT | 597 |
| NC_02 5323 | Metaneph rops sibogae | GAGGGACGATAAGACCCTATAAAGCTTGATAATTTTGTATATAAAT AAATAAGTTGTTAGTATTATACTGTCTACATATAAAATTATTTCGTT GGGGCGACGATAATATAATCTGTAACTGTTTAGGTTTTAACTCAAA TATATTTGTGTTTAATGATCCATTAATTGTTGATTAAAAATTTAAGTT ACTTTAGGGATAACAGCGTTAT | 598 |
| NC_02 8024 | Panulirus cygnus | GGGGGACGATAAGACCCTATAAATCTTGATGATCGGGGTAGTAAT TTTTATGTAGTAGTTATAAACTTAAATTTAATACCTTGATTATTTTGT TGGGGCGACAGGGGTATAATTAGTAACTGCCTTGGATAAACAGCT AATTATTTTTATTGTGTTATGTCTAAGATCCTTCTTTGAAGATATCA GATCAAGTTACTTTAGGGATAACAGCGTAAT | 599 |
| NC_02 7608 | Metaneph rops thomsoni | GAGGGACGATAAGACCCTATAAAGCTTGATAATTTTATATATAAAT AAATAAGTTGTTAGTGTTATATCATCTATTTATAAAATTATTTCGTTG GGGCGACGATAATATAATTTGTAACTGTTTAAGATTTAGATTCAAA TATATTTGTGTTTTAATGATCCATTAGTTGTTGATTAAAAATTTAAGT TACTTTAGGGATAACAGCGTTAT | 600 |
| NC_02 6561 | Faxonius limosus | TTGGGACGATAAGACCCTATAAAACTTTATATTTTAAGGTAGGAGT TAATTTCATTTTAAGGTTACTATTTTAAAATGTTTTGTTGGGGGGAC AAGGATATAAGAAATGATAACTGTCTTTTATTTTTACAATAATATTT GATTTATTGATCCTGAAAGGGATTTAAGATTAAGTTACTTTAGGGA TAACAGCGTAAT | 601 |
| NC_02 7178 | Squilloide s leptosquill a | GAGGGACGATAAGACCCTATAAAGCTTTATATTATATTTCTTTAATA TAAATTGTAATGTTATTAACTTATTTTAGAAAATATATTATTTTACTG GGGCGGTAAAAGTATATTAAAGATTAACTGCTTTTGTTTTGTATAA CAATTATAGTTGAAAATAAAATGATCCATTATTAATGATCATAAAAC CAAGTTACTTTAGGGGTAACAGCGTAAT | 602 |
| NC_02 6226 | Cherax bicarinatu s | GAGGGACGATAAGACCCTATAAAGTTTATACATGGGGCTGGTTGA GAGTGATTCAGAATAGTAAAGTTTATTATTAGCAAGATGTTTAGTT GGGGCGACTAAGATATAAATTATATAACTGTCTTTTGTTTAAATCA GAGGTATCTGTTCGTATGATCCTTTTTTAGGATTGGAGAGTAAATT ACTTTAGGGATAACAGCGTAAT | 603 |
| NC_02 6560 | Austropot amobius pallipes | GGGGGACGATAAGACCCTATAAAACTTTATATTTTAAAAATATTAA CTAGTTTTGTATAAGAGGGTTTTTTTTTGAGTATTTTATTGGGGTGA TAGGGATATAATAAAAGATAACTGTCTCTTTTTTTTTACAGAGATAT TTGAGTAAATGATCCTAATAAGGGAAAGAAGGTTAAGTTACTTTAG GGATAACAGCGTAAT | 604 |
| NC_02 6559 | Cherax tenuiman us | AAGGGACGATAAGACCCTATAAAGTTTATACATTCAAATAATTGAA AATAATTAAGAAAAATTAAAGTTTATTATTAGGAAGATGTTTAGTTG GGGCGACTAGGATATAAGTTAATTTAACTGTCTTAAATTAAAGTCA AAGATATTTGAGTTTGTGATCCTTTTTTAGGATTCAAGAACAAGTTA CTTTAGGGATAACAGCGTAAT | 605 |
| NC_02 6227 | Cherax boesema ni | GAGGGACGATAAGACCCTATAAAGTTTATACATGGAGTTAGTTAA GAGTGATTTAGGATAGTAAAGTTTGTTATTAGCAAGATGTTTGGTT GGGGCGACTAGAATATAAGTTATATAACTGTTTTTTATTTAAGTCA GAGGTATTTGTTTATAGTGATCCCTTTTTAGGATTAAAGAGTAAATT ACTTTAGGGATAACAGCGTAAT | 606 |
| NC_03 6132 | Charybdi s (Charybdi s) natator | GAGGGACGATAAGACCCTATAAAGCTTTATAAATTTAAAGATTTTG TATGTTTTTATATAAAGATAAATTCTAAAGAATTTATTGGGTTGGGG CGACAATGGTATAATGAAAATAACTGCTATTAAAATTAACAACTATA TTTGGATTAAGTTAAATAATTGATCCTTTATAAGGATTTTAAGATCA AGTTACTTTAGGGATAACAGCGTTAT | 607 |
| NC_03 3510 | Procamb arus acutus | AAGGGACGATAAGACCCTATAAAACTTTATATTTATAAGATAGTAG CTAGTTTTATTTAAAAGTATTATTTTGGAGTATTTGGTTGGGGTGAC AAGGATAAAATATAAAATAACTGTCTTTTTTTTTTACAGTAATGTTT GGTTTAATGATCCTAAAAGGGATTAAAGATTAAGTTACTTTAGGGA TAACAGCGTAAT | 608 |
| NC_03 3509 | Pacifasta cus leniusculu s | GAGTGACGATAAGACCCTATAAAACTTTATATTTTAAGATAATAGTT AGTTTTATTTAAGAGTTTTATTTTGAAATATTTTATTGGGGTGATAA AGATATAAATTAAAATAACTGTCTTTTTTTTTTACAATAATCTTTGAA TTAATGATCCTAAGAAAGGAGTAAAAGATCAAGTTACTTTAGGGAT AACAGCGTAAT | 609 |
| NC_03 0255 | Munida gregaria | AAGGGACGATAAGACCCTATAAATCTTTATATTAAATTATTTTTAGT TAATTAATAATGTAATTTTAACAGTAAAATAAAATATATTGTGTTGG GGTGATGAGAATATAAAATTAACTGTTCTAAATTGAAAACAAATTTA TTTGAATTAATAATAGATCCTTAATAGAGATTAAAAGTTTAAGTTAC TTTAGGGATAACAGCGTTAT | 610 |
| NC_02 8627 | Panulirus versicolor | GGGGGACGATAAGACCCTATAAATCTTTATGCTTTAATTAATAAAT CTTAGAAATTAGTTACAAATCTAGCGTCATTGAGTTAAAGTATTTTG TTGGGGCGACAGAAGCATAATAAGTAACTGCTTTCGTTTAAAAAAA TTAATTATTTTTATTGTGATAGTTATATTGATCCTCTTATGGAGAAA CCAGATCAAGTTACTTTAGGGATAACAGCGTAAT | 611 |
| NC_02 9720 | Faxonius rusticus | ATGGGACGATAAGACCCTATAAAACTTTATATTTTAATGTAGAAGT TAATTTTATTTTAAGTTCACTATTTTAAAATTTTTTGTTGGGGCGAC AAGGATATAAAAAATGATAACTATCTTTTATTTTTACAATAATATTTG ATTTATTGATCCTAAAAGGGATTAAAGATTAAGTTACTTTAGGGATA ACAGCGTAAT | 612 |
| NC_02 8225 | Portunus sanguinol entus | GAGGGACGATAAGACCCTATAAAGCTTTATAAATTTAAAGATTTTA CATATTTTATCTATAATGGTAATCTCTGGAGAATTTATTGGGTTGG GGCAACAAAAGTATAATTTATCAATAACTGCTATAAAATAATACAATT ATCTTTGTGTGGGTAGATTAGTTGATCCCTGAAAAGGATTAAAAGA TCAAGTTACTTTAGGGATAACAGCGTAAT | 613 |
| NC_02 8447 | Procamb arus alleni | AAGGGACGATAAGACCCTATAAAACTTTATATTTATAATATAGTAGT TAGTTTTATTTAATAATATTATATTAAAGTATTTGGTTGGGGTGACA AGGATATAATATAAAATAACTGTCTTTTTTTTTTACAGTAATATTTG GTTTAATGATCCTAGAAGGGATTAAAAGATTAAGTTACTTTAGGGA TAACAGCGTAAT | 614 |
| NC_05 0675 | Metacarci nus magister | GGGGGACGATAAGACCCTATAAAGCTTTATATTATCAGCTATGATT TAATTAAATTAAAAAGAAATTAAAGGTTTTAATAAGCTATTATATTTT ATTGGGGAGATATAAGTATAATTTTATGTAACTGCTTATAAGTCGA TACATTGATAATTGGTTTAAATTTTAATGATCCTTTGAATAAAGATT CAAGACCAAGTTACTTTAGGGATAACAGCGTTAT | 615 |
| NC_04 1155 | Puerulus angulatus | GGGGGACGATAAGACCCTATAAATCTTTATATTTGCTCAGCCAAAA AATGTGTATTAGGGCCAAAAATATATTTTTTGGTGGGGTGATATTT TGTTGGGGCGACAGGAGTATAATAAGTAACTGCTTTAAAAGTTTAA | 616 |
| | | TTAGATATATTTAGTTTGAGTGTTAAAAGATCCTTTAGTAAGGATTA TTAAAATAAGTTACTTTAGGGATAACAGCGTAAT | |
| NC_04 0124 | Lupocycl oporus gracilima nus | GAGGGACGATAAGACCCTATAAAGCTTTATAAATTCGATAATTTTG CAAATTTGTACAAATAAAAGTAATTTTGAAAAATTTATTTGGTTGGG GCGACAAAAGTATAATTAAAATAACTGCTAGAATTTCAAACAATAAT TTTTGATTTAGGTTTGTAAATGATCCCTTATAAGGATTAAAAGATCA AGTTACTTTAGGGATAACAGCGTAAT | 617 |
| NC_03 7173 | Monomia gladiator | TAAGGGGACGATAAGACCCTATAAAGCTTTATAAATTCAGAGATTT TAGATATTTTATATATAAATTTATTTTTTGATAATTTATTTGGTTGGG GCGACAAGGGTATAATTATAAATAACTGCCATTAATGTTTATACAT ATATTTTTGATTTAAATTTAATATAGTAGTTGATCCTTTTTAAGGATT TAAAGATCAAGTTACTTTAGGGATAACAGCGTAAT | 618 |
| NC_03 7155 | Varuna yui | TTAAAGTGACGATAAGACCCTATAAAGCTTTATATAGATATATTATT TAATTAAATTTTTAATAAAAATTTAGTAGTAAAATTTATTTTATTGGG GCGATAAGAGTAAAATTTTTATTAACTGCTTTAAGTTTTGAATACAT TTATGTTTGATTAAAATTTTAAATGATCCTAAATAAAGATTAAAAGTT TAAGTTACTTTAGGGATAACAGCGTTAT | 619 |
| NC_03 9671 | Panulirus argus | GGGGGACGATAAGACCCTATAAATCTTTACAATTAGGGCTTTAGTT ATTAGAAAGTAGTTATGGATCTAAATCTAAGGTTTAAATTGTTTTGT TGGGGCGACAGAAGTATAATAAGTAACTGCTTTGGTTTTCATATTA ATTGTTTTTATTATGGTTAGTTTAAAAGATCCTCTTTTGGAGATCAC AGACTAAGTTACTTTAGGGATAACAGCGTAAT | 620 |
| NC_03 9112 | Munida isos | AAGGGACGATAAGACCCTATGAATCTTTATATTATGTTACTTTTAAT TAATTAATGTTGTAGATATAGTGATAAGGTGGGGTATATTGTGTTG GGGTGATGAGAATATAAGAAATAACTGTTCTAAATTTAAAACAAAA TTGTTTGAGGTAATAATAGATCCTTTATTGAGATTAAAAGTTTAAGT TACTTTAGGGATAACAGCGTTAT | 621 |
| NC_04 4425 | Scylla ride s squammo sus | GAGGGACGATAAGACCCTATAAATCTTTATACTTAGGTTTATACGG TTGAAGGTTAGGTTACTATTTAATTTTATAAAGTTAAGTATTTTGTT GGGGTGACAGGGGCATAAGAGTGAATAACTGCCTTAGAGTTAGA GTTAAAAGTATTTAATTTATTAAGTGATCCTTTAGTAGAGAATATAG ACTAAGTTACTTTAGGGATAACAGCGTAAT | 622 |
| NC_01 6925 | Cambaroi des similis | GGGGGACGATAAGACCCTATAAAACTTTATATACTTATAATTTAAT AATAACTTTTGGGTTTAAAAATTTAGATTAGAGTATTTTATTGGGGC GATAAAGATAAAATTTTAGATAACTGTTTTATTTTTTAACAAAAATGT TTGAGTTTTAGATCCTTAAAGAGATTAAAAGATTAAGTTACTTTAGG GATAACAGCGTTAT | 623 |
| NC_03 7695 | Charybdi s bimaculat a | GAGGGACGACAAGACCCTATAAAGCTTTATAAATTTGATAATTTTT TAAATTTATATATAAAAATAATTTTTGTAAAATTTATTTGGTTGGGG CGACAATGGTATAATTAAGATAACTGCTATAAGATTTAACAAAAATA TTTGTTTAATAAATATAATTGATCCTTTTTAAAGATTTAAGATCAAGT TACTTTAGGGATAACAGCGTTAT | 624 |
| NC_03 3507 | Cambaru s robustus | AAGGGACGATAAGACCCTATAAAACTTTATATTTAGAAATAATGGG TAAGTTTTGTTTAGAAGTTATTATTTTAGAGTATTTGGTTGGGGCG ACAAGGATAAAAAGAAGATAACTGTTCTTTTATTTTACAATAATGTT TGGGTATAATGATCCTAATATGGGTTAAAAGATTAAGTTACTTTAG GGATAACAGCGTAAT | 625 |
| NC_03 9640 | Thalamita sima | GAGGGACGATAAGACCCTATAAAGCTTTATAAACTTATAAATTTTA TATGTTTTTTATAAAAATAATCTTTCGAAGGTTTATTTAGTTGGGG CGACAATGATATAAATAATATAACTGTCATAAAATATAACAAATATA TTTGTTTAATAAGGAATAATTGATCCTTTATAAGGATTTTAAGATCA AGTTACTTTAGGGATAACAGCGTTAT | 626 |
| NC_02 4438 | Thranita crenata | AAGGGACGATAAGACCCTATAAAGCTTTATAATTTTAAAGATTTTAT ATATTTTTCTATAGTAATAATTTCTTGAAAACTTATTTGGTTGGGGC GACAATAGTATAATTAGAATAACTGCTTAAAATTAATACAAATATAT CTGAGTTAAAATTGACTGATCCTTTATAAAGATTTTAAGATCAAGTT ACTTTAGGGATAACAGCGTTAT | 627 |
| NC_03 3508 | Orconect es luteus | AGGGGACGATAAGACCCTATAAAACTTTATATTTTAAGATGGAGAT AAATTTTATTTTAAGGTTACTATTTTAAATTGTTTTGTTGGGGCGAC AAGGATATAAGGAAAGGATAACTGTCTTTTGTTCTTACAATAATATT TGAATTATTGATCCTAAAAGGGATTAAAGATTAAGTTACTTTAGGG ATAACAGCGTAAT | 628 |
| NC_03 0768 | Orconect es punctima nus | AAGGGAAGATAAGACCCTATAAAACTTTATATTTTAAGGTAGAAAT TAATTTTATTTTTAGATTACTATTTTAAAATATTTTGTTGGGGTGACA AGGATATAATAAAAGGTAACTGTCTTTTGGTTTAACAATAATATTTG GGTAATTGATCCTAAAATGGATAAAAGATTAAGTTACTTTAGGGAT AACAGCGTAAT | 629 |
| NC_02 9721 | Orconect es sanbornii | TTGGGACGATAAGACCCTATAAAACTTTATATTTTAAGGTAGAAAT TAATTTTATTTTAAGGTTACTATTTTAAAATGTTTTGTTGGGGTGAC AAGGATACAAGAAATGATAACTGTCTTTTATTTTTACAATAATATTT GATTTATTGATCCTGAAAGGGATTAAAGATTAAGTTACTTTAGGGA TAACAGCGTAAT | 630 |
| NC_02 4029 | Cherax crassima nus | AAGGGACGATAAGACCCTATAAAGTTTATATACCGAACTAATTAAA GATAATTAAGAAGGGTAAAGTTTATTATTAGAGAGGTATTTAGTTG GGGCGACTAGGATATAAATTAATTTAACTGTTTTAAATTAAAGTCAA AAATATTTGAGTTTGTGATCCTTTTTTAGGATTAGAGAATAAGTTAC TTTAGGGATAACAGCGTAAT | 631 |
| NC_02 3482 | Cherax preissii | AAGGGACGATAAGACCCTATAAAGTTTATATGTTGAACTAATTAAA GATAATTAGGAAGAGTAAAGTTTATTATTGGTTAGGCATTTAGTTG GGGCGACTGAAATATAAGTTAATTTAACTGTTTTAAATTAAGGTCA AAGATGTTTGAGTTTGTGATCCCTTTTTAGGATGATAGAATAAGTT ACTTTAGGGATAACAGCGTAAT | 632 |
| KF051 309 | Munida spinosa | AAGGGACGATAAGACCCTATGAATCTTTATATTATGTCACTTTAAG TTAGTTAATATCGTAGATATAATGATAAGGTGAGGTGTATTGTGTT GGGGTGATGAGAATATAAGAAATAACTGTTCTAAATTTAAGACAAA ATTGTTTGTGGTAATAATAGATCCTTTATTGAGATTAAAAGTTTAAG TTACTTTAGGGATAACAGCGTTAT | 633 |
| MK847 971 | Munida asprosom a | AAGGGACGATAAGACCCTATAAATCTTTATATTAGATTATTTTTAGT TATTTAATATTGTGAGTATAACAATAAAGTGGTATATATTGTGTTGG GGTGATGAGAATATAATAAATAACTGTTCTAAAATTAAAACAAAATT ATTTGTATAAATAATAGATCCTTTATTGAGATTAAAAGTTTAAGTTA CTTTAGGGATAACAGCGTTAT | 634 |
| MK847 969 | Munida leagora | AAGGGACGATAAGACCCTATAAATCTTTATATTAAATTATTTTTAGT TGATTAATATTGTAAATATAATAATAAAATATCAAATATTGTGTTGG GGCGACGAGAATATAATAAATTAACTGTTCTAAAATTAAAACAAAA TTGTTTGTAAAAATAATAGATCCTTTATAAAGAGTAAAAGTTCAAGT TACTTTAGGGATAACAGCGTTAT | 635 |
| AY351 105 | Munida alonsoi | AAGGGACGATAAGACCCTATAAATCTTTATATTAAATTGTTTAATAT TTAATTAATATTGTGTACTAGATATTTAAATAATATATATTGTGTTGG GGTGACGAGAATATAATAAATAGCTGTTCTAAAGTAAAAACAATTT TTATTGTGGTAATAGAAGATCCTTTATTGAGATTGAAAGTTTAAGTT ACTTTAGGGATAACAGCGTTAT | 636 |
| AY351 177 | Munida taenia | AAGGGACGATAAGACCCTATAAATCTTTATATTAAATTATTTTCAAT TAATTAATACTGTAAATACAATAATAAAATAATAAATATTATGTTGG GGCGATGAGAATATAATAAATAACTGTTCTAAAATTTATACAAAATT ATTTGTTACAATAATAGATCCTTTTATAGAGATTAAAAGTTTAAGTTA CTTTAGGGATAACAGCGTTAT | 637 |
| MK847 957 | Munida gordoae | GAGGGACGATAAGACCCTATAAATCTTTATATTAAGTTGTTTTTTAC TAATTAATATTGTAGATGAAGTAATAAAATTATATATATTGTGTTGG GGCGATGAGAATATAAATAAATAACTGTTCTAGTGTAGAAAAACAA AATTATTTGGATATTGGTAGATCCTTTAGTTAAGATTAAAAGTTTAA GTTACTTTAGGGATAACAGCGTTAT | 638 |
| AY351 190 | Munida zebra | AAGGGACGATAAGACCCTATAAATCTTTATACTAGGATATTTTTAG TTAATTAATATTGTAAATATAATAATAAAATGACATGTATTGTGTTG GGGTGATGAGAATATAATAAATAACTGTTCTAAGATGAAAACAAAA TTATTTGTAGTAATAATAGATCCTTTATTGAGATTGTAAGTTTAAGT TACTTTAGGGATAACAGCGTTAT | 639 |
| AY351 119 | Munida distiza | AAGGGACGATAAGACCCTATAAATCTTTATATTAAACTGTTTTCAAT TAATTAATATTGTTAATATAATAGTAAAAGAGTAAATATTATATTGG GGCGATAAGAATATATAAATTAACTGTTCTAAAGTTAAAACAAAATT ATTTGTAAAAATAATAGATCCTTAATAAAGATTAAAAGTTCAAGTTA CTTTAGGGATAACAGCGTTAT | 640 |
| AY351 158 | Munida psamathe | AAGGGACGATAAGACCCTATAAATCTTTATATTAAGTTATTTTTAGT TAATTAATATTGTAAATGAAATTACAAAATAATATATATTGTGTTGG GGTGATGAGAATATAATAAATATCTGTTCTAAAATAAAAACAATTTT TTTGTGTTAGTAGAAGATCCTTTGTTGAGATTAAAAGTTTAAGTTAC TTTAGGGATAACAGCGTTAT | 641 |
| AY351 181 | Munida thoe | GAGGGACGATAAGACCCTATAAATCTTTATATTAGGTTATTTTATA GTTAGTTAATATTGTAAATATAATAATAAGGTGAAATATATTATGTT GGGGTGATGAGAATATAATAAATAACTGTTCTAAGATAAAAACAAA ATTGTTTGTAAAATAGTAGATCCTTTGTTGAGATTAGAAGTTTAAGT TACTTTAGGGATAACAGCGTTAT | 642 |
| AY351 130 | Munida guttata | AAGGGACGATAAGACCCTATAAATCTTTATATTAAACTGTTTTCAAT TAATTAATATTGTTAATATAATAGTAAAATAGTAAATATTATATTGGG GTGATAAGAATATATAAATTAACTGTTCTAAAATTAAAACAAAATTA TTTGTAGTAATAATAGATCCTTAATAAAGATTAAAAGTTCAAGTTAC TTTAGGGATAACAGCGTTAT | 643 |
| AY351 170 | Munida stia | AAGGGACGATAAGACCCTATAAATCTTTATATCAAATTATTTTTAGT TGATTAATATAGTAGATATTAATAATAAAATAACACATATTATATTG GGGTGATAAGAATATAAAAAATAACTGTTCTAAAATTAAAACAAAAT TATTTGTAGTAGTTATAATAGATCCTTTATTGAGATCCAAAGTTTAA GTTACTTTAGGGATAACAGCGTTAT | 644 |
| AY351 150 | Munida ommata | AAGGGACGATAAGACCCTATAAATCTTTATATTAAGTTAATTTTTTA GTTAATTAATATTGTAAATATAATAGTAAGATGGCGTATATTGTGTT GGGGTGATGAGAATATAATAAATAACTGTTCTAAAATATAAACAAT ATTGTTTGTAAATAATAGTAGATCCTTTATTGAGATTAAAAGTTTAA GTTACTTTAGGGATAACAGCGTTAT | 645 |
| MK847 958 | Munida roshanei | AAGGGACGATAAGACCCTATAAATCTTTATATTAAACTATTTAAAAT TAATTAATATGTAAATTTAATAAATAAAATGGAAAATATTATATTGG GGTGATAAGAATATAAATTATATAACTGTTCTAAAATTTAAACAAAA TTATTTGTACAAATAGTAGATCCTTTTTTAAGATTAAAAATTTAAGTT ACTTTAGGGATAACAGCGTTAT | 646 |
| MK847 944 | Munida compress a | AAGGGACGATAAGACCCTATAAATCTTTATATTAGATTATTTTTAAG TTATTTAATATTGTAAATATAATAATAAAATAATATATATTGTGTTGG GGTGATGAGAATATAATGAATAACTGTTCTAAAATGAAAACAAAAT TATTTGTGAAAATAATAGATCCTTTATTGAGATTAAAAGTTTAAGTT ACTTTAGGGATAACAGCGTTAT | 647 |
| AY351 113 | Munida clinata | AAGGGACGATAAGACCCTATAAATCTTTATATTAAACTACCTTAAAT TAATTAATATTGTAAATAATAACGGTAAGGTAGAAAATATTATATTG GGGTGATAAGAATATAAAATAATATAACTGTTCTAAAATTTAAACAA AATTATTTGTAATAATAATAGATCCTTTTTTAAGATCAAAAATTTAAG TTACTTTAGGGATAACAGCGTTAT | 648 |
| MK847 964 | Munida chydaea | GAGGGACGATAAGACCCTATAAATCTTTATATTAAATTATTTTTAGT TAATTAATATTGTGAACATAATAATAAAGTAAGGAAATATTGTGTTG GGGTGATGAGAATATAAATAATAACTGTTCTAAAATTAAAACAAAAT TATTTGTAAGTAATAATAGATCCTTTATTGAGATTAAAAGTTTAAGT TACTTTAGGGATAACAGCGTTAT | 649 |
| MK847 941 | Munida compacta | AAGGGACGATAAGACCCTATAAATCTTTATATTAGATTATTTTTAGT TGTTTAATATTGTAAATATAATAATAAAATGATATATATTGTGTTGG GGTGATGAGAATATAATAAATAACTGTTCTAAAATGAAAACAATATT ATTTGAATGAATAGTAGATCCTTTATTGAGATTAAAAGTTTAAGTTA CTTTAGGGATAACAGCGTTAT | 650 |
| AY351 122 | Munida eclepsis | AAGGGACGATAAGACCCTATAAATCTTTATATTAGATTATTTTTAGT TATTTAATATTGTAAATATAATAATAAAGTGGTGTATATTATGTTGG GGTGATGAGAATATAATAAATAACTGTTCTAAAATAAAAACAAAATT GTTTGTATAGATAATAGATCCTTTATTGAGATTAAAAGTTTAAGTTA CTTTAGGGATAACAGCGTTAT | 651 |
| AY351 185 | Munida tyche | AAGGGACGATAAGACCCTATAAATCTTAATATTAAGTTATTTTTAGT TAATTAATATCTGTAAATACAATAATAAAATATTAAATATTATGTTGG GGTGACAAGAATATAATAAATAACTGTTCTAAAATTTAAACAAAATT ATTTGTAAAAAAAAAAGATCCTTTTTAAAGATTAAAAGTTTAAGTTA CTTTAGGGATAACAGCGTTAT | 652 |
| MK847 953 | Munida philippine nsis | AAGGGACGATAAGACCCTATAAATCTTTATATTAAATTATTTTTATT TTATTAATACCGTAGATATAATGATAAAGTAATAAATATTATGTTGG GGTGATAAGAATATAAAAAATTAACTGTTCTAAGATTAAAACAAAAT TATTTGAAAAAAAAAATAATAGATCCTTTATAAAGATTAAAAGTTTAA GTTACTTTAGGGATAACAGCGTTAT | 653 |
| AY351 107 | Munida armilla | AAGGGACGATAAGACCCTATAAATCTTTATATTAAATTATTTTCAAT TAATTAATATTGTAAATAAAATAATAAAATAATAAATATTATATTGGG GCGATGAGAATATAACAAAATAACTGTTCTACAATTAATACAAAATT ATTTGTAATAATAATAGATCCTTTATAAAGATTAAAAGTTTAAGTTA CTTTAGGGATAACAGCGTTAT | 654 |
| KY230 470 | Munida mesembri a | AAGGGACGATAAGACCCTATAAATCTTAATATTAAATTATTTTTAGT TAATTAATAATTGTAAATATAATAATAAAATATTAAATATTATGTTGG GGTGACAAGAATATAATAAATAACTGTTCTAAAATTAAAACAAAATT ATTTGTAAAAAAAATGATCCTTTATAAAGATTAAAAGTTCAAGTTAC TTTAGGGATAACAGCGTTAT | 655 |
| AY351 166 | Munida spilota | AAGGGACGATAAGACCCTATAAATCTTTATATTAAATTATTTTTAAT TAATTAATATTGTAAATTTAATAATAAAATATTAAATATTATGTTGGG GCGACAAGAATATAATAAATAACTGTCTAAATTTAAAACAAAATTAT TTGTAATAAAATAGATCCTTTTTAAAGATTAAAAGTTTAAGTTACTTT AGGGATAACAGCGTTAT | 656 |
| KY230 468 | Munida benguela | AAGGGACGATAAGACCCTATAAATCTTTATATTAGATTATTTTTAGT TATTTAATATTGTAAATATAGTAATAAAATGATATATATTGTGTTGG GGTGATGAGAATATAATAAATAACTGTTCTAAAATGAAAACAAAATT ATTTGAATAAATAGTAGATCCTTTATTGAGATTAAAAGTTTAAGTTA CTTTAGGGATAACAGCGTTAT | 657 |
| MK847 934 | Munida endeavou rae | AAGGGACGATAAGACCCTATAAATCTTTATATTAAATTATTTTTAAT TAGTTAATATTGTAAATATAATAGTAAGGTAAAGTATATTGTGTTGG GGTGATGAGAATATAAGAAATAACTGTTCTAAAATAAAAACAAAAT TATTTGAAGAAATAATAGATCCTTTGTTGAGATTAAAAGTTTAAGTT ACTTTAGGGATAACAGCGTTA | 658 |
| MK847 961 | Munida agave | AAGGGACGATAAGACCCTATAAATCTTAATATTAAATTATTTTTAGT TAATTAATAGTTGTAAATATAATAATAAAATATTAAATATTATGTTGG GGTGACAAGAATATAATAAATAACTGTTCTAAAATTTAAACAAAATT ATTTGTAAAAAAAGATCCTTTATAAAGATTAAAAGTTTAAGTTACTT TAGGGATAACAGCGTTAT | 659 |
| MK458 566 | Munida idyia | GAGGGACGATAAGACCCTATAAATCTTTATATTAGGTTATTTTCAG TTGATTAATATTGTAAATATAATAATAAGATAATATATATTGTGTTG GGGTGATGAGAATATAATGAAATAACTGTTCTAGGATAGAAACAAA ATTATTTGAAGTAATAAATGATCCTTTATTGAGATTAAAAGTTTAAG TTACTTTAGGGATAACAGCGTTAT | 660 |
| AY351 143 | Munida militaris | AAGGGACGATAAGACCCTATAAATCTTTATATTAGATTATTTTTAGT TATTTAATATTGTAAATATAATAATAAAGTGATTTATATTGTGTTGG GGTGATGAGAATATAATAAATAACTGTTCTAAAATGAAAACAAAATT ATTTGTGTAAATAATAGATCCTTTATTGAGATTAAGAGTTTAAGTTA CTTTAGGGATAACAGCGTTAT | 661 |
| MF490 158 | Munida flinti | AAGGGACGATAAGACCCTATAAATCTTTATATTAACTTATTTTTTAT ATATTAATCTTGTAAACTCCGTTTAATAAAATAAGATATATTTTGTT GGGGCGACAAGAATATAAACACAACTGTTCTAAATACTAAACAAAA CTATTTGTTTTAAATTCATAGATCCTTCATTTAAGATTATAAGTTTAA GTTACTTTAGGGATAACAGCGTTAT | 662 |
| AY351 115 | Munida congesta | AAGGGACGATAAGACCCTATAAATCTTTATATTAGATTATTTTTAGT TATTTAATATTGTAAATATAGTAATAAAATAATATATATTGTGTTGG GGTGATGAGAATATAATAAATAACTGTTCTAAAATATAAACAAAATT ATTTGAATAAATAGTAGATCCTTTATTGAGATTAAAAGTTTAAGTTA CTTTAGGGATAACAGCGTTAT | 663 |
| AY351 163 | Munida rubridigita lis | AAGGGACGATAAGACCCTATAAATCTTTATATTAGATTATTTTTAAG TTGTTTAATATTGTAAATATAATAATAAAATAATATATATTGTGTTGG GGTGATGAGAATATAATAAATAACTGTTCTAAAATGAAGACAAAAT TATTTGTATAAATAATAGATCCTTTATTGAGATTAAAAGTTTAAGTT ACTTTAGGGATAACAGCGTTAT | 664 |
| KF182 521 | Munida iris | AAGGGACGATAAGACCCTATAAATCTTTATATTAAATTATTTTTAAT TTGTTAATTATGTAAATTTAATAACAAGATAAAATATATTATGTTGG GGTGATGAGAATATATAATCAACTGTTCTAAATTAAAAACAAAATTA TTTGAACAAAATAGTAGATCCTTATTAGAGATTAAAAGTTTAAGTTA CTTTAGGGATAACAGCGTTAT | 665 |
| MF490 159 | Munida microphth alma | AAGGGACGATAAGACCCTATAAATCTTTATATTAAATTATTTTAAAT TAATTAATATTGTAAATAAGATAATAAAATAATATATATTGTGTTGG GGTGATGAGAATATAATAAATAACTGTTCTAAAATAAAAACAAAGC TATTTGTATAAAATAATAGATCCTTTACTAAGATTAAAAGTTTAAGT TACTTTAGGGATAACAGCGTTAT | 666 |
| AY351 164 | Munida rufiantenn ulata | GGGGGACGATAAGACCCTATAAATCTTTATATTAAGTTATTTTTAG TTAATTAATATTGTAAATATAATAATAAAATAGCGTGTATTGTGTTG GGGTGATGAGAATATAATAAATAACTGTTCTAAAATGTAAACAATA TTATTTGTAAATAATAGTAGATCCTTTATTGAGATTAAAAGTTTAAG TTACTTTAGGGATAACAGCGTTAT | 667 |
| KF182 522 | Munida pusilla | AAGGGACGATAAGACCCTATAAATCTTTATATAAAATTATCTTTAAT TTATTTATAATGTAAGTTTAATATAAAGATAAGATATATTACATTGG GGTGATGAGAATATATAATAAACTGTTCTAGATTCAAAACAAATTTA TTTGTATTATAAAATAGTAGATCCTTTTTTAAGATTAAAAGTTTAAGT TACTTTAGGGATAACAGCGTTAT | 668 |
| KY230 472 | Munida remota | AAGGGACGATAAGACCCTATAAATCTTTATATTAGATTATTTTTAGT TATTTAATATTGTGAATATAATAATAAAGTGGTATATATTGTGTTGG GGTGATGAGAATATAATAAATAACTGTTCTAAAATTAAAACAAAATT ATTTGTATAAATAATAGATCCTTTATTGAGATTAAAAGTTTAAGTTA CTTTAGGGATAACAGCGTTAT | 669 |
| AY351 141 | Munida leptosyne | AAGGGACGATAAGACCCTATAAATCTTTATATTAATTTATTTGTTTA TTGATTAATAAGTAGATGATATAATAAGATAATATATATTATGTTGG GGTGATGAGAATATAATAATTAACTGTTCTAAGTTGTAAACAAAATT ATTTGAAATTTTATAGATCCTTTATTGAGATTAAAAGTTTAAGTTAC TTTAGGGATAACAGCGTTAT | 670 |
| AY351 162 | Munida rosula | AAGGGACGATAAGACCCTATAAATCTTTATATTAGATTATTTTTAGT TATTTAATATTGTAAATATAATAATAAAGTGATATATATTGTGTTGG GGTGATGAGAATATAATAAATAACTGTTCTAAAATGAAAACAAAATT ATTTGTGTAAATAATAGATCCTTTATTGAGATTAAAAGTTTAAGTTA CTTTAGGGATAACAGCGTTAT | 671 |
| LC464 553 | Munida munin | TCTTTATATTAAACTATTTAAAATTAATTAATATGTAAATTTAATAAA TAAAATGGAAAATATTATATTGGGGTGATAAGAATATAAATTATATA ACTGTTCTAAAATTTAAACAAAATTATTTGTACAAATAGTAGATCCT TTTTTAAGATTAAAAATTTAAGTT | 672 |
| JN800 548 | Munida valida | AAGGGACGATAAGACCCTATAAATCTTTATATTAAGTTATTTTAAAT TAATTAATGTTGTAAATAAAATAATAAAGTAATATATATTGTGTTGG GGTGATGAGAATATAATAAATAACTGTTACTAAATTTAAAAACAAGT TATTTGAATTTAAAATAATAGATTCCTTTTTGAGTATTAAAAGTTTAA GTTACTTTAGGGATAACAGTCGTTA | 673 |
| AY351 153 | Munida proto | GAGGGACGATAAGNCCCTATAAATCTTAATATTAAATTATTTTTAAT TAATTAATATTGTAAATATAATAATAAAATATTAAATATTATGTTGGG GTGATAAGAATATAAAAAATAACTGTTCTAAATTTAAAACAAAATTA TTTGAAAATAATAGATCCTTTTTTAAAGATTAAAAGTTCAAGTTACT TTAGGGATAACAGCGTTAT | 674 |
| AY351 142 | Enriquea levianten nata | AAGGGACGATAAGACCCTATAAAGCTTTATATTATTTTTTTTTATA GATTATTGTTGTGGATATAATAATAAAATAAGGAATATTATATTGGG GTGATAAGGGTATAAAATTAACTGCTCTAAAATTAAATCAAATTTAT TTGTAAAATAATAATAGATCCTTTATTAAGATTAAAAGTTTAAGTTA CTTTAGGGATAACAGCGTTAT | 675 |
| LC430 735 | Munida multilinea ta | TCTTAATATTAAACTATTTTTAGTTAATTAATTAATTGTAAATATAAA TAATAAAATATTAAATATTATGTTGGGGTGACAAGAATATAAAAAT AACTGTTCTAAATTTAAAACAAAATTATTTGAAAAAAAGATCCTTTA TAAAGATTAAAAGTTCAAGTT | 676 |
| AY351 152 | Munida pagesi | AAGGGACGATAAGACCCTATAAATCTTTATATTATATTATTTTTAGT TAATTAATATTGTAAATATAATAATAAGGTAATATATATTATGTTGG GGTGATAAGAATATAATAAATAATTGTTCTAAAATGAAAACAAAATT GTTTGTGTTAATAATAGATCCTTTATTGAGATTAAAAGTTTAAGTTA CTTTAGGGATAACAGCGTTAT | 677 |
| KY230 475 | Munida stomifera | GAGGGACGATAAGACCCTATAAATCTTTATATTAAGTTGTTTTTAG CTGATTAATGTTGTAGATTCAGTATTAAGACAAAATATATTATGTTG GGGTGATGAGAATACAATAGATACCTGTTCTAAAATATCAAACAAA AATTTTTGTGTGAGTAGAAGATCCTCTTTATTGAGATCAGGAGTTT AAGTTACTTTAGGGATAACAGCGTTAT | 678 |
| AF436 050 | Munida quadrispi na | GGGACGATAAGACCCTATAAATCTTTATATTAAATTGTTTTTAGTTT ATTAATAATGTAATTTAGATAATAAGGCAAAATATATTGTGTTGGGG TGATGGGAATATAAAAAATTAACTGTTCTGAAATAAAAACAAATTTG TTTGGCTTAATAATAGATCCTTTTTTAAGATTAAAAGTTTAAGTTAC TTTAGGGATAACAGCGTTAT | 679 |
| AY351 183 | Munida tiresias | AAGGGACGATAAGACCCTATAAATCTTTATATTAAGTTGTTTTTAGT TAATTTATATTGTAAATAAGATAATAAAGTAAAATATATTGTGTTGG GGTGATGAGAATATAATAAATAACTGTTCTAAAATAAAAACAAAGC TATTTGTATTAAATAATAGATCCTTTATTAAGATTAAAAGTTTAAGTT ACTTTAGGGATAACAGCGTTAT | 680 |
| AY351 159 | Munida psylla | ATGGGACGATAAGACCCTATAAATCTTTATATTAAGTTATTTTTAGT TAATTAATATTGTAAATATAATAATAAAATAATGTATATTGTGTTGG GGTGATGAGAATATAATAAATAACTGTTCTAAAATGTAAACAATATT GTTTGTAATAATAGTAGATCCTTTATTGAGATTAAAAGTTTAAGTTA CTTTAGGGATAACAGCGTTAT | 681 |
| MK847 965 | Munida heteracan tha | AAGGGACGATAAGACCCTATAAATCTTTATATTAAACTGTTTTCAAT TAATTAATATTGTAAATATAATAATAAAATAGTAAATATTATGTTGG GGTGATGAGAATATAAGAAATAACTGTTCTAAAATTAAAACAAAATT GTTTGTAATAATAATAGATCCTTTATAAAGATTAAAAGTTTAAGTTA CTTTAGGGATAACAGCGTTAT | 682 |
| FJ462 645 | Paralomis formosa | AAAAGACGATAAGACCCTATAAATCTTTACAAGAAATATATTTTATG TTTTAGCTTATAAGTATATAAGAAATAAAAATATAAGTTTGTTGCGC TGGGGCGGCGTAGATATATAAATAAACTGTCTATGGTTTAAATACA ATAATTATTGCTTAATACAAATTGATCCTTAAATAGATTAAAAGATT AAGATACTTTAGGGATAACAGCGTTAT | 683 |
| FJ462 646 | Paralomis spinosissi ma | AAAAGACGATAAGACCCTATAAATCTTTACAAGAAATATATTTTATG TTTTAGCTTATAAGTGTATAAGAAATAAAAATATAAGTTTGTTGCGC TGGGGCGGCGTAGATATATAAATAAACTGTCTATGATTTAAATACA ATAATTATTGYTTAATACARATTGATCCTTAAATAGATTAAAAGATT AAGATACTTTAGGGATAACAGCGTTAT | 684 |
| KY426 326 | Paralomis birsteini | AAAAGACGATAAGACCCTATAAATCTTTACAAGAAATATATTTTATG TTTTAGCTTATAAGTGTATAAGAAATAAAAATATAAGTTTGTTGCGC TGGGGCGGCGTAGATATATAAATAAACTGTCTATGGTTTAAATACA ATAATTATTGCTTAATACAGATTGATCCTTAAATAGATTAAAAGATT AAGATACTTTAGGGATAACAGCGTTAT | 685 |
| KY426 327 | Paralomis hirtella | AAAAGACGATAAGACCCTATAAATCTTTACAAGAAATATATTTTATG TTTTAGCTTATAAGTGTATAAGAGATAAAAATATAAGTTTGTTGCGC TGGGGCGGCGTAGATATATAAATAAACTGTCTGTGGTTTAAATACA ATAATTATTGCTTAATACAAATTGATCCTTAAATAGATTAAAAGATT AAGATACTTTAGGGATAACAGCGTTAT | 686 |
| MF460 387 | Scyllarus subarctus | AAGGGACGATAAGACCCTATAAATCTTTATACTTGATTTTATAGAG CAAAATATTAAAATAATTTTTGTTCAATAGGTTTGGGTATTTTGTTG GGGCGACAGGAGTATAAAATGTAACTGCTCTAAAATGATAGTTAG TTGATATTTAGTTAAGTGATCCTTTAATAAAGATGTTAGTTTAAGTT ACTTTAGGGATAACAGCGTAAT | 687 |
| FJ174 908 | Scyllarus pygmaeu s | GGGGGACGATAAGACCCTATAAATCTTTATATTTAAGCTTATAAAA TAAACGATTAAAATAATTTTTGTTTAATAAGCTTATATATTTTGTTGG GGCGCCAGAAGTATAAAATGTAACTGCTTTAAAATAATAGTTAAAT GATATTTAGTTAAGTAATCCTTTAATAAAGATGTTAGTTTAAGTTAC TTTAGGGATAACAGCGTAAT | 688 |
| JN701 734 | Scyllarus chacei | GGGGGACGATAAGACCCTATGAATCTTTATATTTCGGTTTATAAGA TAAAAAATTAATATAATTTTTGTCTTATTAATCTTTTTATTTTGTTGG GGCGACAGAAGCGTAAACTGTAACCGCTTTGAAAAAATAGTTAAT GATATTTAGTTAAGTGACCCTTCTATGGGGGTATCAGTATAAGTTA CTCTAGGGATAACAGCGTAAT | 689 |
| FJ174 909 | Scyllarus caparti | AGGGGACGATAAGACCCTATAAATCTTTATATTTGGGTTTGTGAAA TAAAAAATTAAAATAATTTTTGTTTAATAAGCTTGGGTATTTTGTTG GGGCGACAAGAGTATAAAAATGTAACTGCTTTGAAATAATAGTTAA TTGGTGTTTAGTTAATTGATCCTTTAGTAGAGATGTCAGTTTAAGTT ACTTTAGGGATAACAGCGTAAT | 690 |
| JN701 732 | Scyllarus american us | GGGGGACGATAAGACCCTATGAATCTTTATTTTTGGCTTATAAAAT AAAAAAATAATATCAATTTTTGTTTTATCGGTTTCAATATTTTGTTGG GGCGACGGAAGCGTAAATTGTAACCGCTTTATATTAATAGTTAACG ATATTTAGTTAAGTGATCCTTTATAGAGATATTAGTATAAGTTACTC TAGGGATAACAGCGTAAT | 691 |
| FN295 576 | Episesar ma palawane nse | AAAAGACGATAAGACCCTATAAAACTTAATAAAAATTGATATTTAAA TAAAATTTATTAATAAATATTTAGTAATTAATTTTTATTTTATTGGGG TGATAATGGTAAAATGGATATTAACTGTTAATAAAGTTTATAACAAA AATTATTGAGTAAGTACAATAAATGATCCTTATTAAAGATTAAAAGT TTAAGTTACTTTAGGGATAACAGCGTTAT | 692 |
| FN295 575 | Episesar ma singapore nse | AAAAGACGATAAGACCCTATAAAACTTAATATAAATTGATTATTTGA GTAAAATTTTATTGTATAAATATTTAATAATTAATTTATATTTTATTG GGGTGATAATGGTAAAATGATTATTAACTGTTAATTATTTTATGACA AAAATTATTGAATAAATGTGTAATAAATGATCCTTATTAAAGATTAA AAGTTTAAGTTACTTTAGGGATAACAGCGTTAT | 693 |
| KP712 873 | Austropot amobius fulcisianu s orientalis | GGGGGACGATAAGACCCTATAAAACTTTATATTTTAAAAATATTAAT TAATTTTGTATGAGAAGGCTATTTTTAAATATTTTATTGGGGTGATA GGGATATAATAAAAGATAACTGTTTCTTTTTTTTACAGAGGTGTTTG AGTAAAAGATCCTAATTAGGGAAAGAAGGTTAAGTTACTTTAGGGA TAACAGCGTAAT | 694 |
| FJ152 150 | Achelous tumidulus | GGGGGACGATAAGACCCTATAAAGCTTCATAAATTTATAGATTTTA TGCATTTAATATAAAAATAGTTTCTGCAGTATTTATTTGGTTGGGGC GACAAAAGTATAATTTTTAATAACTGCTATTTGTAAGTTACAATTAT ATTTGGTTAGTGAGTAATTGATCCTTCTGTGAGGAGTAAAAGTTCA AGTTACTTTAGGGATAACAGCGTCAT | 695 |
| MG51 5565 | Achelous asper | GGGGGACGATAAGACCCTATAAAGCTTTATAAATTCAAGAATTTTA TATATTTAGTATAAAAATAATTTTCTCGATGTTTATTTGGTTGGGGC GACAAAAGTATAATTTTGAATAACTGCTAGTTCAGACATACAAAAA TCTCTGATTTATAAATAATGGATCCCTTTATGAGGATTAAAAGTTCA AGTTACTTTAGGGATAACAGCGTTAT | 696 |
| DQ388 067 | Achelous sebae | GAGGGACGATAAGACCCTATAAAGCTTTATAAGTTTATAAATTTTA TACATTTGTAAATAAAAATAATTTTTTTAATACTTATTTGGTTGGGG CGACAAAAGTGTAATTTAAAATAACCGCTAATATATTAATACAATAA TTTTTGGCTAACTAAGTAATGGATCCCTTTTAAGGAATAAAAGACC AAGTTACTTTAGGGATAACAGCGTTAT | 697 |
| MG51 5559 | Portunus acuminat us | GGAAGACGATAAGACCCTATAAAGCTTTATAAGTTTATAAATTTTG TATATTTAACCTAAAAATAATTTTTTAGGTGCTTATTTGGTTGGGGC GACAAAAGTATAATTACATTGATTTTTTATTTAAAAATAATGGATCC TTCTGTGAAGAATAAAAGTTCAAGTTACTTTAGGGATAACAGCGTA AT | 698 |
| MG51 5578 | Achelous tuberculat us | GGGGGACGATAAGACCCTATAAAGCTTTATAAGTTTATGGATTTTA TAGATTTARAATAAAGATAATTTCCTGAATATTTATTTGGTTGGGGC GACAAAAGTATAATTTAAATAACTGCTAGTTGAAGATTACAGTGAT TTTTGATTCTAAAATAATGGATCCCTTATAAGGATTAAAAAATCAAG TTACTTTAGGGATAACAGCGTTAT | 699 |
| MG51 5567 | Achelous iridescen s | GGGGGACGATAAGACCCTATAAAGCTTTATAAATTTATGAATTTTA TTTATTTAATATAAAAATAATTTTTACAATATTTATTAGGTTGGGGC GACAAAAGTATAATTCGAAATAACTGCTATTTTAAAATTACAATAGT TTTTGTTTTAAAAGTAATTGATCCCTTTATGAGGAGTAAAAGTTCAA GTTACTTTAGGGATAACAGCGTTAT | 700 |
| MG51 5580 | Portunus xantusii | GGGGGACGATAAGACCCTATAAAGCTTCATAAATTTATAAATTTTA TGTATYTAATATAAAGATAATTTTTGTAACGTTTATTTGGTTGGGGC GACAAAAGTATAATTTCAAATAACTGCTAGGTTTGATTTACAATGAT AGTTGATTAATAAGTAATTGATCCCTTGTGAGGAATAAAAGTTCAA GTTACTTTAGGGATAACAGCGTTAT | 701 |
| DQ388 065 | Achelous depressifr ons | GGGGGACGATAAGACCCTATAAAGCTTTATAAATTTATTAATTTTG TATATTTAAAATAAAAACAATCTTTTCAATATTTATTTGGTTGGGGC GACAAAAGTGTAATTAAAGTAACCGCTAATAATATAATTACAATAAT TTTTGATTAGCAATTAATGGATCCCTTTTAAGGAATAAAAGATCAA GTTACTTTAGGGATAACAGCGTTAT | 702 |
| DQ388 063 | Achelous rufiremus | AGGGGACGATAAGACCCTATAAAGCTTTATAAGTTTATGGATTTTA TATATTTAATATAAAAATAATTTTTTAAATATTTATTTGGTTGGGGCG ACAAAAGTATAATTGGAAATAACTGCTAACTTTAAGTACCACTACA TCTTGTATAAAAAATAATCGGATTCTTTATAAAGAGTAAAAGTTCAA GTTACTTTAGGGATAAAAGCATCAT | 703 |
| DQ388 057 | Achelous gibbesii | GGGGGACGATAAGACCCTATAAAGCTTTATAAATTTAAAAATTTTA TATATTTATCATAAAGATAATTTTTCAATGTTTATTTGGTTGGGGCG ACAAAAGTATAATTTAAAATAACTGCTAGTTTGAAAACACAATAATT TTTGTTTTAAAAAGTAATGGATCCCTTTATGAGGATTAAAAGTTCAA GTTACTTTAGGGATAACAGCGTCAT | 704 |
| MG51 5573 | Portunus minimus | GGGGGACGATAAGACCCTATAAAGCTTTATAAATTTATAAATTTTA TGTATTTAATATAAAGATAATTTTTGTAACGTTTATTTGGTTGGGGC GACAAAAGTATAATTTCAAATAACTGCTAGGTTTGGTTTACAATGA TAGTTGATTAATAAGTAATTGATCCCTTGTGAGGAATAAAAGTTCA AGTTACTTTAGGGATAACAGCGTTAT | 705 |
| MG51 5576 | Achelous stanfordi | GGAAGACGATAAGACCCTATAAAGCTTTATAAGTCTACGAATTCTG TAGATCTGATATAAAAATAATTTTTTTGATACTTATTTGGTTGGGGC GACAAAAGTATAATTTAAATAACTGCTAATTTAAAATTACAATGATT TTTGATTATAAAAATAATGGATCTTTCTCTGAAAAATGAAAGTTCAA GTTACTTTAGGGATAACAGCGTAAT | 706 |
| MG51 5566 | Achelous breviman us | GGGGGACGATAAGACCCTATAAAGCTTTATAAATTTATAAATTTTA TGTATTTAATATAAAGATAATTTTTGTAACGTTTATTTGGTTGGGGC GACAAAAGTATAATTTTAAATAACTGCTAGGTTTGATTTACAGCGA TAGTTGATTAATAAGTAATTGATCCCTTGTAAGGAATAAAAGTTCAA GTTACTTTAGGGATAACAGCGTTAT | 707 |
| MG51 5560 | Portunus affinis | GGGGGACGATAAGACCCTATAAAGCTTCATAAATTTATAAATTTTA TGTATCTAATATAAAGATAATTTTTGTAACGTTTATTTGGTTGGGGC GACAAAAGTATAATTTCAAATAACTGCTAGGTTTGATTTACAATGAT AGTTGATTAATAAGTAACTGATCCCTTGTGAGGAATAAAAGTTCAA GTTACTTTAGGGATAACAGCGTTAT | 708 |
| MG51 5561 | Achelous angustus | GGGGGACGATAAGACCCTATAAAGCTTTATAAATTTATAGATTTTA TTTATTTAATAATAAAAATAATTTCTACAGTGTTTATTTGGTTGGGG CGACAAAAGTATAATTTTGAATAACTGCTAGTTCTAAGTTACAACA ATATTTGATTTGTAAGTAATTGATCCTTCTATGAGGAGTAAAAGTTC AAGTTACTTTAGGGATAACAGCGTTAT | 709 |
| DQ388 062 | Achelous binoculus | GGGGGACGATAAGACCCTATAAAGCTTTATAAATTTTATAGACTTT ATATATTTAAAATAAAAATAATTTCTAAAACATTTATTTGGTTGGGG CGACAAAAGTATAATTTCAAATAACTGCTATTTTAAAATTACAATAA TTTTTGTTTTATGAATAATTGATCCTTCTATGAGGAGTAAAAGTTCA AGTTACTTTAGGGATAACAGCGTCAT | 710 |
| MH168 220 | Oratosqui llina inornata | GAGGGACGATAAGACCCTATAAAGCTTTATATTACATTTCTTTTTTA TAAATTATTTCGTTATTAACTTATTTTAGAATTTGTATTATTTTACTG GGGCGGTAAAAGTATATTAAAGATTAACTGCTTTTGTTTTGTAAAA CAATTATAGTTGAAAATAAATTGATCCATTATTAATGATCATAAGAA CAAGTTACTTTAGGGATAACAGCGTAAT | 711 |
| MH168 238 | Oratosqui llina asiatica | GAGGGACGATAAGACCCTATAAAGCTTTATGTTATTCTTCTTTTTTA TAAATTATTTAATTATTAACTTATTTCAGAATTAATATCATTTTACTG GGGCGGTAAAAGTATAATAAAGATTAACTGCTTTTGTTTTGTCTAA CAATAATAGTTGAAAATAGATTGATCCATTATTAATGATCATAAGAA CAAGTTACTTTAGGGATAACAGCGTAAT | 712 |
| MH168 217 | Oratosqui llina anomala | GAGGGACGATAAGACCCTATAAAGCTTTATATTACATTTCTTTTTTA TAAATTATTTCGTTATTAACTTATTTTAGAATTTGTATTATTTTACTG GGGCGGTAAAAGTATAATGAAGATTAACTGCTTTTGTTTTGTAAAA CAATTATAGTTGAAAATAATTTGATCCATTATTAATGATCATAAGAA CAAGTTACTTTAGGGATAACAGCGTAAT | 713 |
| MH168 218 | Oratosqui llina perpensa | GGAGGGACGATAAGACCCTATAAAGCTTTATGTTACATTTCTTTTT TATAAATTATTTTGTTATTAACTTATTTTAGAATTTGTATCATTTTAC TGGGGCGGTAAAAGTATAATAAAGATTAACTGCTTTTGTTTTGTAT AACAATTTTAGTTGGAAATAAATTGATCCATTATTAATGATCATAAG AACAAGTTACTTTAGGGATAACAGCGTAAT | 714 |
| MH168 226 | Erugosqu illa graham | GAGGGACGATAAGACCCTATAAAGCTTTATGATATTCTTCTTTTTT ATAAATTGTCTTGTTATTAACTTATTTTAGAATTAATATCATTTTACT GGGGCGGTAAAAGTATAAAAAGATTAACTGCTTTTGTTTTGTATAA CAATCTTAGTTGGAAATAAATTGATCCATTATTAATGATCATAAGAA CAAGTTACTTTAGGGATAACAGCGTAAT | 715 |
| MH168 223 | Busquilla quadratic auda | GAGGGACGATAAGACCCTATAAAGCTTTATGATATTTCTCTTTCTT ATAAATTTTTGTTATTAACCTATTTTAGAGTTAATATCATTTTACTGG GGCGGTAAAAGTATAATAAAGATTAACTGCTTTTGTTTTGTACAAC AATTTTAGTTGGAAATAAATTGATCCATTATTAATGATCATAAGACC AAGTTACTTTAGGGATAACAGCGTAAT | 716 |
| MH168 213 | Kempella stridulans | GAGGGACGATAAGACCCTATAAAGCTTTATAATACCTTTCTTTTTT ATAAAATTTTTTGTTGTTAACATATTTTAGAATTTGTATTATTTTACT GGGGCGGTAAAAGTATAATAAATAACTGCTTTTATTTTGTGTAACA ATTTTAGTTGATGATAAATTGATCCATTATTAATGATTATAAGAACA AGTTACTTTAGGGATAACAGCGTAAT | 717 |
| AF133 678 | Gonodact ylaceus graphuru s | GAGGGACGATAAGACCCTATAAAGCTTTATAGTAGTTCCTTTTTAT TCAAATTATAAAATGTTAACCTTTTGAAAGATTACTACTATTTTACT GGGGCGGTAGAAGTATATTTATGATTAACTGCTTTTGTTTTGACAA ACAATAATAATTGAATATTTATGTGATCCATTATTAATGATCAAAAG TACAAGTTACTTTAGGGATAACAGCGTAAT | 718 |
| MW01 9425 | Gonodact ylaceus randalli | AAGTGACGATAAGACCCTATAAAGCTTTATAGTAATTTCTTTTTATT TAAAATATAAAATTTTAACCTTTTTAAAGATTATTACTATTTTACTGG GGCGGTAAAAGTATATTTTGATTAACTGCTTTTGTTTTAATTAACAA CGATAGTTGAATAGTCATGTGATCCATTATTAATGATCAAAAGTAC AAGTTACTTTAGGGATAACAGCGTAAT | 719 |
| CAU74 327 | Carcinus aestuarii | GGGGGACGATAAGACCCTATAAAACTTTATATACACAACAATACAG TTAAATTAAACTATAAAAACTTATTAGATAAAATATATTTAGTTGGG GCGACTGAGGTATAATTTATAGTAACTGATTAAGAGCTAGACAAAT AATATTTGATTAATTTTAATTGATCCTTTTTAAAGATTAAAAGATTAA GTTACTTTAGGGATAACAGCGTAAT | 720 |
| KF220 514 | Menippe rumphii | GAGGGACGATAAGACCCTATAAAGCTTTATAAATTATTTAAATTTG ATTGAATTAATATAATAAAATTTTGATTTGGATTAATTTATTTTGCTG GGGCGGCATAGGTATAAATTATATTAACTGCTTAGTAGAAATACAA TAATAGTTGAGTTGACATTAAAAGATCCTTTTTAAAGATTTAAGAAC AAGTTACTTTAGGGATAACAGCGTTAT | 721 |
| MK971 425 | Menippe nodifrons | GAGGGACGATAAGACCCTATAAAGCTTTATATATTATCTGAATTTG AGTAAATTGATTTATAAAATTTTAATTTGGAGTAATTTATTTTGCTG GGGCGGCATAGGTATAAGTTATATTAACTGCTTAATAAAAATACAA TTATAGTTGAGTTATTAATTAAAAGATCCTTTTTAAAGATTTGAGAA TAAGTTACTTTAGGGATAACAGCGTTAT | 722 |
| HM637 973 | Menippe adina | GAGGGACGATAAGACCCTATAAAGCTTTATATGTTATTTAAATTTA ATTGAATTGATTTATAAATTTTAAATTTAGAGTAATTTATTTTGCTGG GGCGGCATAAGTATAAGTTATATTAACTGCTTAATAATGATACAAT AATAGTTGAATTGTTGTTAAATGATCCTTTTTAAAGATTTAAGAACA AGTTACTTTAGGGATAACAGCGTTAT | 723 |
| JX514 567 | Procamb arus liberorum | AAGGGACGATAAGACCCTATAAAACTTTATATTTGTAAATTAATAG CTAATTTTATTTAAATATATTATTTAAAAATATTTTGTTGGGGTGACA AGGATAAAATTAAAAGTAACTGTCCTTTTTTATTACAATAATGTTTG GTTTAATGATCCTTAAATGGATTTAAGATTAAGTTACTTTAGGGATA ACAGCGTAAT | 724 |
| AY214 438 | Procamb arus toltecae | AAGGGACGATAAGACCCTATAAAACTTTATATTTATAAGGTAGAAA ATTAGTTTTATTTAATGATGCTACTTTGGAGTATTTGGTTGGGGTG ACAAGGATAAAATATAAAATAACTGTCTTTTTTTTTCTACAGTTATA TTTGATTTAATGATCCTAAAAGGGATTAAAGATTAAGTTACTTTAGG GATAACAGCGTTAT | 725 |
| EF012 344 | Procamb arus curdi | AAGGGACGATAAGACCCTATAAAACTTTATATTTATAAATTAATAGT TAATTTTATTTAAATATATTATTTTAAAATATTTTGTTGGGGTGACAA GGATAAAATTAAAGATAACTGTCCTTTTTTATTACAGTAATATCTGG TTAGGTGATCCTTAAAATGGATTATAAGATTAAGTTACTTTAGGGA TAACAGCGTAAT | 726 |
| AY214 435 | Procamb arus digueti | AAGGGACGATAAGACCCTATAAAACTTTATATCTAGAGGTAGTAG GATAATTTTATTTAAGGTGTATTATTTTAAGATATTTGGTTGGGGTG ACCAGGATAAAATGAAAGATAACTGTCTTATATATTACAATAATTTT TGGGTTGTTGATCCTACAAAGGGATTAAAAGATTAAGTTACTTTAG GCGATAACAGCGTAAT | 727 |
| EF012 345 | Procamb arus nigrocinct us | AAGGGACGATAAGACCCTATAAAACTTTATATTTATAAGTAGTAGT TAATTTTATTTGAGTATATTATTTTAAAGTATTATGTTGGGGTGACA AGGATAAAATTAAAAATAACTGTCTTTTTTTATTACAGTAATGTTTG GTTTAATGATCCTTAAATGGATTATAAGATTAAGTTACTTTAGGGAT AACAGCGTAAT | 728 |
| JF737 390 | Procamb arus versutus | AAGGGACGATAAGACCCTATAAAACTTTATATTCATAAATTAGTAG TTATTTTTATTTTAAAGTATTGTTTTAGAATATTTGGTTGGGGTGGC AAGGATAAAATATTTAATAACTGTCTTTTTTTTTTACAATAATGTTTG GTTTAATGATCCTAAAAGGGATTTAAGATTAAGTTACTTTAGGGAT AACAGCGTAAT | 729 |
| EU433 916 | Procamb arus gibbus | AAGGGACGATAAGACCCTATAAAACTTTATATTTGCAAGGTAGTAG AAAATTTTATTTTAATTTATTATTTTAAAGTATTTGGTTGGGGTGAC AAGGATAAAATATAAAATAACTGTCTTTTTTTTTTACAGTAATGTTT GGTTTAATGATCCTAAAAGGATTAAAGATTAAGTTACTTTAGGGA TAACAGCGTAAT | 730 |
| EU433 911 | Cambaru s pecki | AAGGGACGATAAGACCCTATAAAACTTTATATTTAAGAATGATTAA TGAGTTTTGTTTTAAGGTTATTATTTTGAGATATTTGGTTGGGGCG ACAAGAATAGAAGAAAAAGTAACTGTTTTTTTTTTAATAACAAAAAT GTTTGGGTTAATGATCCTAAAATGGATTAAAGATTAAGTTACTTTA GGGATAACAGCGTAAT | 731 |
| JX514 566 | Procamb arus geminus | AAGGGACGATAAGACCCTATAAAACTTTATATTTATAAGATAGTAG CTAGTTTTATTTAAAAGTATTATTTTGGAGTATTTGGTTGGGGTGAC AAGGATAAAATATAAAATAACTGTCTTTTTTTTTTACAGTAATGTTT GGTTTAATGATCCTAAAAAGGATTAAAGATTAAGTTACTTTAGGGA TAACAGCGTAAT | 732 |
| LC430 785 | Charybdi s acuta | ACTTTATAAATTTAATGATTTTATATATTTTATATAAAGATAATTTTT GGAAAATTTATTTGGTTGGGGCGACAATGGTATAATTTAAATAACT GCTATTAAGTTTAACAAAAATATTTGCTTGAAATACTGATAATTGAT CCTTTATAAGGATTTTAAGATCAAGTT | 733 |
| KC163 442 | Creaserin us fodiens | AAGGGACGATAAGACCCTATAAAACTTTATATTTAAAGGTATAAAT TATTTTTATTTATGTGTTGGTATCTAAAAGTATTTGGTTGGGGCGA CAAGGGCAGAATATTAAGTAACTGTCTTTTTTTTTGACAATAATATT TGGTTTAATGATCCTAAAAAGGATTAAAGATTAAGTTACTTTAGGG ATAACAGCGTAAT | 734 |
| KC163 480 | Fallicamb arus jeanae | AAGGGACGATAAGACCCTATAAAACTTTATATTTATAAATGGTAGA CAATTTTGTTTTGGGGTATTATTTTAAAATATTTGGTTGGGGTGACA AGGATAAAATAAAAAATAACTGTCTTTTTTTTTTACAGTGATATTTG GTTTAATGATCCTAGAAGTGGATTATAAGATTAAGTTACTTTAGGG ATAACAGCGTAAT | 735 |
| KC163 523 | Creaserin us gordoni | AAGGGACGATAAGACCCTATAAAACTTTATATTTAAAGGTATAGGT TATTTTTATTTGTGGGTTATTATCTAAAAATATTGGGTGGGGGCGA CGAGGATAGAATATTAAGTAACTGTCTTTTTTTAAAACAGTAATATT TGATTTAATGATCCTAAAAGGGATTAAAGATTAAGTTACTTTAGGG ATAACAGCGTAAT | 736 |
| KC163 501 | Creaserin us caesius | GAGGGACGATAAGACCCTATAAAACTTTATATTTGAGGGTGGAAA TTATTTTTATTTGGAGGTTGATATCTAAAATATTTTGTTGGGGCGAC AAGGATAAAATATTAAGTAACTGTCTTTTTTTTTTACAATGATATTT GGTTTAATGACCCTAAAAAGGATTAAAGATTAAGTTACTTTAGGGA TAACAGCGTAAT | 737 |
| KC163 463 | Fallicamb arus dissitus | AAGGGACGATAAGACCCTATAAAACTTTATATTTATGAATGATAGT TAATTTTGTTTTGGAATATTATTTTAAAATATTTAGTTGGGGTGACA AGGATAAAATATGAAATAACTGTCTTTTTTTTTTTTACAGTGATATTT GGTTTAATGATCCTAAAGTGGATTATAAGATTAAGTTACTTTAGGG ATAACAGCGTAAT | 738 |
| KC163 518 | Creaserin us danielae | AAGGGACGATAAGACCCTATAAAACTTTATATTTAAGGATATAGAC TATTTTTATTTGTGAACTATTATCAAAAGTATTTGGTTGGGGTGACA GGGATAGAATATAAAGTAACTGTCCTTTTTTTTTAACAATAATATTT GACTTAGCGATCCCTGGGAAGGATTAAAGATTAAGTTACTTTAGG GATAACAGCGTGAT | 739 |
| KC163 509 | Fallicamb arus oryktes | AAGGGACGATAAGACCCTATAAAACTTTATATTTAAGGATATAGAC TATTTTTATTTGTAAACTATTATCAAAAGTATTTGGTTGGGGCGACA GGGATAGAATATAAAGTAACTGTCCTTTTTTTTAACAATAATATTTG ACTTAACGATCCCTGGGAAGGATTAAAGATTAAGTTACTTTAGGGA TAACAGCGTGAT | 740 |
| KC163 506 | Fallicamb arus byersi | AAGGGACGATAAGACCCTATAAAACTTTATATTTTAAGGTATAGAT TATTTTTGTTTATAGGTTGTTATCTGAAAATATTTGGTTGGGGCGA CAGGGATAAAATGAGAGGTAACTGTTTTTTTTTTATTACAACAATGT TTGATTTAATGATCCTGAAAAGGATTAAAAGATTAAGTTACTTTAGG GATAACAGCGTAAT | 741 |
| KC163 511 | Creaserin us burrisi | AGGGGACGATAAGACCCTATAAAACTTTATATTTAAGGGTATAAGT TGTTTTTGTTTGTAAGTTATTATCTGAGAGTATTTGGTTGGGGCGA CAGGAATAGGATATAAAGTAACTGTTCTTTTTTTTGACAATAATATT TGGTTTAATGATCCTAAAAAGGATTGAAGATTAAGTTACTTTAGGG ATAACAGCGTAAT | 742 |
| KC163 450 | Creaserin us gilpini | GAGGGACGATAAGACCCTATAAAACTTTATATTTGAAGGTAGAAGT TATTTTTATTTGAAAGTTAATATCTAAAATATTTTGTTGGGGCGACA AGGATAAAATATTAAGCAACTGTCTTTTTTTTTTACAATGATATTTG GTTTAATGATCCTAAAAAGGATTAAAGATTAAGTTACTTTAGGGAT AACAGCGTAAT | 743 |
| KC163 479 | Fallicamb arus harpi | AAGGGACGATAAGACCCTATAAAACTTTATATTTATAAATAGTAGC TAATTTTGTTTTGAAATATTATTTTAAAATATTTTGTTGGGGTGACA AGGATATAACAGAAAATAACTGTCTTTTATGTTTACAGTAATGTCTG GTTTAATGATCTTAAGAATAGATTATAAGATTAAGTTACTTTAGGGA TAACAGCGTAAT | 744 |
| KC163 444 | Fallicamb arus macnees ei | AAGGGACGATAAGACCCTATAAAACTTTATATTTTATGAATAGTAG TTAATTTTATTTTATAATATTATTTAAAAGTATTTGGTTGGGGTGAC AAGGATAAAATAAAAAATAACTGTCTTTTTTTTTTACAGTAATGTTT GGTTTAATGATCCTAAAATGGATTGAAAGATTAAGTTACTTTAGGG ATAACAGCGTAAT | 745 |
| KC163 468 | Fallicamb arus petilicarp us | GAGGGACGATAAGACCCTATAAAACTTTATATTTATAAATGGTAGT TAATTTTATTTTAGAATATTATTTTGGAATATTTGGTTGGGGTGACA AGGATAAAATATGAAATAACTGTCTTTTTTTTTTACAGTGATATTTG GTTTAGTGATCCTGAAATGGATTATAAGATTAAGTTACTTTAGGGA TAACAGCGTAAT | 746 |
| KC163 434 | Fallicamb arus wallsi | GAGGGACGATAAGACCCTATAAAACTTTATATTTATAAGTGGTAGT TAATTTTATTTTATAATATTATTTTGAAATATTTGGTTGGGGTGACG AGGATAAAATAAAAAATAACTGTCTTTTTTTTTAACAGTAATGTTTG GTATGATGATCCTAAAACGGATTAAAGATTAAGTTACTTTAGGGAT AACAGCGTAAT | 747 |
| KC163 486 | Fallicamb arus strawni | AAGGGACGATAAGACCCTATAAAACTTTATATTTATAAATGGTAGT TAATTTTGTTTTAAAATGTTATTAAAAAATATTTTGTTGGGGTGACA AGGATAAAATAGAAAATAACTGTCCTTTTTTATTACAGTAATGTTTG GTTTAATGATCCTAAAAAGGATTATAAGATTAAGTTACTTTAGGGAT AACAGCGTAAT | 748 |
| KC163 474 | Fallicamb arus devastato r | AAGGGACGATAAGACCCTATAAAACTTTATATTTATAAGTGGTAAT TAATTTTATTTAGGAATATTATTTTAAAATATTTGGTTGGGGTGACA AGGATAAAAGTAAAAATAACTGTCCTTTTTTTTTTCAGTAATATCTG GTTTGATGATCCTAAAATGGATTATAAGATTAAGTTACTTTAGGGA TAACAGCGTAAT | 749 |
| KC163 432 | Fallicamb arus houstone nsis | AAGGGACGATAAGACCCTATAAAACTTTATATTTTATGAATGGTAG TTAATTTTATTTTGTAATATTATTTAAAAGTATTTGGTTGGGGTGAC AAGGATAAAATAAAAAATAACTGTCTTTTTTTTTTACAGTAATGTTT GGTTTAATTGATCCTAAGATGGATTGAAAGATTAAGTTACTTTAGGG ATAACAGCGTAAT | 750 |
| KC163 472 | Fallicamb arus hortoni | AGGGGACGATAAGACCCTATAAAACTTTATATTTAAGGTGGAAATT ATTTTTATTTGTTAAGTACTATTTAAAAATATTTGGTTGGGGCGACA AGGATAGAATGTTAAGTAACTGTCTTTTTTTTTAACAATAATATTTG GTTTAATGATCCTAAAAAGGATTAAAGATTAAGTTACTTTAGGGAT AACAGCGTAAT | 751 |
| FM208 749 | Arenaeus cribrarius | GAGGGACGATAAGACCCTATAAAGCTTTATAAGTTAAAGTATTTTG TATATTTTATATTATAATAATGATTTATTGAGTACTTATTTGGTTGGG GCAACAAGAGTATAATAAAAAGTAACTGCTAATGTTTAAAACAATT ATATTTGATTTAAGAGTAATTGATCCCCCAGGGGATTAAAAGATCA AGCTACTTTAGGGATAACAGCGTAAT | 752 |
| JX514 521 | Cambaru s tenebrosu s | AAGGGACGATAAGACCCTATAAAACTTTATATTGAAAAGTGATAGG TATATTTTGTTTAAAGAATTATTACTTTAAAGTATTTGGTTGGGGTG ACAAGAATAGAAGAGAAAGTAACTGTTTTTTTTTTATACAATAATGT TTGGGTTAGTGATCCTAAAATGGATTAAAGATTAAGTTACTTTAGG GATAACAGCGTAAT | 753 |
| JX514 504 | Cambaru s deweesa e | AAGGGACGATAAGACCCTATAAAACTTTATATTTAGGGGTGGAAAT TAGTTTTATTTAAAGGTTACTATTTTATAATATTTGGTTGGGGCGAC AGGGATAGAAAAAAAGTTAACTGTCTTTTTTTTTTTACAGTAATATT TGGTTGAATGATCCTAAAATGGATTAAAGATTAAGTTACTTTAGGG ATAACAGCGTAAT | 754 |
| JX514 513 | Cambaru s striatus | AAGGGACGATAAGACCCTATAAAACTTTATATTAAAAAGTGATGGG TAAGTTTTGTTTAAAGGTTATTACTTTAAAGTATTTGGTTGGGGTGA CAGGAATAGAATAGAAAGTAACTGTTCTTTTTTTTTTACAGTAGTGT TTGAGTTAGTGATCCTAAAATGGATTAAAGATTAAGTTACTTTAGG GATAACAGCGTAAT | 755 |
| JX514 506 | Cambaru s graysoni | AAGGGACGATAAGACCCTATAAAACTTTATATTGAAAAGTGGTAGG TATGATTTGTTTAAAAGGTTACTACTTTAAAGTATTTGGTTGGGGT GACAAAAATAGAATAGAAAGTAACTGTTTTTTTTTTTAACAATAATG TTTGGGTTAGTGATCCTAAAATGGATTAAAGATTAAGTTACTTTAG GGATAACAGCGTAAT | 756 |
| JX514 537 | Cambaru s monongal ensis | AAGGGACGACAAGACCCTATAAAACTTTATATTGCAAAGTGATTAG TAAGTTTTATTTAAAGGTTATTATTTTGAAATATTTGGTTGGGGTGA CAAGGATTGAAGAAAAAGTAACTGTATTTTTTTTTACAATAATGTTT GGTTTAGTGATACTAAAATGGATTAAAGATTAAGTTACTTTAGGGA TAACAGCGTAGT | 757 |
| JX514 511 | Cambaru s pyronotus | AAGGGACGATAAGACCCTATAAAACTTTATATTTAAAAGTGATAGG TAGGTTTTGTTTTAAGGTTATTTCTTTAAAATATTTGGTTGGGGTGA CAGGAATAGAATAGAAAGTAACTGTTCTTTTTTTTTACAGTAGTGTT TGAGTTAGTGATCCTAAAATGGATTAAAGATTAAGTTACTTTAGGG ATAACAGCGTAAT | 758 |
| AF235 988 | Cambaru s maculatu s | AAGGGACGATAAGACCCTATAAAACTTTATATTGAGAGGTGAGAG GTAATTTTGTTTAAAAGTTATTGCTTTAAAGTATTTGGTTGGGGCG ACAAGGATACAAGGTAAAGTAACTGTTTTTTTTTTCTACAATAATGT TTGAGTGAATGATCCTAAGATGGATTAAAAGATTAAGTTACTTTAG GGATAACAGCGTAAT | 759 |
| JX514 524 | Cambaru s coosawat tae | AAGGGACGATAAGACCCTATAAAACTTTATATTGAGAAGTAATGGG CAGGTTTTGTTTAGAAGTTATTATTTTTAGAGTATTTGGTTGGGGC GACAAGGATAAAAAGAAAGTAACTGTTCTTTTATTTTACAATAATGT TTGGGTATAATGATCCTAATATGGATTAAAGATTAAGTTACTTTAG GGATAACAGCGTAAT | 760 |
| JX514 509 | Cambaru s latimanus | GAAGGGACGATAAGACCCTATAAAACTTTATATTAAAAAGTGATGT GTAAGTTTTGTTTAAAGGTTATTACTTTAAAGTATTTGGTTGGGGT GACAGGAATAGAATAGAAAGTAACTGTTCTTTTTTTTTTACAGTAGT GTTTGAGTTAGTGATCCTAAAATGGATTAAAGATTAAGTTACTTTA GGGATAACAGCGTAAT | 761 |
| JX514 517 | Cambaru s strigosus | AAGGGACGATAAGACCCTATAAAACTTTATATTGAAAAGTGATAGG TACAATTTGTTTGAAGGGTTGTTATTTTAAAGTATTTGGTTGGGGT GACAAGGATAAAATGGAAAGTAACTGTTTTTTTTTTTTACAGTGAT GTTTGGGTTAATGATCCTAAAATGGATTAAAGATTAAGTTACTTTA GGGATAACAGCGTAAT | 762 |
| JX514 556 | Cambaru s parrishi | AAGGGACGATAAGACCCTATAAAACTTTATACTGAGAGGTGATGG ATGAGTTTTGTTTAGAAGTTATTATTTTAGAGTATTTGGTTGGGGC GACAAGGGTAAAAAGAAAGTAACTGTTCTTTATTTTTACAGTTATG TTTGAGTGAATGATCCTAAAACGGGTTAAAGATTAAGTTACTTTAG GGATAACAGCGTAAT | 763 |
| JX514 532 | Cambaru s bouchardi | AAGGGACGATAAGACCCTATAAAACTTTATATTTAGAAGTGATAGG TAAGTTTTGTTTGTAGTTTAATTACTTTAAAATATTTGGTTGGGGTG ACAAGGATAGAATAACAAGTAACTGTTTTTTTTTTTAACAATGATGT TTGGATCGGTGATCCTAAAAGGATTAAAAGATTAAGTTACTTTAGG GATAACAGCGTAAT | 764 |
| JX514 525 | Cambaru s fasciatus | AAGGGACGATAAGACCCTATAAAACTTTATATTAAGAAGTAATGGG TGAATTTTGTTTAGAAGTTATTATTTTAAAATATTTGGTTGGGGCGA CAAGGATAAAAAGAAAATAACTGTTCTTTTATTTTACAATAATGTTT GGGTATAATGACCCTAATAGGGGTTAAAAGATTAAGTTACTTTAGG GATAACAGCGTAAT | 765 |
| JX514 508 | Cambaru s harti | AAGGGACGATAAGACCCTATAAAACTTTATATTGAAAAGTGGTAGG TATGGTTTGTTTAAAAGGTTATTGCTTTAAAGTATTTGGTTGGGGT GACAAGGATAGAATGGAAAGTAACTGTTTTTTTTTTTTACAATGAT GTTTGAGCTAATGATCCTAAAATGGATTAAAGATTAAGTTACTTTA GGGATAACAGCGTAAT | 766 |
| JX514 555 | Cambaru s nerterius | AAGGGACGATAAGACCCTATAAAACTTTATATTTGGGGGTAATAG GTAGGTTTTGTTTGTTAGTTATTATTTTAAAGTATTTGGTTGGGGTG ACAAGGATAAAAAAAAAGTAACTGTTCTTTATTTTTACAATAATGTT TGGTATAATGATCCTAATATGGATTAAAGATTAAGTTACTTTAGGG ATAACAGCGTAAT | 767 |
| JX514 539 | Cambaru s setosus | AAGGGACGATAAGACCCTATAAAACTTTATATTTAGGAGTAGAGTT AGTTTTATTTTAATATCTTATTTTAAAGTATTTGGTTGGGGTGACAT GAACAAAAAATGAAATAATTGTTTTTATTTTTTACAATGATATTTGG ACTTATGATCCTAAAGTGGATTAAAGATTAAGTTACTTTAGGGATA ACAGCGTAAT | 768 |
| JX514 531 | Cambaru s batchi | AGGGGACGATAAGACCCTATAAAACTTTATATTGAAAAGTGGTAG GTACGATTTGTTTTAAGGTTACTACTTTAAAGTATTTGGTTGGGGC GACAAGAATAGAATAGAAAGTAGCTGTTTTTTTTATTTTACAATGAT GTTTGGGTTAGTGATCCTAAAACGGATTAAAAGATTAAGTTACTTT AGGGATAACAGCGTAAT | 769 |
| JX514 507 | Cambaru s halli | AAGGGACGATAAGACCCTATAAAACTTTATATTGAAAGTGGTGG GTATGGTTTGTTTAAAGATTATTACTTTAAAGTATTTGGTTGGGGT GACAAGAATAGAATAGAAATTAACTGTTTTTTATTTTTACAATGATG TTTGAGTTAGTGATCCTAAAATGGATTAAAGATTAAGTTACTTTAG GGATAACAGCGTAAT | 770 |
| JX514 533 | Cambaru s crinipes | AAGGGACGATAAGACCCTATAAAACTTTATATTTAGAAGTGATAGG TAAGTTTTGTTTGTAGTTTAATTACTTTAAAATATTTGGTTGGGGTG ACAAGGATAGAATAACAAGTAACTGTTTTTTTTTTTAACAATGATGT TTGGATCGGTGATCCTGAAAGGATTAAAAGATTAAGTTACTTTAGG GATAACAGCGTAAT | 771 |
| JX514 541 | Cambaru s unestami | AAGGGACGATAAGACCCTATAAAACTTTATATTTAGAAGTGGTGG GTAAGTTTTGTTTGTAGTTTAATTACTTTAAAATATTTGGTTGGGGT GACAAGGATAGAATAGCAAGTAACTGTTTTTTTTTAAAACAATGAT GTTTGGATTAATGATCCTAAAGGGATTAAAAGATTAAGTTACTTTA GGGATAACAGCGTAAT | 772 |
| JX514 557 | Cambaru s reburrus | AAGGGACGATAAGACCCTATAAAACTTTATATTTGGAAGTGATGG GTAAGTTTTGTTTGCCGGTTATTATTTTAAAGTATTTGGTTGGGGT GACAAGGATAAAAAAAAGTAACTGTTCTTTATTTTTTACAATAATGT TTGGTATAATGATCCTAATATGGATTAAAAGATTAAGTTACTTTAGG GATAACAGCGTAAT | 773 |
| AY853 664 | Cambaru s gentry | AGGGACGATAAGACCCTATAAAACTTTATTTGAAAAGTGATGAGTA TGGTTTATTTAAAAGGTTATTATTTTAAAGTATTTGGTTGGGGCGA CAAGAACAAAACGGAAAGTAACTGGTTTTTTTTTTTACAATGATGTT TGAGTTAATGATCCTAAAATGGATTAAAGATTAAGTTACTTTAGGG ATAACAGCGTAAT | 774 |
| JX514 518 | Cambaru s hubbsi | AAGGGACGATAAGACCCTATAAAACTTTATATTAGGAAGTGATAAG TATATTTTGTTTAAAGAATTATTACTTTAAAGTATTTGGTTGGGGTG ACAAAAATAAAAAAAAAGTAACTGTTTTTTTTTTTAACAATAATGTTT GGGTTAGTGATCCTAAAATGGATTAAAGATTAAGTTACTTTAGGGA TAACAGCGTAAT | 775 |
| DQ411 733 | Cambaru s friaufi | AAGGGACGATAAGACCCTATAAAACTTTATATTAAAAAGTGGTAGG TATGGTTTGTTTGAAGATTATTATTTTTGAGTATTTGGTTGGGGCG ACAGGAATAAAATAAAAGTAACTGTTTTTTTTTTTTTACAATGATGTT TGAGTTAATGATCTTAAAATGGATTAAAGATTAAGTTACTTTAGGG ATAACAGCGTAAT | 776 |
| JX514 538 | Cambaru s obeyensi s | AAGGGACGATAAGACCCTATAAAACTTTATATTTAGAAGTGATAGG TAAGTTTTGTTTGTAGTTTAATTATTTTAAAATATTTGGTTGGGGTG ACAAGGATATAATAGCAAGTAACTGTTTTTTTTTTTAACAATAATGT TTGGGTTGATGATCCTAAAAGGATTAAAAGATTAAGTTACTTTAGG GATAACAGCGTAAT | 777 |
| JX514 522 | Cambaru s cracens | AAGGGACGATAAGACCCTATAAAACTTTATACTGAAAAGTGGTGG GTATGGTTTGTTTTAAAGATTATTACTTTAAAGTATTTGGTTGGGGT GACAAGAATAAAATAGAAACTAACTGTTTTTTATTTTTACAATGATG TTTGAGTTAGTGATCCTAAAACGGATTAAAGATTAAGTTACTTTAG GGATAACAGCGTAAT | 778 |
| JX514 530 | Cambaru s asperima nus | AAGGGACGATAAGACCCTATAAAACTTTATATTTAAAAGGAGTAAG CAAATTTTATTTAGAAGTTATTGCTTGAAAATATTTTGTTGGGGCGA CAAGAATAGGGAGGTAAATAACTGTTTTTTTTTTTTTGACAATAATGT TTGGATTAGTGATCCTGAAAGGATAATAAGATTAAGTTACTTTAGG GATAACAGCGTAAT | 779 |
| JX514 554 | Cambaru s hobbsoru m | CTGTTTTTTTTTTTTACAATAATGTTTGGTTTAGTGATCCTAAAATG GATTAAAGATTAAGTTACTTTAGGGATAACAGCGTAAT | 780 |
| JX514 523 | Cambaru s williami | AAGGGACGATAAGACCCTGTAAAACTTTATATTTAAGAGTGGTGG GTAAGGTTTGTTTGGAGGTAATTATTTTAAAGTATTTGGTTGGGGC GACAAGAATAGAAAAAAAAATAACTGTTTTTTTTTCTTACAATGATG TTTGAGTTAATGATCCTAAAACGGATTAAAGATTAAGTTACTTTAG GGATAACAGCGTAAT | 781 |
| JX514 502 | Cambaru s howardi | AAGGGACGATAAGACCCTATAAAACTTTATATTTGGAAGTGATGGT AGATTTTGTTTGTTAGTTATTATTTTAAAGTATTTGGTTGGGGCGAC AAGGATAAAAAGAAAGTAACTGTTCTTTGTTTTTACAATAATGTTTG GTTTAGTGATCCTAATATGGATTAAAAGATTAAGTTACTTTAGGGA TAACAGCGTAAT | 782 |
| JX514 510 | Cambaru s obstipus | AAGGGACGATAAGACCCTATAAAACTTTATATTAAAAAGTAATGGG TAAGTTTTGTTTAAAGGTTATTACTTTAAAGTATTTGGTTGGGGTGA CAGGAATAGAATAGAAAGTAACTGTTCTTCTTTTTTACAGTAGTGT TTGAGTTAGTGATCCTAAAATGGATTAAAGATTAAGTTACTTTAGG GATAACAGCGTAAT | 783 |
| JX514 526 | Cambaru s girardianu s | AAGGGACGATAAGACCCTATAAAACTTTATATTAAGAAGTAATGGG TAAATTTTGTTTAGAAGTTATTATTTTAAAGTATTTGGTTGGGGCGA CAAGGATAAAAAGAAAATAACTGTTCTTTCATTTTACAATAATGTTT GGAGATAGTGATCCTAAGATGGGTTAAAGATTAAGTTACTTTAGG GATAACAGCGTAAT | 784 |
| JX514 534 | Cambaru s cryptodyt es | GGGGGACGATAAGACCCTATAAAACTTTATATTGAAAGGGGGTAA GTAAATTTTGTTTAAGGTTATGTGCTTAAAATATTTGGTTGGGGTG ACAGGGATAGAAGAAAAGGTAACTGTTTTTTTTTATTACAAATAATA TTTGGATTAGTGATCCTGAAGTGGATTTAAGATTAAGTTACTTTAG GGATAACAGCGTAAT | 785 |
| JX514 528 | Cambaru s speciosus | AAGGGACGATAAGACCCTATAAAACTTTATATTGAGAAGTAATGGG CAGGTTTTGTTTAGAAGTTATTATTTTTAGAGTATTTGGTTGGGGC GACAAGGATAAAAAGAAAGTAACTGTTCTTTTATTTTACAATAATGT TTGGGTATAATGATCCTAATATGGATTAAAGATTAAGTTACTTTAG GGATAACAGCGTAAT | 786 |
| JX514 503 | Cambaru s sciotensis | AAGGGACGATAAGACCCTATAAAACTTTATATTAAGAAGTAATGGG TAAGTTTTGTTTAAAAGTTATTATTTTAAAGTATTTGGTTGGGGCGA CAAGGATAAAAAGAGAGTAACTGTTCTTTTGTTTTACAATAATGTTT GATATTATGATCCTAAGAAGGATTAAAGATTAAGTTACTTTAGGGA TAACAGCGTAAT | 787 |
| JX514 553 | Cambaru s georgiae | AAGGGACGATAAGACCCTATAAAACTTTATATTAAGAAGTAATGGG TGAATTTTGTTTGGAAGTTATTATTTTAGAGTATTTGGTTGGGGTG ACAAGGATAAAAAGAAAGTAACTGTTCTTTTATTTTACAATAATGTT TGGGTTTGATGATCCTAAGACGGATTAAAGATTAAGTTACTTTAGG GATAACAGCGTAAT | 788 |
| JX514 561 | Cambaru s pristinus | AAGGGACGATAAGACCCTATAAAACTTTATATTGAGAAGTGATAGG TAAGTTTTGTTTATAGGTTAATTACTTTAAAATATTTGGTTGGGGCG ACCGGGATAGAATAACAAGTAACTGTCTTTTTTTAAAACAATTGTG TTTGGATTAGTGATCCTAAGATGGATTAAAAGATTAAGTTACTTTA GGGATAACAGCGTAAT | 789 |
| JX514 529 | Cambaru s aculabru m | AAGGGACGATAAGACCCTATAAAACTTTATATTTAGGAGTAGAGTT AGTTTTATTTTGGGATCTTATTTTAAAGTATTTGGTTGGGGTGACAT GAACAAAAAATGAAATAATTGTTTTTATTTTTTACAATGATATTTGG GTTTATGATCCTAAAGTGGATTAAAGATTAAGTTACTTTAGGGATA ACAGCGTAAT | 790 |
| JX514 505 | Cambaru s englishi | AAGGGACGATAAGACCCTATAAAACTTTATACTAAAAAGTGATGGG TAAGTTTTGTTTAAAGGTTATTACTTTAAAGTATTTGGTTGGGGTGA CAGGAATAGAATAGAAAGTAATTGTTCTTTTTTTTTACAGTAATGTT TGAGTTAGTGATCCTAAAATGGATTAAAGATTAAGTTACTTTAGGG ATAACAGCGTAAT | 791 |
| DQ411 732 | Cambaru s brachyda ctylus | AAGGGACGATAAGACCCTATAAAACTTTATATTGAAAAATGGTAGG TATGGTTTATCTAAGGATCATTATTTTTAGTATTTGGTTGGGGCG ACAAAAATAGAATAATAAGTAACTGTTTTTTTTTTTAACAATGATGT TTGAGTTAGTGATCCTAAAATGGATTAGAGATTAAGTTACTTTAGG GATAACAGCGTAAT | 792 |
| JX514 552 | Cambaru s cumberla ndensis | AAGGGACGATAAGACCCTATAAAACTTTATATTAAAAAGTAGTGGG TAAATTTTGTTTAGAAATTATTATTTTAAAGTATTTGGTTGGGGCGA CAGGGATAAAAGGAAAGTAACTGTTCTTTTATTTTACAATAATGTTT GGGTACACGATCCTAAGATGGATTAAAGATTAAGTTACTTTAGGGA TAACAGCGTAAT | 793 |
| JX514 536 | Cambaru s dubius | AAGGGACGATAAGACCCTATAAAACTTTATATTGAAAGGGGGTAA GTAGATTTTGTTTAAAAGTTATTTCTTAGAAATATTTGGTTGGGGC GACAAGAATAGGAAGGCAAATAACTGTTTTTTTTTTTTACAATAATT TTTGGGTTAATGATCCTGAAGAGGATTAAAAGATTAAGTTACTTTA GGGATAACAGCGTAAT | 794 |
| JX514 512 | Cambaru s reflexus | AAGGGACGATAAGACCCTATAAAACTTTATATTGAAAAGTGATAGG TATAATTTGTTTAAAAGGTTATTATTTTAAAGTATTTGGTTGGGGTG ACAAGGATANAATGAAAAGTAACTGTTTTTTTTTTTTACAATGATGT TTGGGTTAATGATCCTAAAATGGATTAAAGATTAAGTTACTTTAGG GATAACAGCGTAAT | 795 |
| JX514 520 | Cambaru s rusticifor mis | AAGGGACGATAAGACCCTATAAAACTTTATATTGAAAAGTGATAGG TATATTTTGTTTAAAGAATTATTACTTTAAAGTATTTGGTTGGGGTG ACAAGAATAGAAGAGAAAGTAACTGTTTTTTTTTTATACAATAATGT TTGGGTTAGTGATCCTAAAATGGATTAAAGATTAAGTTACTTTAGG GATAACAGCGTAAT | 796 |
| JX514 559 | Cambaru s scotti | AAGGGACGATAAGACCCTATAAAACTTTATATTAAGAAGTAGTAGG GTGAATTTTGTTTAGAAGATATTATTTTAAAGTATTTGGTTGGGGC GACAAGGATAAAAAGAAAATAACTGTTCTTTTATTTTACAATAATGT TTGGGTATAATGATCCTAAGACGGGTTAAAAGATTAAGTTACTTTA GGGATAACAGCGTAAT | 797 |
| JX514 551 | Cambaru s coosae | AAGGGACGATAAGACCCTATAAAACTTTATATTGAGAAGTAATGGG CAGGTTTTGTTTAGAAGTTATTATTTTTAGAGTATTTGGTTGGGGC GACAAGGATAAAAAGAAAGTAACTGTTCTTTTATTTTACAATAATGT TTGGGTATAATGATCCTAATATGGATTAAAGATTAAGTTACTTTAG GGATAACAGCGTAAT | 798 |
| JX514 535 | Cambaru s distans | AAGGGACGATAAGACCCTATAAAACTTTATATTTAGAAGTGATAGG TAAGTTTTGTTTGTAGTTTAATTACTTTAAAATATTTGGTTGGGGTG ACAAGGATAGAATAACAAGTAACTGTTTTTTTTTTTAACAATGATGT TTGGATCGGTGATCCTGAAAGGATTAAAAGATTAAGTTACTTTAGG GATAACAGCGTAAT | 799 |
| JX514 527 | Cambaru s longirostri s | AAGGGACGATAAGACCCTATAAAACTTTATATTTGGGAGTGATGG GCAGGTTTTGTTTGTTAGTTATTATTTTAAAGTATTTGGTTGGGGT GACAAGGATAAAAAGAAAGTAACTGTTCTTTATTTTTACAATAATGT TTGGTATAATGATCCTAATGTGGATTAAAGATTAAGTTACTTTAGG GATAACAGCGTAAT | 800 |
| JX514 519 | Cambaru s hubrichti | AAGGGACGATAAGACCCTATAAAACTTTATATTGAGAAGTGATGG GTATATTTGTTTAAAGATTATTACTTTAAAGTATTTGGTTGGGGTGA CAAGAATAGAATAGAAAGTTACTGTTTTTTTTTTTTACAATGATATT TGAGTTGGTGATCCTAAAAGGGATTGAAGATTAAGTTACTTTAGGG ATAACAGCGTAAT | 801 |
| MG56 3792 | Monomia lucida | AGGGGACGATAAGACCCTATAAAGCTTTATAAGTCTAGGAATTTTA GATATTTTGTAGGATAAATTTATTTTCTGATAACTTATTTGGTTGGG GCGACAAAGGTATAATTATGGATAACTGCTATTAATTTTGATACATT TATTGGTGATTGAAATTTAGGGTAATTGATCCTCTTTGGGGATTAA AAGATCAAGTTACTTTAGGGATAACAGCGTAAT | 802 |
| MH168 232 | Faughnia serenei | GAGGGACGATAAGACCCTATAAAGCTTTATAATGTTTTTTTTTATTA TAAATTGTGCAATTTTAGTTTACTAAAAAGAAATTATTATTTTACTG GGGCGGTAAAAGTATGTTCTTGACCAACTGCTTTTGTTTTTAAAAA CAAGTATTTTTGAAAATAAATTGATCCATTATTAATGATTATAAGAA CAAGTTACTTTAGGGATAACAGCGTAAT | 803 |
| MH168 201 | Harpiosq uilla melanour a | GAGGGACGATAAGACCCTATAAAGCTTTATAACGTATTTTTTTTTTA TAAACTATTTAATTATTAACTTAATACAAAATGTACGTTATTTTACTG GGGCGGTAAAAGTATAATCAAGAATAACTGCTTTTTTTATTAATAA CAATTATAATTGAAAATAAATTGATCCATTATTAATGATCATAAGAA CAAGTTACTTTAGGGATAACAGCGTAAT | 804 |
| MH168 202 | Harpiosq uilla annandal ei | GAGGGACGATAAGACCCTATAAAGCTTTATAGTATATTTTTTTTTA TAAATTATTTTGTTATTAACTTATTATAAAATATATATTATTTTACTG GGGCGGTAAAAGTATAATTAAGAATAACTGCTTTTCTTTTTAATAAC AGTGATAACTGGAAATAAATTGATCCATTATTAATGATCACAAGAA CAAGTTACTTTAGGGATAACAGCGTAAT | 805 |
| KJ920 795 | Cherax communi s | GAGGGACGATAAGACCCTATAAAGTTTATACATGGAGTTAATCAA GAGTGATTTGGAATAGTAAAGTTCATTATTAGCAAGATGTTTAGTT GGGGTGACTAGAATATAAGTTATATAACTGTTTTTTGTTTTAATCAG AGATATCTGTTTATATGGTCCCTTTTTAGGATTAGAGAGTAAATTAC TTTAGGGATAACAGCGTAAT | 806 |
| EU977 342 | Cherax cuspidatu s | GAGGGACGATAAGACCCTGTAAAGTTTGTACATCAGGGTTAATGG GTAGCGATTTAGGTTAATAAGGCTAACTCTTATGGAGGTGTTTAGT TGGGGCGACTGGGATATAAGTTATCTAACTGTTTCTTTATTGGAAT CAATAATATTTGAGTTTATGGTCCTCTTTAAGGATTTCAGAATAAAT TACTTCAGGGATAACAGCGTAAT | 807 |
| KJ920 829 | Cherax paniaicus | GAGGGACGATAAGACCCTATAAAGTTTATACATGGAGTTAATCAA GAGTGATTTGGAATAGTAAAGTTCATTATTAGCAAGATGTTTAGTT GGGGTGACTAGAATATAAGTTATATAACTGTTTTTTGTTTTAATCAG AGATATCTGTTTATATGATCCCTTTTTAGGATTAGAGAGTAAATTAC TTTAGGGATAACAGCGTAAT | 808 |
| KJ920 812 | Cherax lorentzi | GAGGGACGATAAGACCCTATAAAGTTTATACATGAAGCTGGTTAA GAGTGATTTAGGGTGATAAAGTTTATTATTAGCAGGGTGTTTAGTT GGGGCGACTGAGATATAAATCATATAACTGTTTTTTGTTTAAATCA GAGATACTTGCTGGTATTGGTCCCTTTTTAGGATTAGAGAAAAAT TACTTTAGGGATAACAGCGTAAT | 809 |
| KJ920 772 | Cherax albertisii | GAGGGACGATAAGACCCTATAAAGTTTATACATGAAGCTGGTTAA GAGTGATTTGAGGTATTAAAGTTTATTATCAGCAGGGTGTTTAGTT GGGGCGACTAGGATATAAATTATATAACTGTTTTTTGTTTAAATCA GAGATACTTGTTTATATGATCCCTTTTTAGGATTAGAGAATAAATTA CTTTAGGGATAACAGCGTAAT | 810 |
| AF135 973 | Cherax rotund us | GAGGGACGATAAGACCCTATAAAGTTTTGACACTAAATTAATTAAA GGTGATTTGGGTTATAAAGTTTTATTATTAAATAAGTGTTTAGTTGG GGCGACTAGGATATAAGTGATTTAACTGTTTCTTCGTTTAAACCAA AAATATTTGGGTTTATGATCCTTTTTTAGGATTTTTAGAGTAAATTA CTTTAGGGATAACAGCGTAAT | 811 |
| KM039 077 | Cherax leckii | GAGGGACGATAAGACCCTGTAAAGTTTGTACATCAGGGTTAATGG GTAGAGATTTAGGTTAATAAGGCTAACTCTTATGGGGGTGTTTAGT TGGGGCGACTAGGATATAAGTTATCTAACTGTTTCTTTGTTGGAAT CAATAATATTTGAGTTTATGATCCTCTTTAAGGATTTCAGAATAAAT TACTTCAGGGATAACAGCGTAAT | 812 |
| KJ920 825 | Cherax murido | GAGGGACGATAAGACCCTATAAAGTTTATACATGGAGTTAATCAA GAGTGATTTGGAATAGTAAAGTTCATTATTAGCAAGATGTTTAGTT GGGGTGACTAGAATATAAGTTATATAACTGTTTTTTGTTTTAATCAG AGATATCTGTTTGTATGGTCCCTTTTTAGGATTAGAGAGTAAATTA CTTTAGGGATAACAGCGTAAT | 813 |
| KM039 082 | Cherax wasselli | GAGGGACGATAAGACCCTATAAAGTTTGTACAGTGAATTAGTATG AAATAGATTAGATGGGTAAAATTTGTTACTAAGGAGATGTTTGGTT GGGGCGATTAGAATATAGGTGAAGTAACTGTTTCTTTTTTTGAGTC AAAAATTTTTGATTTAATGACCCTTAATAAGGATTATGAAAGCAAGT TACTTTAGGGATAACAGCGTAAT | 814 |
| KM039 078 | Cherax parvus | GGGGGACGATAAGACCCTATAAAGTTTGTACATTAGGCTAATTAA GAGTGATTTAGATTTATAAGGCTTTATTATTAGGGGGTGTTAAGTT GGGGCGACTAGGATATAATTTATATAACTGTTTCTTTATTATAATCA GAAATATTTGAGTTTATGATCCTTTTTTAGGATTTTTAGAGTAAATT ACTTTAGGGATAACAGCGTAAT | 815 |
| KJ920 828 | Cherax pallid us | GAGGGACGATAAGACCCTATAAAGTTTATACATAGAGTTAATCAAG AGTGATTTGGAATAGTAAAGTTCATTATTAGCAAGATGTTTAGTTG GGGTGACTAGAATATAAGTTATATAACTGTTTTTTGTTTTAATCAGA GATATCTGTTTATATGGTCCCTTTTTAGGATTAGAGAGTAAATTACT TTAGGGATAACAGCGTAAT | 816 |
| KM039 079 | Cherax cartalaco olah | GAGGGACGATAAGACCCTATGAAGTTTGTACATTGGATTAATTGG GAATGATTTATGTTTAGTAAGGTTTGCTATTGAGAAGATGTTTAGTT GGGGCGACTAGGATATAATATATAACTGTTTCTTGGTAGGAGTCA AAAATATTTGAGTTAATGATCCTTTTTTAGGATTTTAGAGTAAATTA CTCTAGGGATAACAGCGTAAT | 817 |
| KJ920 806 | Cherax longipes | GAGGGACGATAAGACCCTATAAAGTTTATACATGGAGTTAATCAA GAGTGATTTGGAATAGTAAAGTTCATTATTAGCAAGATGTTTAGTT GGGGTGACTAGAATATAAGTTATATAACTGTTTTTTGTTTTAATCAG AGATATCTGTTTATATGGTCCCTTTTTAGGATTAGAGAGTAAATTAC TTTAGGGATAACAGCGTAAT | 818 |
| KJ920 765 | Cherax rhynchotu s | GAGGGACGATAAGACCCTATAAAGTTTATACATGGGGCTGGTTGA GAGTGATTCAGAATAGTAAAGTTTATTATTAGCAAGATGTTTAGTT GGGGCGACTAAGATATAAATTATATAACTGTCTTTTGTTTAAATCA GAGGTGTCTGTTCGTATGATCCTTTTTTAGGATTGGAGAGTAAATT ACTTTAGGGATAACAGCGTAAT | 819 |
| KY654 091 | Cherax pulcher | GAGGGACGATAAGACCCTATAAAGTTTATACATAGGGTTAGTTAAA AGTGATTTAGGATAGTAAAGTTTGTTACTAGCAAGATGTTTGGTTG GGGTGACTAGAATATAAGTTATATAACTGTTTTTTATTTAAGTCAGA AGTATTTGTTTATAGTGATCCCTTATTTAGGATTAAAGAGTAAATTA CTTTAGGGATAACAGCGTAAT | 820 |
| KJ920 835 | Cherax peknyi | GAGGGACGATAAGACCCTATAAAGTTTATACACAGAATTAATCGAA AATAATTTGGAATAATAAAATTTCTTATTAGTGAGGTGTTTGGTTGG GGCGACTAGGATATAAGTTATATAACTGTTTTTTGTTTAAATCAAAG ATATTTGTTTATATGATCCCTTTTTAGGATTAAAGAGTAAATTACTT TAGGGATAACAGCGTTAT | 821 |
| AF135 972 | Cherax setosus | GAGGGACGATAAGACCCTATAAAGTTTGACATTAAATTAATTAAAG GCGATTTAGGTCGTTAAAGTCTTATTATTATATAAGTGTTTAGTTGG GGCGACTAGGATATAAGTGATTTNNAACTGTTTTCTTCGTTCGAGT CAAGAATATTTGAGTGTATGATCCTTTTTTTAGGATTTTAGAGTAAAT TACTTTAGGGATAACAGCGTAAT | 822 |
| KJ920 813 | Cherax misolicus | GAGGGACGATAAGACCCTATAAAGTTTATACATGGAGTTAGTTAA GAGTAATTTAGAATAGTAAAGTTTATTATTAACAAGATGTTTGGTTG GGGCGACTGGAATATAAGTTATATAACTGTTTTTTATTTAAGTCAG AAATATTTGTTTATAATGATCCTTTTTTTGGATCAAAGAGTAAATTA CTTTAGGGATAACAGCGTAAT | 823 |
| KY654 088 | Cherax warsams onicus | GAGGGACGATAAGACCCTATAAAGTTTTATACATGGAGTTAGTTAA GAGTAATTTAGAGTAGTAAAGTTTATTATTAGCAAGATGTTTGGTT GGGGCGACTGGAATATAAGTTATATAACTGTTTTTTATTTAAGTCA GAAATATTTGTTTATAATGATCCCTTTTTTGGATTTAAGAGTAAATT ACTTTAGGGATAACAGCGTAAT | 824 |
| KJ920 836 | Cherax solus | GACGATAAGACCCTATAAAGTTTATACATGGAGTTAATCAAGAGTG ATTTGGAATAGTAAAGTTCATTATTAGCAAGATGTTTAGTTGGGGT GACTAGAATATAAGTTATATAACTGTTTTTTGTTTTAATCAGAGATA TCTGTTTATATGGTCCCTTTTTAGGATTAGAGAGTAAATTACTTTAG GGATAACAGCGTAAT | 825 |
| KY654 087 | Cherax snowden | GAGGGACGATAAGACCCTATAAAGTTTAAACACTGAGTTAGTTAA GAGTGATTTAGAGTAGTAAGGTTTATTATTAGTTAGGTGTTTGGTT GGGGCGACTAGAATATAAGTTATATAACTGTTTTTTATTTGAATCA GAGATATCTGTTCACATGATCCCTTTTTAGGATTAAAGAGTAAATT ACTTTAGGGATAACAGCGTAAT | 826 |
| KJ920 785 | Cherax buitendijk ae | GAGGGACGATAAGACCCTATAAAGTTTATACATGGAGTTAATCAA GAGTGATTTGGAATAGTAAAGTTCATTATTAGCAAGATGTTTAGTT GGGGTGACTAGAATATAAGTTATATAACTGTTTTTTGTTTTAATCAG AGATATCTGTTTATATGGTCCCTTTTTAGGATTAGAGAGTAAATTAC TTTAGGGATAACAGCGTAAT | 827 |
| KJ920 784 | Cherax boschmai | GAGGGACGATAAGACCCTATAAAGTTTATACATGGAGTTAATCAA GAGTGATTTGGAATAGTAAAGTTCATTATTAGCAAGATGTTTAGTT GGGGTGACTAGAATATAAGTTATGTAACTGTTTTTTGTTTTAATCA GAGATATCTGTTTATATGATCCCTTTTTAGGATTAGAGAGTAAATTA CTTTAGGGATAACAGCGTAAT | 828 |
| KJ920 760 | Cherax nucifraga | GGGGGACGATAAGACCCTATAAAGTTTGTACATGGGGCCGCTTAG GAGCGATTTAGAGAAGTAAAGTTTATTACTAGCAAGGTGTTTCGTT GGGGCGACTAAGATATAAATTATATAACTGTTTTCTGTTAGAATCA GAGATGTCTGTACATATGATCCCTTTTTAGGATGAAAGAGTAAATT ACTTTAGGGATAACAGCGTAAT | 829 |
| KJ920 752 | Cherax barrette | GAGGGACGATAAGACCCTATAAAGTTTATACATGGGGCTGGTTGA GAGTGATTCAGAATAGTAAAGTTTATTATTAGCAAGATGTTTAGTT GGGGCGACTAAGATATAAATTATATAACTGTCTTTTGTTTAAACCA GAGGTATCTGTTCGTATGATCCTTTTTTAGGATTGGAGAGTAAATT ACTTTAGGGATAACAGCGTAAT | 830 |
| MH168 227 | Oratosqui lla fabricii | GAGGGACGATAAGACCCTATAAAGCTTTATGGTATTTTTCTTTTTT ATAAATTATGTTATTATTAACTTATTTTAGAACTAATATTATTTTACT GGGGCGGTAAAAGTATAATAAAGACTAACTGCTTTTGTTTTGTATA ACAATTATAATTGAAAATAAATTGATCCATTATTAATGATCACAAGA ACAAGTTACTTTAGGGATAACAGCGTAAT | 831 |
| DQ006 549 | Astacopsi s tricornis | GGGGGACGATAAGACCCTATAAAGTTTAACATAATAATGATAAAAA ATTAATTAGGTTATAAAGTTTATTATTACAAATAATGTTTTGTTGGG GCGACAAGAATAAAAATAATTTAACTGTTCTTTTTTTTATCAAAAAT ATTTGGGTGGGTGATCTTTTTTAAAAGTATTAGAGTAAATTACTTTA GGGATAACAGCGTAAT | 832 |
| FJ152 163 | Thalamita admete | GAGGGACGATAAGACCCTATAAAGCTTTATAAGTTTAGGTATTCTA TAGATTTCCACATAAAAATAGATTTTCAAATGCTTATTTTGTTGGGG CGACAATGGTACAATAATAGTAACTGCTTTTAAGTATAACATATAT GGATGAGTCATATTTAATTGATCCTTTCTAAAGATTTTAAGATCAAG TTACTTTAGGGATAACAGCGTTAT | 833 |
| KT959 518 | Faxonius virilis | AGGGGACGATAAGACCCTATAAAACTTTATATTTTAAGGTGGAAGA TAATTTTATTTTAAAATTACTATTTTAAATTATTTTGTTGGGGCGACA AGGATATAAGAAAGGGTAACTGTCTTTTATTTTTACAATAATATTTG AATTATTGATCCTAAAAGGGATTAAAGATTAAGTTACTTTAGGGAT AACAGCGTAAT | 834 |
| KJ132 641 | Thranita prymna | AAGGGACGATAAGACCCTATAAAGCATTTGGTTGGGGCGACAATA GTATAATTAATAACTGCTTTAAAATAATACAAATATATTTGAAGTTG ATCCTTTATAAAGATCTAAGATCAAGTTACTTTAGGGATAACAGCG TTAT | 835 |
| AF044 240 | Astacopsi s franklinii | GGGGGACGATAAGACCCTATAAAGTTTAACATAATAAGGATAAAAA ATTAATTAGGTTTATAAAGTTTATTATTACAAATAATGTTTTGTTGG GGCGACAAGAATAAAAGTAATTTAACTGTTCTTTTTTTAATCAAAAA TATTTGGGTGGGTGATCTTTTCTAAAAGTATTAGAGTAAATTACTTT AGGGATAACAGCGTAAT | 836 |
| KX268 737 | Cambaroi des schrenckii | GAGGGACGATAAGACCCTATAAAACTTTATATACTTATAATTTAATA GAAATCTTTGTTTATAAAAATTTAGATTAGAGTATTTTATTGGGGCG ATGAAAATAAAATCTTAGGTAACTGTTTTTGTTTTTTAACAATAATAT TTGAATTTTAGATCCCGAAAAAGATCAAAAGATTAAGTTACTTTAG GGATAACAGCGTTAT | 837 |
| EU433 912 | Orconect es australis | GAGGGACGATAAGACCCTATAAAACTTTATATTAAAAAGTGATGAG TGTGTTTTGTTTAAGGGTTATTATTTTAAAGTATTTGGTTGGGGCG ACAAAAATAGAAGGGAAAGTAACTGTTTTTTTTTATTACAATAATGT TTGAGTGGATGATCCTAAAGTGGATTAAAGATTAAGTTACTTTAGG GATAACAGCGTAAT | 838 |
| LC469 670 | Thalamita chaptalii | GCTTTATAANTTTAGGGAGATCTTATAAATATTTAAGTAACAATAAT TTTTTAAATGTTTATTCGGTTGGGGCGACAAGAGTATAACGAAAAT AACTGCTTTAAATTAAGACAGAAATATCTGAATCATAGTTAATTGAT CCTTTTTAAAGATTCTAAGATCAAGTTA | 839 |
| LC469 673 | Zygita longifrons | GCTTTATAAATTTAGAGATCTTATATATTTTTATATAGCTGTAATTTC TTAAATGTTTATTTGGTTGGGGCGACAATAGTATAACAGAAGTAAC TGCTTTTAGGTAGTACAAGCTTATTTGAGTAATACCGTTAATTGAT CCTTTATAAAGATTTAAAGACCAAGTT | 840 |
| LC469 671 | Thalamita picta | GCTTTATAAGTTTAAAGATTTTGTATATTTTATTATAGCTATAATTTC TTGAATACTTATTTAATTGGGGCGATAACAGTATAATTATAGTAACT GCTTGTAAGTAATACAAAAATACTTGAATTGAAATTATAATTGATCC TTTATAAAGATTTTAAGATCAAGTT | 841 |
| LC469 672 | Thalamita seurati | GCTTTACAAGTTTAAAGATCTTATATATTTTTCTATATCAATAATTTC TTGATTATTTGTTTGGTTGGGGCGACGATAGTATAATAATAGTAAC | 842 |
| | | TGCTTTAAAGTGTTTCAAATGTATTTGAATCGAAATAATAACTGNAT CCTTAAATAAAGATTTAAAGATCAAGTT | |
| LC430 787 | Thranita pelsarti | GCTTTATAAGTTTGGAGATTTTATATATTTTTCTATAGCAATAATTTC TTGAAAGCTTATTTGGTTGGGGCGACAATAGTATAATTGAAATAAC TGCTTTAAAATAATACAAATATATTTGAATTATAATTAGTTGATCCTT TATAAAGATTTAAGATCAAGTT | 843 |
| EU433 913 | Orconect es barri | GAGGGACGATAAGACCCTATAAAACTTTATATTGAAAAGTGATGG GTATGTTTTGTTTAGGGATTATTATTTTAAAGTATTTGGTTGGGGC GACAAAAATAGAAGAGAAAGTAACTGTTTTTTTTTATTACAATAATG TTTGAGTTGATGATCCTAAAATGGATTAAAGATTAAGTTACTTTAG GGATAACAGCGTAAT | 844 |
| JX514 565 | Faxonius ronaldi | ATGGGACGATAAGACCCTATAAAACTTTATATTTCAAGGTAGAAAA TTAATTTTATTTTAAGGTTACTATTTTAAAATATTTTGTTGGGGCGA CAAGGGTATAAGAAATGATAACTGTCTTTTATTTTTACAATAATATT TGATTTATTGATCCTAAAAGGGATTAAAGATTAAGTTACTTTAGGG ATAACAGCGTAAT | 845 |
| JX514 564 | Faxonius neglectus | AGGGGACGATAAGACCCTATAAAACTTTATATTTTAAGGTGGAAAT TAATTTTATTTTGAGGATTACTATTTTAAAATGTTTCGTTGGGGCGA CAAGGATATAAGAAAGGGTAACTGTCTTTTGGTTTAACAGTAATAT TTGGTTTGTTGATCCTAAAAAGGATTAAAGATTAAGTTACTTTAGG GATAACAGCGTGAT | 846 |
| EU433 917 | Orconect es compress us | AAGGGACGATAAGACCCTATAAAACTTTATATTAAAAAGTAGAAGT TAATTTTATTTGAGTGTTACTATTTAAAATATTTGGTTGGGGCGATC GGAATAAAAGGTCTAATAACTGTCCTTTTTTTTTACAATAATATTTG GTTTAATGATCCTAAAAGGGATTTAAGATTAAGTTACTTTAGGGAT AACAGCGTAAT | 847 |
| EU433 919 | Orconect es forceps | AAGGGACGATAAGACCCTATAAAACTTTATATTTTGAGGTAGAAAT TAATTTTATTTCATATCACTATTTTAAAGTATTTCGTTGGGGCGACA AGGATACAAGAAAAGGTAACTGTCTTTTGTTTTTACAAAAATATTTG GTTTGTTGATCCTTAAAGGGATTAAAGATTAAGTTACTTTAGGGAT AACAGCGTAAT | 848 |
| EU433 914 | Orconect es pellucid us | AAGGGACGATAAGACCCTATAAAACTTTATATTGAAAAGTGGTGG GTATGTTTTATTTTGAGGTTATCACTTAAAAGTATTTGGTTGGGGT GACAAAAATAGAAGGAAAAGTAACTGTTTTTTTTTCTTACAGTGAT GTTTGGGTTAGTGATCCTAAAATGGATTAAAAGATTAAGTTACTTT AGGGATAACAGCGTAAT | 849 |
| NC_04 1211 | Neoerioc heir leptognat hus | ATGGGACGATAAGACCCTATAAAGCTTAATATTAAAATTTTATTTGA TTAAATTTTTTTTCAATAAAAATCTAATAATTAAATTTATTTTATTGG GGCGATAAAAGTATAATGTTTGATAACTGCTTAATTTTAATTACACC TATGCGTGAATTAATTTATGATTGATCCTAATTAAAGATTAAAAGTT TAAGTTACTTTAGGGATAACAGCGTTAT | 850 |
| AF279 826 | Penaeus kerathuru s | GGGGGACGATAAGACCCTATAAAGCTTGACAATAATCTTATTAAAT TATAAATTGTTAGTATAACTTGATTTTAATTAGAGTTTGTTTCGTTG GGGCGACGAGAATATAATAAATAACTGTTCTTTTAAATATAATTACA AAAATAATTGGTAAAATATTGATCCTTTATTAAAGATTAAAAGATTA AGTTACTTTAGGGATAACAGCGTAAT | 851 |
| AF279 824 | Penaeus marginatu s | GGGGGACGATAAGACCCTATAAAGCTTGACAATAATCCTGCTAAA TTATAAATTGTTAGTATAACTTAATTCTAGCTAGAATTTGTTTCGTT GGGGCGACGGGAATATAATAAATAACTGTTCTTTTAAATATAGTTA CAGAAATGTCTGCTATAAGATTGATCCTTTATTAAAGATTAAAAGAT TAAGTTACTTTAGGGATAACAGCGTAAT | 852 |
| AF279 823 | Penaeus longistylu s | GGGGGACGATAAGACCCTATAAAGCTTGACAATAACTTCGTTAAA TTATAAATTGTTAGTATAACTTGATTTTAACTGGGGTTTGTTTCGTT GGGGCGACGAGAATATAATAAAATAACTGTTCTTTTAAATATAATT ACAAAAATGCTTGGTGGATAATTGATCCTTTAATAAAGATTAAAAG ATTAAGTTACTTTAGGGATAACAGCGTAAT | 853 |
| AF279 822 | Penaeus plebejus | GGGGGACGATAAGACCCTATAAAGCTTGACAATAACTTTGTTAAAT TATAAATTGTTAGTATAACTTGATTTTAACTAGGGTTTGTTTCGTTG GGGCGACGAGAATATAATAAATAACTGTTCTTTTAAATATAATTACA AAAATGCTTGGTGGATGATTGATCCTTTATTAAAGATTAAAAGATA AAGTTACTTTAGGGATAACAGCGTAAT | 854 |
| AF105 044 | Metapena eopsis liui | AGAGGGACGATAAGACCCTATAAAGCTTTACAATTATTTAGTTAAA TTATAAATTGTTGGTATAACTTGATTTAATTAATATTTGTTTCGTTG GGGCGACGAGAATAAAATTAAAATAACTGTTCTTTTATTAGTAGTT ACAAATTTGTTTGGTTAAAAATTGATCCTTTATTAGAGATCAAAAGA TTAAGTTACTTTAGGGATAACAGCGTAAT | 855 |
| AF105 043 | Metapena eopsis lamellata | AAGGGACGATAAGACCCTATAAAGCTTTACAATTATTTAATTAAATT ATAAATTATTGGTTTAACTTAATTTAATTAATATTTGTTTCGTTGGG GCGACGAGAATAAAATTAAAATAACTGTTCTTTTATAATTAATTACA AATTTGTTTGGTTAAATATTGATCCTTTATTAAAGATTAAAAGATTA AGTTACTTTAGGGATAACAGCGTAAT | 856 |
| AF105 040 | Metapena eopsis acclivis | AAGGGACGATAAGACCCTATAAAGCTTTACAATTATTTAGTTAAAT TATAAATTGTTGGTTTAACTTGATTTAATTAATATTTGTTTCGTTGG GGCGACGAGAATAAAATTTAAGTAACTGTTCTTTTATAAATAGTCA CAAATTTATTTGGTTAGAAATTGATCCTTTCTTAAAGATCAAAAGAT TAAGTTACTTTAGGGATAACAGCGTAAT | 857 |
| AF105 042 | Metapena eopsis commens alis | AAGGGACGATAAGACCCTATAAAGCTTTACAATTATTTGGCTAATT TATAAATTGTTGGTTTAACTTAAGTGAGTCAATGTTTGTTTCGTTGG GGCGACGAGAATAAAATTAAGTAACTGTTCTTTAAAAGTAATTACA AAGTTAATTGATAAATAATTGGTCCTTTACTAAAGATTAAAAGATTA AGTTACTTTAGGGATAACAGCGTAAT | 858 |
| AY622 201 | Atypopen aeus stenodact ylus | GTGGGACGATAAGACCCTATAAAGCTTTACAATTATTTGATTAAAT TATAAATTGTTAGTATAAACTTAATTTAGTTAATATTTGTTTTGTTGG GGCGACGAGAATATAATTTTAATAACTGTTCTTTTATAATAATTTAC TTTGATAATTGGAGAATAATTGATCCTTTACTAAAGATTAAAAGATT AAGTTACTTTAGGGATAACAGCGTAAT | 859 |
| AY601 738 | Aristeus antillensis | AGGGGACGATAAGACCCTATAAAGCTTTACGATTTGTTTATTAAAT CAAAAATTGTAAGTATAAATTTTATTTAATAGACATTTGTTTCGTTG GGGCGACGAGAATAAAATTTAGTAACTGTTCTTTTAGAGTATAACA ATTATAATTGAAGGAAAATTGATCCCTTATTAAGGATTAAAAATTTA AGTTACTTTAGGGATAACAGCGTAAT | 860 |
| AY601 736 | Solenoce ra vioscai | GGGGGACGATAAGACCCTATAAAGCTTTACAATAAGTTGGTTGAA TTATAAATTGTTAGTTTAACTTAATTTAATTGGTGTTTGTTTCGTTG GGGCGACGAGAATATAATAATATAACTGTTCTTTTGAAAATTTAAC AAAGTTGATTGGATAATAATTGATCCTTTAATAAAGATTAAAAGATT AAGTTACTTTAGGGATAACAGCGTAAT | 861 |
| NC_05 0695 | Trachysal ambria curvirostri s | GGGGGACGATAAGACCCTATAAAGCTTTACAATTATTTAGTTAAAT TATAAATTGTTAGTATAAACTTAATTTAATTGATGTTTGTTTCGTTG GGGCGACGAGAATATAATTTTGTAACTGTTCTTTTATAGTTTGTTA CAATTATTTTTGGTAAAAGATTGATCCTTTATTAAAGATTAAAAGAT TAAGTTACTTTAGGGATAACAGCGTAAT | 862 |
| AY264 908 | Penaeus chinensis | AGGGGACGATAAGACCCTATAAAGCTTGACAATAATTTAATTATAC TATCAATTGTTAGTGTAACTTGGTTTTAATTAATATTTGTTACGTTG GGGCGACGGGAATATAACAAGTAACTGTTCTTAAATATTTAATAAC AAATATAATTGGAAAACTAGCATGATCCTCTATTAGTGATTAAAAG ATTAAGTTACTTTAGGGATAACAGCGTAAT | 863 |
| AY264 907 | Penaeus canalicula tus | GGGGGACGATAAGACCCTATAAAGCTTGACAATAACTTCGTTATAT TATAAATTGTTAGTATAACTTGATTTTAACGGGGGTTTGTTTCGTTG GGGCGACGGGAATATAATAAATAACTGTTCTTTTAAATATAATTAC AAAAATGTTTGGTAAATAATTGATCCTCTATTAGAGATTAAAAGATT AAGTTACTTTAGGGATAACAGCGTAAT | 864 |
| NC_04 0139 | Metapena eopsis barbata | AAGGGACGATAAGACCCTATAAAGCTTTACAATTATTTAATTAAGT TATAAATTGTTGGTTTAACTTAACTTAGTTAATATTTGTTTCGTTGG GGCGACGAGAATAAAATTAAGTAACTGTTCTTTTGTAGTTAATTAC AAATTTGTTTGGTAGAAAATTGATCCTTTATTAAAGATTAAAAGATT AAGTTACTTTAGGGATAACAGCGTAAT | 865 |
| AF279 828 | Penaeus esculentu s | AGGGGACGATAAGACCCTATAAAGCTTGACAGTAAGTTGATTATAT TATAAATTGTTAGTATAACTTGATTTTAGTTAGCGTCTGTTACGTTG GGGCGACGAGAATATAATAAGTAACTGTTCTTAAGTATCTAATAAC AACTATAGTTGGTAAATAAATGATCCTCTATTAGGGATTAGAAGAT TAAGTTACTTTAGGGATAACAGCGTAAT | 866 |
| AY622 202 | Heterope naeus longiman us | GGGGGACGATCAGACCCTATTAAGCTTGACTAGTATTTATTTAAAT TACAAACTGTCAGTATAACGTAATCTTAAATAAAAGTTGTTTCGTTG GGGCGACGGGAATATAAACAATAACTGTTCTTTTGATTATCTTACA AAAATAATTGGTAAATAAGTGATCCTTTATTAAAGATTAAAAGATTA AGTTACTTTAGGGATAACAGCGTAAT | 867 |
| EF601 684 | Atypopen aeus dearmatu s | GAGTGACGATAAGACCCTATAAAGCTTTACAATTATTTGGTTGAAT TATAAATTATTAGTATTAACTTAATTCAATTAATATTTGTTTCGTTGG GGCGACGGGAATATAATTTTAGTAACTGTTCTTTTATAATTAATTAC TTTTATTATTAGGAAAATAATTGATCCTTTATTAAAGATTAAAAGATT AAGTTACTTTAGGGATAACAGCGTAAT | 868 |
| AY622 218 | Funchalia taaningi | AGGGGACGATAAGACCCTATAAAACTTAACAATAATTTAATTAAAT TATAAATTGTAAGTGTAACTTAATTTTAATTAATACTTGTTTCGTTG GGGCGACGAGAATATAAAAAATAACTGTTCTTTTAAATATTTACAT GTGTATAGTTGGTAAAAATTGATCCTTTATTAAAGATTAAAAGATTA AGTTACTTTAGGGATAACAGCGTAAT | 869 |
| AY622 217 | Xiphopen aeus kroyeri | AGGGGACGATAAGACCCTATAAAGCTTTACAAATATCTAGTTAATT TATAAATTGTTATATAAACTTAGGTTAATTAGTGCTTGTTTCGTTGG GGCGACGAGAATATAATTTTGTAACTGTTCTTTTATGGTTTAGTAC AATAATTTTTGGTAAAAAATTGATCCTTTATTAAAGATTAAAAGATT AAGTTACTTTAGGGATAACAGCGTAAT | 870 |
| AY622 216 | Trachype naeopsis mobilispin is | AAGGGACGATAAGACCCTATAAAGCTTTACAAACACTTAGCTGGA TTTTGAAATGTTAGTATAAATTTAATTCAGTTAAAGATTGTTCCGTT GGGGCGACGAAAATATAAATTTAAGTAACTGTTTTGAAAATAAATT ACAGATATATCTGAAAATAAATGATCCTTTAATAAAGATTAAGAGAT CAAGTTACTTTAGGGATAACAGCGTAAT | 871 |
| AY622 215 | Rimapen aeus similis | AGGGGACGATAAGACCCTATAAAGCTTTACAATTATCTTGTTAAAT TATAAATTGTTAGTTTAATCTTAATTTAATTAGTGTTTGTTTCGTTGG GGCGACGGGAATATAATGTTTTGTAACTGTTCTTTTATAATTTGTTA CAATTAATGTTTGGTAAAAAATTGATCCTTTATTAGAGATTAAAAGA TTAAGTTACTTTAGGGATAACAGCGTAAT | 872 |
| AY622 214 | Megokris granulosu s | GGGGGACGATAAGACCCTATAAAGCTTTACAATTATTTAATTAGAT TATAAACTGTTAGTATAAACTTAATTTAATTAATACTTGTTTCGTTG GGGCGACGAGAATATAATTTTGGTAACTGTTCTTTTGTAAATTATT ACAATAAATTTTTGGAGAAAAAATGATCCTTTATTAGAGATTAAAAG ATTAAGTTACTTTAGGGATAACAGCGTAAT | 873 |
| AY622 210 | Parapena eus politus | GGGGGACGATAAGACCCTATAAAGCTTTACAATTATTTAATTAGAT TATAAATTGTTAGTATAACTTAATTTAATTGATATTTGTTTCGTTGG GGCGACGAGAATATAATTTTAAGTAACTGTTCTTTTATGGTTAATTA CAGATTTATTTGGGTAAAAATTGATCCTTTATTAGAGATTAAAAGAT TAAGTTACTTTAGGGATAACAGCGTAAT | 874 |
| FR849 633 | Solenoce ra membran acea | GGGGGACGATAAGACCCTATAAAGCTTTACAATAATTTAATTAAAT TATAAATTGTTAGTATAACTTAATTTAATTAATATTTGTTTCGTTGGG GCGACGGGAATAAAATAGTATAACTGTTCTTTTATAAAATTAAACA GATTTAATTGGATAAAGATTGATCCTTTAATAAAGATTAAAAGATTA AGTTACTTTAGGGATAACAGCGA | 875 |
| FJ435 649 | Solenoce ra koelbeli | AGGGGACGATAAGACCCTATAAAGCTTTACAATAAGTTAATTAAAT TATAAATTGTTAGTATAACTTGGTTTAGTTATTATTTGTTTCGTTGG GGCGACGAGAATATAATTATATATAACTGTTCTTTTAAGAAGAAAA CAAGGTTAATTGGATTAGAATTGATCCTTTAATAAAGATTAAAAGAT TAAGTTACTTTAGGGATAACAGCGTAAT | 876 |
| NC_03 8069 | Alcockpe naeopsis hungerfor dii | GGGGGACGATAAGACCCTATAAAGCTTTACAATTATTTAATTAAAT TGTAAATTGTTTATTTAACATGGTTTAGTTAATGTTTGTTTCGTTGG GGCGACGGGAATATAATTTAGTAACTGTTCTTTATGAGAGACAATA ATTTTTGGTATTATGATCCTCTATTAGAGATTAAAAGATTAAGTTAC TTTAGGGATAACAGCGTAAT | 877 |
| FJ435 641 | Batepena eopsis tenella | GGGTGACGATAAGACCCTATAAAACTTTACAATTATTTAATTGAAT GATGAATTGTTAGTATAAATTTGATTCAATTAATGTTTGTTTCGTTG GGGCGACGAAAATATAATTTTATAACTGTTTTATAATTTTGTAACAA TGATTTTTGGTGAAGATGATCCTTTATTAGAGATTAAAAGATTAAGT TACTTTAGGGATAACAGCGTAAT | 878 |
| EU548 178 | Pandalus platycero s | ATAAAATTTTACAAGATGGGGATTTTTTTTTGAATTAAGGTTTAAAT TCTATAAGTTCCTGTTTGTTGTGTTGGGGCGGCAAAGATAAAAATT AGTAACTGTCATTTAATTAGAATAATTATAATTAGTTTAGTTGATCC TTTATTAGAGATTAAAAG | 879 |
| FJ435 639 | Metapena eus moyebi | GGGGGACGATAAGACCCTATAAAGCTTTACAGTGTATTGATTAAAT TATAAACTGTTAGTATAAACTTAATTTAGTTAATAACTGTTTCGTTG GGGCGACGAGAATATAATAAACAGTAACTGTTCTTTTTTTATAATA ACAATAAATATTTGGTATTAATTGATCCTTTATTAAAGATTAAAAGA TTAAGTTACTTTAGGGATAACAGCGTAAT | 880 |
| NC_04 2173 | Metapena eus joyneri | GGGGGACGATAAGACCCTATAAAGCTTTACAGTGTGTTAATTAAAT TTTAAACTGTTAGTATTAACTTAATTTATTTAATAACTGTTTCGTTGG GGCGACGAAAATATAATGAATAGTAACTGTTTTTCTTTGTAAAATAA CAATAAATTTTTGGAAGTAATTGATCCTTTTTTTAAAGATTAAAAGAT TAAGTTACTTTAGGGATAACAGCGTAAT | 881 |
| EU868 698 | Pandalus montagui | GGGGGACGATAAGACCCTATAAAATTTTACAAAATCGGGGTTTTTT TCTGAATTAAGGTGTAAATTTTATAAATCCCTGTTTGTTGTGTTGG GGCGGCAAAGATAAAAATTAGTAACTGTCATTTAATTAGAATAATT ATAATTAGTTTAATTGATCCTTTATTAGAGATTAAAAGATTAAGTTA CTTTAGGGATAACAGCGTAAT | 882 |
| HQ241 514 | Heterocar pus parvis pin a | GGGGGACGATAAGACCCTATAAAACTTTACAAGCGAAGATTTGTA TTTTTTAAATTAAAGTATAACGTTATGTACTGTTCTGTTTGTTATGTT GGGGCGACAAAGATATAATAAGTAACTGTTAATTTTATTGAATAGT GATAATTAGTTTAATTGATCCTCTCTTAGAGATTACAAGATTAAGTT ACTTTAGGGATAACAGCGTAAT | 883 |
| HM014 402 | Penaeus brasiliensi s | AGGGGACGATAAGACCCTATAAAGCTTGACAATAAATCGTTTATAT TATAAACTGTTAGTGTAACTTGATTTTAAACGGTGTTTGTTGCGTT GGGGCGACGGGAATATAAAAGGTAACTGTTCTTATAACATTTAATA ACAGAAATATCTGAAAAATTAATGATCCTTTTTTTAAAGATCATAAGA TTAAGTTACTTTAGGGATAACAGCGTAAT | 884 |
| GU972 651 | Aristeus antennatu s | GGGGGACGATAAGACCCTATAAAGCTTTACAATAACCAGAATGCA TTAAGATTTGTTAGTATAATTTGGTCTATTTCGGGTTTGTTTCGATG GGGCGACGAGAATATAAGTTAGATAACTGTTCTTTTTAGGTATACA ATATTAATTGGTTAAAAATTGATCCTTTATTAAAGATTATAAGATTAA GTTACTTTAGGGATAACAGCGTAAT | 885 |
| GQ302 761 | Heterocar pus laevigatu s | GGGGGACGATAAGACCCTATAAAACTTTACAGGTAAATCTGTGCT TCTTAAATTAAAGTTTAACATTGGTCATCGTCTTGCCTGTTGTGTTG GGGCGACAAAGATATAATTTAAATAACTGTCAATTTTATTAAATAGC GATAATTAGATTAGTTGATCCTTTATTAGAGATTATAAGATTAAGTT ACTTTAGGGATAACAGCGTAAT | 886 |
| GQ302 759 | Heterocar pus lepidus | GGGGGACGATAAGACCCTGTAAAACTTTACAGACATTGTTTGTATT TTTGAATTAAGGTTTAATATCAATTGTTATTGTTGTTTGTTATGTTG GGGCGACAAAGATATAATTAGAATAACTGTCAATTTTATTAAATAAT GATAATTAGTTAATTGATCCTTTATTAGAGATTATAAGATTAAGTTA CTTTAGGGATAACAGCGTAAT | 887 |
| GQ302 745 | Heterocar pus gibbosus | GGGGGACGATAAGACCCTATAAAACTTTACAGACATTGTTTGTATT TTTGAATTAAAGTTTAATATCAATTGTTATTGTTGTTTGTTATGTTG GGGCGACAAAGATATAATTGGAATAACTGTCAATTTTATTAAATAG TAATAATTAGTTAATTGATCCTTTATTAGAGATTATAAGATTAAGTT ACTTTAGGGATAACAGCGTAAT | 888 |
| JX403 852 | Funchalia villosa | AGGGGACGATAAGACCCTATAAAACTTGACAATAATTTAATTAAAT TATAAATTGTAAGTATAACTTGATTTAATTGATACTTGTTCCGTTGG GGCGACGAGAATATAAAATAACTGTTCTTTTAAATATTTACATATAT ATTTGGTAAAGATTGATCCTTTATTAAAGATTAAAAGATTAAGTTAC TTTAGGGATAACAGCGTAAT | 889 |
| JX403 847 | Hemipen aeus carpenter | GGGGGACGATAAGACCCTATAAAGCTTTACAATAATCTAAATATTC CAAGATTTGTTAGTCTAATTTGGGTTATTTAGAGTTTGTTTCGTTGG GGCGACGAGAATATAAATTAAATAACTGTTCTTTTCAGTACTACAA TATTAATTGATTAAAAATTGATCCTTTATTAAAGATTAAAAGATTAA GTTACTTTAGGGATAACAGCGTAAT | 890 |
| JX403 849 | Mesopen aeus tropicalis | AGGGGACGATAAGACCCTATAAAGCTTTACAATAAAATTATTAAAT TATAAATTGTTAGTCTAACTTAGTTTAATAATTGTTTGTTTCGTTGG GGCGACGAGAATATAATAATATAACTGTTCTTTTAAAAATTATTTAA CAGATTTAATTGGATTATAATTGATCCTTTAATAAAGATTAAAAGAT TAAGTTACTTTAGGGATAACAGCGTAAT | 891 |
| JF899 809 | Pelagope naeus balboae | AGGGGACGATAAGACCCTATAAAACTTAACAATATTTTAACTAAAT TATAAATTGTAAACGTAACTTAATTTTAGTTAATACTTGTTTCGTTG GGGCGACGAGAATATAAATAATAACTGTTCTTTTAAATATAATTCA CATATATGTATGGTAAAAATTGATCCTTTATTAAAGATTAAAAGATT AAGTTACTTTAGGGATAACAGCGTAAT | 892 |
| JF899 806 | Penaeus hathor | GGGGGACGATAAGACCCTATAAAGCTTGACAATAACTTAGTTAAAT TATAAATTGTTAGTATAACTTGATTTTAGCCAGGGTTTGTTTCGTTG GGGCGACGAGAATATAATGAATAACTGTTCTTTTAAATATAATTAC AAAGATAATTGGTGAGCGATTGATCCTCTAATAGAGATTAAAAGAT AAAGTTACTTTAGGGATAACAGCGTAAT | 893 |
| JF899 805 | Metapena eopsis provocato ria | AAGGGACGATAAGACCCTATAAAGCTTTACAATTATTTAATTAGAT TATAAATTGTTGGTGTAACTTGATTTAATTAATATTTGTTTCGTTGG GGCGACGGGAATAAAATTGAAATAACTGTTCTTTTATTAGTAGTCA CAGATTTGTTTGATTAAAAATTGATCCTTTATTAGAGATTAAAAGAT TAAGTTACTTTAGGGATAACAGCGTAAT | 894 |
| NC_03 9154 | Aristeus virilis | GGGGGACGATAAGACCCTATAAAGCTTTACAATAACCAAGATGCA CTAAGATTTGTTAGTATAATTTGGTCTATTTGGGGTTTGTTTCGTTG GGGCGACGAGAATATAAATTAAATAACTGTTCTTTTTAGATATACA ATATTAATTGGTTAAAAATTGATCCTTTATTAAAGATTACAAGATTA AGTTACTTTAGGGATAACAGCGTAAT | 895 |
| KJ486 492 | Aristeus alcock | GGGGGACGATAAGACCCTATAAAGCTTTACAATAACTGAAATACAT TAAGATTTGTTAGTATAATTTGGTTTATTTTGGGTTTGTTTCGTTGG GGCGACGAGAATATAAGTTAGATAACTGTTCTT | 896 |
| KF983 532 | Penaeus aztecus | AGGGGACGATAAGACCCTATAAAGCTTGACAATAAGTCATTTATAT TATAAATTATTAGTATAACTTGATATTAAGTGGTGTTTGTTGCGTTG GGGCGACGAGAATATAAAAGGTAACTGTTCTTATAACATTTAATAA CAGAAATATCTGGAAATTTAATGATCCTTTTTTTAAAGATCATAAGAT TAAGTTACTTTAGGGATAACAGCGTAAT | 897 |
| KF023 186 | Heterocar pus abulbus | GGGGGACGATAAGACCCTATAAAACTTTACAGACATTGTTTGTATT TTTGAATTAAGGTTTAATATCAATTGTTATTGTTGTTTGTTATGTTG GGGCGACAAAGATATAATTGGAATAACTGTCAATTTTATTAAATAG TAATAATTAGTTAATTGATCCTTTATTAGAGATTATAAGATTAAGTT ACTTTAGGGATAACAGCGTAAT | 898 |
| JX403 862 | Penaeus setiferus | AGGGGACGATAAGACCCTATAAAGCTTTACAATAAATTACTTATAT TATAAACTGTTAGTATAACTTGATTTTAGGTAATATTTGTTGCGTTG GGGCGACGAGAATATAATGAGTAACTGTTCTTAAGTTAATTAATAA CATAAATATATGGAAGATGAATGATCCTCTTTTAGAGATCATAAGA TTAAGTTACTTTAGGGATAACAGCGTAAT | 899 |
| JX403 860 | Cerataspi s monstros us | GGGGGACGATAAGACCCTATAAAGCTTTACAATAACTCAGGTGTA CTAAGATTTGTTTGTATAATTTGGTTTACCTGAGACTTGTTTCGTTG GGGCGACGAGAATATAATTAAATAACTGTTCTTTTGGAGTTTACAG TATTAATTGGTTAAAAATTGATCCTTTATTAAAGATTAAAAGATTAA GTTACTTTAGGGATAACAGCGTAAT | 900 |
| JX403 857 | Pleoticus robustus | GGGGGACGATAAGACCCTATAAAGCTTTATAATAATTTAATTAAAT TATAAATTGTTAGTATAACTTAATTTAATTAGTATTTATTTCGTTGGG GCGACGAGAATATAATAATATAACTGTTCTTTTATAGATTAAACAAA TTTAATTGGATAAAAATTGATCCTTTTATAAAGATTAAAAGATTAAG TTACTTTAGGGATAACAGCGTAAT | 901 |
| JX403 854 | Aristaeop sis edwardsi ana | GGGGGACGATAAGACCCTATAAAGCTTTACAATAACTTGAATATAC TAAAATTTGTTAGTGTAACTTAATATATTTGAGATTTGTTTCGTTGG GGCGACGAGAATATAAATAAATAACTGTTCTTTTGCTTAAATATAC AATAATAATTGGTTAAATATTGATCCTTTATTAAAGATTAAAAGATT AAGTTACTTTAGGGATAACAGCGTAAT | 902 |
| JX403 853 | Solenoce ra necopina | GAGGGACGATAAGACCCTATAAAGCTTTACAATAAGATAATTAAAT TATAAATTGTTAGTTTAACTTAATTTAATTATTATTTGTTTCGTTGGG GCGACGAGAATATAATAGTATAACTGTTCTTTTGCAATTTAAACAA AATTAATTGGATAAAAATTGATCCTTTAATAAAGATTAAAAGATTAA GTTACTTTAGGGATAACAGCGTAAT | 903 |
| KP059 272 | Parapena eus indicus | GGGGGACGATAAGACCCTATAAAGCTTTACAATTATTTAATTAAAT TATAAATTGTTAGTATAACTTAATTTAGTTAATATTTGTTTCGTTGG GGCGACGAGAATATAATTTTAAGTAACTGTTCTTTTATAGTTAATTA CAAATTTATTTGGGTAAAGATTGATCCTTTATTATAGATTAAAAGAT TAAGTTACTTTAGGGATAACAGCGTAAT | 904 |
| KP059 270 | Parapena eus cayrei | GGGGGACGATAAGACCCTATAAAGCTTTACAATTATTTAATTAAAT TATAAATTGTTAGTATAACTTAACTTAGTTAATATTTGTTTCGTTGG GGCGACGAGAATATAATTTTAAGTAACTGTTCTTTTATAGTTAATTA CAAATTTATTTGGGTAAAGATTGATCCTTTATTATAGATTAAAAGAT TAAGTTACTTTAGGGATAACAGCGTAAT | 905 |
| KP059 273 | Parapena eus fissurus | GGGGGACGATAAGACCCTATAAAGCTTTACAATTATTTAATTAAAT TATAAATTGTTAGTATAACTTAGTTTAGTTGATATTTGTTTCGTTGG GGCGACGAGAATATAATTTTAAGTAACTGTTCTTTTATAGTTAATTA CAAATTTATTTGGGTAAAGATTGATCCTTTATTATAGATTAAAAGAT TAAGTTACTTTAGGGATAACAGCGTAAT | 906 |
| KP059 274 | Parapena eus investigat oris | GGGGGACGATAAGACCCTATAAAGCTTTACAATTATTTAATTGGAT TATAAATTGTTAGTATAACTTAATTTAGTTGATATTTGTTTCGTTGG GGCGACGAGAATATAATTTTTAAGTAACTGTTCTTTTGTAGTTAATT ACAGATTTATTTGGGTAAAAATTGATCCTTTATTAGAGATTAAAAGA TTAAGTTACTTTAGGGATAACAGCGTAAT | 907 |
| KP059 271 | Parapena eus fissuroide s | GGGGGACGATAAGACCCTATAAAGCTTTACAATTATTTAATTAAAT TATAAATTGTTAGTATAACTTAATTTAGTTAATATTTGTTTCGTTGG GGCGACGAGAATATAATTTTAAGTAACTGTTCTTTTATAGTTAATTA CAAATTTATTTGGGTAAAGATTGATCCTTTATTATAGATTAAAAGAT TAAGTTACTTTAGGGATAACAGCGTAAT | 908 |
| KP059 269 | Parapena eus australien sis | GGGGGACGATAAGACCCTATAAAGCTTTACAATTATTTAATTAAAT TATAAATTGTTAGTATAACTTAATTTAGTTGATATTTGTTTCGTTGG GGCGACGAGAATATAATTTTAAGTAACTGTTCTTTTATAGTTAATTA CAAATTTATTTGGGTAAAGATTGATCCTTTATTATAGATTAAAAGAT TAAGTTACTTTAGGGATAACAGCGTAAT | 909 |
| KP059 267 | Parapena eus american us | GGGGGACGATAAGACCCTATAAAGCTTTACAATTATTTAATTAAAT TATAAATTGTTAGTATAACTTAATTTAATTAATATTTGTTTCGTTGGG GCGACGAGAATATAATTTTAAGTAACTGTTCTTTTGTGGTTAATTAC AGATTTATTTGGGTAAAAATTGATCCTTTATTAGAGATTAAAAGATT AAGTTACTTTAGGGATAACAGCGTAAT | 910 |
| KP059 268 | Parapena eus ruberocul atus | GGGGGACGATAAGACCCTATAAAGCTTTACAATTATTTAATTAAAT TATAAATTGTTAGTATAACTTAATTTAGTTGATATTTGTTTCGTTGG GGCGACGAGAATATAATTTTAAGTAACTGTTCTTTTATAGTTAATTA CAAATTTATTTGGGTAAAGATTGATCCTTTATTATAGATTAAAAGAT TAAGTTACTTTAGGGATAACAGCGTAAT | 911 |
| NC_04 0855 | Heterocar pus ensifer | GGGGGACGATAAGACCCTATAAAACTTTACAAGCGAAGATTTGTA TTTTTTAAATTAAAGTATAACGTTATGTACTGTTCTGTTTGTTATGTT GGGGCGACAAAGATATAATAAGTAACTGTTAATTTTATTGAATAGT GATAATTAGTTTAATTGATCCTCTCTTAGAGATTACAAGATTAAGTT ACTTTAGGGATAACAGCGTAAT | 912 |
| KP059 284 | Kishinouy epenaeop sis cornuta | GGGGGACGATAAGACCCTATAAAGCTTTACAATTGTTTAATTAGAT TGTAAATTGTTTATTTAACATAATTTAATTAATGCTTGTTTCGTTGG GGCGACGGAAATATAATTAGTAACTGTTTTTTATAAAAAACAATTAT TTTTGGTAAAATGATCCTCTATTAGAGATTAAAAGATCAAGTTACTT TAGGGATAACAGCGTAAT | 913 |
| KP059 283 | Parapena eus sextuberc ulatus | GGGGGACGATAAGACCCTATAAAGCTTTACAATTATTTAATTAAAT TATAAATTGTTAGTATAACTTAATTTAGTTAATATTTGTTTCGTTGG GGCGACGAGAATATAATTTTAAGTAACTGTTCTTTTATAGTTAATTA CAAATTTATTTGGGTAAAGATTGATCCTTTATTATAGATTAAAAGAT TAAGTTACTTTAGGGATAACAGCGTAAT | 914 |
| KP059 281 | Parapena eus perezfarf antae | GGGGGACGATAAGACCCTATAAAGCTTTACAATTATTTAATTAGAT TATAAATTGTTAGTATAACTTAATTTAGTTAATATTTGTTTCGTTGG GGCGACGAGAATATAATTTTAAGTAACTGTTCTTTTATAGTTAATTA CAAATTTATTTGGGTAAAAATTGATCCTTTATTATAGATTAAAAGAT TAAGTTACTTTAGGGATAACAGCGTAAT | 915 |
| KP059 280 | Parapena eus murrayi | GGGGGACGATAAGACCCTATAAAGCTTTACAATTATTTAATTGGAT TATAAATTGTTAGTATAACTTAGTTTAGTTGATATTTGTTTCGTTGG GGCGACGAGAATATAATTTTTAAGTAACTGTTCTTTTGTAGTTAATT ACAGATTTATTTGGGTAAAATTGATCCTTTATTAGAGATTAAAAGAT TAAGTTACTTTAGGGATAACAGCGTAAT | 916 |
| KP059 278 | Parapena eus longipes | AGGGGACGATAAGACCCTATAAAGCTTTACAATTGTTTAGTTGGAT TATAAATTGTTAGTATAACTTAATTTAGTTAGTATTTGTTTCGTTGG GGCGACGAGAATATAATTTAAGATAACTGTTCTTTTTAATTAATTAC AAATTTGTTTGGGAGAAGATTGATCCTTTATTAAAGATTATAAGATT AAGTTACTTTAGGGATAACAGCGTAAT | 917 |
| KP059 276 | Parapena eus lanceolat us | GGGGGACGATAAGACCCTATAAAGCTTTACAATTATTTAATTAAAT TATAAATTGTTAGTATAACTTAATTTAGTTAATATTTGTTTCGTTGG GGCGACGAGAATATAATTTTAAGTAACTGTTCTTTTATAGTTAATTA CAAATTTATTTGGGTAAAGATTGATCCTTTATTATAGATTAAAAGAT TAAGTTACTTTAGGGATAACAGCGTAAT | 918 |
| KP059 275 | Parapena eus kensleyi | GGGGGACGATAAGACCCTATAAAGCTTTACAATTATTTAATTAAAT TATAAATTGTTAGTATAACTTAATTTAGTTAATATTTGTTTCGTTGG GGCGACGAGAATATAATTTTAAGTAACTGTTCTTTTATAGTTAATTA CAAATTTATTTGGGTAAAGATTGATCCTTTATTATAGATTAAAAGAT TAAGTTACTTTAGGGATAACAGCGTAAT | 919 |
| KT372 710 | Heterocar pus chani | GGGGGACGATAAGACCCTATAAAACTTTACAGACATTGTTTGTATT TTTGAATTAAGGTTTAATATCAATTGTTATTGTTGTTTGTTATGTTG GGGCGACAAAGATATAATTGGAATAACTGTCAATTTTATTAAATAG TGATAATTAGTTAATTGATCCTTTATTAGAGATTATAAGATTAAGTT ACTTTAGGGATAACAGCGTAAT | 920 |
| KP725 531 | Heterocar pus woodmas oni | AGGGGACGATAAGACCCTATAAAACTTTACAGATATAATTATACTT ATTAAAATAAAGTTTAATAGTATTTATTATTTTATCTGTTATGTTGGG GCGACAAAGATATAATAAAGTAACTGTTAGTTTTATTTGATAATAAT AATTAGTTTAATTGATCCTTTATTAAAGATTATAAGAATAAGTTACTT TAGGGATAACAGCGTAAT | 921 |
| KP725 530 | Heterocar pus sibogae | GGGGGACGATAAGACCCTATAAAACTTTACAAGCAAAGATTTGTAT TTTTTAAATTAAAGTATAACGTTATGTACTGTTCTGTTTGTTATGTT GGGGCGACAAAGATATAATAAGTAACTGTTAATTTTATTGAATAAT GATAATTAGTTTAATTGATCCTCTCTTAGAGATTACAAGATTAAGTT ACTTTAGGGATAACAGCGTAAT | 922 |
| KP725 528 | Heterocar pus dorsalis | GAGGGACGATAAGACCCTATAAAACTTTACAAATATATTTGTAATT TTTAAATTTTAGTTTAACATCAATTACTGGTTTGTTTGTTGTGTTGG GGCGACAATGATATAATTAAATAACTGTCAATTTTATTAAATAGTGA TAATTAGTTTAATTGATCCTTTACTAGAGATTATAAGATTAAGTTAC TTTAGGGATAACAGCGTAAT | 923 |
| KP721 230 | Metapena eopsis andaman ensis | GAGGGACGATAAGACCCTATAAAGCTTTACAATTATTTAATTAGAT TATAAATTGTTGGTATAACTTGATTTAATTAATATTTGTTTCGTTGG GGCGACGAGAATAAAATTAAAATAACTGTTCTTTTATTAGTAGTTA CAAATTTGTTTGGTTAAAAATTGATCCTTTATTAGAGATCAAAAGAT TAAGTTACTTTAGGGATAACAGCGTAAT | 924 |
| KP725 527 | Heterocar pus corona | GGGGGACGATAAGACCCTATAAAACTTTACAGACATTGTTTGTATT TTTGAATTAAAGTTTAATATCAATTGTTATTGTTGTTTGTTTATGTTG GGGCGACAAAGATATAATTGGAATAACTGTCAATTTTATTAAATAG TAATAATTAGTTAATTGATCCTTTATTAGAGATTATAAGATTAAGTT ACTTTAGGGATAACAGCGTAAT | 925 |
| KP721 226 | Metapena eopsis coniger | GAGGGACGATAAGACCCTATAAAGCTTTACAATTATTTAATTAGAT TATAAATTGTTGGTATAACTTGATTTAATTAATATTTGTTTCGTTGG GGCGACGAGAATAAAATTAAAATAACTGTTCTTTTATTAGTAGTTA CAGATTTGTTTGGTTAAAAATTGATCCTTTATTAGAGATCAAAAGAT TAAGTTACTTTAGGGATAACAGCGTAAT | 926 |
| KP081 666 | Macrobra chium idella | AATGGGACGATAAGACCCTATAAAACTTAATATAATTTTGGCTTAA TTTGTGATTATTAGTGAAAAGTAGTTTTGCCGGGTTTATATTTCGTT GGGGAGATGTAGATATAATATGTAACTGTCTATATAATTTAATAATT ATAATTAGTATTTGATCCTTCTTTGTGGATTAGAAGAATAAGTTACT TTAGGGATAACAGCGTGAT | 927 |
| KX162 742 | Trachysal ambria brevisutur ae | GAGGGACGATAAGACCCTATAAAGCTTTATAACTAGCTGATTAAAT TATAAATTATTAGTATAAACTTAGTTTAATTGGTGCTTATTTTGTTG GGGCGACAAGAACATAAAATTGTAACTGTTCTTTTATAGTTTATAT CATTAATGTTTGGTAAATAATTGATCCTTTGTTAAAGATTAAAAGAT TAAGTTACTTTAGGGATAACAGCGTAAT | 928 |
| KX162 734 | Trachysal ambria aspera | GGGGGACGATAAGACCCTATAAAGCTTTACAATTATCTAGTTGAAT TATAAATTATTAGTATAAACTTAGTTTAATTAGTGTTTGTTTCGTTG GGGCGACGAGAATATAATTTTGTAACTGTTCTTTTATGATTTGTTA CAATGATTTTTGGAAAAAATTGATCCTTTATTAAAGATTAAAAGATT AAGTTACTTTAGGGATAACAGCGTAAT | 929 |
| KX162 733 | Trachysal ambria albicoma | GGGGGACGATAAGACCCTATAAAGCTTTACAATTATTTGATTAACT TATAAATTATTAGTATAAACTTAACTTAATCAATGTTTGTTTCGTTG GGGCGACGAGAATATAATTTTGTAACTGTTCTTTAGTAGTTTGTTA CAATAATTTTTGGTAAAAAATTGATCCTTTATTAAAGATTAAAAGAT TAAGTTACTTTAGGGATAACAGCGTAAT | 930 |
| NC_04 0987 | Euphausi a superba | AAGTGACGATAAGACCCTATAAAACTTGATAAATAAAATAAAAAAA GTGGAGTAGTTGATTAAAACTTTTATTGTGGAATTTATTTTATTGGG GCGATAAAAATATAAAAAGTAACTGTTTTTAAAGTTTTAATGATTTA TAATCAGATTGTGTGAATGATCCTTTAATAAAGATTATAAGATTAAG TTACTTTAGGGATAACAGCGTAAT | 931 |
| KU133 278 | Solenoce ra hextii | GGGGGACGATAAGACCCTATAAAGCTTTACAATAAATTAATTAAAT TATAAATTATTAGTTTAACTTAATTTAATTAGTGTTTGTTTCGTTGG GGCGACGAGAATATAAATTATATAACTGTTCTTTTAGGTGTATAAC AAGATTAATTGGAGAAATATTGATCCTTTAGTAAAGATTAAAAGATT AAGTTACTTTAGGGATAACAGCGTAAT | 932 |
| KU133 288 | Hymenop enaeus equalis | AAGGGACGATAAGACCCTATAAAGCTTTACAATAAGTTAATTAAAT TATAAATTGTTAGTATAACTTAATTTAATTAATGTTTGTTTCGTTGG GGCGACGGGAATATAATTATATAACTGTTCTTTTATAAATTTAACAA AATTAATTGGATAATAATTGATCCTTTAATAAAGATTAAAAGATTAA GTTACTTTAGGGATAACAGCGTAAT | 933 |
| KT959 496 | Rimapen aeus constrictu s | AGGGGACGATAAGACCCTATAAAGCTTTACAATTATCTAATTAAAT TATAAATTGTTAGTTTAATCTTAATTTAATTAGTGTTTGTTTCGTTGG GGCGACGAGAATATAATGTTTTGTAACTGTTCTTTTGTAATTTGTTA CAGTTAATAGTTGGTAAAAGATTGATCCTTTATTAAAGATTAAAAGA TTAAGTTACTTTAGGGATAACAGCGTAAT | 934 |
| KT952 497 | Crangon crangon | AATGGGAAGACAAGACCCTATAAAACTTTACAAAATATATTAATAA ATAATTTGGTAGTTTAAAATTATATTAGGTTATTTTTGTTGAGCTGG GGCGGTTTTTATAAAAAAATTAACTGTAAGTTAAATTAAAATATAAA TATATAGTTTAATAAGATCCTTAGGTTTGGAGATTAAAAGACTAAGT TACTTTAGGGATAACAGCATAATTT | 935 |
| KX162 771 | Trachype naeus anchorali s | GAGGGACGATAAGACCCTATAAAGCTTTACAATTATTTAATTAGAT TATAAATTGTTAGTATAAACTTAGTTTAATTAATGCTTGTTTCGTTG GGGCGACGAGAATATAATTTTGGTAACTGTTCTTTTGTAAGCTATT ACAATAAATTTTTGGAGAAAAAATGATCCTTTATTAAAGATTAAAAG ATTAAGTTACTTTAGGGATAACAGCGTAAT | 936 |
| KX162 770 | Megokris pescador eensis | GGGGGACGATAAGACCCTATAAAGCTTTACAATTATTTAATTAGAT TATAAACTGTTAGTATAAACTTAATTTAATTAATACTTGTTTCGTTG GGGCGACGAGAATATAATTTTGGTAACTGTTCTTTTGTAAATTATT ACAATAAATTTTTGGAGAAAAAATGATCCTTTATTAGAGATTAAAAG ATTAAGTTACTTTAGGGATAACAGCGTAAT | 937 |
| KX162 768 | Trachysal ambria longipes | GAGGGACGATAAGACCCTATAAAGCTTTACAATTATTTAACTAAAT TATGAACTGTTAGTATAAATTTAATTTAATTAATGTTTGTTTCGTTG GGGCGACGAGAATATAATTTTTGTAACTGTTCTTTTGTAGTTTGTT GCAATAGTTTTTGGAAAAAGATTGATCCTTTATTAAAGATTAAAAGA TTAAGTTACTTTAGGGATAACAGCGTAAT | 938 |
| KX162 764 | Trachysal ambria staroboga tovi | GGGGGACGATAAGACCCTATAAAGCTTTACAATTATTTGGTTAAAT TATAAATTGTTAGTATAAACTTAATTTAATTGATGTTTGTTTCGTTG GGGCGACGAGAATATAATTTAGTAACTGTTCTTTTATAATTTGTTAC AATGATTTTTGGAAAAGAAATGATCCTCTATTAGAGATTAAAAGATT AAGTTACTTTAGGGATAACAGCGTAAT | 939 |
| KX162 758 | Trachysal ambria nansei | GGGGGACGATAAGACCCTATAAAGCTTTACAATTATTTGGTTAAAT TATGAATTGTTAGTATAAATTTAATTTAATTGATGTTTGTTTCGTTG GGGCGACGGGAATATAATTTTGTAACTGTTCTTTTATAATTTGTTA CAATAATTTTTGGTAAAAGATTGATCCTTTATTAAAGATTAAAAGAT TAAGTTACTTTAGGGATAACAGCGTAAT | 940 |
| KX162 757 | Trachysal ambria malaiana | GAGGGACGATAAGACCCTATAAAGCTTTACAATTATTTAATTAGAT TATAAATTGTTAGTATAAACTTAATTTAGTTAGTGTTTGTTTCGTTG GGGCGACGAGAATATAATTTTGGTAACTGTTCTTTTGTAAATTATT ACAATAAATTTTTGGAGAAAAAATGATCCTTTATTAGAGATTAAAAG ATTAAGTTACTTTAGGGATAACAGCGTAAT | 941 |
| KX162 755 | Trachysal ambria dentata | GGGGGACGATAAGACCCTATAAAGCTTTACAATTATTTAGTTAAAT TATAAATTGTTAGTATAAACTTAATTTAATTGATGTTTGTTTCGTTG GGGCGACGAGAATATAATTTTGTAACTGTTCTTTTATAGTTTGTTA CAATTATTTTTGGTAAAAGATTGATCCTTTATTAAAGATTAAAAGAT TAAGTTACTTTAGGGATAACAGCGTAAT | 942 |
| KX162 749 | Trachysal ambria parvis pin a | GGGGGACGATAAGACCCTATAAAGCTTTACAATTATTTAGTTGGAT TATAAATTGTTAGTATAAACTTAATTTAACTGGTGTTTGTTTCGTTG GGGCGACGGGAATATAATTTTGTAACTGTTCTTTTATAACTTGTTA CAATTATTTTTGGTAAAAATTGATCCTTTATTAAAGATTAAAAGATT AAGTTACTTTAGGGATAACAGCGTAAT | 943 |
| LC464 546 | Crangon uritai | ACTTTACATAATATATTAATAAATAATTTGGTAGTTTAAAATTATGTT AGGTTATTTATGTTGAGCTGGGGCGGTTTTTATAAAAAAAATAACT GTAAGTTAAATTAAAATATAAATATATAGTTTAATAAGATCCTTAAAT TTGGAGATTGGAAGACTAAGTT | 944 |
| LC341 266 | Pandalus borealis | GGGGGACGATAAGACCCTATAAAATTTTACAAAATTGGGATTTTTT TTTGAATTAAGGTTTAAATTCTATAAGTTCTTATTTGTTTTGTTGGG GCGGCAAAGATAAAAAATTAGTAACTGTCATTTAATTAGAATAATTAT AATTAGTTTAATTGATCCTTTATTAGAGATTAAAAGATTAAGTTACT TTAGGGATAACAGCGTAAT | 945 |
| LC150 203 | Metapena eus monocero s | GGGGGACGATAAGACCCTATAAAGCTTTACGGTTTGTTAATTAAAT TATAAATTGTTAGTATAAACTTAATTTAGCTAACAGCTGTTTCGTTG GGGCGACGAAAATATAATAGATAGTAACTGTTTTTCTTTTATAATAA CAATGATTTTTGGTAGTAATTGATCCTTTATTAAAGATTAAAAGATT AAGTTACTTTAGGGATAACAGCGTAAT | 946 |
| LC121 756 | Pandalus nipponen sis | GGGGGACGATAAGACCCTATAAAATTTACAGAGTCGGATCTTT TTTTAAATTAGTGTTTAAATTCTATAGGTTCTTGTTTGTTTTGTTGG GGCGACAAAGATAAAATTTAGTAACTGTCATTTAATTAGAATAAGT ATAATTAGTTTAGTTGATCCTTTATTAGAGATTAAAAGATTAAGTTA CTTTAGGGATAACAGCGTAAT | 947 |
| KY419 832 | Hadropen aeus lucasii | GAGGGACGATAAGACCCTATAAAGCTTTACAATAAAATGGTTAAAT TATAAATTATTAGTATAACTTAATTTAATTATTGTTTGTTTCGTTGGG GCGACGAGAATATAATAATATAACTGTTCTTTATGAAAATAAACAAA ATTAATTGGATAAAGATTGATCCTTTAATAAAGATTAAAAGATTAAG TTACTTTAGGGATAACAGCGTAAT | 948 |
| KY316 150 | Ganjamp enaeopsi s uncta | TTAAAAAGTTTATTATACCCAGGTAATAAGCAGTTATGATCTTGAAA CTTAAAGGATTTGGCGGTAATTTAGTCTAGTTAGAGGAACCTGTCC TGTAATCGATAAACCACGCAGTATCTTACTTTATCTTGAAAATTTAT CAGTTTATATACCATCATTATTAGATAACTTTAAAAAGGTAGAGAAG TTATTGAAATAATTTTAAGTTAGTATATTAGATCAAGGTGTAGCTAA TGATAAAGTAGAGAT | 949 |
| KX530 803 | Solenoce ra annecten s | GGGGGACGATAAGACCCTATAAAGCTTTACAATAATTTAATTAAAT TATAAATTATTAGTTTAACTTAATTTAATTAATAATTGTTTCGTTGGG GCGACGAGAATATAAATTATATAACTGTTCTTTTAAAAATTTAACAA ATTTAATTGGATGAAAATTGATCCTTTAATAAAGATTAAAAGATTAA GTTACTTTAGGGATAACAGCGTAAT | 950 |
| KX574 332 | Solenoce ra melantho | AGGGGACGATAAGACCCTATAAAGCTTTACAATAAGTTAATTAAAT TATAAATTATTAGTTTAACTTAATTTAATTAGTGTTTGTTTCGTTGG GGCGACGAGAATATAAATTATATAACTGTTCTTTTAAAGTAAATAAC AAGATTAATTGGAGGAAGATTGATCCTTTAATAAAGATTAAAAGAT TAAGTTACTTTAGGGATAACAGCGTAAT | 951 |
| MH045 067 | Parapena eopsis stylifera | TTAGAGGGACGATAAGACCCTATAAAGCTTTACAATTATTAAATTG AGCTGTAAATTGTTTGTTATAACGTGGCTAAGTTAATGTTTGTTTC GTTGGGGCGACGGGAATATAATTAGTAACTGTTCTTATATATATAT ATATAACAATAATTTTTGGAAAAATGATCCTCTAATAGAGATTAAAA GATTAAGTTACTTTAGGGATAACAGCGTAAT | 952 |
| MG77 2559 | Penaeus japonicus | GGGGGACGATAAGACCCTATAAAGCTTGACAATAATTTCGTTATAT TATAAATTGTTAGTATAACTTGATTTTAACGGGAGTTTGTTTCGTTG GGGCGACGGGAATATAATAAATAACTGTTCTTTTAAATATAATTAC AAAAATACTTGGTAAATAATTGATCCTCTATTAGAGATTAAAAGATT AAGTTACTTTAGGGATAACAGCGTAAT | 953 |
| MG00 1109 | Penaeus brevirostri s | AGGGGACGATAAGACCCTATAAAGCTTGACAATAAGTTGTTTATAT TATAAATTGTTAGTATAACTTGATTTTAAGCGGTGTTTGTTACGTTG GGGCGACGAGAATATAAAAGGTAACTGTTCTTATATTGTTTAGTGA CAGATATATTTGGAGAGTTAATGATCCTTTCTTAAAGATCACAAGA TTAAGTTACTTTAGGGATAACAGCGTAAT | 954 |
| MG00 1088 | Penaeus notialis | GGGGGACGATAAGACCCTATAAAGCTTGACAATAAGCCGTTTATA TTATAAATTGTTAGTATAACTTGATTTTAAATGGTGTTTGTTGCGTT GGGGCGACGAGAATATAAAAGATAACTGTTCTTATAATATTTAATA ACAGAAATAGTTGGAAAATTAATGATCCTTTCTTTAAGATCATAAGA TTAAGTTACTTTAGGGATAACAGCGTAAT | 955 |
| MG00 1081 | Penaeus duorarum | GGGGGACGATAAGACCCTATAAAGCTTGACAATAAGCCGTTTATA TTATAAATTGTTAGTATAACTTGATTTTAAATGGTGTTTGTTGCGTT GGGGCGACGAGAATATAAAAGATAACTGTTCTTATAATATTTAATA ACAGAAATAGCTGGAAAATTAATGATCCTTTCTTTAAGATCATAAG ATTAAGTTACTTTAGGGATAACAGCGTAAT | 956 |
| MF490 229 | Penaeus schmitti | AGGGGACGATAAGACCCTATAAAGCTTTACAATAAATTACTTATAT TATAAACTGTTAGTATAACTTGATTTTAGGTAATATTTGTTGCGTTG GGGCGACGAGAATATAACGAGTAACTGTTCTTAAGTTAATTAATAA CATAGATATATGGAAGATGAATGATCCTCTTTTAGAGATCATAAGA TTAAGTTACTTTAGGGATAACAGCGTAAT | 957 |
| MF490 228 | Artemesi a longinaris | AAGGGACGATAAGACCCTATAAAGCTTTACAATTATTTAATTAAATT GAAAATTATTAGTATAACTTGATTTAGTTAATAGTTGTTTCGTTGGG GCGACGAGAATATAATTATAAATAACTGTTCTTTTTACAAATAACAA ATTTGTTTGGTTAAAAATTGATCCTTTATTAAAGATTAAAAGATTAA GTTACTTTAGGGATAACAGCGTAAT | 958 |
| MF490 143 | Penaeus subtilis | AGGGGACGATAAGACCCTATAAAGCTTAACAATAAGTTGTCTATAT TATAAATTGTTAGTATAACTTGATATCAGACGGCGTTTGTTGCGTT GGGGCGACGAGAATATAAGAGGTAACTGTTTTTAGATTATTTAATA ACAGAAATATCTGGAAATTTAATGATCCTTTTTTAGAGATCATAAGA TTAAGTTACTTTAGGGATAACAGCGTAAT | 959 |
| NC_01 2060 | Penaeus stylirostris | AGGGGACGATAAGACCCTATAAAGCTTTACAATAAGTTATCTATAT TATAAATTGTTAGTATAACTTGATTTTAGATAATATTTGTTGCGTTG GGGCGACGGGAATATAATGAGTAACTGTTCTTAAGCTATTTAATAA CAGGAATTTCTGGAAAATTAATGATCCTCTTTTAGAGATCATAAGA TTAAGTTACTTTAGGGATAACAGCGTAAT | 960 |
| NC_00 9626 | Penaeus vannamei | AGGGGACGATAAGACCCTATAAAGCTTTACAATAAGTTACCTATAT TATAAATTGTTAGTATAACTTGAGTTTAGGTAACGTTTGTTGCGTTG GGGCGACGAGAATATAATAAGTAACTGTTCTTAAGTTATTTAATGA CAGAAATTTCTGGAAAATTAATGATCCTCTACTAGAGATCATAAGA TTAAGTTACTTTAGGGATAACAGCGTAAT | 961 |
| NC_00 6880 | Macrobra chium rosenber gii | GGGGGACGATAAGACCCTATAAAACTTAATATAATTTAGGCTTAAC TTGCGATGTGGGTGAAAAGTAGTTTTGTCTGGTTTATATTTCGTTG GGGAGATGAAGATATAATGAGTAACTGTCTATAAAATTTTATAGCA TTGACTAGAATTTGATCCTTCCTTGGGGATTAGGAGAATAAGTTAC TTTAGGGATAACAGCGTGAT | 962 |
| NC_00 2184 | Penaeus monodon | AGGGGACGATAAGACCCTATAAAACTTAACAATAATTTGATTAAAT TATAAATTGTTAGTATAACTTGATTTTAATTAATGTTTGTTGCGTTG GGGCGACGGGAATATAATTAGTAACTGTTCTTAAATATTTTATTAA CAAGTATAATTGAAGAATAATTGATCCTTTATTAAAGATTAAAAGAT TAAGTTACTTTAGGGATAACAGCGTAAT | 963 |
| MK470 792 | Pandalus hypsinotu s | GGGGGACGATAAGACCCTATAAAATTTACAAAATTAGGATTTTTT TTTAAATTAAGGTTTAAATTCTATAAGTTCTTGTTTGTTGTGTTGGG GCGGCAAAGATAAAAATTAGTAACTGTCATTTAATTAGAATAAGTA TAATTAGTTTAGTTGATCCTTTATTAGAGATTAAAAGATTAAGTTAC TTTAGGGATAACAGCGTAAT | 964 |
| MK430 861 | Ganjamp enaeopsi s uncta | GGGTGACGATAAGACCCTATAAAACTTTACAATTATTTAATTGAAT GATGAATTGTTAGTATAAATTTGATTCAATTAATGTTTGTTTCGTTG GGGCGACGAAAATATAATTTTATAACTGTTTTATAATTTTGTAACAA TGATTTTTGGTGAGGATGATCCTTTATTAGAGATTAAAAGATTAAG TTACTTTAGGGATAACAGCGTAAT | 965 |
| MK000 270 | Pandalus jordani | GGGGGACGATAAGACCCTATAAAATTTTACAAAATTGGGATTTTTT TTTTGAATTAATGTTTAAATTTTATGAGTTCCTGTTTGTTTTGTTGG GGCGGCAAAGATAAAAATTAGTAACTGTCATTTAATTAGAATAATT ATAATTAGTTTAATTGATCCTTTATTAGAGATTAAAAGATTAAGTTA CTTTAGGGATAACAGCGTAAT | 966 |
| NC_02 7602 | Macrobra chium bullatum | ATGGGACGATAAGACCCTATAAAACTTGATATAAATTTGACTTAAT TTGCGATTGTTTGTGAAAAGTAGTTTTGCTGTGTTTATATTTCGTTG GGGAGATGTAGATATAATATGTAACTGTCTGTTTAATGTAATAATTA TAATTAGTTTGTGATCCTTCATTTGTGGATTAGAAGAATAAGTTACT TTAGGGATAACAGCGTGAT | 967 |
| NC_02 6885 | Penaeus penicillatu s | AGGGGACGATAAGACCCTATAAAGCTTGACAATAATTTAATTATAC TATCAATTGTTAGTATAACTTGGTTTTAATTAAGATTTGTTGCGTTG GGGCGACGAGAATATAATAGGTAACTGTTCTTAAATATTTAATAAC AAATATAATTGAAAATTAGTGTGATCCTCTATTAGCGATTAAAAGAT TAAGTTACTTTAGGGATAACAGCGTAAT | 968 |
| NC_02 6884 | Penaeus merguien sis | AGGGGACGATAAGACCCTATAAAGCTTGACAATAATTTAATTATAC TATCAATTGTTAGTGTAACTTGGTTTTAATTAAAATTTGTTGCGTTG GGGCGACGAGAATATAATAGGTAACTGTTCTTAAATATTTAATAAC AAATATAATTGAAAATTAGTGTGATCCTCTATTAGCGATTAAAAGAT TAAGTTACTTTAGGGATAACAGCGTAAT | 969 |
| NC_02 6834 | Metapena eus ensis | GGGGGACGATAAGACCCTATAAAGCTTTACAGTGTGCTAATTAAA TTATAAATTGTTAGTATAAACTTGGTTTGGTTAGTAGCTGTTTCGTT GGGGCGACGAAAATATAATTGATAGTAACTGTTTTTCTTTTATAATA ACAATAAATTTTTGGTAGAAATTGATCCTTTATTAAAGATTAAAAGA TTAAGTTACTTTAGGGATAACAGCGTAAT | 970 |
| NC_01 7600 | Acetes chinensis | GAGGGACGATAAGACCCTATAAAGCTTTACTGTTTAAATTTTTTGA TATTAGTATTAAATGATCCTCTAATAAAGATTAAAAATTTAAGTTAC TTTAGGGATAACAGCGTAAT | 971 |
| NC_01 5073 | Macrobra chium nipponen se | AGTGGGACGATAAGACCCTATAAAACTTTATATAAGTTTAATTTGG CTTACAATTTGAGTGAAAAGTAGCTTTATTAGGCTTATATTTCGTTG GGGAGATGTAGATGGGATAATCGACTGTAGAGGCCTGCATGCTAT TTAATTTAATAATTATAATTAGTGTGTGATCCTTCTTTGTGGATTAA AAGTATAAGTTACTTTAGGGATAACAGCGTGAT | 972 |
| NC_01 2738 | Penaeus californie nsis | AGGGGACGATAAGACCCTATAAAGCTTGACAATAAGTCGTTTATAT TATAAATTGTTAGTGTAACTTGATTTTAAACGGTGTTTGTTGCGTTG GGGCGACGGGAATATAAAAGGTAACTGTTCTTATAATATTTAATAA CAGAAATATTTGGAAAATTAATGATCCTTTCTTAAAGATCATAAGAT TAAGTTACTTTAGGGATAACAGCGTAAT | 973 |
| NC_01 2217 | Macrobra chium lancheste ri | ATGGGACGATAAGACCCTATAAAACTTAATATAATCTAGTTAATAC TTGCGATTTAGGTAAAAAGGAGTTTTGTTTAGATTATATTTCGTTG GGGAGATGAAGATATAATGTATAACTGTCTATTGAATAATATAATG CTAATTAGAGTTTGATCCTTCTTTGTGGATTAAAAGAATAAGTTACT TTAGGGATAACAGCGTAAT | 974 |
| NC_03 9964 | Pleoticus muelleri | AGGGGACGATAAGACCCTATAAAGCTTTACAATAAATTAATTAAAT TATAAATTATAAGTTTAACTTAATTTAATTAGTATTTGTTTCGTTGGG GCGACGAGAATATAATTATATAACTGTTCTTTTATTGTTATAACAAA TTTAATTGGGTGAAAATTGATCCTTTTATAAAGATTAAAAGATTAAG TTACTTTAGGGATAACAGCGTAAT | 975 |
| NC_03 9179 | Metapena eus affinis | GGGGGACGATAAGACCCTATAAAGCTTTACAGTTTGTTGATTAAAT TATAAATTGTTAGTGTGAACTTAATTTATTTGATAGCTGTTTCGTTG GGGCGACGAAAATATAATAAATAGTAACTGTTTTTCTTTTATAATAA CAATGATTTTTGGTAGTAATTGATCCTTTATTAAAGATTAAAAGATT AAGTTACTTTAGGGATAACAGCGTAAT | 976 |
| NC_03 9169 | Hymenop enaeus neptunus | AAGGGACGATAAGACCCTATAAAGCTTTACAATAAGAAAATTAAAC TATAAATTATTAGTATAACTTAATTTAATTGGTATTTGTTTCGTTGG GGCGACGGGAATATAATAATATAACTGTTCTTTTATAAGTTTAACA AAATTAATTGGATAAAAATTGATCCTTTAATAAAGATTAAAAGATTA AGTTACTTTAGGGATAACAGCGTAAT | 977 |
| NC_03 1366 | Penaeus indicus | AGGGGACGATAAGACCCTATAAAGCTTGACAATAATTTAATTATAC TATCAATTGTTAGTATAACTTGGTTTTAATTAATATTTGTTGCGTTG GGGCGACGAGAATATAATAGGTAACTGTTCTTAAATATTTAATAAC AAATATAATTGGAAATTAGTATGATCCTCTATTAGCGATTAAAAGAT TAAGTTACTTTAGGGATAACAGCGTAAT | 978 |
| NC_03 9153 | Aristaeo morpha foliacea | AGGGGACGATAAGACCCTATAAAGCTTTACAATAAATTAAATATAT TAAAATCTGTTAGTGTAACTTATTATATTTAGAGTTTGTTTCGTTGG GGCGACGAGAATATAATGAATAACTGTTCTTTTTAAAAATAGACAA TAATAATTGGTTAAAGATTGATCCTTTATTAAAGATTAAAAGATTAA GTTACTTTAGGGATAACAGCGTAAT | 979 |
| NC_03 0280 | Solenoce ra crassicor nis | AGGGGACGATAAGACCCTATAAAGCTTTACAATAAGTTAATTAAAT TATAAATTGTTAGTATAACTTGGTTTAGTTATTATTTGTTTCGTTGG GGCGACGAGAATATAATTATATATAACTGTTCTTTTAAGAAGAAAA CAAGGTTAATTGGATTAGAATTGATCCTTTAATAAAGATTAAAAGAT TAAGTTACTTTAGGGATAACAGCGTAAT | 980 |
| NC_03 0277 | Mierspen aeopsis hardwickii | AGGGGACGATAAGACCCTATAAAGCTTTACAATAAATTAATTAAAT TATAAATTGTTAGTTTAACTTAATTTAATTAGTGTTTGTTTCGTTGG GGCGACGAGAATATAAATTATATAACTGTTCTTTTAGATAAATAACA AGGTTAATTGGAGGAAGATTGATCCTTTAATAAAGATTAAAAGATT AAGTTACTTTAGGGATAACAGCGTAAT | 981 |
| NC_04 0140 | Penaeus latisulcatu s | GGGGGACGATAAGACCCTATAAAGCTTGACAATAACTTCGTTAAA TTGTAAATTGTTAGTGTAACTTGATTTTAACTAGGGTTTGTTTCGTT GGGGCGACGGGAATATAATAAATAACTGTTCTTTTAAGTATAGTTA CAAAAATGCTTGGTGAATAATTGATCCTCTACTAGAGATTAAAAGA TAAAGTTACTTTAGGGATAACAGCGTAAT | 982 |
| MG82 1354 | Penaeus semisulca tus | AGGGGACGATAAGACCCTATAAAGCTTGACAATAAGTTAATTATAT TATAAATTGTTAGTATAACTTGATTTTAATTGACGTTTGTTACGTTG GGGCGACGAGAATATAATGAGTAACTGTTCTTAAATGTTTATTGAC AAATATAATTGGTATTTGATTGATCCTTTATTAAAGATTAAAAGATT AAGTTACTTTAGGGATAACAGCGTAAT | 983 |
| AF401 305 | Penaeus silasi | AGGGGACGATAAGACCCTATAAAGCTTGACAATAATTTAATTATAC TATCAATTGTTAGTATAACTTGGTTTTAATTAAGATTTGTTGCGTTG GGGCGACGAGAATATAATAGGTAACTGTTCTTAAATATTTAATAAC AAATATAATTGAAAATTAGTGTGATCCTCTATTAGCGATTAAAAGAT TAAGTTACTTTAGGGATAACAGCGTAAT | 984 |
| MG00 1049 | Penaeus isabelae | AGGGGACGATAAGACCCTATAAAGCTTAACAATAAGCTGTCTATAT TATAAACTGTTAGTATAACTTGATACTAGATGGTATTTGTTGCGTTG GGGCGACGAGAATATAAAAGGTAACTGTTCTTATAACATATAATAA CAGAAATATCTGGAAATTTAATGATCCTTTTTTAAAGATCATAAGAT TAAGTTACTTTAGGGATAACAGCGTAAT | 985 |
| NC_03 9168 | Sicyonia lancifer | GGGGGACGATAAGACCCTATAAAGCTTTACAAACCCTTTTTTTAGT TAAAAATTATAAGTGTAAACTAACTTAAAAATTATTTGTTTCGTTGG GGCGACGAGAATATAATTTAGTAACTGTTCTTTTAAAATATTACAGT ATTAGTTGATTGAAAATTGATCCCTTAATAAGGATTAAAAGTTTAAG TTACTTTAGGGATAACAGCGTAAT | 986 |
| NC_02 9457 | Metapena eopsis dalei | GAGGGACGATAAGACCCTATAAAGCTTTACAATTATTTAGTTAAGT TATAAATTGTTGGTCTAACTTAATTTAATTAATATTTGTTTCGTTGG GGCGACGAGAATAAAATTAAGTAACTGTTCTTTTGTAAATAATTAC AAATCTATTTGGTAAAAAATTGATCCTTTATTAAAGATTAAAAGATT AAGTTACTTTAGGGATAACAGCGTAAT | 987 |
| MK971 537 | Metapena eopsis gerardoi | AAGGGACGATAAGACCCTATAAAGCTTTACAATTAGTCAACTAAAT CATAAATTATTGGTTTAACTTGGTTTAGTTGATGTTTGTTTCGTTGG GGCGACGAGAATATAATTAAAATAACTGTTCTTTAATAATTAATTAC AGTTTTTTCTGATATAAAATTGATCCTTTATTAAAGATTAAAAGATTA AGTTACTTTAGGGATAACAGCGTAAT | 988 |
| KP059 279 | Parapena eus longirostri s | GGGGGACGATAAGACCCTATAAAGCTTTACAATTATTTAATTAAAT TATAAATTGTTAGTATAACTTAATTTAATTGATATTTGTTTCGTTGG GGCGACGAGAATATAATTTTAAGTAACTGTTCTTTTATGGTTAATTA CAGATTTATTTGGGTAAAAATTGATCCTTTATTAGAGATTAAAAGAT TAAGTTACTTTAGGGATAACAGCGTAAT | 989 |
| MK470 790 | Pandalus eous | GGGGGACGATAAGACCCTATAAAATTTTACAAAATTGGGATTTTTT TTTGAATTAAGGTTTAAATTCTATAAGTTCTTATTTGTTTTGTTGGG GCGGCAAAGATAAAAATTAGTAACTGTCATTTAATTAGAATAATTAT AATTAGTTTAATTGATCCTTTATTAGAGATTAAAAGATTAAGTTACT TTAGGGATAACAGCGTAAT | 990 |
| LC431 729 | Pandalus miyakei | GGGGGACGATAAGACCCTATAAAATTTTACAAAATCGGGATTTTTT TTGAATTAAGGTTTAAGTTCTATAATTCCTTGTTTGTTGTGTTGGGG CGGCAAAGATAAAAATTAGTAACTGTCATTTAATTAGAATAAGTATA ATTAGTTTAGTTGATCCTTTATTAGAGATTAAAAGATTAAGTTACTT TAGGGATAACAGCGTAAT | 991 |
| MK470 785 | Pandalus japonicas | GGGGGACGATAAGACCCTATAAAATTTTACAAAATTGGAATTTTTA TTTGAATTAGGGTTTAAATTCTATGATTTCTTGTTTGTTGTGTTGGG GCGACAAAGATAAAAGTTAGTAACTGTCATTTAAGTAGAATAAGTA TAATTAGTTTAGTTGATCCTTTATTAGAGATTAAAAGATTAAGTTAC TTTAGGGATAACAGCGTAAT | 992 |
| LC431 728 | Pandalus glabrus | GGGGGACGATAAGACCCTATAAAATTTTACAAAATCGGGATCTTTT TTGAATTAAGGTTTAAATTCTATGATTCCTTGTTTGTTGTGTTGGGG CGGCAAAGATAAAAATTAGTAACTGTCATTTAATTAGAATAAGTATA ATTAGTTTAGTTGATCCTTTATTAGAGATTAAAAGATTAAGTTACTT TAGGGATAACAGCGTAAT | 993 |
| MK470 796 | Pandalus teraoi | GGGGGACGATAAGACCCTATAAAATTTTACAGAGTTGGGACCTTT ATTTTAAATTAGTGTTTAAATCTTATGGGTTCTTATTTGTTTTGTTG GGGCGACAAAGATAAAATTTAGTAACTGTCATTTAATTAGAATAAG TATAATTAGTTTAGTTGATCCTTTATTAGAGATTAAAAGATTAAGTT ACTTTAGGGATAACAGCGTAAT | 994 |
| MK470 793 | Pandalus ivanovi | GGGGGACGATAAGACCCTATAAAATTTTACAAAATTAGGATTTTTT TTTAAATTAAGGTTTAAATTCTATAAGTTCTTGTTTGTTGTGTTGGG GCGGCAAAGATAAAAATTAGTAACTGTCATTTAATCAGAATAAGTA TAATTAGTTTAGTTGATCCTTTATTAGAGATTAAAAGATTAAGTTAC TTTAGGGATAACAGCGTAAT | 995 |
| MK470 784 | Pandalus coccinatu s | GGGGGACGATAAGACCCTATAAAATTTTACAAAATCGGGGTTTTTT TTTAATTAAGGTTTAAATTCTGTAATTTCTTGTTTGTTGTGTTGGGG CGGCAAAGATAAAAATTAGTAACTGTCATTTAATTAGAATAAGTATA ATTAGTTTAGTTGATCCTTTATTAGAGATTAAAAGATCAAGTTACTT TAGGGATAACAGCGTAAT | 996 |
| MK470 791 | Pandalus formosan us | GGGGGACGATAAGACCCTATAAAATTTTACAGAGTCGGGTCTTTT TTTAAATTAGTGTTTAAATTCTGCAGGTTCTTATTTGTTTTGTTGGG GCGACAAAGATAAAATTTAGTAACTGTCATTTAATCAGAATAGATA TAATTAGTTTAGTTGATCCTTTATTAGAGATTAAAAGATTAAATTAC TTTAGGGATAACAGCGTAAT | 997 |
| LC431 732 | Pandalus princeps | GGGGGACGATAAGACCCTATAAAATTTTACAAAATTGGGATTTTTT TTGAATTAAGGTTTAAATTCTATAATTCCTTGTTTGTTGTGTTGGGG CGGCAAAGATAAAAATTAGTAACTGTCATTTAATTAGAATAAGTATA ATTAGTTTAGTTGATCCTTTATTAGAGATTAAAAGATTAAGTTACTT TAGGGATAACAGCGTAAT | 998 |
| MK470 789 | Pandalus chani | GGGGGACGATAAGACCCTATAAAATTTACAGAGTCGGATCTTT TTTTAAATTAGTGTTTAAATTCTATGGGTTCTTATTTGTTTTGTTGG GGCGACAAAGATAAAATTTAGTAACTGTCATTTAATTAGAATAAGT ATAATTAGTTTAGTTGATCCTTTATTAGAGATTAAAAGATTAAGTTA CTTTAGGGATAACAGCGTAAT | 999 |
| LC431 731 | Pandalus houyuu | GGGGGACGATAAGACCCTATAAAATTTTACAAAATCGGAGTTTTTT TTGAATTAAGGTTTAAATTCTTTAATTCCTTGTTTGTTGTGTTGGGG CGACAAAGATAAAAATTAGTAACTGTCATTTAATTAGAATAAGTATA ATTAGTTTAGTTGATCCTTTGTTAGAGATTAAAAGATTAAGTTACTT TAGGGATAACAGCGTAAT | 1000 |
| LC431 730 | Pandalus capillus | GGGGGACGATAAGACCCTATAAAATTTTACAAAATTGGGATTTTTT TTGAATTAAGGTTTAAATTCTATAATTCCTTGTTTGTTGTGTTGGGG CGGCAAAGATAAAAATTAGTAACTGTCATTTAATTAGAATAAGTATA ATTAGTTTAGTTGATCCTTTATTAGAGATTAAAAGATTAAGTTACTT TAGGGATAACAGCGTAAT | 1001 |
| MK470 786 | Pandalus longirostri s | GGGGGACGATAAGACCCTATAAAATTTTACAAAATTGGGATTTTTT TTGAATTAAGGTTTAAATTCTATAATTCCTTGTTTGTTGTGTTGGGG CGGCAAAGATAAAAATTAGTAACTGTCATTTAATTAGAATAAGTATA ATTAGTTTAGTTGATCCTTTATTAGAGATTAAAAGATTAAGTTACTT TAGGGATAACAGCGTAAT | 1002 |
| MK470 787 | Pandalus ochotensi s | GGGGGACGATAAGACCCTATAAAATTTTACAAAATCGGAGTTTTTT TTGAATTAAGGTTTAAATTCTTTAATTCCTTGTTTGTTGTGTTGGGG CGACAAAGATAAAAATTAGTAACTGTCATTTAATTAGAATAAGTATA ATTAGTTTAGTTGATCCTTTGTTAGAGATTAAAAGATTAAGTTACTT TAGGGATAACAGCGTAAT | 1003 |
| AB244 633 | Pandalus latirostris | AGGGGACGATAAGACCCTATAAAATTTTACAAAATAGGGATTTTTT TTAAATTAAGGTTTAAATTCTATAAGTCTCTGTTTGTTGTGTTGGGG CGGCAAAGATAAAAATTAGTAACTGTCATTTAATTAGAATAAGCAT AATTAGTTTAGTTGATCCTTTATTAGAGATTAAAAGATTAAGTTAGT TTAGGGATAACAGCGTAAT | 1004 |
| MK500 702 | Metapena eus brevicorni s | GGGGGACGATAAGACCCTATAAAGCTTTACAGTGTATTAATTAAAT TTTAAATTGTTAGTATTAACTTAGTTTATTTAGTAACTGTTTCGTTG GGGCGACGAAAATATAATAAATAGTAACTGTTTTTCTTTATAATATA ACAAAAATTTTTGGAAGTAATTGATCCTTTCTTAAAGATTAAAAGAT TAAGTTACTTTAGGGATAACAGCGTAAT | 1005 |
| MK500 704 | Metapena eus dobsoni | GGGGGACGATAAGACCCTATAAAGCTTTACAGTGTATTAATTAAAT TTTAAATTGTTAGTATTAACTTAGTTTATTTAGTAACTGTTTCGTTG GGGCGACGAAAATATAATAAATAGTAACTGTTTTTCTTTATAATATA ACAAAAATTTTTGGAAGTAATTGATCCTTTCTTAAAGATTAAAAGAT TAAGTTACTTTAGGGATAACAGCGTAAT | 1006 |
| MK470 779 | Heterocar pus hayashii | GGGGGACGATAAGACCCTATAAAACTTTACAAGCGAAGATTTGTA TTTTTTAAATTAAAGTATAACGTTATGTACTGTTCTGTTTGTTATGTT GGGGCGACAAAGATATAATAAGTAACTGTTAATTTTATTGAATAGT GATAATTAGTTTAATTGATCCTCTCTTAGAGATTACAAGATTAAGTT ACTTTAGGGATAACAGCGTAAT | 1007 |
| MK470 780 | Heterocar pus fascirostr atus | AGGGGACGATAAGACCCTATAAAACTTTACAGATATAATTATACTT ATTAAAATAAAGTTTAATAGTATTTATTATTTTATCTGTTATGTTGGG GCGACAAAGATATAATAAAGTAACTGTTAGTTTTATTTGATAATAAT AATTAGTTTAATTGATCCTTTATTAAAGATTATAAGAATAAGTTACTT TAGGGATAACAGCGTAAT | 1008 |
| NC_05 0168 | Palaemo n adspersu s | AGGGGACGATAAGACCCTATAAAACTTTATAAATGAAGTATGTAAT CTCTAAATTATATTAAAATGTTGATAAGTATTTTATTTATTTCGTTGG GGCGACGTTGATATAATTTGTAACTGTCTAAATGAATAATATAATC GTCATTATACTTTGATCCTTCTTTGTGGATTATAAGACTAAGTTACT TTAGGGATAACAGCGTAAT | 1009 |
| NC_05 0266 | Palaemo n serratus | AGGGGACGATAAGACCCTATAAAACTTTATACATAGAATATAAAAT CTTTAAATTATATTAAAATATTGATGGATATTTTATGTATTTCGTTGG GGAGACGTTGATATAATTTGTAACTGTCTGAATGAATAATATAATTA TCATTATAGTTTGATCCTTCTTTGTGGATTATAAGAATAAGTTACTT TAGGGATAACAGCGTAAT | 1010 |
| NC_04 5090 | Palaemo n sinensis | AGGGGACGATAAGACCCTATAAAACTTAATATTTATTTATATTTTGT TTTTAAATTTTAAATAAACTTTACAAAGTTTAGTATATATTTTGTTGG GGAGACATTGACATAATTAATAACTGTCTAATTTAAATGTTTATAGC TATTGTTATTATATGATCCTTCTTTGAGGATGAAAAGAGCAAGTTA CTTTAGGGATAACAGCGTAAT | 1011 |
| NC_03 9373 | Palaemo n capensis | AGGGGACGATAAGACCCTATAAAACTTTATATAAATTAAGAATTAT TGTGAATTGGACTTAAATATTTTAATGCTTAATTTATATTTCGTTGG GGAGACGCTGATATAATTTGTAACTGTCAGTTAAATAAAATATTTAT AATTAGTAAATTGATCTTTCAATGAGAATTTAAGAGAAGTTACTTTA GGGATAACAGCGTAAT | 1012 |
| NC_03 8117 | Palaemo n annandal ei | GGGGGACGATAAGACCCTATAAAACTTGATAAAAAGTTTATTTTAA CTTTAAATTATATTTAAAAATTGTGAAGTTTTCTTTTTATTTTGTTGG GGCGACATTGATATAAATTGTAACTGTTTTAGCAAAAAGTATAATTA TCATTATAGTTTGATCCTTCTTTGGGGATAAAAAGAATAAGTTACTT TAGGGATAACAGCGTGAT | 1013 |
| NC_02 9240 | Palaemo n gravieri | AAGGGACGATAAGACCCTATAAAACTTTATAAGAGATTTATTTTATT TTTAAATTCTTCTTTAAAAATTGTGAAGTATTTCTCTTATTTTGTTGG GGCGACATTGATATAAGCTGTAACTGTTTAAATAAAAACTATAATTA TCATTATTGTTTGATCCTTCTTTGTGGATAAAAAGACTAAGTTACTT TAGGGATAACAGCGTAAT | 1014 |
| NC_02 7601 | Palaemo n serenus | GTGGGACGATAAGACCCTATAAAACTTTATATGTTGCCTGCTTTTT GTTAAAATTCTACCTAAAATTTGAAAAGCACTCTACATATTTTGTTG GGGAGACATTGATATAAATTGTAACTGTCTAAATAAAAAATATAATT ATCATTATAATCTGATCCTTCTTTGTGGATAAAAAGAATAAGTTACT TTAGGGATAACAGCGTGAT | 1015 |
| NC_01 2566 | Palaemo n carinicau da | AGGGGACGATAAGACCCTATAAAACTTAATAAAAGGTTAATTTTAA CTATGAATTGTATTTAAAAGTTGTGAAATTCTCTTTTTATTTTGTTG GGGCGACATTGATATAAGCTGTAACTGTCTTGATAATAAAATAGTT ATCACTATAAGCTGATCCTTTTTTTAGGGATAATAAGTATAAGTTACT TTAGGGATAACAGCGTAAT | 1016 |
| KT959 474 | Palaemo n pugio | AAGGGACGATAAGACCCTATAAAACTTGACACGCCACTGTATTTA GCTTTAAATTTTAATCTAAAATTGTAAGGGTAGCTGTGTGTTTTGTT GGGGAGACATTGATATAAGTAGTAACTGTCTGATCAAAAATTATAA TTATAATTAGCATATGATCCCTATTTTAGGATTATAAGAATAAGTTA CTTTAGGGATAACAGCGTAAT | 1017 |
| KF923 713 | Palaemo n pandalifor mis | AGGGGACGATAAGACCCTATAAAACTTCACATGAAACGTTGTAGT TTATGGATCTTGTTTAATGTAATTTCAGCGTTTTTTGTTTTGTTGGG GCGACATGGATAAAAAGTAACTGTCTATTAAAATTCAATAACAATA ATTAGTTTGTGATCCTTTATTAAGGATTAAAAGAATAAGTTACTTTA GGGATAACAGCGTGAT | 1018 |
| MT340 087 | Palaemo n elegans | AGGGGACGATAAGACCCTATAAAACTTTATGCACGACATGTCTTG TCTTTAAATTATATCTAAATATTGGTTGGTTAAAAGTGCATTCCGTT GGGGAGACGTTGATATAAGCTGTAACTGTTTTATTAAATAGAATAA TTATTATTATAATTTGATCCTTCTTTGTGGATAAAAAGAATAAGTTA CTTTAGGGATAACAGCGTAAT | 1019 |
| MT340 091 | Palaemo n longirostri s | ATTACGCTGTTATCCCTAAAGTAACTTATTCTTATAACCCACAAAGA AGGATCAAAATATAATGATAGTTATTTTATTCACTCAGACAGTTACA AATTATATCAACGTCTCCCCAACGAAATATATTAAGAATTTATCAGT ATTTTAATATAATTTAAAGATTATATACTTTATATATATAAAGTTTTATA GGGTCTTATCGTCCCTC | 1020 |
| JQ042 296 | Palaemo n peringuey i | GTGGGACGATAAGACCCTGTAAAACTTTATACGCATAGTATTTAGC CTTTAAATTATATTAAAATATTGATATATACTTTGGGTATTTCGTTG GGGAGACGTTGATATAATTTGTAACTGTCTAATTAAATAAAATAATT ATCATTATACTTTGATCCTTCTTTGTGGGTTATAAGAATAAGTTACT TCAGGGATAACAGCGTAAT | 1021 |
| KP725 611 | Palaemo n debilis | GGGGGACGATAAGACCCTATAAAACTTTATGTGATTTGTGGTTATT TATTAAAATTTTACTTAAAATTTAGAAAATCTCATTTACATTTTGTTG GGGAGACATAGATATAATTTGTAACTGTCTGTTGTAAAAATATAGC TATCGTTAGAATTTGATCCCTCTGTGGGGATAATAAGAATAAGTTA CTTTAGGGATAACAGCGTAAT | 1022 |
| KF923 720 | Palaemo n carteri | AGGGGACGATAAGACCCTATAAAACTTTATATAAAATAAGTATATC TACATATTATGTTAATAGGGGTATGCTATTTTATATTTTGTTGGGGC GACATAGATATGTAAGCAAATTAACTGTTTATTAAAATAAAATAATA ATAATTAGTTTATGATCCTTTAGTGAAGATTAAAAGAGTAAGTTACT TTAGGGATAACAGCGTGAT | 1023 |
| JN674 344 | Palaemo n ritteri | AGGGGACGATAAGACCCTATAAAACTTGATATAGAGCTGACTTTA GTTTTAAATTTTAAAATAAAATTGTAAAGTAAACTTTATATTTTGTTG GGGAGACATTGATATAAGTAGTAACTGTCTGTTAAAAATTATAATT ATATTTAGTTTTTGATCCTTCTTTGTGGATTAGAAGAATAAGTTACT TTAGGGATAACAGCGTGAT | 1024 |
| KC515 036 | Palaemo n orientis | AGGGGACGATAAGACCCTATAAAACTTAATAAAAGGTTAATTTTAA CTATGAATTGTATTTAAAAGTTGTGAAATTCTCTTTTTATTTTGTTG GGGCGACATTGATATAAGCTGTAACTGTCTTGATAATAAAATAGTT ATCACTATAAGCTGATCCTTTTTTTAGGGATAATAAGTATAAGTTACT TTAGGGATAACAGCGTAAT | 1025 |
| DQ194 924 | Macrobra chium gracilirost re | TGTGTAATTTATTACAAAGTCTAGCCTGCCCACTGATTTAAGGGGA CGATAAGACCCTATAAAACTTAATATAAGTTGGGCTTAATTTATGAT TTAGGTGAAAAATGATTTTGTTTGGTTTATATTTCGTTGGGGAGAT GAAGATATAAAATGTAACTGTCTATTTAAATTTAATAATATTAATTA GTATGTGATCCTTCTTTGTGGATTATAAGAATAAGTTACTTTAGGG ATAACAGCGTAAT | 1026 |
| KT959 473 | Palaemo n vulgaris | AAGGGACGATAAGACCCTATAAAACTTAACACATTGTTATTTCTAG CTTTGAATTTTAACCTAAAATTTTAGAGATAATCTTGTGTTTTGTTG GGGAGACATTGATAAAACTAGTAACTGTCTAAGTAAAAATTATAAT TATAATTAGCATGTGATCCCTCTATGAGGATTAGAAGAATAAGTTA CTTTAGGGATAACAGCGTAAT | 1027 |
| JQ042 295 | Palaemo n serrifer | GGGGGACGATAAGACCCTATAAAACTTTATAAAAGACTAATTTCAT TTTTAAATTCTGTTAAAAAATTATGAAATTTTTCTTTTATTTTGTTGG GGCGACATTAATATAAGCTGTAACTGTTTAAATAAAAATTATAATTA TCATTATTGTTTGATCCCTCTTTGAGGATAAGAAGACTAAGTTACTT TAGGGATAACAGCGTAAT | 1028 |
| MT340 090 | Palaemo n varians | GGGGGACGATAAGACCCTATAAAACTTTATACACGACTCAGTTCA TCTTTAAATTTTAATTAAATTAAAGTGAAATGGGACGTGTATTTCGT TGGGGAGACGTTGATATAAACTGTAACTGTCTAATTGAAAAATATA AAAATAATTATTATATGATCCTTCTTTGTGGATAAAAAGATTAAGTT ACTTTAGGGATAACAGCGTAAT | 1029 |
| JQ042 297 | Palaemo n macrodac tylus | AAGGGACGATAAGACCCTATAAAACTTTATAAGAGATTTATTTTATT TTTAAATTCTTCTTTAAAAATTGTGAAGTAATTCTTTTATTTTGTTGG GGCGACATTGATATAAGCTGTAACTGTTTAAATAAAAACTATAATTA TCATTATTGTTTGATCCTTCTTTGTGGATAAAAAGACTAAGTTACTT TAGGGATAACAGCGTAAT | 1030 |
| KC515 035 | Palaemo n tonkinens is | GGGGGACGATAAGACCCTATAAAACTTTATATAAATATAGGTTTTT TTTAATTTTACTAAAATATTAGAGATCCTTATTTATATTTTGTTGGG GAGACATTGATATAAGCAGTAACTGTCTGATTAAAAATAATAATGA TATTTATAAAGTGAACCCTCACTGAGGATTAAAAGAATAAGTTACT TTAGGGATAACAGCGTAAT | 1031 |
| JQ042 294 | Palaemo n xiphias | CGGGGACGATAAGACCCTATAAAACTTTATACATAAAGTGTGTAAT CTCTATATTATATTGAAATGTCGATAAGTACTCTATTTATTTCGTTG GGGAGAGGTCCATATACCTTGTAACTGTCTGAGTGAACAAAATAA TTATCATTATATTTTGATCCTTCTTTGTGGATTATAAGAGTAAGTTA CTTTAGGGATAACAGCGTAAT | 1032 |
| KF923 729 | Palaemo n ivonicus | TGGGGACGATAAGACCCTATAAAACTTTATATAAAATATATTTGTTT ATAAATTATGTTTATAAGAAATTTATTATTTTATATTTTGTTGGGGC GACATAAATAAAACATGTAACTGTTTATATAAATAAAATAATGATAA TTAAATTTTGATCCTTTAATAAGGATTACAAGAATAAGTTACTTTAG GGATAACAGCGTAAT | 1033 |
| JN674 340 | Palaemo n pacificus | GGGGGACGATAAGACCCTGTAAAACTTTATACGTAGGGTATTTAG TCTTTAAATTATACTAAATTGTTGATATATATCTTAGGTATTTCGTTG GGGAGACGTGGATATAATTTGTAACTGTCTAATTAATAATATAATTA TCTTTATATTTTGAACCTTCTTTGTGGGTTATAAGAATAAGTTACTT CAGGGATAACAGCGTAAT | 1034 |
| JN674 352 | Palaemo n atrinubes | GTGGGACGATAAGACCCTATAAAACTTTATATGAATTGAGATTATT TGTCAAAATTTTAATTAAAATTTAAAAGATCTTATTTATATTTTGTTG GGGAGACATTGATATAACTTGTAACTGTCTAGTTGAAAAATATAGT TATTGCTAGAATTTGATCCCTCTATGAGGATAAAAAGAATAAGTTA CTTTAGGGATAACAGCGTGAT | 1035 |
| KF923 725 | Palaemo n intermedi us | GTGGGACGATAAGACCCTATAAAACTTTACATGTGGTACGCTTTTT TTTAAAATTTTAATTAAACTTTGGAAAGTGTTCTTCATGTTTTGTTG GGGAGACATTGATATAATTTGTAACTGTCTAAATAAAACATATAATA ATCATTAGAATTTGATCCTTCTTTGTGGATAAAAAGAATAAGTTACT TTAGGGATAACAGCGTAAT | 1036 |
| KC515 043 | Palaemo n concinnu s | GGGGGACGATAAGACCCTATAAAACTTTACATAAGTTACATTTAGT TTGTGAATAAAGTAAAAAACTGTTTTAGATACCTTATGTTTCGTTGG GGAGACGTTTATATAACTGTAACTGTCAGATAAATTTAATAATTCTA ATTAGTATATGATCCTTCTGTGGGGATTATAAGAGTAAGTTACTTT AGGGATAACAGCGTGAT | 1037 |
| KF923 716 | Palaemo n yuna | GGGGGACGATAAGACCCTATAAAACTTTATATAAAATATGTTTGTT TATAAATTGCGTTTAAAGGAAATTTATTGTTTTATATTTTGTTGGGG CGACATAAATAAAATTTGTAACTGTTTATATAAATAAAATAACGATA ATTAGATTTTGATCTTTTAATAAGGATTATAAGAATAAGTTACTTTA GGGATAACAGCGTAAT | 1038 |
| JQ042 306 | Palaemo n antennari us | GGGGGACGATAAGACCCTATAAAACTTTATACGCTGTTTAATTTGT CTTTGAATTTTAAATAAATTAAAGTGAATTAGCCAGTGTATTTCGTT GGGGAGACGTTAATATAAATTGTAACTGTTTAATTAAAAAATATAAA TATAATTATGGTATGATCCTTCTTTGTGGATTAAAAGATTAAGTTAC TTTAGGGATAACAGCGTAAT | 1039 |
| JN674 333 | Palaemo n dolospinu s | GTGGGACGATAAGACCCTATAAAACTTTATATGTGGCCTGCTTTTT CTTAAAATTTCAGATAAACTTTGAAAAGCAGTTCTCGTATTTTGTTG GGGAGACATTGATATAAATCGTAACTGTCTGAATAAAATATATAGT AATCACTATAATTTGATCCTCCTATGCGGCTAAAAAGAACAAGTTA CTTTAGGGATAACAGCGTAAT | 1040 |
| KF923 714 | Palaemo n gracilis | GGGGGACGATAAGACCCTATAAAACTTTACATAAAAAACTATAGTC TATGAATTATATTTAAGGGGAGTCTAGTCTTTTGTGTTTTGTTGGG GCGACATGGATAAAAAGTAACTGTCTATTAAAATTTAATAATAATAA TTAGTTTTTGATCCTTTAGTATGGATTAAAAGAATAAGTTACTTTAG GGATAACAGCGTGAT | 1041 |
| JN674 354 | Palaemo n mundusn ovus | AAGGGACGATAAGACCCTATAAAACTTGATACGCCGTTATGCTTA GCTTTAAATTTTAATCTAAAATTGTAAGGGTGGCTGTGTGTTCTGT TGGGGAGACATTGATATAAATAGTAACTGTCTGATTAAAAAATATA ATTATAATTAGTATGTGATCCTTCTTTGTGGATTATAAGAGTAAGTT ACTTTAGGGATAACAGCGTAAT | 1042 |
| KF923 712 | Palaemo n suttkusi | ATGGGACGATAAGACCCTATAAAACTTTATATGTTATATATTTTATT TTTGAATTATTTTAAAATATTGTTTAATGTTTTACATATTTCGTTGGG GAGACGTTGACATAAAATGTAACTGTTTAAATAAAAAATATAATTAT CATTATATTTTGATCCTTCTTTGTGGATAAAAAGAATAAGTTACTTT AGGGATAACAGCGTAAT | 1043 |
| JQ042 299 | Palaemo n zariquieyi | GGGGGACGATAAGACCCTATAAAACTTTATACATTATTTAGTTCGT CTTTAAATTTTAAGTAAATTATAGTGAATTGATATGTGTATTTCGTT GGGGAGACGTTGATATAAATTGTAACTGTCTAATTAAAAAATATAA ATATGATTATGATGCGATCCTTCTTTGTGGATAAAAAGATTAAGTTA CTTTAGGGATAACAGCGTAAT | 1044 |
| JN674 353 | Macrobra chium australien se | GTGGGACGATAAGACCCTATAAAACTTTATATGTCGTTTGCTTTTT CTTAAAATTTTATGTAAACTTTGAGAAGCATTTCTCATATTTTGTTG GGGAGACATTGATATAAGCTGTAACTGTCTAAATAAAATATATAAT AATCATTAGAATTTGATCCTTCTTTGTGGATAAAAAGAATAAGTTAC TTTAGGGATAACAGCGTAAT | 1045 |
| JN674 345 | Palaemo n semmelin kii | GGGGGACGATAAGACCCTATAAAACTTTATATTTTAATTTTTTGCTT TAGATTATAGATTAAAATTGCAAAGTTATTTAAATATTTCGTTGGGG CGACGTTGATAAAATCTTAACTGTCTGTGTAAAAGTTATAGTAATTA CTAGAATATGATCCTTCTTTTGAGGATAACAAGAATAAGTTACTTTA GGGATAACAGCGTAAT | 1046 |
| JN674 337 | Palaemo n litoreus | GTGGGACGATAAGACCCTATAAAACTTTATATGTTGCCTGCTTTTT GTTAAAATTCTATCTAAAATTTGAAAAGCACTCTACATATTTTGTTG GGGAGACATTGATATAAATTGTAACTGTCTAAATAAAAAATATAATT ATCATTATAATTTGATCCTTCTTTGTGGATAAAAAGAATAAGTTACT TTAGGGATAACAGCGTGAT | 1047 |
| LC425 608 | Palaemo n septemtri onalis | AAGGGACGATAAGACCCTATAAAACTTAATATAAATTATACTATATT TTTAAATTATAAATAATCATGATAAAGTTTAGTTTATATTTTGTTGGG GAGATATTGATATAAATTGTAACTGTCTAATTTAAAAAAAATAATAA TCATTATAATGTGATCCTTCATTGAGGATAAAAAGAATAAGTTACTT TAGGGATAACAGCGTAAT | 1048 |
| JN674 335 | Palaemo n guangdon gensis | AAGGGACGATAAGACCCTATAAAACTTTATAAGAGATTTGTTTTAT TTTTAAATTCTTCTTTAAAAGTTGTGAAGTAATTCTTTTATTTTGTTG GGGCGACATTGATATAAGCTGTAACTGTTTAAATAAAAACTATAAT TATCATTATTGTTTGATCCTTCTTTGTGGATAAAAAGACTAAGTTAC TTTAGGGATAACAGCGTAAT | 1049 |
| KF923 715 | Palaemo n hancocki | GGGGGACGATAAGACCCTATAAAACTTTACATTAAAAACTATAGTC TATAAATTATATTTAAGGGGAGTCTAGCGTTTATATGTTTTGTTGG GGCGACATGGATAAAAAGTAACTGTCTATAAAAATTTAATAATAAT AATTAGTTTTTGATCCTTTATTATGGATTAAAAGAATAAGTTACTTT AGGGATAACAGCGTGAT | 1050 |
| KC515 037 | Palaemo n vietnamic us | GGGGGACGATAAGACCCTATAAAACTTAATAAAAGGTTTATTTTAA CTATAAATTATATTTAAAAGTTGTGAAATCTTCTTTTTATTTTGTTGG GGCGACATTGATATAAACTGTAACTGTCTTAATAATAAAATAATTAT CATTATGAGCTGATCCTTTTTTTAGGGATAATAAGTATAAGTTACTTT AGGGATAACAGCGTAAT | 1051 |
| JQ042 303 | Palaemo n texanus | AAGGGACGATAAGACCCTATAAAACTTGATACGCCGTTATGCTTA GCTTTAAATTTTAATCTAAAATTGTAAGGATGGCTGTGTGTTCTGTT GGGGAGACATTGATATAAATAGTAACTGTCTGATTAAAAAATATAA TTATAATTAGTATGTGATCCTTCTTTGTGGATTATAAGAGTAAGTTA CTTTAGGGATAACAGCGTAAT | 1052 |
| JN674 339 | Palaemo n ortmanni | AAGGGACGATAAGACCCTATAAAACTTTATAAGAGACTTATTTTAT TTTTAAATTCTTTTTAAAAATTATAAAATATTTCTTTTATTTTGTTGG GGCGACATTGATATAACCTGTAACTGTTTAAATAAAAATTATAATTA TCATTATTGTTTGATCCTTCTTTGTGGATAAAAAGAATAAGTTACTT TAGGGATAACAGCGTAAT | 1053 |
| JQ042 302 | Palaemo n turcorum | GGGGGACGATAAGACCCTATAAAACTTTATACGTTACCCTATTCAT CTTTGAAGTTTAGATAAATTATAGTGAATGGGGGGTGTATTTCGT TGGGGAGACGTTGATATAATCTGTAACTGTCTAATTGAAAAATATA AATATAATTAGTACATGATCCTTCTTTGTGGATAAAAAGATTAAGTT ACTTTAGGGATAACAGCGTAAT | 1054 |
| KF923 718 | Palaemo n kadiaken sis | AAGGGACGATAAGACCCTATAAAACTTTATATGTAAGGTATTTTGT TTTTAAATTATTTTGAAATATTAGTTAATATTTTACATATTTCGTTGG GGAGACGTTGATATAACATGTAACTGTCTAAATAAAAAAAATAATT ATTATTATATTTTGATCCTTCTTTGTGGATAAAAAGAATAAGTTACT TTAGGGATAACAGCGTAAT | 1055 |
| EU493 137 | Macrobra chium asperulu m | GAGGGACGATAAGACCCTATAAAACTTTATATAGACTTAATTTAAC TTGTGACTTAAGTGAAAAATAGTTTTGTTGAGTTTATATTTTGTTGG GGAGATGTAGATATAATATGTAACTGTCTATTTAATTTAATAATTAT AATTAGTGTTTGATCCTTCTTTGTGGATTAAAAGTGTAAGTTACTTT AGGGATAACAGCGTGAT | 1056 |
| GU987 057 | Macrobra chium australe | ATGGGACGATAAGACCCTATAAAACTTAATATAAATTTAGTTTAATT TGTGACATAAGTGAAAAGTGGTTATACTTCATTTATATTTCGTTGG GGAGATGTAGATATAAAAGTAACTGTCTATTAAATTTAATAGCCAT AGTTAGTAATTGATCCTTCTTTGGGGATTAAAAGAGTAAGTTACTT TAGGGATAACAGCGTGAT | 1057 |
| MH253 292 | Macrobra chium olfersii | GTGGGACGATAAGACCCTATAAAACTTGATATAAGTTGAACTTGAT TTGTGATTTAAGTGAAAAGCAGTTTTGTTTGACTTATATTTTGCTGG GGCGGCATAGATATAAAATGTAACTGTCTATTAAATTTAATAGTGC TAATTAGTATTTGATCCTTCCTTGTGGATTAAAAGAATAAGTTACTT TAGGGATAACAGCGTGAT | 1058 |
| GU929 448 | Macrobra chium jelskii | GAGGGACGATAAGACCCTATAAAACTTAACATAAGTTTTGTTTTAT TTATAATTTTAGTGTAAAATGGTTTTACTAAGTTTATGTTTCGTTGG GGAGATGAAGATATAAAGAGTAACTGTCTTTTGAATAGAATAATTA TAATTAGTTTTTGATCCTTCTTTGGGGATTAAGAGAATAAGTTACTT TAGGGATAACAGCGTGAT | 1059 |
| KJ544 746 | Macrobra chium villosiman us | GGGGGACGATAAGACCCTATAAAACTTTATATAATCTAAACTTGAC TTGGGATTTAAGTGAAAAGTAGTTTTGTTCGGTTTATATTTCGTTG GGGAGATGAAGATATAATTTGTAACTGTCTATTGAATATTATAGTAT TAATTAGTATTTGATCCTTCTTTGAGGATTAGGAGAATAAGTTACTT TAGGGATAACAGCGTGAT | 1060 |
| KP763 693 | Macrobra chium equidens | GTGGGACGATAAGACCCTATAAAACTTAATATAAGTTAAATTGGAT TTGTGATTTCAGTAAAAAGTGGTCCTTTTAAGTTTATATTTCGTTGG GGAGATGTAGATATAATGTGTAACTGTTTATAAAACTAAATAATCAT AATTAGTTTGTGATCCTGCAGATGTGGATTGAAAGAGTAAGTTACT TTAGGGATAACAGCGTAAT | 1061 |
| JX466 930 | Macrobra chium potiuna | GAGGGACGATAAGACCCTATAAAACTTAATATAAATTGTAATTTTG TTTATGATTTAGGTGGAAAATAATTTAATTTAATTTATATTTCGTTG GGGAGATGTAGATATAAAATGTAACTGTCTATAAAATTTAATAATTA TTGTTAGTGGTTGATCCTTCTTTGGGGATTAAAAGAATAAGTTACT TTAGGGATAACAGCGTAAT | 1062 |
| MK782 955 | Macrobra chium malcolms onii | GAGGGACGATAAGACCCTATAAAACTTGATATAATTTGAACTTGAC TTGTGATTTGAGTGAAAAATAGTCTTGTTTGGTTTATATTTCGTTGG GGAGATGAAGATATAATGTGTAACTGTCTATTGAATTTTATAGTATT AATTAGAATTTGATCCTTCTTTGAGGATTAGGAGAATAAGTTACTTT AGGGATAACAGCGTGAT | 1063 |
| KC515 041 | Macrobra chium superbum | AGGGGACGATAAGACCCTATAAAACTTTATATAAAGTTCATATTCG ATTAAATTTTAATGTTAATAAAAATTGGATGTTAGCTTTATATTTTGT TGGGGCGACAAAAATAAAATTGATAGCAACTGTTTAAATAATTAGA CAAAAATATTTGATTATATAATTAAATGATCCTAATTAAAGATTAAAA GTTTAAGTTACTTTAGGGATAACAGCGTTAT | 1064 |
| KM610 135 | Macrobra chium striatum | GTGGGACGATAAGACCCTATAAAACTTGATATAAGTTTGATTGGAT TTGTGATTTCAGTAAAAAATGGTCCTCTGAGGCTTATATTTCGTTG GGGAGATGTAGATATAATATGTAACTGTTTGTAAAATTAAATAATTA TAATTAGTTTGTGATCCTGCCGTGTGGATTGAAAGAGTAAGTTACT TTAGGGATAACAGCGTAAT | 1065 |
| JF310 722 | Macrobra chium latidactylu s | AAGGGACGATAAGACCCTATAAAACTTGATACGAGTTTAGCTTAAT TTGTGATTTATGTGAAAAGTAGTTTTGCTTGGTTCGTATTTCATTGG GGAGATGTAGATATAATCTGTAACTGTCTATGAAATTTAATAATCAT AATTAGTAGTTGATCCTTCTTTGTGGATTAAAAGAGTAAGTTACTTT AGGGATAACAGCGTGAT | 1066 |
| JQ805 822 | Macrobra chium hancocki | GTGGGACGATAAGACCCTATAAAACTTGATATAAGTGAACCTTGAT TTGTGATTTGTGTGAAAAATAGTGTTGCTTTACTTATATTTTGCTGG GGCGGCATAGATATAAAATGTAACTGTCTGTTAAATTTAATAGTGT TAATTAGTTTTTGATCCTTCCTTGTGGATTAGAAGAATAAGTTACTT TAGGGATAACAGCGTGAT | 1067 |
| GU929 449 | Macrobra chium acanthuru s | GGGGGACGATAAGACCCTATAAAACTTGATATTGATCTGGCATGA TTTGCGATTTTAGTGAAAAGTAGTTTTGTTTGATCTATATTTCGTTG GGGAGATGAAGATATAAATTGTAACTGTCTATTTAATTTAATAATTA TAGTTAGTGTTTGATCCTTCTTTGTGGATTAAGAGATTAAGTTACTT TAGGGATAACAGCGTGAT | 1068 |
| DQ194 931 | Macrobra chium inflatum | GTGGGACGATAAGACCCTATAAAACTTTATATAAGTTTAATTTGGC TTACAATTTGAGTGAAAAGTAGCTTTATTAGGTTTATATTTCGTTGG GGAGATGTAGATATAATGTGTAACTGTCTGTTTAATTTAATAATTAT AATTAGTGTGTGATCCTTCTTTGTGGATTAAAAGTATAAGTTACTTT AGGGATAACAGCGTGAT | 1069 |
| JQ805 801 | Macrobra chium crenulatu m | GTGGGACGATAAGACCCTATAAAACTTGATATAAGTGAAGCTTGAT TTGTGATTTGGGTGAAAAATAGTGTTGCTTTGCTTATATTTTGCTG GGGCGGCATGGATATAAAATGTAACTGTCTGTTAAATTTAATAGTG TTAATTAGTTTATGATCCTTCTTTGTGGATTAAAAGAATAAGTTACT TTAGGGATAACAGCGTGAT | 1070 |
| KM101 474 | Macrobra chium carcinus | GTGGGACGATAAGACCCTATAAAACTTTATACAGGTTGGGTCAAA CTTGTGATTTGTGTGAAAAGTAGTTTTATTTGATCTGTATTTTGTTG GGGCGACATAGATATAATGTGTAACTGTCTATTAAATTAAATAGTG TTAATTAGTCTTTGATCCTTCTTTGTGGATTAGAAGAATAAGTTACT TTAGGGATAACAGCGTGAT | 1071 |
| GU929 463 | Macrobra chium american um | GTGGGACGATAAGACCCTATAAAACTTTATACAGGTTGGGTTCAAT TTGTGATTTGTGTGAAAAGTAGTTTTATTCGGTCTGTATTTTGTTGG GGCGACATAGATATAATATGTAACTGTCTATCAAATTAAATAGTGT TAATTAGTTTTTGATCCTTCTTTGTGGATTAGAAGAATAAGTTACTT TAGGGATAACAGCGTGAT | 1072 |
| EU493 143 | Macrobra chium latimanus | GTGGGACGATAAGACCCTATAAAACTTGATATATGCTGAGTTTAAT TTATGATATAAGTGAAAAGTAGTTATACTTGGTTTATATTTCGTTGG GGAGATGTAGATATAAATTGTAACTGTCTATTTAATTTAATAATGTT AATTAGTGTGTGATCCCTCTTTGGGGATTAAAAGAATAAGTTACTT TAGGGATAACAGCGTGAT | 1073 |
| FM986 629 | Macrobra chium mammillo dactylus | GTGGGACGATAAGACCCTATAAAACTTGATATAAGCTTGATTTAAT TTGCGATTATTAGTGAAAAGTAGTTTTGTTAGGTTTATATTCCGTTG GGGAGATGTAGATATAATGTGTAACTGTCTATTAAATTTAATAGTTA TAATTAGTTTTTGATCCTTCTTTGTGGATTAGAAGAATAAGTTACTT TAGGGATAACAGCGTGAT | 1074 |
| JQ805 812 | Macrobra chium faustinum | GTGGGACGATAAGACCCTATAAAACTTAATATGAGTTAAGCTTGAT TTGTGATCTAAGTGAAAAATAGTTTTGTTTGATTTATATTTTGCTGG GGCGGCATAGATATAAAATGTAACTGTCTATTAAATTTAATAGTGT TAATTAGTCTTTGATCCTTCTCTGTGGATTAAAAGAATAAGTTACTT TAGGGATAACAGCGTGAT | 1075 |
| AY377 842 | Macrobra chium heterochir us | GTGGGACGATAAGACCCTATAAAACTTGATATTATCTAGATTTGAT TTGCGATTTGAGTGAAGAGCGGTTTTATTTATATGATATTTTGTTG GGGAGACATAGATATAATTAGTAACTGTCTATTAAATTTAATAGTGT TAGTTAGCATTTGATCCTTCTTTGTGGATTAAAAGAATAAGTTACTT TAGGGATAACAGCGTGAT | 1076 |
| KP756 688 | Macrobra chium scabricul um | GTGGGACGATAAGACCCTATAAAACTTGATATAAGTTTAGCTTGAT TTGTGATTTAGGTGAAAGACGATTTTGTTTGGTTTATATTTCGTTG GGGAGATGTAGATATAATGTGTAACTGTCTATTTAATTTAATAAATA TAATTAGTATGTGATCCTTCATTTGTGGATTGGAAGAATAAGTTAC TTTAGGGATAACAGCGTGAT | 1077 |
| KM101 476 | Macrobra chium digueti | GTGGGACGATAAGACCCTATAAAACTTGATATAAGCTGGACTTGA TTTGTGATTTAAGTGAAAAGCAGTTTTGTTTAACTTATATTTTGCTG GGGCGGCATAGATATAAAATGTAACTGTCTGTTAAATTTAATAGCG CTAACTAGTATTTGATCCTTCTTTGTGGATTAAAAGAATAAGTTACT TTAGGGATAACAGCGTGAT | 1078 |
| KF383 310 | Macrobra chium tenellum | GGGGGACGATAAGACCCTATAAAACTTAATATCAATTTGGTTTGAT TTGCGATTTTAGTGAAAAGTAGTCGTGCTTGACTGATATTTCGTTG GGGAGATGAAGATATAAATTGTAACTGTCTATTTAATTCAATAGCT ATGGTTAGTGTGTGATCCTTCTTTGTGGATTAAGAGAGTAAGTTAC TTTAGGGATAACAGCGTGAT | 1079 |
| KP037 053 | Macrobra chium idae | GTGGGACGATAAGACCCTATAAAACTTAATATAATTTTGATTTGATT TGCGATTTTCGGTGAAAAGTAGTCTTGTCGGGTTTATATTTCGTTG GGGAGATGTAGATATAATATGTAACTGTCTATTTAATTTAATAGCTA TAACTAGTCTTTGATCCTTCTTTGTGGATTAAAAGAGTAAGTTACTT TAGGGATAACAGCGTGAT | 1080 |
| JX025 200 | Macrobra chium japonicu m | GGGGGACGATAAGACCCTATAAAGCTTGACAATAACTTCGTTATAT TATAAATTGTTAGTATAACTTGATTTTAACGGGGGTTTGTTTCGTTG GGGCGACGGGAATATAATAAATAACTGTTCTTTTAAATATAATTAC AAAAATGTTTGGTAAATAATTGATCCTCTATTAGAGATTAAAAGATT AAGTTACTTTAGGGATAACAGCGTAAT | 1081 |
| EU493 150 | Macrobra chium formosen se | GTGGGACGATAAGACCCTATAAAACTTTATATAAGTTTAATTTGGC TTACAATTTGAGTGAAAAGTAGCTCTATTAAGCTTATGTTTCGTTG GGGAGATGTAGATATAATGTGTAACTGTCTGTTTAATTTAATAATTA TAATTAGTGTGTGATCCTTCTTTGTGGATTAAAAGTATAAGTTACTT TAGGGATAACAGCGTGAT | 1082 |
| JQ390 474 | Macrobra chium dienbienp huense | GTGGGACGATAAGACCCTATAAAACTTAATATGGGTGTGGCTTGA TTTGCGATTAGTAGGTGAAAAGTAATTTTGTTTGGCTCATATTTTGT TGGGGAGATGTGGATATAATGTGTAACTGTCTGTTGAATTTAATAG TTATAATTAGTGTATGATCCTTCATTTGTGGATTAGGAGAATAAGTT ACTTTAGGGATAACAGCGTGAT | 1083 |
| EU493 139 | Macrobra chium placidulu m | GGGGGACGATAAGACCCTATAAAACTTTATATAAGTTTGGTTTAGT CTATGATTTAGGTAAAAGTGGTTTTGTCTGGTTTATATTTCGTTG GGGAGATGAGGATATAAAATGTAACTGTCTGTTTAAATTTAATAGT TTTAATTAGTTTGTGATCCCCCTTTGGGGATTAAAAGAGTAAGTTA CTTTAGGGATAACAGCGTGAT | 1084 |
| JQ362 449 | Macrobra chium sintangen se | GTGGGACGATAAGACCCTATAAAACTTTATATAAGAGTAACTTGGT TTATGATTTAGGTGAAAAGTAGCTTTGTTGGGTTTATATTTCGTTG GGGAGATGTAGATATAATATATAACTGTCTGTTTAATTTAATAGCT GTAGTTAGTGTGTGATCCTTCTTTGTGGATTAAAAGTATAAGTTAC TTTAGGGATAACAGCGTGAT | 1085 |
| JQ359 750 | Macrobra chium niphanae | GTGGGACGATAAGACCCTATAAAACTTAATATAGGTTTGACTTAAT TTTATAAAATGGGTGATAAAGTGATTTTGTTTAGTTTATATTTTGTT GGGGCGATATAGATATAATATGTAACTGTCTATTAAATTTAATAATT ATAATTAGTTTTTGATCCTACTTTGTGGATTGAAAGAATAAGTTACT TTAGGGATAACAGCGTGAT | 1086 |
| KF383 311 | Macrobra chium totonacu m | GTGGGACGATAAGACCCTATAAAACTTGATATAAGTTTAGTTTAAT TTGTGATTTGAGTGAAGAGCAATTTTGTTAAATTTATATTTTGTTGG GGAGATGTAGATATAAAATATAACTGTCTATTGAATTTAATAGTAAT AATTAATTTTTGATCCTTCTNTGAAGATTAGGAGAATAAGTTACTTT AGGGATAACCGCGCTGA | 1087 |
| JF491 346 | Macrobra chium tuxtlaens e | GTGGGACGATAAGACCCTATAAAACTTGATATAAGTTTAATTTAAT TTGTGATTTAAGTGAAGAACAATTTTGTTAAATTTATATTTTGTTGG GGAGATGTAGATATAAAATATAACTGTCTATTAAATTTAATAGTAAT AATTAATTTTTTGATCCTTCTGTGGGGATTAGGAGAATAAGTTACTTT AGGGATAACAGCGTGAT | 1088 |
| KF383 313 | Macrobra chium vicconi | ATGGGACGATAAGACCCTATAAAACTTAATATAAATTTAGTTTAATT TGTGATTTAAGTGAAGAGCAGTTTTATTAAATTTATATTTTGTTGGG GAGATGTAGATATAAAGTGTAACTGTCTAATAAATTTAATAATAATA ATTAATTTTTGATCCTTCCATGAGGATTAGGAGAGTAAGTTACTTTA GGGATAACAGCGTGAT | 1089 |
| KF383 314 | Macrobra chium villalobosi | GTGGGACGATAAGACCCTATAAAACTTGATATAAGTTTAATTTAAT TTGTGATTTAAGTGAAGAGCGATTTTGTTGAATTTATATTTTGTTGG GGAGATGTAGATATAAAGTGTAACTGTCTATTGAATTTAATAGTAA TAATTAGTTTTTGATCCTTCTGTGGAGATTAAGAGAGTAAGTTACTT TAGGGATAACAGCGTGAT | 1090 |
| KM101 468 | Macrobra chium amazonic um | GGGGGACGATAAGACCCTATAAAACTTAATATCTGTTTAGCTTGAT TTGTGATTTTAGTGAAAAGTAGTTTTGTTAAGTTGATATTTCGTTGG GGAGATGAAGATATAAATTGTAACTGTCTATTATAATTTGATAATTA TAGTTAGTGTGTGATCCTTCTTTGTGGATTAAAAGAATAAGTTACTT TAGGGATAACAGCGTAAT | 1091 |
| JF774 072 | Macrobra chium canarae | AAGGGACGATAAGACCCTATAAAACTTGATATAGTTCAAGCTTGAC TTGTGATTATGAGTAAAAAGTAGTTTTGTTTGGTTTATATTTTGTTG GGGAGATAAAGATATAATGTGTAACTGTCTATTGAATTTTATAGTAT TAATTAGAATTTGATCCTTCTTTGAGGATTAAGAGAATAAGTTACTT TAGGGATAACAGCGTGAT | 1092 |
| JQ362 452 | Macrobra chium tratense | GTGGGACGATAAGACCCTATAAAACTTTATATAAAAGTAACTTGGT TTATGATTTAGGTGAAAAGTAGCTTTGTTGGGTTTATATTTCGTTG GGGAGATGTAGATATAATATATAACTGTCTGTTTAATTTAATAGCT GTAGTTAGTGTGTGATCCTTCTTTGTGGATTAAAAGTATAAGTTAC TTTAGGGATAACAGCGTGAT | 1093 |
| JQ362 454 | Macrobra chium forcipatu m | GTGGGACGATAAGACCCTATAAAACTTAATATGGGTTTGGCTTGAT TTGCGATTAGTGGGTGAAAAGTAATTTTGTTTAGCTCATATTTTGTT GGGGAGATGTGGATATAATGTGTAACTGTCTGTTGAATTTAATAGT TATGATTAGTGTGTGATCCTTCATTTGTGGATTAGGAGAATAAGTT ACTTTAGGGATAACAGCGTGAT | 1094 |
| JQ390 475 | Macrobra chium hirsutima nus | GTGGGACGATAAGACCCTATAAAACTTAATATAGGTTTAGCTTGAT TTGCAATTATGGGTGAAAAGTGGTTTTGTCTAGGCTTATATTTTGT TGGGGAGATGTAGATATAATGTGTAACTGTCTGTTGAATTTAATAG TTATGATTAGTGTGTGATCCTTCATTTGTGGATTAAGAGAATAAGT TACTTTAGGGATAACAGCGTAAT | 1095 |
| GU929 445 | Macrobra chium borellii | GGGGGACGATAAGACCCTATAAAATTTAATATAAGTTTAATTTTATT TGTAATTTAAGTGAAAAATAGTTTAATTAAATTTATATTTCGTTGGG GAGATGTGGATATAAAGTGTAACTGTCTATAAAATTTAATAGTAATT ATTAGTAAGTGATCCTTCTTTGTGGATTTGAAGAATAAGTTACTTTA GGGATAACAGCGTAAT | 1096 |
| GU929 446 | Macrobra chium brasiliens e | GGGGGACGATAAGACCCTATAAAACTTGATATAAATTTAGTTTTAC TAGTGATTTAAGTGAAAAGTGGTTTAATTAAAGTTATATTTCGTTGG GGAGATGTAAATATAAATGTAACTGTTTATAAAATTAAATAATTATT GTTAGTAGGTGATCCTGCATATGCGGATTGAGAAAATAAGTTACTT TAGGGATAACAGCGTAAT | 1097 |
| JF310 709 | Macrobra chium aemulum | GTGGGACGATAAGACCCTATAAAACTTGATATAAATTTAGTTTGAT TTATGATGTGAGTGAAAAGTTGTTTTGCTAGGTTTATATTCCGTTG GGGCGATGAAGATATAAAATGTAACTGTCTATTTAATTTAATAGTAT TAATTAGTACTTGATCCTTCTTTGGGGATTAAAAGAATAAGTTACTT TAGGGATAACAGCGTGAT | 1098 |
| JF310 717 | Macrobra chium handschi ni | GTGGGACGATAAGACCCTATAAAACTTGATATAAGTTTTGCTTGAT TTGTGATTTGTAGTGAAAAGTGGTTTCGTTTAATTTATATTTCGTTG GGGAGATGTAGATATAATATGTAACTGTCTATTTAATTTAATAGTTA TAATTAGTATTTGATCCTTCTTTGTGGATTAAGAGAATAAGTTACTT TAGGGATAACAGCGTGAT | 1099 |
| JF310 719 | Macrobra chium horstii | ATGGGACGATAAGACCCTATAAAACTTAATATAAGTTGATCTTGAT TTGTGATTTGGGTGAAAAATGATTTTGCTTGGCTTATATTTCGTTG GGGAGATGAAGATATAAAATGTAACTGTCTGTGAAATTTAATAGTG TTAATTAGTTTTTGATCCTTCTTTGTGGATTAAGAGAATAAGTTACT TTAGGGATAACAGCGTGAT | 1100 |
| GU929 447 | Macrobra chium ferreirai | GGGGGACGATAAGACCCTATAAAACTTAATATAAATTTGATTTTAT TTATAATTTTAGTGAAAAGAAGTTTTAATAAGTTTATATTTTGTTGG GGTGACGTGGATATAAAGTGTAACTGTCCGTAAAATTTAATAATTT TAATTAGTGTCTGATCTTTCTTATGGGGATTAAAAGAGTAAGTTAC TTTAGGGATAACAGCGTGAT | 1101 |
| DQ194 911 | Macrobra chium lanatum | GTGGGACGATAAGACCCTATAAAACTTGATATGAGTTTAGCTTGAT TTGTGATTTAGGTGAAAGACGATCTTGTTTGGTTTATATTTCGTTG GGGAGATGTAGATATAATGTGTAACTGTCTATTTAATTTAATAAATA TAGTTAGTATGTGATCCTTCATTTGTGGATTAGAAGAATAAGTTAC TTTAGGGATAACAGCGTGAT | 1102 |
| JF310 725 | Macrobra chium novaeholl andiae | GTGGGACGATAAGACCCTATAAAACTTGATATAAGTCTGAGGGGA TTTGTGATTTCAGTAAAAAATAGTCCTATTAGGTATATATTTCGTTG GGGAGATGTAGATATAATGTGTAACTGTCTTTAAAATTGAATAAAT ATAGTTAGTTTGTGATCCTGTATTTACGGATTAAAAGAGTAAGTTA CTTTAGGGATAACAGCGTAAT | 1103 |
| EF588 319 | Macrobra chium tolmerum | ATGGGACGATAAGACCCTATAAAACTTAATATAAGTCTGATTTAAT TTGTGATTTAAGTGAAAATCGATTTTGTTTGACTTATATTTCGTTGG GGAGATGAAGATATAAAGTGTAACTGTCTGTTATAATTTAATAATAT TAATTAGTTTTTGATCCTTCTTTGTGGATTAAAAGAATAAGTTACTT TAGGGATAACAGCGTAAT | 1104 |
| HM352 432 | Macrobra chium iheringi | AAGGGACGATAAGACCCTATAAAACTTAATATAAATTTTATTTTTTA TGTGATTTAAGTGAAACTCAGTTTAGTTTGATTTATATTTCGTTGGG GAGATGTGGATATAAAGTGTAACTGTCTGCTGAATTTAATAATTAT TGTTAGTGCGCGATCCTTCTTTGTGGGTTGTAAGAATAAGTTACTT TAGGGATAACAGCGTGAT | 1105 |
| JF310 728 | Macrobra chium saigonen se | GTGGGACGATAAGACCCTATAAAACTTTATATAAAAGTAGTTTGGT TTATAATTTAAGTAAAAAGTAACTTTGCTAAATTTATATTTCGTTGG GGAGATGTAGATATAATGTGTAACTGTCTGTTTAATTTGATAATTGT AGTTAGTATATGATCCTTCTTTGTGGATTAAAAGTATAAGTTACTTT AGGGATAACAGCGTGAT | 1106 |
| HM352 428 | Macrobra chium nattereri | GGGGGACGATAAGACCCTATAAAACTTGATATAGATTTAATTTTGC TTGTGATTTCAGTAGAGAGGGGTTTTGATTAGATCATGTTTCGTTG GGGAGATGTGGATATAAAGTGTAACTGTCTGTAGAATTATATAATT ATTATTAGTAGATGATCCTTCTTTGGGGATTAGAAGAATAAGTTAC TTTAGGGATAACAGCGTAAT | 1107 |
| HM352 430 | Macrobra chium aracamun i | GGGGGACGATAAGACCCTATAAAACTTAATATAAATTTAATTTATTA TATGATTTAAGTGAAAAGTAGTTTAAGGTAGTTTATATTTTGTTGGG GTGATATAGATATAAAATGTAACTGTCTGGAAAATTTAATAATTTTA ATTAGTGTTTGATCCTTCTTTGTGGATTAAGAGAATAAGTTACTTTA GGGATAACAGCGTGAT | 1108 |
| HM352 433 | Macrobra chium inpa | GGGGGACGATAAGACCCTGTAAAACTTAATATAAATTTGGTTTTAT TTGTGATTTTAGTGAAAAGAAGTTTAAATTAGTTTATATTTTGTTGG GGTGACATAGATATAAAATGTAACTGTCTAAAAAATTTAATAATTTT AATTAGTGTTTGATCCTTCTTTGGGGATTTAAAGAGTAAGTTACTTT AGGGATAACAGCGTAAT | 1109 |
| HM352 435 | Macrobra chium depressi manum | GGGGGACGATAAGACCCTATAAAACTTTATATAAATTTAATTTAGT TTATGATATAGGTGAAAAATTGTTTTAATAATTTTATATTTTGTTGG GGAGACATAGATATAATATGTAACTGTTTGTTAAATTTAATAATTAT AGTTAATATTTGATCCTTCCTTGAGGATTAAGAGAATAAGTTACTTT AGGGATAACAGCGTAAT | 1110 |
| HM352 446 | Macrobra chium surinamic um | GTGGGACGATAAGACCCTATAAAACTTGATATAGGTTAAGTTTGAT TTGTGATTTAAGTGAAGAGTGGTTTTGCTTGACTTATATTTTGCTG GGGCGGCATGGATATAAAGTGTAACTGTCTATTAAATTTAATAACA TTAATTAGTTTCTGATCCTTCTTTGTGGATTAAAAGAATAAGTTACT TTAGGGATAACAGCGTGAT | 1111 |
| HM352 462 | Macrobra chium denticulat um | GTGGGACGATAAGACCCTATAAAACTTGATATAAGTTGAACTTGGT TTGTGATTTAGGTGAAAAATAGTCTTGCTTGACTTATATTTCGCTG GGGCGGCATAGATATAAAATGTAACTGTCTATTAAATTTAATAGTG CTAATTAGTCTTTGATCCTTCTCTGTGGATTAAAAGAATAAGTTACT TTAGGGATAACAGCGTGAT | 1112 |
| GQ487 497 | Macrobra chium pilimanus | GTGGGACGATAAGACCCTATAAAACTTAATATAGGTTTAGTTTGAT TCGCGATTGTGGGTGAAAAGTAGTGTTGCTAGTCCTATATTTTGTT GGGGCGATGCGGATAAAATCGGTAACTGTTTGTAAAATTTAATAAT TATGATTGATCCTTCGTTTGTGTGGATTAGGAGAATAAGTTACTTT AGGGATAACAGCGTGAT | 1113 |
| HM352 464 | Macrobra chium ohione | GTGGGACGATAAGACCCTATAAAACTTGATATTAGTTTAATTTGAT TTGCGATTTGAGTGAAGAGCGGTTTTATCTATATTATATTTTGTTG GGGAGACATAGATATAAACAGTAACTGTCTATTAAATTTAATAGCG TTAGTTAGTACTTGATCCTTCTTTGTGGATTAAAAGAATAAGTTACT TTAGGGATAACAGCGTGAT | 1114 |
| EF501 999 | Macrobra chium hainanen se | GTGGGACGATAAGACCCTATAAAACTTGATATAAGTTTAATTAACT TATAATTTAAGTGAAAAGTATTTTAAGTGGTTTTATATTTTGTTGGG GAGATGTAGATATAATGTGTAACTGTTTATAAAATTTAATAATTATA ATTAGTATGTGATCCTTCTTTGTGGATTAAAAGTATAAGTTACTTTA GGGATAACAGCGTAAT | 1115 |
| EU493 138 | Macrobra chium lepidactyl oides | GAGGGACGATAAGACCCTATAAAACTTGACATAATCCAAATATGAC TTGAGATTTGAGTGAAGATCGGTCCCGTTTGGCTTGCGTTTCGTT GGGGAGATGAAGATATAAAATGTAACTGTCTGTTTAAATTAAATAG TGTTCAGTAGTTTGTGATCCTTCTCTGTGGATTAAAAGAATAAGTT ACTTTAGGGATAACAGCGTGAT | 1116 |
| EU493 146 | Macrobra chium jaroense | ATGGGACGATAAGACCCTATAAAACTTAATATGATCCGACCTTGAT TTGTGATTTGAGTGAAAAGTGATCTTGTTTGGTTTATATTTCGTTG GGGAGATGAAGATATAAAATGTAACTGTCTATGAAATTTAATAGTG TTAATTAGTTTTTGATCCTTCTTTGTGGATTAAGAGAATAAGTTACT TTAGGGATAACAGCGTGAT | 1117 |
| EU493 145 | Macrobra chium esculentu m | GTGGGACGATAAGACCCTATAAAACTTGATATAAGTTTTGCTTGAT TTGTGATTTATGGTGAAAAATGGTTTTGTTTAACTTATATTTTGTTG GGGAGATGTAGATATAATATGTAACTGTCTATTTAATTTAATAATTA TAATTAGTGTTTGATCCTTCTTTGTGGATTAAGAGAATAAGTTACTT TAGGGATAACAGCGTGAT | 1118 |
| DQ194 910 | Macrobra chium maculatu m | GTGGGACGATAAGACCCTATAAAACTTAATATAAATTTATTTAATTT ATGATTTAAGTGAAAAGTAATTTAAGTGGGTTTATATTTTGTTGGG GAGATGTAGATATAATATGTAACTGTTTATTAAATTTAATAATTATA ATTAGTGTGTGATCCTTCTTTGTGGATTAAAAGTATAAGTTACTTTA GGGATAACAGCGTGAT | 1119 |
| DQ194 912 | Macrobra chium edentatu m | GTGGGACGATAAGACCCTATAAAACTTGATATAAATATAATTTAAC TTGTGATTTAGGTAAAAAGTGGTTTTATTGGGTTTATATTTCGTTGG GGAGATGTGGATATAATATGTAACTGTCTGTTTAATTTAATAATTAT GATTAGTGTGTGATCCTTCTTTGGGGATTAAGAGTTTAAGTTACTT TAGGGATAACAGCGTGAT | 1120 |
| DQ194 926 | Macrobra chium grandima nus | GAGGGACGATAAGACCCTATAAAACTTTACATGAATTCAGTTTGAT TCTGTGAGCTGATGTGAAAAACAATCCTGCTTAATTCATGTTTCGT TGGGGAGACGAATATATAATTTGTAACTGTCTGTTTAATTAAATAG TGATAGTTAGTATCTGATCCCTCTATGAGGATTAAAAGAATAAGTT ACTTTAGGGATAACAGCGTGAT | 1121 |
| DQ194 947 | Macrobra chium malayanu m | GAGGGACGATAAGACCCTATAAAACTTAATACAGGTTTGATTAGGT TACGATTTTAGTATAAAGTGGTATTATTAAGTTTGTATTTCGTTGGG GCGATGAAGATATAATTTGTAACTGTCTGTTAAATATAATAGTTATT GCTAGAATTTGATCCTGCTTTGTGGATTATAAGAATAAGTTACTTTA GGGATAACAGCGTAAT | 1122 |
| DQ194 949 | Macrobra chium meridiona le | ATGGGACGATAAGACCCTATAAAACTTGATATAGGTTTAGCTTGGT TTGTGATGTAAGTGAAAAGTTGCTTTGCTTGGCTTATATTTCGTTG GGGAGATGAAGATATAAAGTGTAACTGTCTATTAAATTTAATAGTG CTAATTAGTGTTTGATCCTTCTTTGTGGATTAAAAGAATAAGTTACT TTAGGGATAACAGCGTGAT | 1123 |
| DQ194 953 | Macrobra chium neglectu m | GTGGGACGATAAGACCCTATAAAACTTAATATAAGTTTTGCTTGAT TTATGATGTGTGTGAAAAGTAGTTTTGTTTGGTTTATATTTTGTTGG GGAGATATAGATATAATTTGTAACTGTCTATTTAATTTAATAGTTAT TGTTAGTGTGTGATCCTTCTTTGTGGATTAAAAGAATAAGTTACTTT AGGGATAACAGCGTGAT | 1124 |
| DQ194 954 | Macrobra chium platychel es | GTGGGACGATAAGACCCTATAAAACTTGATATAGGTAAGGCTTGG TTTGTAATTGTAGGTTAAAAGTTGTTTTGCTTGGCTTATATTTTGTT GGGGAGATGTAGATATAATGTGTAACTGTCTGTTGAATTTAATAGT TATAATTAGTGTGTGATCCTTCATTTGTGGATTAGGAGAATAAGTT ACTTTAGGGATAACAGCGTGAT | 1125 |
| DQ194 950 | Macrobra chium naso | GAGGGACGATAAGACCCTATAAAACTTGATATAAATTTATCTTAAT TTGTGATGTGAGTTAAAAGTAATTTTGTTTAAATTATATTTCGTTGG GGAGATGAAGATATAACATGTAACTGTCTGATTAATTTAATAATCAT AATTAGTATTTGATCCTTCTTTGTGGATTTTAAGAAAAAGTTACTTT AGGGATAACAGCGTGAT | 1126 |
| DQ194 957 | Macrobra chium placidum | GGGGGACGATAAGACCCTATAAAACTTTATATAAGTTTGGTTTAGT CTATGATTTAGGTGAAAAGTGGTTTTGTCTAGTTTATATTTCGTTG GGGAGATGAGGATATAAAATGTAACTGTCTGTTTTAATTTAATAGT TCTAACTAGTTTGTGATCCTTCTTTGGGGATTAAAAGAATAAGTTA CTTTAGGGATAACAGCGTGAT | 1127 |
| DQ194 960 | Macrobra chium yui | ATGGGACGATAAGACCCTATAAAACTTAATATAGGTTTAGTTTAAT TTGTAATTTACGTAAAAAGGGGTTTTATTAAATTTATATTTCGTTGG GGAGATGTGGATATAATTTGTAACTGTCTGTAGAATTTAATAATTAT AGTTAGAGTTTGATCCTTCGTTGGGGATTAGTAGAATAAGTTACTT TAGGGATAACAGCGTAAT | 1128 |
| DQ194 961 | Macrobra chium shokitai | GGGGGACGATAAGACCCTATAAAACTTTATATAAATTTAATTTAAC TTGTGAGTTAAGTGAAAAGTAGTTTTGTTGAGTTTATATTTCGTTG GGGAGATGTAGATATAATATGTAACTGTCTATTTAATTTAATAATAA TGATTAGTGTGTGATCCTTCTTTGTGGATTAAAAGTTTAAGTTACTT TAGGGATAACAGCGTGAT | 1129 |
| AY377 852 | Macrobra chium sundaicu m | GTGGGACGATAAGACCCTATAAAACTTGATATGAGTGAAGTTTAGT TTGTGGTATAGGTGGAAAGTAATTAAATTTAGTTTATATTTTGTTGG GGTGATGTAGATATAATGTGTAACTGTCTGTTAGAATTTAATAATTA TAATTAGTGTTTGATCCTGCTTTGCGGATTAAAAGAATAAGTTACTT TAGGGATAACAGCGTAAT | 1130 |
| AY858 836 | Macrobra chium rude | GTGGGACGATAAGACCCTATAAAACTTGACACAGACTTAGCTGAA TTTGTGATTTTAGTAAAAAGTGATTCTGCGAAGCCTTTGTTTCGTT GGGGAGATGTAGATATAACTTGTAACTGTCTATAAAATTAAATAAC TGTGATTAGTTGTTGATCCTTCACTTGTGGATTAAAAGAATAAGTT ACTTTAGGGATAACAGCGTGAT | 1131 |
| AY730 051 | Macrobra chium lamarrei | AAGGGACGATAAGACCCTATAAAACTTGATATAGTTTGAACTTAAC TTGCGGTTTAAGTGAAAAGTAGTTTTGTTTGTTTTATATTTTGTTGG GGAGATAGAGATATAATGTGTAACTGTCTATTGAATTTTATAGTTTT AATTAGTATTTGATCCTTCTTTGGGGATTAAGAGAGTAAGTTACTTT AGGGATAACAGCGTGAT | 1132 |
| AY730 052 | Macrobra chium sankolli | GAGGGACGATAAGACCCTATAAAACTTAATATAGTTTAAATTTAGC TTTCAACTTAAGTAAAAAGTAGTTTTGTTTAGTTTATATTTTGTTGG GGAGATGAAGATATAATGTGTAACTGTCTATTGAATTTTATAGTGT TAATTAGTATTTGATCCTTCTTTGGGGATTAAGAGAATAAGTTACTT TAGGGATAACAGCGTGAT | 1133 |
| AY730 054 | Macrobra chium gangeticu m | GGGGGACGATAAGACCCTATAAAACTTTATATAATCTAAACTTGAC TTGCGATTTAAGTGAAAAGTAGTTTTGTTCGGTTTATATTTCGTTG GGGAGATGAAGATATAATTTGTAACTGTCTATTGAATTTTATAGTAT TAAATTAGTATTTGATCCTTCTTTGAGGATTAGGAGAATAAGTTACT TTAGGGATAACAGCGTGAT | 1134 |
| KX162 763 | Trachysal ambria palaestin ensis | GGGGGACGATAAGACCCTATAAAGCTTTACAGTTATTTAGTTGAAT TATAAATTGTTAGTATAAAGTTAATTTAACTAATGTTTGTTTCGTTG GGGTGACGAGAATATAATTTTGTAATTGTTCTTTTATGTTTTATTAC AATGATTTTTGGTATAAGAATGATCCTTTATTAAAGATTAAAAGATT AAGTTACTTTAGGGATAACAGCGTAAT | 1135 |
| NC_01 6184 | Euphausi a pacifica | AAGTGACGATAAGACCCTATAAAACTTGATAAATAAAATTAAAAAA GCGTATTAGTTTATTAAAACTTTTTTTCTGGAATTTATTTTATTGGG GCGATAAAAATATAAAAAGTAACTGTTTTTAAAATTTTAATGGTTAA TAACTAAATTAAATAAATGATCCTTTAATAAAGATTAAAAGATTAAG TTACTTTAGGGATAACAGCGTAAT | 1136 |
| GQ890 518 | Euphausi a lucens | AAGTGACGATAAGACCCTATAAAACTTAATAAGAAAGATAAAAAAA GTGCAGTAGTTAATTAAAACTTTTTTTATTAAACTTATTTTATTGGG GCGATAAAAATGTAAAAAGTAACTGTTTTTAACTTATTAATGATTAT AGTCAGAAAGATTGAATGATCCTTTACTAAAGATTATAAGATTAAG TTACTTTAGGGATAACAGCGTAAT | 1137 |
| MG67 7874 | Euphausi a vallentini | AAGTGACGATAAGACCCTATAAAACTTAATAAGAAAGATAAAAAAA GTGTAGTAGTTAATTAAAACTTTTTTTGTTAAACTTATTTTATTGGG GCGATAAAAATGTAAAAAGTAACTGTTTTTAACTTCTTAATGATTAT AGTCAGTAAGATTGAATGATCCTTTAGTAAAGATTATAAGATTAAG TTACTTTAGGGATAACAGCGTAAT | 1138 |
| DQ356 240 | Euphausi a triacantha | AAGTGACGATAAGACCCTATAAAACTTGATAAATAAAATTAAAAAA GGATATTAGTTAAATTAAAACTTTTTTTGTGGAATTTATTTTATTGG GGCGATAAAAATATAATTAGTAACTGTTTTTAATATTTTAATGATTA ATAATCAGAGTTTAGTAAATGATCCTCTAATAGAGATTATAAGATTA AGTTACTTTAGGGATAACAGCGTGAT | 1139 |
| AF177 180 | Euphausi a longirostri s | AAGTGACGATAAGACCCTATAAAACTTGATAAATAAAATTAAAAAAT GATATTAGTTAATTTAAACTTTTTTTTATGGTATTTATTTTATTGGGG CGATAAAAATATAATAAGTAACTGTTTTTCATATTTTAATGATAAATA ATCAGAGTAAATAAATGATCCTTTAATAAAGATTGTAAGATTAAGTT ACTTTAGGGATAACAGCGTAAT | 1140 |
| AF177 178 | Euphausi a similis | AAGTGACGATAAGACCCTATAAAACTTTATAAATAAAATAAAAAAAA GATATTAGTTGATTGAAACTTTTTTTTATGGAATTTATTTTATTGGG GCGATAAAAATATAATAAGTAACTGTTTTTAATATTATAATGATTAA TAGTCAAAATATAAATAAATGATCCTTTAATAAAGATTGCAAGATTA AGTTACTTTAGGGATAACAGCGTAAT | 1141 |
| MG67 7872 | Euphausi a recurve | AAGTGACGATAAGACCCTATAAAACTTTATAAATTAAACTAAAAAAA AATATTAGTTAATTGAAATTTTTTATTGTTGAATTTATTTTATTGGGG CGATACAAATATAATAAGTAACTGTTTTTCATATTTAAATGATAAAT AATCATGAACAATTAAATGATCCTTTAATAAAGATTGTAAGATTAAG TTACTTTAGGGATAACAGCGTAAT | 1142 |
| MG67 7869 | Euphausi a krohni | AAGTGACGATAAGACCCTATAAAACTTTATAAACTAAACTAAAAAA AAATATTAGTTAATTGAAATTTTTTATTGTTGAATTTATTTTATTGGG GCGATTAAAATATAATAAGTAACTGTTTTTTATATTTAAATGATAATT AGTCATGAGTAATTAAATGATCCTTTAGTAAAGATTGTAAGATTAA GTTACTTTAGGGATAACAGCGTAAT | 1143 |
| DQ356 239 | Euphausi a frigida | AAGTGACGATAAGACCCTATAAAACTTAATAAGGAAAACAAAAAAA GTGCACTAGTTTATTAAAGCTTCTCTTGTTAAACTTATTTTATTGGG GCGATAAAAATGTAACAAGTAACTGTTTTTAACTTCTTAATGATTAT AGTCAGTAAGATTGAATGATCCTTTAATAAAGATTATAAGATTAAGT TACTTTAGGGATAACAGCGTAAT | 1144 |
| MG67 7867 | Euphausi a gibboides | AAGTGACGATAAGACCCTATAAAACTTGATAAATAAAGTTAAAAAA AGATATTAGTTAATTGAAACTTTTTTTTGTGGTGTTTATTTTATTGG GGCGATTAAAATATAATAAGTAACTGTTTTTTACATTTTTATGATAA ATAGTCAAGACAAATAAATGATCCTTTATTAGAGATTATAAGATTAA GTTACTTTAGGGATAACAGCGTAAT | 1145 |
| DQ079 713 | Euphausi a eximia | AGGGGACGATAAGACCCTATAAAACTTTATGCACGACATGTCCTG TCTTTGAATTATATCCAAATATTGGTTGGCTAAAAGTGCATTCCGTT GGGGAGACGTTGATATAAGCTGTAACTGTCTTATTAAATAGAATAA TTATTATTATAATTTGATCCTTCTTTGTGGATAAAAAGAATAAGTTA CTTTAGGGATAACAGCGTAAT | 1146 |
| MG67 7865 | Euphausi a american a | AAGTGACGATAAGACCCTATAAAACTTTATAAATTAAACTAAAAAAA AATATTAGTTAATTGAAATTTTTTATTGTTAAATTTATTTTATTGGGG CGATACAAATATATTAAGTAACTGTTTTTTATATTTAAATGATAAATA ATCATAATTAATTAAATGATCCTCTATTAGAGATTGAAAGATTAAGT TACTTTAGGGATAACAGCGTAAT | 1147 |
| MG67 7873 | Euphausi a tenera | AAGTGACGATAAGACCCTATAAAACTTGATAAATAATATTAAAAAT GAGCATTAGTTAGCTAAATCTTTTTTTGTAAAGTTTATTTTATTGGG GCGATAAAAATATAAATAGTAACTGTTTTTAAAATTTTAATGGTAGT AAACAAAAATATTAAATGATCCTTTAATAAAGATTATAAGATTAAGT TACTTTAGGGATAACAGCGTAAT | 1148 |
| MG67 7871 | Euphausi a pseudogi bba | AAGTGACGATAAGACCCTATAAAGCTTAATAAACAAAACTAAAAAT AAATATTAGTTAATTAAATTTTTTTTTGTGTAGTTTATTTTATTGGGG CGATAAAAATATAAATAGTAACTGTTTTTAATATTTTAATGATAAATA ATCAAAAATTAAATGATCCTTTAATAAAGATTATAAGATTAAGCTAC TTTAGGGATAACAGCGTAAT | 1149 |
| MG67 7868 | Euphausi a hemigibb a | AAGTGACGATAAGACCCTATAAAACTTGATAAATAAAATTAAAAATA AATATTAGTTAATTAAATTTTTTTTTGTGAAGTTTATTTTATTGGGGC GATAAAAATATAAATAGTAACTGTTTTTAATATTTTAATGATAATAGT CAAAAATTAAATGATCCTTTAATAAAGATTATAAGATTAAGTTACTT TAGGGATAACAGCGTGAT | 1150 |
| MG67 7866 | Euphausi a brevis | AAGTGACGATAAGACCCTATAAAACTTTATAAATTTAAATTAAAAAA AGTATTAGTAGTTTAAAATTTTTTATTGTTAAATTTATTTTATTGGGG CGATACAAATATAATAAATAACTGTTTATATATTTAAATGACGAATA ATCATGAATAATTAAATGATCCTTTAATAAAGATTGTAAGATTAAGT TACTTTAGGGATAACAGCGTAAT | 1151 |
| KF182 582 | Hymenop enaeus debilis | AAGGGACGATAAGACCCTATAAAGCTTTACAATAAATAAATTAAAT TATAAATTATTAGTTTAACTTAATTTAATTTGTGTTTGTTTCGTTGGG GCGACGAGAATATAATAATAACTGTTCTTTTATAAAATTAACAAAAT TAATTGGATAATAATTGATCCTTTAATAAAGATTAAAAGATTAAGTT ACTTTAGGGATAACAGCGTAAT | 1152 |
| KC515 042 | Nematop alaemon tenuipes | GTGGGACGATAAGACCCTATAAAACTTTATATAAATGGATTGAGCC TGTGATTTATCTGGTAAAGCGGGCTGGGTTTTTTTATATTTTGTTG GGGCGACATTGATAAAATTTGTTGGTAACTGTTCTGTGAATTTTAT AATGGTGGTTAGGTAAAGATCCTTTTATAGGGATTAAGAGTTTAAG TTACTTTAGGGATAACAGCGTAAT | 1153 |

### Primer systems

Primers were designed manually on a multiple DNA sequence alignment of the mitochondrial 16S rDNA of approximately 90 bivalve species using the CLC Genomics Workbench 10.1.1. The designed primers were checked for their physical and structural properties (e.g. formation of dimers, secondary structure, annealing temperature) using Oligo Calc, the OligoAnalyzer Tool provided by Integrated DNA Technologies (IDT) and the online product descriptions from TIB Molbiol (Berlin, Germany). The primers, listed in Table 5, were synthesized by TIB Molbiol. Table 5 also shows the Illumina overhang adapter sequences which were linked to the target-specific primers.

**Table 5**

| **Name** | **Sequence 5'→ 3'** | **SEQ ID NO:** |
|---|---|---|
| **mussel** | | |
| For_Mu | CCTTTTGCATAAGGGTTTTTCAAG | 14 |
| Rev1_Mu | CGAATAGTATCTAGCCGCCATTC | 24 |
| Rev2_Mu | GCAAATAGCATATCACTTTCACCTC | 25 |
| scallop | | |
| For_Mu | TGCTAAGGTAGCTAAATTATGGCC | 13 |
| Rev_Mu | CTTCACGGGGTCTTCTCGTC | 23 |
| **oyster** | | |
| For_Mu | GGTAGCGAAATTCCTTGCCTT | 12 |
| Rev_Mu | AAAGTTGCACGGGGTCTT | 22 |
| overhang | | |
| Forward | TCGTCGGCAGCGTCAGATGTGTATAAGAGACAG | 26 |
| Reverse | GTCTCGTGGGCTCGGAGATGTGTATAAGAGACAG | 27 |

All in-house designed primers were tested in real-time PCR with DNA extracted from the eleven reference samples, comprising three mussel, six scallop, and two oyster species. During optimization, the following PCR conditions/ parameters were kept constant and applied as published previously: DNA input amount of 12.5 ng, 'ready-to-use' HotStarTaq Master Mix Kit, annealing temperature (62°C), 25 cycles (Dobrovolny, S. et al., 2019). Only one variable, the addition of magnesium chloride solution, was modified (addition of 1.5 mM or 3 mM MgCl₂). Real-time PCR reactions were carried out using a fluorescent intercalating dye (EvaGreen^{®} (20x in water)) in strip tubes or in 96-well plates, depending on the thermocycler used, the Rotor-Gene Q (Qiagen) or the LightCycler^{®} 480 System (Roche, Penzberg, Germany), respectively. The total volume of the PCR reactions was 25 µL, consisting of 22.5 µL reaction mix and 2.5 µL of template DNA (diluted DNA samples (5 ng/µL)) or water as negative control. In the reaction mix, the HotStarTaq Master Mix Kit (Qiagen) was used at a final concentration of 1x and the final concentration of primers was 0.2 µM, expect the forward primer for mussels (0.4 µM). PCR cycling conditions were 15 min initial denaturation at 95°C, 25 cycles at 95°C, 62°C and 72°C for 30 s each, and a final elongation for 10 min at 72°C. The primer pairs for mussels, scallop and oysters with and without Illumina overhang adapter sequences were first used in singleplex PCR assays. Then, the seven primers (three forward and four reverse primers) listed in Table 5 were combined in a triplex assay. The identity of the PCR products was confirmed by melting curve analysis and/or agarose gel electrophoresis.

### Library preparation and NGS

In general, samples were sequenced by using either the MiSeq^{®} or the iSeq^{®} platform (Illumina, San Diego, California, USA). DNA extracts were diluted to a DNA concentration of 5 ng/µL. Extracts with a DNA concentration < 5 ng/µL were used undiluted.

DNA library preparation was performed according to Dobrovolny, S. et al. (2019) with minor modifications (excess of magnesium chloride, final concentration 3 mM; average library size: 278 bp; diluted libraries of the iSeq^{®}system were denatured automatically on the instrument).

For the MiSeq^{®} and iSeq^{®}platform, the DNA library was adjusted to 4 nM and 1 nM, respectively, with 10 mM Tris-HCL, pH 8.6. After pooling individual DNA libraries (5 µL MiSeq^{®}, 7 µL iSeq^{®}), the DNA concentration was determined using Qubit^{®} 2.0 fluorimeter.

All sequencing runs were performed using either the MiSeq^{®} Reagent Kit v2 (300-cycles) or the iSeq^{®}100 i1 Reagent v2 (300-cycles) with a final loading concentration of 8 pM. The pooled DNA libraries contained a 5% PhiX spike-in.

Reference samples were sequenced in six replicates (three sequencing runs, two replicates per run), while DNA extract mixtures were sequenced in nine replicates (three sequencing runs, three replicates per run). Commercial food products (O5-Mi86, above the double line in Table 9) were sequenced in one replicate (three sequencing runs, one replicate per run) and food products (O1-S61, below the double line in Table 9) were sequenced at least once by using either the MiSeq^{®} or the iSeq^{®}platform.

### NGS data analysis using Galaxy

After paired-end sequencing, the resulting FastQ files, generated by the instrument control software, were used as input for data analysis. The sequencing output in FastQ format was then processed with an analysis pipeline as described previously by using Galaxy (Version 19.01) (Dobrovolny, S. et al., 2019). The published amplicon analysis workflow was modified as follows: the target-specific primers were trimmed from both ends using the tool Cutadapt and reads were not clustered into Operational Taxonomic Units (OTUs) (Martin, M., 2011). Completely identical sequences were collapsed into a single representative sequence with the tool Dereplicate to minimize the number of reads, and then compared against a customized database for bivalves using BLASTn (Edgar, R.C., 2010).

### Results

### Barcode region and primer systems

The aim was to develop a DNA metabarcoding method allowing the differentiation between species belonging to the bivalves Pectinidae, Ostreidae, and Mytilidae. To be applicable in routine analysis, the method should allow identifying the economically most important bivalve species in raw and highly processed food products.

Three primer sets were designed, one for each of the three bivalves, Pectinidae, Ostreidae, and Mytilidae. Primer pairs consisting of one forward and one reverse primer allowed amplifying the DNA barcode region in scallop and oyster species (Table 5). However, in case of mussels, a primer set consisting of one forward primer and two reverse primers (Table 5) was necessary to obtain a PCR product for the mussel species listed in Table 3. With the three primer sets, PCR products differing in at least one base should be obtained for all bivalve species of interest.

Further sequence alignments indicated that the DNA barcode region selected does not allow distinguishing between all species of the following genera: *Chlamys* spp., *Euvola* spp., *Pecten* spp., *Crassostrea* spp., *Magallana* spp., *Ostrea* spp. and *Saccostrea* spp. These species cannot be distinguished: *Chlamys rubida* and *Chlamys behringiana; Pecten albicans, Pecten fumatus, Pecten jacobaeus, Pecten keppelianus, Pecten novaezelandiae, Pecten sucicostatus, Crassostrea hongkongensis* and *Crassostrea rivularis; Ostrea angelica* and *Ostrea lurida;* as well as *Ostrea permollis* and *Ostrea pu̅elchana;* and *Saccostrea echinata, Saccostrea glomerata* and *Saccostrea mytiloides.* In addition, two mussel species, *Mytilus platensis* and *Mytilus chilensis,* can also not be distinguished (for *Mytilus platensis* only one DNA sequence entry was in the public databases provided by NCBI). However, differentiation at the genus level *(Chlamys* spp., *Pecten* spp., *Crassostrea* spp. *Ostrea* spp., *Mytilus* spp.) is sufficient according to the "Codex Alimentarius Austriacus" chapter B35 (see BMGF-75210/0026-II/B/13/2017, 2007).

When the primers were tested in singleplex PCR assays, for each of the reference samples a PCR product of about 150 bp in length was obtained by increasing the concentration of the forward primer for mussels to 0.4 µM and keeping the concentration of the other six primers at 0.2 µM. In addition, it was tested whether the seven primers could be combined to a triplex system. PCR products for the bivalve species of interest were obtained in one and the same vial by increasing the magnesium chloride concentration to a final concentration of 3 mM. Thus, it was achieved to perform the triplex PCR assay.

### Library preparation, pooling of libraries and sequencing

Library preparation, pooling of 5 µL or 7 µL per normalized DNA library and the sequencing process were performed as described previously (Dobrovolny, S. et al., 2019). Sequencing runs were performed in triplicates and the average run metrics were as follows: cluster density (969 K/mm²) on the flow cell, cluster passing filter (70.22%) as well as the Q-scores (Q30) for read1 and read2 were 92.6% and 89.28%, respectively. 5.02% of the total reads were identified as PhiX control sequences with an error rate of 1.49%.

### Analysis of DNA extracts from reference samples

PCR products were obtained for each of the reference samples, comprising three mussel samples (M12, M13 and M27, Table 2), six scallop samples (S42, S46, S47, S49, S50, and S55, Table 2) and two oyster samples (02 and 03, Table 2). Sequencing results for reference samples are summarized in Table 6.

Table 6 shows results for DNA extracts from reference samples. The numbers are mean values (n=6, three sequencing runs, 2 replicates per run). The table shows the mean values of the total number of raw reads, the total number of reads that passed the analysis pipeline in Galaxy as well as the total number and percentage of reads that were assigned correctly to the eleven species (based on 6 replicates).

No significant differences were observed in the total number of reads (before data analysis process) between these species, except *Mytilus galloprovincialis* (162843), *Perna canaliculus* (169631), and *Mytilus edulis* (134500). With the exception of *Perna canaliculus,* > 70% of the reads passed the amplicon analysis workflow. All three mussel species, six scallop species and two oyster species could be identified with this workflow at high rate (>97.5%), except *Mytilus edulis.*

**Table 6**

| **Sample ID** | **Declaration on the product: Scientific/Latin name** | **Species identified¹** | **Total number of raw reads** | **Total number of reads passing the workflow** | **Number of reads assigned correctly** | **Percentage of reads assigned correctly%** |
|---|---|---|---|---|---|---|
| O2 | *Ostrea edulis* | *Ostrea edulis* | 78559 | 63491 | 61875 | 97.46 |
| O3 | *Crassostrea gigas* | *Magallana gigas* | 76143 | 65389 | 64125 | 98.07 |
| M12 | *Mytilus galloprovincialis* | *Mytilus galloprovincialis* | 162843 | 150678 | 149315 | 99.09 |
| M13 | *Perna canaliculus* | *Perna canaliculus* | 169631 | 104861 | 103350 | 98.56 |
| M27 | *Mytilus edulis* | *Mytilus edulis* | 134500 | 120686 | 105024 | 87.02 |
| S42 | *Mizuhopecten yessoensis* | *Mizuhopecten yessoensis* | 75927 | 58069 | 57058 | 98.26 |
| S46 | *Pecten jacobaeus* | *Pecten* spp. | 79472 | 61484 | 60514 | 98.42 |
| S47 | *Zygochlamys patagonica* | *Zygochlamys patagonica* | 77747 | 59245 | 58429 | 98.62 |
| S49 | *Placopecten magellanicus* | *Placopecten magellanicus* | 79131 | 61531 | 60886 | 98.95 |
| S50 | *Argopecten purpuratus* | *Argopecten purpuratus* | 77383 | 55455 | 54588 | 98.44 |
| S55 | *Aequipecten opercularis* | *Aequipecten opercularis* | 79141 | 56064 | 55800 | 99.53 |

### Analysis of DNA extract mixtures

Six ternary DNA extract mixtures were analysed containing the DNA of the three bivalve species Pectinidae, Ostreidae, and Mytilidae in ratios of 98.0:1.5:0.5 (v/v/v). The composition of the DNA extract mixtures and the results obtained by DNA metabarcoding are summarized in Table 7. Table 7 shows the results for ternary DNA extract mixtures representing the three bivalve species of interest. DNA extracts (5 ng/µL) were mixed in a ratio of 98.0:1.5:0.5 (v/v/v). Numbers are mean values (n=9, three sequencing runs, 3 replicates per run). The total number of raw reads ranged from 80856 to 147443 and the reads that passed the workflow were in the range from 65961 to 147196. For the main components (98.0%), the number of reads assigned correctly ranged from 62434 to 140147. In addition, both minor components (1.5% and 0.5%) could be identified. The number of reads assigned correctly was in the range from 1710 to 4356 and 555 to 1478, respectively.

**Table 7**

| | **Proportion** | | **Total number of raw reads** | **Total number of reads passing the workflow** | **Reads assigned correctly** | | | | | |
|---|---|---|---|---|---|---|---|---|---|---|
| **Species 1 (98%)** | **Species 2 (1.5%)** | **Species 3 (0.5%)** | | | **Species 1** | **(%)** | **Species 2** | **(%)** | **Species 3** | **(%)** |
| *Crassostrea gigas* | *Mytilus galloprovincialis* | *Pecten* spp. | 80856 | 69506 | 66430 | 95.57 | 1985 | 2.86 | 658 | 0.95 |
| *Crassostrea gigas* | *Pecten* spp. | *Mytilus galloprovincialis* | 89552 | 76669 | 73114 | 95.36 | 2182 | 2.85 | 894 | 1.17 |
| *Pecten* spp. | *Crassostrea gigas* | *Mytilus galloprovincialis* | 88971 | 69682 | 66291 | 95.13 | 1710 | 2.45 | 922 | 1.32 |
| *Pecten* spp. | *Mytilus galloprovincialis* | *Crassostrea gigas* | 84085 | 65961 | 62434 | 94.65 | 2281 | 3.46 | 555 | 0.84 |
| *Mytilus galloprovincialis* | *Pecten* spp. | *Crassostrea gigas* | 159737 | 147196 | 140147 | 95.21 | 4356 | 2.96 | 1478 | 1.00 |
| *Mytilus galloprovincialis* | *Crassostrea gigas* | *Pecten* spp. | 147443 | 136629 | 130986 | 95.87 | 3304 | 2.42 | 1156 | 0.85 |

In addition, three DNA extract mixtures containing DNA from species belonging to one bivalve species were analysed (Table 8). Table 8 shows the results for DNA extract mixtures representing one bivalve species. DNA from minor components was present in a proportion of 1% each. In addition, results for a DNA extract mixture containing DNA from a squid species *(Sepiella inermis)* as main component (97.0%) and DNA from three bivalve species (1% each) is shown. Numbers are mean values (n=9, three sequencing runs, 3 replicates per run). The mixtures contained DNA from a scallop or mussel species, respectively. DNA from other bivalve species was present in a proportion of 1.0% each. Both species being present as main components, *Placopecten magellanicus* and *Perna canaliculus,* could be identified, with the number of reads assigned correctly ranging from 58156 to 77483. However, quite different numbers of reads were correctly assigned to the minor components, ranging from 626 *(Mizuhopecten yessoensis)* to 50391 *(Mytilus galloprovincialis).*

A further DNA extract mixture was analysed containing DNA from the squid species *Sepiella inermis* as main component (97.0%) and DNA from the bivalve species *Placopecten magellanicus, Ostrea edulis* and *Perna canaliculus* as minor components (1.0% each). As expected, in this mixture, the main component could not be detected because the primers are not suitable for amplification of the target region for *Sepiella inermis.* 31424, 28162, and 806 reads, respectively, were assigned correctly to the three bivalve species.

**Table 8**

| **Main component** | **Minor component (1.0 % each)** | **Total number of raw reads** | **Total number of reads passed the workflow** | **Reads assigned correctly** | **Percentage of reads assigned correctly (%)** |
|---|---|---|---|---|---|
| *Placopecten magellanicus* | | 83526* | 65446 | 58156 | 88.86 |
| *Mizuhopecten* | *Mizuhopecten yessoensis* | | | 626 | 0.96 |
| | *Pecten spp.* | | | 817 | 1.25 |
| | *Zygochlamys patagonica* | | | 4534 | 6.93 |
| | *Argopecten purpuratus* | | | 663 | 1.01 |
| | *Aequipecten opercularis* | | | 35 | 0.05 |
| *Placopecten magellanicus* | | 84282* | 66691 | 63628 | 95.41 |
| | *Magallana gigas* | | | 1298 | 1.95 |
| | *Ostrea edulis* | | | 1088 | 1.63 |
| *Perna canaliculus* | | 179227* | 128882 | 77483 | 60.12 |
| | *Mytilus galloprovincialis* | | | 50391 | 39.10 |
| | *Mytilus edulis* | | | 824 | 0.64 |
| *Sepiella inermis* | | 78467 | 61415 | | |
| | *Placopecten magellanicus* | | | 31424 | 51.17 |
| | *Ostrea edulis* | | | 28162 | 45.86 |
| | *Perna canaliculus* | | | 806 | 1.31 |

| | | | | | |
|---|---|---|---|---|---|
| *Number of values (n=6, three sequencing runs, 2 replicates per run) | | | | | |

### Analysis of commercial seafood samples

In order to investigate the applicability of the DNA metabarcoding method to foodstuffs, DNA extracts from 75 commercial food products were analysed. According to declaration, eight samples (O1 and 04-010) contained oyster species, 27 samples (M11, M14-M26, and M28-M40) mussel species, 15 samples (S41, S43-45, S48, S51-S55, and S56-S61) scallop species and 25 samples (Mi62-Mi86) were mixed-species seafood products (Table 9). Table 9 shows the results obtained for commercial seafood samples. Samples listed above the double line were sequenced with the MiSeq^{®} (three sequencing runs, 1 replicate per run, numbers are mean values); samples listed below the double line were sequenced either with the MiSeq^{®} or the iSeq^{®}. The ingredient list of 30 out of 75 food products did not give any information on the bivalve species. 39 samples were declared to contain *"Crassostrea gigas", "Mytilus galloprovincialis", "Mytilus chilensis", "Mytilus edulis", "Zygochlamys patagonica", "Chlamys opercularis", "Placopecten magellanicus", "Pecten maximus",* or *"Patinopecten yessoensis".* The remaining samples (n=6) were labelled with *"Mytilus* spp." and *"Pecten* spp."

Three oyster species (*Saccostrea malabonensis, Magallana bilineata, Magallana gigas*), three mussel species *(Mytilus galloprovincialis, Mytilus edulis, Perna canaliculus),* and three scallop species (*Aequipecten opercularis, Placopecten magellanicus, Pecten* spp.) were detected in food products (04, 08, M17, M19, M23, M25, M26, M28, M31, M32, M35, M38-M40, S51, S56, S58-S60, Mi63, Mi65, Mi70, Mi71, Mi73-Mi76, Mi81, Mi83, Mi85, and Mi86) although they were not declared on the label. In four (O1 and 05-07) out of eight oyster products declared to contain *"Crassostrea gigas",* this species was identified.

In 21 products (M11, M16, M18, M21, M24, M33-M35, M37, M39, M40, Mi62, Mi64, Mi66, Mi69, Mi72, Mi77-Mi80, and Mi84), the mussel species *Mytilus galloprovincialis* was detected, indicating that it is one of the most commonly used bivalve species. In addition to *Mytilus galloprovincialis, Mytilus edulis* was identified (percentage of reads assigned correctly >1%) in 13 products (M24, M33, M34, M39, Mi62, Mi64, Mi66, Mi69, Mi72, Mi78-Mi80, and Mi84). However, in none of the products declared to contain *Mytilus chilensis, Mytilus chilensis* was detected. In four products, *Mytilus edulis* could not be detected although it was declared on the label.

*Placopecten magellanicus* and *Patinopecten yessoensis* were listed as ingredients in samples S41, S45, S54, and S57 and samples S48, S52, and S61, respectively. The results confirmed the presence of these two species, except for sample S57. In sample S43, declared to contain *Pecten maximus,* the species *Mizuhopecten yessoensis* was detected. In sample S44 and S53, declared as *Pecten* spp., the species *Mizuhopecten yessoensis* was also analysed.

Thus, the method of the invention allows to specifically control food declaration.

**Table 9**

| **Sample ID** | **Declaration on the product** | | **Species identified** | **Total number of raw reads** | **Total number of reads passed the workflow** | **Reads assigned correctly** | **Percentage of reads assigned correctly %** |
|---|---|---|---|---|---|---|---|
| | **Scientific/Latin name** | **Product description [Eng]** | | | | | |
| O5 | *Crassostrea gigas* | Oyster in sunflower oil | *Magallana gigas* | 76930¹ | 65728 | 64369 | 97.93 |
| O6 | *Crassostrea gigas* | Oyster in sunflower oil | *Magallana gigas* | 44848¹ | 38547 | 37610 | 97.57 |
| O7 | *Crassostrea gigas* | Oyster in water | *Magallana gigas* | 76247 | 64917 | 63700 | 98.13 |
| O8 | not declared | Oyster sauce | *Saccostrea malabonensis* | 14470 | 11658 | 5442 | 46.68 |
| | | | *Magallana bilineata* | | | 4652 | 39.91 |
| M23 | not declared | Mussel with sherry vinegar | *Mytilus galloprovincialis* | 33517 | 30794 | 30358 | 98.58 |
| M25 | not declared | Mussel in marinade sauce | *Mytilus galloprovincialis* | 163188 | 151688 | 150700 | 99.35 |
| M26 | not declared | Grilled blue mussel | *Mytilus galloprovincialis* | 163106 | 151608 | 150433 | 99.23 |
| M29 | *Mytilus galloprovincialis* | Blue mussel in tomato sauce | *Mytilus galloprovincialis* | 153435 | 140475 | 132354 | 94.22 |
| | | | *Mytilus edulis* | | | 7937 | 5.65 |
| M30 | *Mytilus galloprovincialis* | Blue mussel | *Mytilus galloprovincialis* | 185479 | 171890 | 170624 | 99.26 |
| | | A la mariniere | *Mytilus edulis* | | | 1156 | 0.67 |
| M31 | not declared | Blue mussel in | *Mytilus galloprovincialis* | 170303 | 158379 | 157015 | 99.14 |
| | | organic marinade | *Mytilus edulis* | | | 1267 | 0.80 |
| M32 | not declared | Marinated blue | *Mytilus galloprovincialis* | 159181 | 144788 | 143399 | 99.04 |
| | | mussel | *Mytilus edulis* | | | 1308 | 0.90 |
| M33 | *Mytilus chilensis* | Mussel in | *Mytilus galloprovincialis* | 167903 | 151219 | 118879 | 78.61 |
| | | Escabeche | *Mytilus edulis* | | | 31737 | 20.99 |
| M34 | *Mytilus chilensis* | Mussel | *Mytilus galloprovincialis* | 152112 | 138768 | 87964 | 63.39 |
| | | | *Mytilus edulis* | | | 49601 | 35.74 |
| M36 | *Mytilus galloprovincialis* | Blue mussel | *Mytilus galloprovincialis* | 176963 | 163721 | 162224 | 99.09 |
| | | marinated | *Mytilus edulis* | | | 1323 | 0.81 |
| M37 | *Mytilus edulis* | Mussel in honey | *Mytilus galloprovincialis* | 149364 | 136868 | 135249 | 98.82 |
| | | mustard sauce | *Mytilus edulis* | | | 1400 | 1.02 |
| M38 | not declared | Blue mussel | *Mytilus galloprovincialis* | 138801 | 127244 | 125980 | 99.01 |
| | | in marinade | *Mytilus edulis* | | | 1056 | 0.83 |
| S58 | not declared | Rillettes de Saint-Jacques | *Aequipecten opercularis* | 62787 | 44307 | 42716 | 96.41 |
| | | | *Mytilus galloprovincialis* | | | 1330 | 3.00 |
| S59 | not declared | Small scallop in galician sauce | *Aequipecten opercularis* | 82550 | 59722 | 58296 | 97.61 |
| Mi62 | *Mytilus chilensis* | Seafood mix | *Mytilus galloprovincialis* | 618324 | 569815 | 433439 | 76.07 |
| | | | *Mytilus edulis* | | | 134543 | 23.61 |
| Mi63 | not declared | Sauce with seafood | *Mytilus edulis* | 152170 | 139306 | 73550 | 52.80 |
| | | | *Mytilus galloprovincialis* | | | 64729 | 46.47 |
| Mi64 | *Mytilus chilensis, Mytilus edulis* | Seafood mix | *Mytilus galloprovincialis* | 131285 | 119350 | 81590 | 68.36 |
| | | | *Mytilus edulis* | | | 37211 | 31.18 |
| Mi65 | not declared | Bouillabaisse Marseille | *Mytilus galloprovincialis* | 157311 | 143479 | 138535 | 96.55 |
| | | | *Mytilus edulis* | | | 4777 | 3.33 |
| Mi66 | *Mytilus chilensis* | Seafood mix | *Mytilus galloprovincialis* | 152535 | 140047 | 92024 | 65.71 |
| | | | *Mytilus edulis* | | | 47415 | 33.86 |
| Mi67 | *Mytilus* spp. | Seafood mix | *Mytilus galloprovincialis* | 76544 | 69081 | 48275 | 69.88 |
| | | | *Mytilus edulis* | | | 20459 | 29.62 |
| Mi68 | *Mytilus galloprovincialis* | Sea fruit salad in sunflower oil | *Mytilus galloprovincialis* | 157861 | 145671 | 144468 | 99.17 |
| | | | *Mytilus edulis* | | | 1046 | 0.72 |
| Mi69 | *Mytilus chilensis* | Seafood mix | *Mytilus galloprovincialis* | 140227 | 128007 | 85679 | 66.93 |
| | | | *Mytilus edulis* | | | 41686 | 32.57 |
| Mi70 | not declared | Sea fruit salad fantasy | *Mytilus galloprovincialis* | 120677 | 106674 | 101121 | 94.80 |
| | | | *Mytilus edulis* | | | 5413 | 5.07 |
| Mi71 | not declared | Seafood mix | *Mytilus galloprovincialis* | 160546 | 147278 | 79680 | 54.10 |
| | | | *Mytilus edulis* | | | 66675 | 45.27 |
| Mi72 | *Mytilus chilensis* | Seafood mix | *Mytilus galloprovincialis* | 160059 | 146539 | 91557 | 62.48 |
| | | | *Mytilus edulis* | | | 54271 | 37.03 |
| Mi73 | not declared | Seafood mix | *Mytilus edulis* | 150500 | 137634 | 78942 | 57.36 |
| | | | *Mytilus galloprovincialis* | | | 57608 | 41.86 |
| Mi74 | not declared | Seafood mix | *Mytilus galloprovincialis* | 168841 | 155701 | 79035 | 50.76 |
| | | | *Mytilus edulis* | | | 75612 | 48.56 |
| Mi75 | not declared | Pizza Frutti di mare | *Mytilus galloprovincialis* | 181822¹ | 172620 | 95184 | 55.14 |
| | | | *Mytilus edulis* | | | 71440 | 41.39 |
| Mi76 | not declared | Paella | *Mytilus galloprovincialis* | 150431 | 139511 | 138335 | 99.16 |
| | | | *Mytilus edulis* | | | 1070 | 0.77 |
| Mi77 | *Mytilus edulis,* | Paella | *Mytilus galloprovincialis* | 141816 | 132092 | 130768 | 99.00 |
| | *Mytilus chilensis* | | *Mytilus edulis* | | | 1242 | 0.94 |
| Mi78 | *Mytilus chilensis* | Seafood all'Olio | *Mytilus galloprovincialis* | 134717 | 122906 | 73482 | 59.79 |
| | | | *Mytilus edulis* | | | 48774 | 39.68 |
| Mi79 | *Mytilus chilensis* | Seafood mix | *Mytilus galloprovincialis* | 148773 | 137122 | 73035 | 53.26 |
| | | | *Mytilus edulis* | | | 63249 | 46.13 |
| Mi80 | *Mytilus chilensis* | Seafood mix | *Mytilus galloprovincialis* | 136695 | 126608 | 88130 | 69.61 |
| | | | *Mytilus edulis* | | | 37970 | 29.99 |
| Mi81 | not declared | Sea fruit salad | *Mytilus galloprovincialis* | 153499 | 142736 | 141578 | 99.19 |
| | | | *Mytilus edulis* | | | 1022 | 0.72 |
| Mi82 | *Zygochlamys patagonica, Chlamys opercularis* | Scallop terrine | *Zygochlamys patagonica* | 76554 | 59181 | 57329 | 96.87 |
| Mi83 | not declared | Terrine of salmon and great scallop | *Pecten* spp. | 64675¹ | 51387 | 75476 | 146.88 |
| Mi84 | *Mytilus chilensis* | Seafood mix | *Mytilus galloprovincialis Mytilus edulis* | 163885 | 150852 | 124468 25916 | 82.51 17.18 |
| Mi85 | not declared | Instant noodle seafood, mild | *Mytilus galloprovincialis* | 15409 | 14118 | 13750 | 97.39 |
| Mi86 | not declared | Instant noodle seafood, spicy | *Mytilus galloprovincialis* | 9787 | 8892 | 8473 | 95.29 |
| O1 | *Crassostrea gigas* | Oyster | *Magallana gigas* | 139319² | 134073 | 133493 | 99.57 |
| O4 | not declared | Oyster | *Magallana gigas* | 46089² | 40991 | 40279 | 98.26 |
| O9 | not declared | Oyster sauce | | not evaluable³ | | | |
| O10 | not declared | Oyster sauce | | not evaluable³ | | | |
| M11 | *Mytilus edulis* | Mussel | *Mytilus galloprovincialis* | 23766² | 22546 | 22147 | 98.23 |
| M14 | *Mytilus* spp. | Blue mussel | *Mytilus galloprovincialis* | 126880² | 119717 | 79522 | 66.42 |
| M15 | *Mytilus* spp. | Blue mussel | *Mytilus edulis* | 126880² | 119717 | 39555 | 33.04 |
| | | | *Mytilus galloprovincialis* | | | 220226 | 99.79 |
| M16 | *Mytilus edulis* | Bouchot mussel | *Mytilus galloprovincialis* | 51292² | 49604 | 48832 | 98.44 |
| M17 | not declared | Grilled blue | *Mytilus galloprovincialis* | 9888² | 6750 | 3956 | 58.61 |
| | | mussel | *Mytilus edulis* | | | 1998 | 29.60 |
| M18 | *Mytilus chilensis* | Blue mussel | *Mytilus galloprovincialis* | 53710² | 51670 | 50733 | 98.19 |
| M19 | not declared | Blue mussel | *Mytilus galloprovincialis* | 57238² | 54822 | 53829 | 98.19 |
| M20 | *Mytilus* spp. | Blue mussel | *Mytilus galloprovincialis* | 72113² | 69576 | 68969 | 99.13 |
| M21 | *Mytilus edulis* | Mussel | *Mytilus galloprovincialis* | 51328² | 49908 | 49459 | 99.10 |
| M22 | *Mytilus galloprovincialis* | Blue mussel | *Mytilus galloprovincialis* | 115950² | 110777 | 109262 | 98.63 |
| | | | *Mytilus edulis* | | | 1466 | 1.32 |
| M24 | *Mytilus chilensis* | Blue mussel in tomato sauce | *Mytilus galloprovincialis Mytilus edulis* | 113942² | 107150 | 94449 12505 | 88.15 11.67 |
| M28 | not declared | Dry cat food | *Pecten* spp. | 128693³ | 126380 | 79764 | 63.11 |
| | | with green | *Mytilus galloprovincialis* | | | 40450 | 32.01 |
| | | lipped mussel | *Perna canaliculus* | | | 4712 | 3.73 |
| M35 | *Mytilus chilensis* | Mussel in tomato sauce | *Mytilus galloprovincialis* | 197899³ | 190771 | 189540 | 99.35 |
| M39 | *Mytilus chilensis* | Blue mussel | *Mytilus galloprovincialis* | 182612³ | 175982 | 96502 | 54.84 |
| | | | *Mytilus edulis* | | | 75204 | 42.73 |
| M40 | *Mytilus edulis* | Blue mussel | *Mytilus galloprovincialis* | 182958³ | 179399 | 178024 | 99.23 |
| S41 | *Placopecten magellanicus* | Deep-sea scallop | *Placopecten magellanicus* | 143794² | 132140 | 131583 | 99.58 |
| S43 | *Pecten maximus* | Great scallop | *Mizuhopecten yessoensis* | 122156² | 113706 | 113128 | 99.49 |
| S44 | *Pecten* spp. | Great scallop | *Mizuhopecten yessoensis* | 2873135² | 2718126 | 2717426 | 99.97 |
| S45 | *Placopecten magellanicus* | Deep-sea scallop | *Placopecten magellanicus* | 111673² | 107119 | 106632 | 99.55 |
| S48 | *Patinopecten yessoensis* | Great scallop/ Yesso scallop | *Mizuhopecten yessoensis* | 47397² | 41076 | 407873 | 99.51 |
| S51 | not declared | Great scallop | *Placopecten magellanicus* | 51565² | 45007 | 44915 | 99.80 |
| S52 | *Patinopecten yessoensis* | Great scallop | *Mizuhopecten yessoensis* | 46673² | 39769 | 39627 | 99.64 |
| S53 | *Pecten* spp. | Great scallop | *Mizuhopecten yessoensis* | 42857² | 36443 | 35265 | 96.77 |
| S54 | *Placopecten magellanicus* | Great scallop | *Placopecten magellanicus* | 55475² | 48703 | 47915 | 98.38 |
| S56 | not declared | Great scallop | *Placopecten magellanicus* | 1268169³ | 1061137 | 1060653 | 99.95 |
| S57 | *Placopecten magellanicus* | Great scallop | *Pecten* spp. | 174497³ | 171299 | 170404 | 99.48 |
| S60 | not declared | Deep-sea scallop | *Placopecten magellanicus* | 364474³ | 350953 | 350869 | 99.98 |
| S61 | *Patinopecten yessoensis* | Great scallop | *Mizuhopecten yessoensis* | 159145³ | 152930 | 152849 | 99.95 |

| | | | | | | | |
|---|---|---|---|---|---|---|---|
| ¹ value of two replicates; ² samples were analysed with the MiSeq instrument; ³ samples were analysed with the iSeq instrument | | | | | | | |

### Example 2 - Crustacean

In example 2, the identification of Crustacean in food samples according to the invention is shown. The identification was done similar to example 1 using the primer set specific for the identification of seafood species of the respective family in a singleplex setup (two forward primer SEQ ID NO: 1 and SEQ ID NO: 2; and one reverse primer SEQ ID NO: 15). The final concentration of two forward Crustacean primers (SEQ ID NO: 1 and SEQ ID NO: 2) was 0.2 µM each and 0.4 µM for reverse Crustacean primer (SEQ ID NO: 15). Then, the primers of Crustacean und Cephalopods (two Crustacean forward primers (SEQ ID NO: 1 and SEQ ID NO: 2), one Crustacean reverse primer (SEQ ID NO: 15), one Cephalopod forward primer (SEQ ID NO: 3 respectively SEQ ID NO: 4 and SEQ ID NO: 5) and one Cephalopod reverse primer (SEQ ID NO: 16)) were combined in a duplex assay. Furthermore, duplex setups are shown for the combined identification of species of the family of Crustacean and Gastropoda (two forward Crustacean primer SEQ ID NO: 1 and SEQ ID NO: 2, one reverse Crustacean primer SEQ ID NO: 15, one Gastropoda primer SEQ ID NO: 6 and two reverse Gastropoda primer SEQ ID NO: 17 and SEQ ID NO: 18) and also for the combined identification of species of the family of Crustacean and Cephalopods (two Crustacean forward primers (SEQ ID NO: 1 and SEQ ID NO: 2), one Crustacean reverse primer (SEQ ID NO: 15), one Cephalopod forward primer (SEQ ID NO: 3 respectively SEQ ID NO: 4 and SEQ ID NO: 5) and one Cephalopod reverse primer (SEQ ID NO: 16)). Sanger Sequencing was performed as a control experiment for the identification of seafood species.

### Results

The results are shown in the following Tables 10 and 11.

**Table 10**

| | **Product Description** | | | **Sanger Sequencing** | | **Singelplex Crustacean [MiSeq]** | **Duplex Crustacean + Gastropoda [MiSeq]** | **Duplex Crustacean + Cephalopods [MiSeq]** |
|---|---|---|---|---|---|---|---|---|
| **Sampl e No** | **Scientific name of species** | **Product description [Eng]** | **Treat ment** | **Customer Database** | **Sanger 16S [310bp]** | **Customer Database** | **Customer Database** | **Customer Database** |
| 3 | *Nephrops norvegicus* | Norway lobster/ scampie | cooled | *Nephros norvegicus* | no result | *Nephros norvegicus* | *Nephros norvegicus* | *Nephros norvegicus* |
| 14 | *Procambarus clarkii* | Lusiana crayfish | cooled | *Procambarus clarkii* | *Procambarus clarkii* | *Procambarus clarkii* | not determined | *Procambarus clarkii* |
| 15 | *Panulirus argus* | Crayfish | cooled | *Panulirus argus* | no result | *Panuliurs spp* | *Panuliurs argus* | *Panuliurs argus* |
| 17 | *Homarus american us* | Lobster | frozen | *Homarus american us* | *Homarus american us* | *Homarus american us* | *Homarus american us* | *Homarus american us* |
| 20 | | Swimming crab | proces sed | *Monomia gladiator* | no result | *Monomia gladiator* | *Monomia gladiator* | |
| 26 | *Paralithodes camtschaticus* | Kamchatka Crab/ King Crab | cooled | *Paratithodes camtschaticus* | no result | *Paratithodes camtschaticus* | *Paratithodes camtschaticus* | *Paratithodes camtschaticus* |
| 40 | *Chionoecetes opilio* | Snow crab | frozen | *Chionnoecetes opilio Chionocetes bairdi* | no result | *Chionnoecetes spp* | *Chionnoecetes spp* | *Chionnoecetes spp* |
| 45 | *Procambarus clarkii* | Crayfish | frozen | no result | *(Pont)Astacus leptodactylus* | *Pontastacus leptodactylus* | *Pontastacus leptodactylus* | *Pontastacus leptodactylus* |
| 66 | *Homarus gammarus* | European lobster | frozen | *Homarus gammarus* | no result | *Homarus gammarus* | *Homarus gammarus* | *Homarus gammarus* |

**Table 11**

| | **Product Description** | | | **Sanger Sequencing** | | **Singelplex Crustacean [MiSeq]** | **Duplex Crustacea + Gastropoda [MiSeq]** | **Duplex Crustacean + Cephalopods [MiSeq]** |
|---|---|---|---|---|---|---|---|---|
| **Sample No** | **Scientific name of species** | **Product description [Eng]** | **Treat ment** | **Customer Database** | **Sanger 16S [310bp]** | **Customer Database** | **Customer Database** | **Customer Database** |
| 4 | *Pleoticus muelleri* | Argentine red shrimp | frozen | *Pleoticus muellerie* | *Pleoticus muellerie* | *Pleoticus muellerie* | not determined | *Pleoticus muellerie* |
| 6 | *Penaeus monodon* | Partei Crevetten | cooled | *Penaeus monodon* | *Penaeus monodon* | no result | *Penaeus monodon* | *Penaeus monodon* |
| 8 | *Macrobrachiu m rosenbergii* | Rosenberg freshwater shrimp | cooled | *Macrobrachiu m rosenbergii* | *Macrobrachium rossenbergeri* | *Macrobrachium rosenbergii* | not determined | *Macrobrachium rosenbergii* |
| 12 | *Pandalus borealis* | Ice sea shrimps | cooled | *Panadalus borealis* | *Panadalus borealis = Panadalus eous* | *Pandalus spp.* | not determined | *Pandalus spp.* |
| 16 | *Crangon crangon* | North Sea crab | cooled | *Crangon crangon* | no result | *Crangon crangon* | *Crangon crangon* | *Crangon crangon* |
| 31 | *Litopenaeus vannamei* | White Tiger Shrimp | frozen | *Penaeus vannamei* | *Litopenaeus vanamei* | *Penaeus vannamei* | *Litopenaeus vannamei* | *Litopenaeus vannamei* |
| 33 | *Macrobrachiu* m *rossenbergerii* | Rosenberg freshwater shrimp | cooled | *Peanaeus monodon* | *Peanaeus monodon* | *Penaeus spp. no match to Rosenberqerii* | *Genus Penaeus no match to Rosenbergerii* | *Penaeus spp. no match to Rosenbergerii* |
| 34 | *Litopenaeus vannamei* | White Tiger Shrimp | cooled | *Penaeus vannamei* | *Penaeus vannamei* | *Penaeus vannamei* | *Penaeus vannamei* | *Penaeus vannamei* |
| 36 | *Metapenaeus monoceros* | Shrimp | cooled | *Mierspenaeop sis hardwickii Xiphopenaeus kroyeri* | *Parapenaeopsis sculptilis Parapenaeopsis hardwickii* | *Ganjampenaeopsi s uncta* | *Ganjampenaeopsi s uncta* | *Ganjampenaeo psis uncta* |
| 38 | *Heterocarpus reedi* | Shrimp | cooled | *Heterocarpus spp.* | no result | *Heterocarpus spp.* | *Heterocarpus spp.* | *Heterocarp us spp.* |
| 42 | *Penaeus monodon* | Tiger shrimp | frozen | *Penaeus vannamei* | *Peanaeus monodon* | *Penaeus vannamei* | *Penaeus vannamei* | *Penaeus vannamei* |
| 43 | *Macrobrachiu* m *rossenbergerii* | Rosenberg freshwater shrimp | frozen | *Peanaeus monodon* | *Peanaeus monodon* | *Genus Penaeus no match to Rosenbergerii* | *Genus Penaeus no match to Rosenbergerii* | *Genus Penaeus no match to Rosenbergerii* |
| 50 | *Penaeus monodon* | Organic Black Tiger Prawn | frozen | *Panaeus monodon* | *Panaeus monodon* | *Penaeus spp.* | not determined | *Penaeus spp.* |
| 58 | *Crangon crangon* | North Sea crab | cooled | *Crangon crangon* | no result | *Crangon crangon* | *Crangon crangon* | *Crangon crangon* |
| 59 | *Crangon crangon* | North Sea crab | cooled | *Crangon crangon* | no result | *Crangon crangon* | *Crangon crangon* | *Crangon crangon* |
| 64 | *Aristaeopsis edwardsiana* | Carabineros | cooled | *Aristaeopsis edwardsiana* | *Aristaeopsis edwardsiana Aristaeomorpha foliacea* | *Aristaeopsis spp* | *Aristaeopsis spp* | *Aristaeopsis spp)* |
| 65 | *Pleoticus muelleri* | Shrimp ring | frozen | *Pleoticus muelleri* | no result | no result | *Pleoticus muellerie* | not determined |
| 70 | *Penaeus occidentalis* | Pacific white shrimp | cooled | *Pleoticus muelleri* | no result | *Pleoticus muelleri* | *Pleoticus muellerie* | *Pleoticus muellerie* |
| 71 | *Penaeus notialis* | white shrimp | cooled | *Penaeus duorarum* | *Penaeus duorarum Penaeus nr. notialis* | no result | *Penaeus spp.* | *Penaeus spp.* |
| 73 | *Penaeus borealis* | Greenland shrimp | cooled | *Pandalus borealis* | *Pandalus eous = Pandalus borealis* | no result | *Pandalus spp.* | *Pandalus spp.* |
| 76 | Dendrobranchi ata (Suborder) | Red shrimp | proces sed | | not determined | *Penaeus vannamei* | *Penaeus vannamei* | *Penaeus vannamei* |

### Example 3 - Cephalopods

In example 3, the identification of Cephalopods in food samples according to the invention is shown. The identification was done similar to example 1 using the primer set specific for the identification of Cephalopods seafood species of the respective family in a singleplex setup (one forward primer SEQ ID NO: 3 respectively SEQ ID NO: 4 and SEQ ID NO: 5; and one reverse primer SEQ ID NO: 16). The final concentration of forward (SEQ ID NO: 3 respectively SEQ ID NO: 4 and SEQ ID NO: 5) and reverse primer (SEQ ID NO: 16) was 0.2 µM. Furthermore, a duplex setup is shown for the combined identification of species of the family of Crustacean and Cephalopods. The primers of Crustacean und Cephalopods (two Crustacean forward primers (SEQ ID NO: 1 and SEQ ID NO: 2), one Crustacea reverse primer (SEQ ID NO: 15), one Cephalopod forward primer (SEQ ID NO: 3 respectively SEQ ID NO: 4 and SEQ ID NO: 5) and one Cephalopod reverse primer (SEQ ID NO: 16)) were combined in a duplex assay. Sanger Sequencing was performed as a control experiment for the identification of seafood species.

### Results

The results are shown in the following Table 12.

**Table 12**

| Cephalopods | | | | | | | | |
|---|---|---|---|---|---|---|---|---|
| | **Product Description** | | | **Sanger Seguencing** | | **Singelplex Cephalopods [MiSeq]** | **Duplex Crustacean + Cephalopods [MiSeq]** | **Duplex Crustacean + Cephalopods [Iseq]** |
| **Sampl e No** | **Scientific name of species** | **Product description [Engl** | **Treat ment** | **Customer Database** | **Sanger COI [720bp]** | **Customer Database** | **Customer Database** | **Customer Database** |
| 2 | *Loligo edulis* | Squid cleaned | frozen | *Illex illecebrosus Uroteuthis (photololigo) chinensis* | *Uroteuthis chinensis* | no result | no result | *Illex illecebrosus Uroteuthis (photololigo) chinensis* |
| 7 | *Octopus vulgaris* | Octopus tentacle | cooled | no result | *Octopus vulgaris* | no result | *Octopus spp* | *Octopus spp.* |
| 9 | *Loligo chinensis* | Squid | cooled | *Doryteuthis (Amerigo) gahi* | kein Ergebnis | no result | *Doryteuthis (Amerigo) gahi* | *Doryteuthis (Amerigo) gahi* |
| 24 | *Loligo duvauceli* | Squid | frozen | *Loligo duvauceli* | *Loligo gahi* | *Loligo spp.* | *Loligo spp.* | not determined |
| 28 | *Sepiella japonica* | Sepie | frozen | *Sepiella inermis* | *Sepiella inermis* | no result | *Sepiella inermis* | *Sepiella inermis* |
| 29 | *Octopus vulgaris* | Octopus | cooled | *Octopus vulgaris* | *Octopus vulgaris* | no result | *Octopus spp.* | *Octopus spp.* |
| 32 | *Sepia officinalis* | Squid | cooled | *Sepia pharaonis* | *Sepia pharaonis* | *Sepia spp.* | *Sepia spp* | not determined |
| 35 | *Loligo vulgaris* | Squid | cooled | *Doryteuthis (Amerigo) gahi* | *Loligo gahi* | *Doryteuthis (Amerigo) gahi* | *Doryteuthis (Amerigo) gahi* | not determined |
| 37 | *Octopus aegina* | Octopus | cooled | *Amphioctopus aegina* | *Octopus aegina* | *Amphioctopus aegina* | not determined | *Amphioctopus aegina* |
| 41 | *Loligo opalescens* | Squid | frozen | *Doryteuthis opalescens* | kein Ergebnis | *Doryteuthis opalescens* | not determined | *Doryteuthis opalescens* |
| 44 | *Sepiella inermis* | Sepie | frozen | *Sepiella inermis* | *Sepiella inermis* | *Sepiella inermis* | not determined | *Sepiella inermis* |
| 48 | *Octopus maya* | Ocotpus | frozen | *Octopus maya* | *Octopus maya* | *Octopus maya* | not determined | *Octopus maya* |
| 51 | *Sepiella inermis* | Sepie | frozen | *Sepiella inermis* | *Sepiella inermis* | *Sepiella inermis* | not determined | *Sepiella inermis* |
| 56 | *Loligo edulis* | Squid | frozen | *Illex illecebrosus Uroteuthis (photololigo) chinensis* | *Loligo chinensis* | *Uroteuthis chinensis Ilex ilecebrosus* | not determined | *Uroteuthis chinensis Ilex ilecebrosus* |
| 60 | *Eledone moschata* | Musky octopus in oil | proces sed | *Amphioctopus aegina* | *Amphioctopus aegina* | *Amphioctopus aegina* | *Amphioctopus aegina* | not determined |
| 69 | *Loligo chinensis* | Squid | cooled | *Illex illecebrosus Uroteuthis (photololigo) chinensis* | *Loligo chinensis* | *Uroteuthis chinensis Ilex ilecebrosus* | *Uroteuthis chinensis Ilex ilecebrosus* | not determined |
| 72 | *Loligo galhi* | Squid | cooled | *Doryteuthis (Amerigo) gahi* | *Loligo gali* | *Doryteuthis (Amerigo) qahi* | not determined | *Doryteuthis (Amerigo) gahi* |
| 124 | | fried calamari | proces sed | not determined | not determined | not determined | not determined | *Doryteuthis (Amerigo) gahi* |
| 129 | | Ocopus Carpaccio | proces sed | not determined | not determined | not determined | not determined | *Octopus cyanea* |
| 132 | *Uroteuthis duvaucelii* | Squid tubes | frozen | not determined | not determined | not determined | not determined | *Uroteuthis spp.* |
| 137 | *Dosidicus gigas* | Squid in ink sauce | proces sed | not determined | not determined | not determined | not determined | *Dosidicus gigas* |
| 141 | *Dosidicus gigas* | Squid in vegetable oil | proces sed | not determined | not determined | not determined | not determined | *Dosidicus gigas* |
| 142 | *Dosidicus gigas* | Squid in its own ink | proces sed | not determined | not determined | not determined | not determined | *Todarodes pacificus* |
| 149 | | Squid in ink sauce | proces sed | not determined | not determined | not determined | not determined | *Todarodes pacificus* |
| 156 | *Octopus membranaceus* | Musky octopus in oil | proces sed | not determined | not determined | not determined | not determined | *Amphioctopus spp.* |
| 194 | | fried calamari rings | proces sed | not determined | not determined | not determined | not determined | *Dosidicus gigas* |

### Example 4 - Gastropoda

In example 4, the identification of Gastropoda in food samples according to the invention is shown. The identification was done similar to example 1 using the primer set specific for the identification of seafood species of the respective family in a singleplex setup (one forward primer SEQ ID NO: 6 and two reverse primer SEQ ID NO: 17 and SEQ ID NO: 18). The final concentration of forward Gastropoda primer (SEQ ID NO: 6) was 0.4 µM and 0.2 µM each for the two reverse Gastropoda primer (SEQ ID NO: 17 and SEQ ID NO: 18). Furthermore, a duplex setup is shown for the combined identification of species of the family of Crustacean and Gastropoda. Sanger Sequencing was performed as a control experiment for the identification of seafood species.

### Results

The results are shown in the following Table 13.

**Table 13**

| Gastropoda | | | | | | | | |
|---|---|---|---|---|---|---|---|---|
| | **Product Description** | | | **Sanger Sequencing** | | **Singelplex Gastropoda [MiSeq]** | **Duplex Crustacean + Gastropoda [MiSeq]** | **Gastropoda [Iseq]** |
| **Sample No** | **Scientific name of species** | **Product description [Eng]** | **Treatment** | **Customer Database** | **Sanger COI [720 bp]** | **Customer Database** | **Customer Database** | **Customer Database** |
| 5 | *Helix lucorum* | Snail with butter and white wine | processed | *Helix lucorum* | no result | *Helix spp.* | *Helix spp* | not determined |
| 39 | | Agate snail | cooled | *Achatina reticulata* | no result | *Achantina spp* | *Achantina spp.* | not determined |
| 53 | *Helix lucorum* | Roman snail | frozen | *Helix promatia* | *Helix promatia* | *Helix spp.* | no result | *Helix spp.* |
| 75 | | Food supplement with snail powder | processed | not determined | not determined | no result | *Helix spp.* | *Helix thessalica* |
| 144 | | Roman snail | processed | not determined | not determined | not determined | not determined | *Helix spp.* |
| 145 | | Vine snail with balsamic onion | processed | not determined | not determined | not determined | not determined | *Helix spp.* |
| 146 | | Agate snail | processed | not determined | not determined | not determined | not determined | *Achantina fulica* |

### Example 5 - Veneridae

In example 5, the identification of Veneridae in food samples according to the invention is shown. The identification was done similar to example 1 using the primer set specific for the identification of seafood species of the respective family in a singleplex setup (five forward primer SEQ ID NO: 7, SEQ ID NO: 8, SEQ ID NO: 9, SEQ ID NO: 10, and SEQ ID NO: 11 and three reverse primer SEQ ID NO: 19, SEQ ID NO: 20, and SEQ ID NO: 21). The final concentration of forward primer (SEQ ID NO: 7, SEQ ID NO: 8, SEQ ID NO: 9, SEQ ID NO: 10, and SEQ ID NO: 11) was 0.2 µM each, 0.2 µM each for two reverse primer (SEQ ID NO: 20 and SEQ ID NO: 21) and 0.6 µM for the reverse primer (SEQ ID NO: 19). Sanger Sequencing was performed as a control experiment for the identification of seafood species.

### Results

The results are shown in the following Table 14.

**Table 14 ____**

| Veneridae | | | | | | |
|---|---|---|---|---|---|---|
| | **Product Description** | | | **Sanger Sequencing** | | **Singelplex Veneridae [MiSeq]** |
| **Sample No** | ***Scientific name* of species** | **Product description [Eng]** | **Treatment** | **Customer Database** | **Sanger COI [720bp]** | **Customer Database** |
| 10 | *Ensis ensis* | Clam | cooled | *Ensis directus Ensis terranovensis* | *Ensis directus* | *Ensis spp* |
| 11 | *Ruditapes philippinarum* | Venus clam | cooled | *Ruditapes philippinarum* | *no result* | *Ruditapes philipinarum* |
| 18 | not declared | Pickled cockles | processed | *Cerastoderma edule* | *no result* | *Cerastoderma edule* |
| 21 | *Meretrix lyrata* | Venus clam | frozen | *Meretrix lyrata* | *Meretrix lyrata* no result | *Meretrix lyrata* no result |
| 52 61 | *Callista one Solen marinatus* | Venus clam Vagina shell | frozen cooled | no result *Ensis directus Ensis terranovensis* | no result *Ensis directus* | *Ensis spp* |
| 63 | *Ruditapes philippinarum* | Venus clam | cooled | *Ruditapes philippinarum* | *kein Ergbenis* | *Ruditapes philipinarum* |
| 67 | *Cerastoma edulis* | Cockle | cooled | *Cerastoma edulis* | *Cerastoma edulis* | *Cerastoderma edule* |
| 68 | *Chamelea gallina* | Venus clam | cooled | *Chamelea gallina* | *Chamelea gallina* | *Chamelea gallina* |
| 86 | *Cerastoderma edule* | Cockle | cooled | not determined | not determined | *Cerastoderma edule* |
| 87 | *Ruditapes philipinarum* | Carpet shell | cooled | not determined | not determined | *Ruditapes philipinarum* |
| 93 | *Ensis direktus* | Seaside | cooled | not determined | not determined | *Ensis spp* |
| 95 | *Venus spp* | Venus clam | cooled | not determined | not determined | *Ruditapes philipinarum* |
| 96 | *Metrix lyrate* | Brown clam | frozen | not determined | not determined | Metrix lyrate |
| 100 | not declared | Clams au naturel | processed | not determined | not determined | *Chamelea gallina* |
| 121 | not declared | Clams au naturel | cooled | not determined | not determined | *Ruditapes philipinarum* |

### REFERENCES

Dobrovolny, S.; et al. Development of a DNA metabarcoding method for the identification of fifteen mammalian and six poultry species in food. Food Chemistry 2019, 272, 354-361, doi:10.1016/j.foodchem.2018.08.032.
Edgar, R.C. Search and clustering orders of magnitude faster than BLAST. Bioinformatics 2010, 26, 2460-2461, doi:10.1093/bioinformatics/btq461.
Espiñeira, M.; et al.. Development of a method for the genetic identification of commercial bivalve species based on mitochondrial 18S rRNA sequences. J. Agric. Food Chem. 2009, 57, 495-502, doi: 10.1021/jf802787d.
Fernández, A.; et al. Identification of the clam species Ruditapes decussatus (Grooved carpet shell), Venerupis pullastra (Pullet carpet shell), and Ruditapes philippinarum (Japanese carpet shell) by PCR-RFLP. J. Agric. Food Chem. 2000, 48, 3336-3341, doi:10.1021/jf0002185.
Martin, M. Cutadapt removes adapter sequences from high-throughput sequencing reads. EMBnet j. 2011, 17, 10, doi:10.14806/ej.17.1.200.
Näumann, G.; Stumme, B.; Rehbein, H. Differenzierung von Kammmuscheln durch DNA-Analyse. Informationen aus der Fischereiforschung - Information on Fishery Research 2012, 1-7, doi:10.3220/Infn59_1-7_2012.
Pardo, Miguel Angel; Jiménez, Elisa; Pérez-Villarreal, Begoña: Misdescription incidents in seafood sector. In: Food Control, 2016, 62, S. 277-283. DOI: 10.1016/j.foodcont.2015.10.048.
Robson, K., Dean, M., Haughey, S., Elliott, C. A comprehensive review of food fraud terminologies and food fraud mitigation guides. In: Food Control, 2021, 120, S. 107516. DOI: 10.1016/j.foodcont.2020.107516.
Rodriguez, E.M.; Ortea, I. Food Authentication of Seafood Species. In Proteomics in food science: From farm to fork, 3rd; Colgrave, M.L., Ed.; Academic Press: London, 2017; pp 331-342, ISBN 9780128040072.
Stephan, R.; et al. Rapid and reliable species identification of scallops by MALDI-TOF mass spectrometry. Food Control 2014, 46, 6-9, doi:10.1016/j.foodcont.2014.04.047.
Fernandes, T. J. R.; Amaral, J. S.; Mafra, I. DNA barcode markers applied to seafood authentication: an updated review. Critical reviews in food science and nutrition 2020, 1-32. DOI: 10.1080/10408398.2020.1811200

## Claims

1. A method for identifying seafood species in a sample, comprising the steps of
a) isolating DNAfrom the sample,
b) amplifying fragments of said DNA with one or more primer sets (ps) selected from the group of
i. ps 1 for identifying seafood species of the family of Crustacean comprising one or more primer pairs of one forward primer selected from any one of SEQ ID NOs: 1 and 2, and the reverse primer SEQ ID NO: 15 and/or the reverse complement sequences of the primer sequences;
ii. ps 2 for identifying seafood species of the family of Cephalopods comprising one or more primer pairs of one forward primer selected from any one of SEQ ID NOs: 3 to 5, and the reverse primer SEQ ID NO: 16 and/or reverse complement sequences of the primer sequences;
iii. ps 3 for identifying seafood species of the family of Gastropoda comprising one or more primer pairs of the forward primer SEQ ID NO: 6, and one reverse primer selected from any one of SEQ ID NOs: 17 to 18 and/or reverse complement sequences of the primer sequences;
iv. ps 4 for identifying seafood species of the family of Veneridae comprising one or more primer pairs of one forward primer selected from any one of SEQ ID NOs: 7 to 11, and one reverse primer selected from any one of SEQ ID NOs: 19 to 21 and/or reverse complement sequences of the primer sequences;
v. ps 5 for identifying seafood species of the family of Ostreidae comprising the forward primer SEQ ID NO: 12 and the reverse primer SEQ ID NO: 22 and/or reverse complement sequences of the primer sequences;
vi. ps 6 for identifying seafood species of the family of Pectinidae comprising the forward primer SEQ ID NO: 13 and the reverse primer SEQ ID NO: 23 and/or reverse complement sequences of the primer sequences; and
vii. ps 7 for identifying seafood species of the family of Mytilidae comprising one or more primer pairs of the forward primer SEQ ID NO: 14, and one reverse primer selected from any one of SEQ ID NOs: 24 to 25 and/or reverse complement sequences of the primer sequences,
c) sequencing the amplified DNA fragments of step b), and
d) identifying the seafood species by comparison of the sequences obtained by steps a) to c) with reference sequences of seafood species.

2. The method of claim 1, wherein amplifying of fragments of DNA in step b) is performed by a polymerase chain reaction (PCR), preferably by a PCR comprising 25-30 cycles, more preferably by 25 cycles, and preferably at an annealing temperature of 60-65 °C, more preferably at an annealing temperature of 62°C.

3. The method of claim 1 or 2, wherein amplifying fragments of the DNA in step b) is performed with at least 1, 2, 3, 4, 5, 6, or 7 primer sets.

4. The method of any one of claims 1 to 3, wherein the amplified DNA fragments of step b) are 16S rDNA fragments, preferably the amplified DNA fragments comprise 120bp-220bp of the 16S rDNA.

5. The method of any one of claims 1 to 4, wherein the reference sequences of seafood species comprise the DNA sequence of the 16S rDNA of said seafood species.

6. The method of claim 5, wherein the reference sequences are SEQ ID NOs: 28 to 1153.

7. The method of any one of claims 1 to 6, wherein the identified species of the family of Crustacean are selected from Group 1.

8. The method of any one of claims 1 to 6, wherein the identified species of the family of Cephalopods are selected from Group 2.

9. The method of any one of claims 1 to 6, wherein the identified species of the family of Gastropoda are selected from Group 3.

10. The method of any one of claims 1 to 6, wherein the identified species of the family of Veneridae are selected from Group 4.

11. The method of any one of claims 1 to 6, wherein the identified species of the family of Ostreidae are selected from Group 5.

12. The method of any one of claims 1 to 6, wherein the identified species of the family of Pectinidae are selected from Group 6.

13. The method of any one of claims 1 to 6, wherein the identified species of the family of Mytilidae are selected from Group 7.

14. A kit for identifying seafood species in a sample, comprising one or more primer sets (ps) selected from the group of
i. ps 1 for identifying seafood species of the family of Crustacean comprising one or more primer pairs of one forward primer selected from any one of SEQ ID NOs: 1 and 2, and the reverse primer SEQ ID NO: 15 and/or the reverse complement sequences of the primer sequences;
ii. ps 2 for identifying seafood species of the family of Cephalopods comprising one or more primer pairs of one forward primer selected from any one of SEQ ID NOs: 3 to 5, and the reverse primer SEQ ID NO: 16 and/or reverse complement sequences of the primer sequences;
iii. ps 3 for identifying seafood species of the family of Gastropoda comprising one or more primer pairs of the forward primer SEQ ID NO: 6, and one reverse primer selected from any one of SEQ ID NOs: 17 to 18 and/or reverse complement sequences of the primer sequences;
iv. ps 4 for identifying seafood species of the family of Veneridae comprising one or more primer pairs of one forward primer selected from any one of SEQ ID NOs: 7 to 11, and one reverse primer selected from any one of SEQ ID NOs: 19 to 21 and/or reverse complement sequences of the primer sequences;
v. ps 5 for identifying seafood species of the family of Ostreidae comprising the forward primer SEQ ID NO: 12 and the reverse primer SEQ ID NO: 22 and/or reverse complement sequences of the primer sequences;
vi. ps 6 for identifying seafood species of the family of Pectinidae comprising the forward primer SEQ ID NO: 13 and the reverse primer SEQ ID NO: 23 and/or reverse complement sequences of the primer sequences; and
vii. ps 7 for identifying seafood species of the family of Mytilidae comprising one or more primer pairs of the forward primer SEQ ID NO: 14, and one reverse primer selected from any one of SEQ ID NOs: 24 to 25 and/or reverse complement sequences of the primer sequences,
optionally further comprising PCR components, buffers, reagents and/or an instruction manual.

15. A library of primer sequences comprising any one of SEQ ID NOs: 1 to 25.
